# EUROPEAN PATENT SPECIFICATION

(11) **EP 2 082 060 B1**
(45) Date of publication and mention of the grant of the patent: **01.11.2017**
(21) Application number: 07835512.0
(22) Date of filing: 19.10.2007
(51) Int. Cl.: C12Q 1/68

(54) **BREAST TUMOUR GRADING**
GRADIERUNG VON BRUSTTUMOREN
CATÉGORISATION DES TUMEURS DU SEIN

(30) Priority: 20.10.2006 SG 200607354; 23.10.2006 US 862519 P
(43) Date of publication of application: 29.07.2009
(73) Proprietor: Agency for Science, Technology and Research, Singapore 138632 (SG)
(72) Inventor: MILLER, Lance D., Singapore 138672 (SG); KUZNETSOV, Vladimir, Singapore 138672 (SG); IVSHINA, Anna, Singapore 138672 (SG)
(74) Representative: Richards, William John
(86) International application number: PCT/SG2007/000357
(87) International publication number: WO 2008/048193

(56) References cited:
- WO-A-2005/001138
- WO-A-2005/016962
- WO-A-2007/095186
- SORLIE T ET AL: "Gene expression patterns of breast carcinomas distinguish tumor subclasses with clinical implications" PROCEEDINGS OF THE NATIONAL ACADEMY OF SCIENCES OF USA, NATIONAL ACADEMY OF SCIENCE, WASHINGTON, DC, US, vol. 98, no. 19, 11 September 2001 (2001-09-11), pages 10869-10874, XP002215483 ISSN: 0027-8424
- MA X-J ET AL: "GENE EXPRESSION SIGNATURES ASSOCIATED WITH CLINICAL OUTCOME IN BREAST CANCER VIA LASER CAPTURE MICRODISSECTION" BREAST CANCER RESEARCH AND TREATMENT, NIJHOFF, BOSTON, US, vol. 82, no. SUPPL 1, 2003, page S15, XP009035626 ISSN: 0167-6806

## Description

### FIELD

The present invention relates to the fields of medicine, cell biology, molecular biology and genetics. More particularly, the invention relates to a method of assigning a grade to a breast tumour which reflects its aggressiveness.

### BACKGROUND

The effective treatment of cancer depends, to a large extent, on the accuracy with which malignant tissue can be subtyped according to clinicopathological features that reflect disease aggressiveness.

Some clinical subtypes, despite phenotypic homogeneity, are associated with substantial clinical heterogeneity (e.g., refractory response to treatment) confounding their clinical meaning. Recent studies using DNA microarray technology suggest that such clinical heterogeneity may be resolvable at the molecular level (1-4). Indeed, some have demonstrated that gene expression signatures underlying specific biological properties of cancer cells may be superior indicators of clinical subtypes with robust prognostic value (1,2). Thus, global analysis of gene expression has the potential to uncover molecular determinants of clinical heterogeneity providing a more objective and biologically-rational approach to cancer subtyping.

Accordingly, there is a need in the art for gene markers which are diagnostic or reflective of tumourigenicity.

In breast cancer, histologic grade is an important parameter for classifying tumours into morphological subtypes informative of patient risk. Grading seeks to integrate measurements of cellular differentiation and replicative potential into a composite score that quantifies the aggressive behaviour of the tumour.

The most studied and widely used method of breast tumour grading is the Elston-Ellis modified Scarff, Bloom, Richardson grading system, also known as the Nottingham grading system (NGS) (5, 6, Haybittle et al, 1982). The NGS is based on a phenotypic scoring procedure that involves the microscopic evaluation of morphologic and cytologic features of tumour cells including degree of tubule formation, nuclear pleomorphism and mitotic count (6). The sum of these scores stratifies breast tumours into Grade I (G1) (well-differentiated, slow-growing), Grade II (G2) (moderately differentiated), and Grade III (G3) (poorly-differentiated, highly-proliferative) malignancies.

Multivariate analyses in large patient cohorts have consistently demonstrated that the histologic grade of invasive breast cancer is a powerful prognostic indicator of disease recurrence and patient death independent of lymph node status and tumour size (6-9). Untreated patients with G1 disease have a ∼95% five-year survival rate, whereas those with G2 and G3 malignancies have survival rates at 5 years of ∼75% and ∼50%, respectively.

However, the value of histologic grade in patient prognosis has been questioned by reports of substantial inter-observer variability among pathologists (10-13) leading to debate over the role that grade should play in therapeutic planning (14, 15). Furthermore, where the prognostic significance of G1 and G3 disease is of more obvious clinical relevance, it is less clear what the prognostic value is of the more heterogeneous, moderately differentiated Grade II tumours, which comprise approximately 50% of all breast cancer cases (9, 15, 16).

There is therefore a need for methods which are capable of discriminating between heterogenous tumour grades, particularly Grade II breast tumours.

### SUMMARY

We have now demonstrated that a gene expression signature comprising one or more of a set of 232 genes, represented by 264 probesets (e.g., Affymetrix probesets), is capable of discriminating between high and low grade tumours. Such a gene expression signature may be used to provide an objective and clinically valuable measure of tumour grade.

We further describe a novel strategy of clinical class discovery that combines gene discovery and class prediction algorithms with patient survival analysis, and between-group statistical analyses of conventional clinical markers and gene ontologies represented by differentially expressed genes.

Our findings show that the genetic reclassification of histologic grade reveals new clinical subtypes of invasive breast cancer and can improve therapeutic planning for patients with moderately differentiated tumours.

Furthermore, our results support the view that tumours of low and high grade, as defined genetically, may reflect independent pathobiological entities rather than a continuum of cancer progression.

In one aspect, the invention relates to an *in vitro* method of assigning a grade to a breast tumour, which grade is indicative of the aggressiveness of the tumour, the method comprising detecting the expression of FLJ11029, GenBank Accession Number BG165011 as defined in the claims. In another aspect, the invention relates to a the *in vitro* method as described above, in which the method comprises detecting a high level of expression of the gene, preferably above 7.7063, and assigning the survival chances associated with histological Grade 3 to the breast tumour or detecting a low level of expression of the gene, preferably below 7.7063, and assigning the survival chances associated with histological Grade 1 to the breast tumour, in which the level of gene expression is measured as the natural log transform normalised signal intensity measurement for Affymetrix arrays, preferably, global mean normalisation with scaling factor of 500, and in which the breast tumour preferably is a histological Grade 2 breast tumour.

In another aspect, the invention relates to a the *in vitro* method as described above, in which the expression of a plurality of genes is detected, preferably all the genes set out in Table D1 (SWS Classifier 0), preferably in the form of an expression profile of the plurality of genes, in which the expression profile is preferably derived from microarray hybridisation, preferably hybridisation to an Affymetrix microarray preferably comprising a probe set consisting of a probe or probes having Affymetrix ID numbers as set out in Column 6 ("Affi ID") of Table D1, or by real time polymerase chain reaction (RT-PCR). In another aspect, the invention relates to a the *in vitro* method as described above, in which the expression level of 5 or more genes is detected, in which the 5 or more genes preferably comprises the genes set out in Table D2 (SWS Classifier 1), or in which the expression level of the genes is preferably detected using microarray analysis with a probe set consisting of probes having Affymetrix ID numbers as set out in Column 6 ("Affi ID") of Table D2 (SWS Classifier 1).

In another aspect, the invention relates to a the *in vitro* method as described above, in which the method comprises:
(a) detecting a high level of expression of the gene if the expression level of the gene is above the expression level set out in Column 9 ("Cut-Off") of Table D2 (SWS Classifier 1) and assigning the grade set out in Column 7 ("Grade with Higher Expression") of Table D2 (SWS Classifier 1) to the breast tumour; or
(b) detecting a low level of expression of the gene if the expression level of the gene is below the expression level set out in Column 9 ("Cut-Off") of Table D2 (SWS Classifier 1) and assigning the grade set out in Column 8 ("Grade with Lower Expression") of Table D2 (SWS Classifier 1) to the breast tumour;
in which the level of gene expression is measured as the natural log transform normalised signal intensity measurement for Affymetrix arrays, preferably, global mean normalisation with scaling factor of 500.

In another aspect, the invention relates to a the *in vitro* method as described above, in which the expression level of 17 or more genes in Table D1 (or all the genes in Table D1) is detected, in which the genes preferably comprise the genes set out in Table D3 (SWS Classifier 2), or in which the expression level of the genes is preferably detected using microarray analysis with a probe set consisting of probes having Affymetrix ID numbers as set out in Column 6 ("Affi ID") of Table D3.

In another aspect, the invention relates to a the *in vitro* method as described above, in which the method comprises:
(a) detecting a high level of expression of the gene if the expression level of the gene is above the expression level set out in Column 9 ("Cut-Off") of Table D3 (SWS Classifier 2) and assigning the grade set out in Column 7 ("Grade with Higher Expression") of Table D3 (SWS Classifier 2) to the breast tumour; or
(b) detecting a low level of expression of the gene if the expression level of the gene is below the expression level set out in Column 9 ("Cut-Off") of Table D3 (SWS Classifier 2), and assigning the grade set out in Column 8 ("Grade with Lower Expression") of Table D3 (SWS Classifier 2) to the breast tumour;
in which the level of gene expression is measured as the natural log transform normalised signal intensity measurement for Affymetrix arrays , preferably, global mean normalisation with scaling factor of 500.

In another aspect, the invention relates to a the *in vitro* method as described above, in which the grade is assigned by applying:
(a) a class prediction algorithm comprising a nearest shrunken centroid method to the expression data of the plurality of genes;
(b) a class prediction algorithm comprising Prediction Analysis of Microarrays (PAM) to the expression data of the plurality of genes;
   (c) a class prediction algorithm comprising the steps of:
      (i) obtaining a set of predictor parameters;
      (ii) re-coding the parameters to obtain discrete-valued variables;
      (iii) selecting statistically robust discrete-valued variables and combinations thereof;
      (iv) obtaining a sum of the statistically weighted discrete-valued variables and combinations thereof; and
      (v) obtaining a predictive outcome of breast cancer subtype based on the sum; or
(c) a class prediction algorithm comprising Statistically Weighted Syndromes (SWS) to the gene expression data.

In another aspect, the invention relates to an *in vitro* method of classifying a histological Grade 2 breast tumour into a low aggressiveness tumour or a high aggressiveness tumour, the method comprising assigning the grade to the histological Grade 2 breast tumour as described above and as defined in the claims, in which the histological Grade 2 breast tumour that is assigned the low aggressiveness grade has preferably at least one feature of a histological Grade 1 breast tumour, and/or in which the breast tumour that is assigned the high aggressiveness grade has preferably at least one feature of a histological Grade 3 breast tumour, preferably in which the feature comprises any one or more of: (a) likelihood of tumour recurrence post-surgery or survival rate, preferably disease free survival rate; and (b) susceptibility to treatment.

In another aspect, the invention relates to a the *in vitro* method as described above, in which the method is capable of classifying a breast tumour preferably a histological Grade 1 and histological Grade 3 tumour to an accuracy of at least 85%, at least 90% accuracy, or at least 95% accuracy, with reference to the grade obtained of the breast tumour by histological grading.

In another aspect, the invention relates to a the *in vitro* method as described above, in which the histological grading comprises the Nottingham Grading System (NGS) or the Elston-Ellis Modified Scarff, Bloom, Richardson Grading System.

In another aspect, the invention relates to a computer implemented *in vitro* method of assigning a grade indicative of aggressiveness to a breast tumour as defined in the claims, the method comprising processing expression data for FLJ11029, GenBank Accession Number BG165011, detecting a high level of expression of the gene, preferably above 7.7063, and assigning the survival chances associated with histological Grade 3 to the breast tumour or detecting a low level of expression of the gene, preferably below 7.7063, and assigning the survival chances associated with histological Grade 1 to the breast tumour, in which the level of gene expression is measured as the natural log transform normalised signal intensity measurement for Affymetrix arrays, preferably, global mean normalisation with scaling factor of 500.

In another aspect, the invention relates to a program storage device readable by a machine as defined in the claims, tangibly embodying a program of instructions executable by the machine to perform method of assigning a grade indicative of aggressiveness to a breast tumour, the method comprising: processing expression data for FLJ11029, GenBank Accession Number BG165011, detecting a high level of expression of the gene, preferably above 7.7063, and assigning the survival chances associated with histological Grade 3 to the breast tumour or detecting a low level of expression of the gene, preferably below 7.7063, and assigning the survival chances associated with histological Grade 1 to the breast tumour, in which the level of gene expression is measured as the natural log transform normalised signal intensity measurement for Affymetrix arrays.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual: Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855, Lars-Inge Larsson "Immunocytochemistry: Theory and Practice", CRC Press inc., Baca Raton, Florida, 1988, ISBN 0-8493-6078-1, John D. Pound (ed); "Immunochemical Protocols, vol 80", in the series: "Methods in Molecular Biology", Humana Press, Totowa, New Jersey, 1998, ISBN 0-89603-493-3, Handbook of Drug Screening, edited by Ramakrishna Seethala, Prabhavathi B. Fernandes (2001, New York, NY, Marcel Dekker, ISBN 0-8247-0562-9); Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3; and The Merck Manual of Diagnosis and Therapy (17th Edition, Beers, M. H., and Berkow, R, Eds, ISBN: 0911910107, John Wiley & Sons).

### BRIEF DESCRIPTION OF THE FIGURES

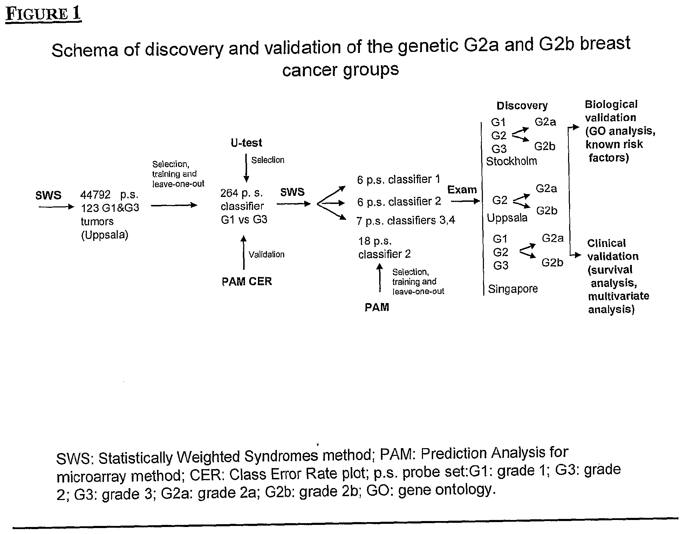

Figure 1. Schema of discovery and validation of the genetic G2a and G2b breast cancer groups. SWS: Statistically Weighted Syndromes method; PAM: Prediction Analysis for Microarray method; CER: Class Error Rate Function; p.s. probe set:G1: Grade 1; G3: Grade 2;G3: Grade 3; G2a: Grade 2a; G2b: Grade 2b; GO: gene ontology.

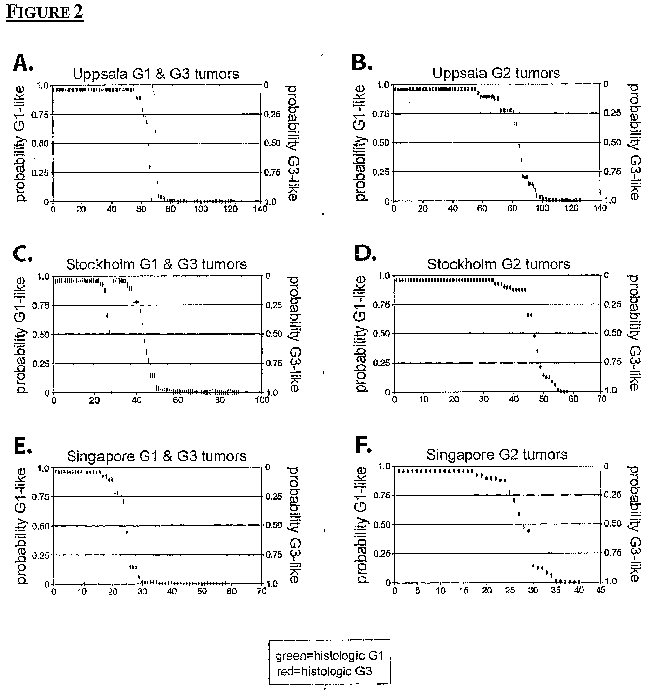

Figure 2. Probability (Pr) scores from the SWS classifier. Pr scores (0-1) generated by the class prediction algorithm are shown on the y-axes. Number of tumours per classification exercise is shown on the x-axis. Green indicates Grade 1 tumours; red denotes Grade 3 tumours.

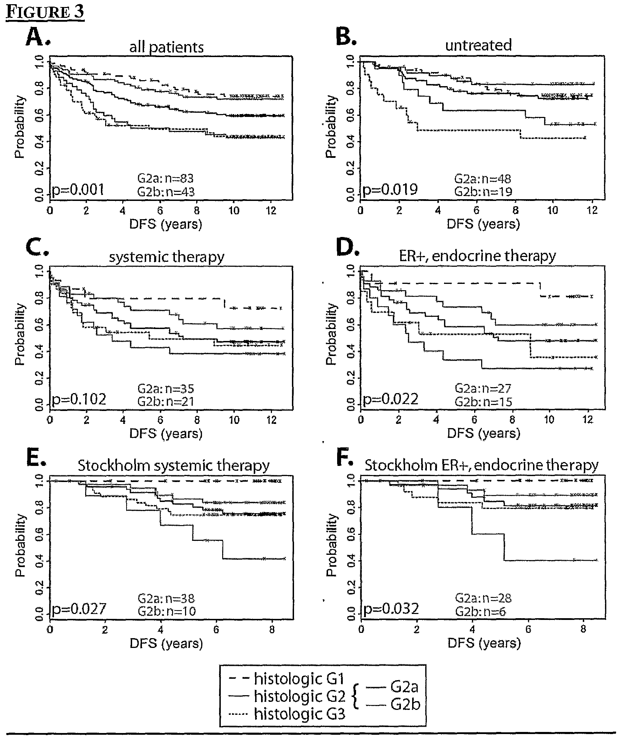

Figure 3. Survival differences between G2a and G2b genetic grade subtypes. Kaplan-Meier survival curves for G2a and G2b subtypes are shown superimposed on survival curves of histologic grades 1, 2, and 3 (see key). Uppsala cohort survival curves are shown for all patients (A), patients who did not receive systemic therapy (B), patients treated with systemic therapy (C), and patients with ER+ disease who received anti-estrogen therapy only (D). Stockholm cohort survival curves are shown for patients treated with systemic therapy (E) and those with ER+ cancer treated with anit-estrogen therapy only (F). The p-value (likelihood ratio test) reflects the significance of the hazard ratio between the G2a and G2b curves.

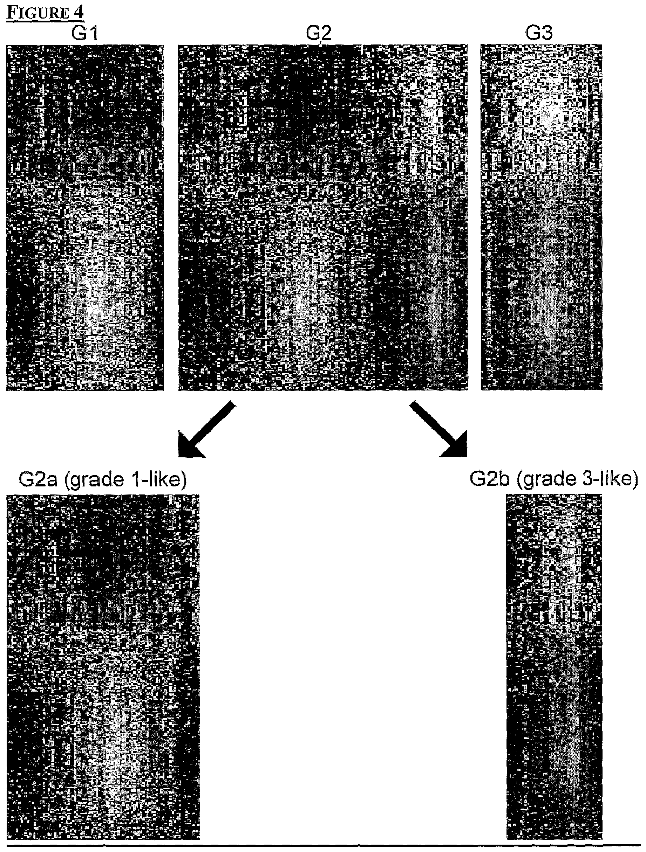

Figure 4. Expression profiles of the top 264 grade (G1-G3) associated gene probesets. Gene probesets (rows) and tumours (columns) were hierarchically clustered by average linkage (Pearson correlation), then tumours were grouped according to grade while maintaining original cluster order within groups. Red reflects above mean expression, green denotes below mean expression, and black indicates mean expression. The degree of color saturation reflects the magnitude of expression relative to the mean.

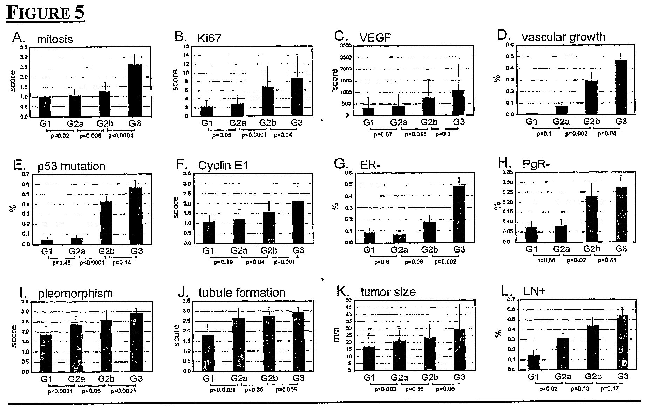

Figure 5. Statistical analysis of clinicopathological markers. Measurements (or percentages of binary measurements) of clinicopathological variables assessed at the time of surgery were compared between different tumour subgroups: G1 vs. G2a, G2a vs. G2b, and G2b vs. G3. P-values are noted below subgroup designations. Average scores (or percentages) within each subgroup are shown as vertical bars with standard deviations.

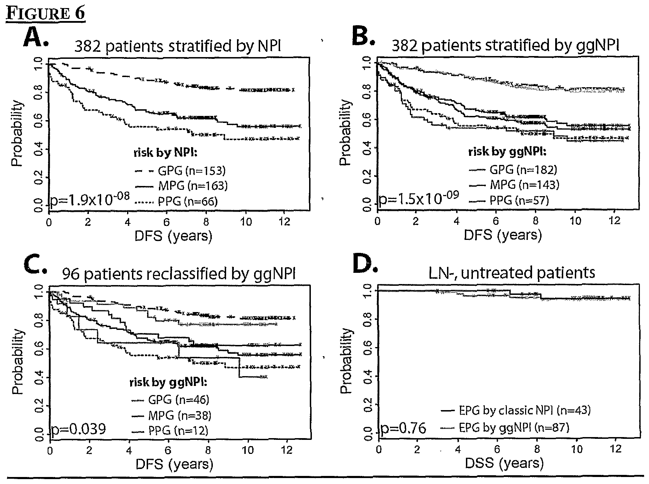

Figure 6. Stratification of patient risk by classic NPI and ggNPI. (A) Kaplan-Meier survival curves are shown for the classic NPI categories: Good Prognostic Group (GPG); Moderate Prognostic Group (MPG); Poor Prognostic Group (PPG). (B) Kaplan-Meier survival curves are shown for risk groups determined by the classic NPI (black curves) and the NPI calculated with genetic grade assignments (ggNPI; colored curves). (C) Kaplan-Meier survival curves are shown for patients reclassified by ggNPI (colored curves indicate that reclassified patients have survival curves similar to the good, moderate and poor prognostic groups of the classic NPI (black curves)). (D) The disease-specific survival curves of node negative, untreated patients classified into the Excellent Prognostic Group (EPG) by classic NPI (black curve) or ggNPI (green curve) are compared.

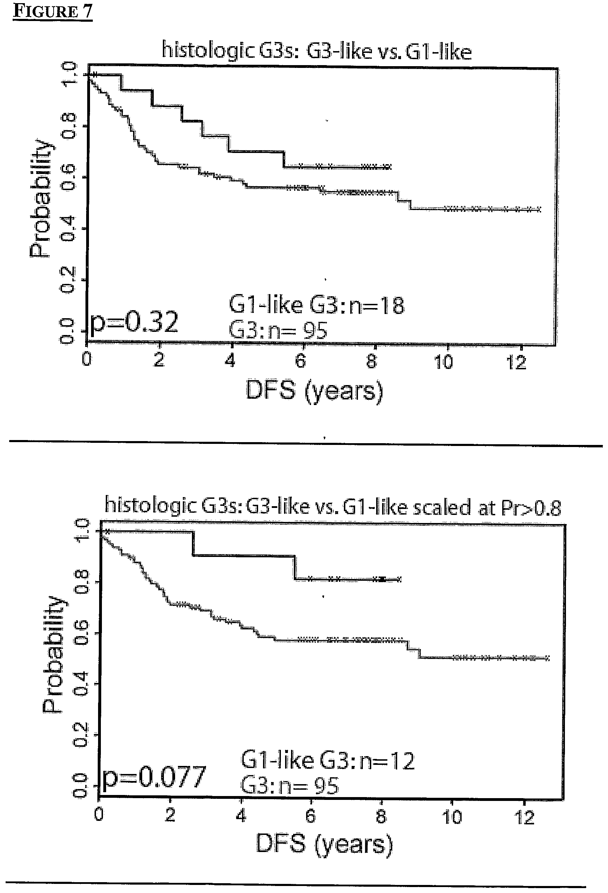

Figure 7. Stratification of patient risk by classic NPI and ggNPI. Kaplan-Meier survival curves are shown for risk groups determined by the classic NPI (A) and the NPI calculated with genetic grade predictions (ggNPI) (B). Survival curves of patients reassigned to new risk groups by the ggNPI are shown (C). The disease-specific survival curve of the EPG patients (by classic NPI) is compared to that of patients identified as EPG exclusively by the ggNPI (D). Classic NPI curves from (A) are shown superimposed on (B-D).

### DETAILED DESCRIPTION

### BREAST TUMOUR GRADING

We have identified a number of genes whose expression is indicative of breast tumour aggressiveness. Accordingly, we provide for methods of grading breast tumours, and therefore assigning a measure of their aggressiveness, by detecting the level of expression of one or more of these genes. The genes are provided in a number of gene sets, or classifiers.

In a general aspect, we provide for the detection of any one or more of a small set of 264 gene probesets, which we term the "SWS Classifier 0". This classifier represents 232 genes. In some embodiments, the expression of all of the 264 gene probesets are detected. For example, the expression of all the 232 genes represented by such probesets may be detected.

The genes comprised in this classifier are set out in Table D1 in the section "SWS Classifier 0" below, in Table S1 in Example 20, as well as in Appendix A1. This and the other tables D2, D3, D4 and D5 (see below) contain the GenBank ID and the Gene Symbol of the gene, as well as the "Affi ID", or the "Affymetrix ID" number of a probe. Affymetrix probe set IDs and their corresponding oligonucleotide sequences, as well as the GenBank mRNA sequences they are designed from, can be accessed on the world wide web at the ADAPT website (http://bioinformatics.picr.man.ac.uk/adapt/ProbeToGene.adapt) hosted by the Paterson Institute for Cancer Research.

In such an embodiment, therefore, our method comprises determining the expression level of at least one of the genes of the 264 gene probesets (for example, at least one of the 232 genes) in the classifier which we term the "SWS Classifier 0". More than one, for example, a plurality of the genes of such a set may also be detected. The 264 gene probesets of the SWS Classifier 0 gene set are set out in Table D1 below.

In some embodiments, the expression level of more than one gene is detected. For example, the expression level of 5 or more genes may be detected. The expression level of a plurality of genes may therefore be determined. In some embodiments, the expression level of all 264 gene probesets (for example, the expression level of all 232 genes) may be detected, though it will be clear that this does not need to be so, and a smaller subset may be detected.

We therefore provide for the detection of one or more, a plurality or all, of subsets comprising 17 genes and several subsets of 5-17 genes from the 264 gene probesets.

Thus, alternatively, or in addition, our method may comprise determining the expression level of at least one, a plurality, or all of the genes of an 5 gene set which we term the "SWS Classifier 1". The 5 genes of the SWS Classifier 1 gene set are set out in Table D2 below.

In other embodiments, our method may comprise determining the expression level of at least one, a plurality, or all of the genes of an 17 gene set which we term the "SWS Classifier 2". The 17 genes of the SWS Classifier 2 gene set are set out in Table D3 below.

In other embodiments, our method may comprise determining the expression level of at least one, a plurality, or all of the genes of an 7 gene set which we term the "SWS Classifier 3". The 7 genes of the SWS Classifier 3 gene set are set out in Table D4 below.

In other embodiments, our method may comprise determining the expression level of at least one, a plurality, or all of the genes of an 7 gene set which we term the "SWS Classifier 4". The 7 genes of the SWS Classifier 4 gene set are set out in Table D5 below.

In specific embodiments, the methods comprise detection of the expression level of all of the genes in the gene set of interest. For example, all 5 genes in the "SWS Classifier 1" are detected, all 17 genes in the "SWS Classifier 2" are detected, all 7 genes in the "SWS Classifier 3" are detected and all 7 genes of the SWS Classifier 4 gene set are detected in these embodiments.

Where the SWS Classifier 1, the SWS Classifier 2, the SWS Classifier 3 or the SWS Classifier 4 are used, each of Tables D2, D3, D4 and D5 provide indications of the grades to be assigned to the tumour depending on the level of expression of the relevant gene which is detected (in Columns 7 and 8 respectively).

Thus, the tables also contain columns showing the grades associated with high and low levels of expression of a particular gene, in Columns 7 and 8 of Table D1 for example. Thus, for example, the gene Barren homolog (*Drosophila*) is annotated to the effect that the "Grade with Higher Expression" is 3, while the "Grade with Lower Expression" is 1. Accordingly, our method provides that the tumour has a grade of 3 if a high level of expression of Barren homolog (*Drosophila*) is detected in or from the tumour. If a low level of this gene is detected in or from the tumour, then a grade of 1 may be assigned to that tumour.

Detection of gene expression, for example for tumour grading, may suitably be done by any means as known in the art, and as described in further detail below.

The methods described here for gene expression analysis and tumour grading may be automated, or partially or completely controlled by a controller such as a microcomputer. Thus, any of the methods described here may comprise computer implemented methods of assigning a grade to a breast tumour. For example, such a method may comprise processing expression data for one or more genes set out in Table D1 (SWS 0 Classifier) and obtaining a grade indicative of aggressiveness of the breast tumour.

The methods described here are suitably capable of classifying a breast tumour to an accuracy of at least 85%, at least 90% accuracy, or at least 95% accuracy, with reference to the grade obtained by conventional means, such as for example grading of the breast tumour by histological grading. For example, the methods may be capable of classifying tumours with grades corresponding to histological Grade 1 and histological Grade 3 tumours with an accuracy of 70% or above, 80% or above, or 90% or above.

### DETECTION OF HIGHER AND LOWER EXPRESSION

In a refinement of our methods, we provide for a "cut-off" level of expression, by which the expression of a gene in or from a tumour may be judged in order to establish whether the expression is at a "high" level, or at a "low" level. The cut-off level is set out in Column 9 of Tables D1, D2, D3, D4 and D5.

Accordingly, in some embodiments, our methods include assigning a grade based on whether the level of expression falls below or exceeds the cut-off. In some embodiments, the cut-off values are determined as the natural log transform normalised signal intensity measurement for Affymetrix arrays. In such embodiments, the cut-off values may be determined as a global mean normalisation with a scaling factor of 500.

For example, referring back to Table 1, the cut-off level of expression for the gene Barren homolog (*Drosophila*) is 5.9167 units (see above and formula (1), Microarray Method). Where a given tumour contains a level of expression of this gene that exceeds this level, then it is determined to be a "high" level of expression. A grade of 3 may then be assigned to that tumour. On the other hand, if the expression of the Barren homologue falls below this cut-off level, then the expression is judged to be a "low" level of expression. A grade of 1 may be assigned to the tumour in this event.

Thus, we provide for a method which comprises detecting a high level of expression of a gene in SWS Classifier 0 and assigning the grade set out in Column 7 of Table D1 to the breast tumour. The method may comprise, or optionally further comprise detecting a low level of expression of the gene and assigning the grade set out in Column 8 of Table D1 to the breast tumour. A high level of expression may be detected if the expression level of the gene is above the expression level set out in Column 9 of Table D1, and a low level of expression is detected if the expression level of the gene is below that level.

### DETECTION OF GENE EXPRESSION

There are various methods by which expression levels of a gene may be detected, and these are known in the art. Examples include RT-PCR, RNAse protection, Northern blotting, Western blotting etc. The gene expression level may be determined at the transcript level, or at the protein level, or both. The detection may be manual, or it may be automated. It is envisaged that any one or a combination of these methods may be employed in the methods and compositions described here.

The detection of expression of a plurality of genes is suitably detected in the form of an expression profile of the plurality of genes, by conventional means known in the art. In some embodiments, the detection is by means of microarray hybridisation.

For example, a sample of a tumour may be taken from a patient and processed for detection of gene expression levels. Gene expression levels may be detected in the form of nucleic acid or protein levels or both, for example. Analysis of nucleic acid expression levels may be suitably performed by amplification techniques, such as polymerase chain reaction (PCR), rolling circle amplification, etc. Detection of expression levels is suitably performed by detecting RNA levels. This can be performed by means known in the art, for example, real time polymerase chain reaction (RT-PCR) or RNAse protection, etc. For this purpose, we provide for sets of one or more primers or primer pairs which are capable of amplifying any one or more of the genes in the classifiers disclosed herein. Specifically, we provide for a set of primer pairs capable of amplifying all of the genes in the SWS Classifier 0, SWS Classifier 1, SWS Classifier 2, SWS Classifier 3 or SWS Classifier 4 sets.

Suitably, RNA expression levels may be detected by hybridisation to a microchip or array, for example, a microchip or array comprising the genes or probesets corresponding to the specific classifier of interest, as described in the Examples. In some embodiments, the gene expression data or profile is derived from microarray hybridisation to for example an Affymetrix microarray.

Detection of protein levels may be performed by for example, immunoassays including ELISA or sandwich immunoassays using antibodies against the protein or proteins of interest (for example as described in US6664114. The detection may be performed by use of a "dip stick" which comprises impregnated antibodies against polypeptides of interest, such as described in US2004014094.

We provide therefore for sets of one or more antibodies which are capable of binding specifically to any one or more of the proteins encoded by the genes in the classifiers disclosed herein. Specifically, we provide for a set of antibodies capable of amplifying all of the genes in the SWS Classifier 0, SWS Classifier 1, SWS Classifier 2, SWS Classifier 3 or SWS Classifier 4 sets.

The grade may be assigned by any suitable method. For example, it may be assigned applying a class prediction algorithm comprising a nearest shrunken centroid method (Tibshirani, et al., 2002, Proc Natl Acad Sci USA. 99(10): 6567-6572) to the expression data of the plurality of genes. The class prediction algorithm may suitably comprise Statistically Weighted Syndromes (SWS) or Prediction Analysis of Microarrays (PAM).

In some embodiments, the grade of the tumour may be assigned by applying a class prediction algorithm comprising one or more of the steps set out here. First, a set of predictor parameters (i.e., probesets) may be obtained, based on predictors which discriminate the histologic tumours G1 and G3. Next, the potentially predictive parameters (i.e. signal intensity values of micro-array) may be recoded to obtain cut-off values for robust discrete-valued variables. The recoding may be done in such a way as to maximize an informativity measure of discrimination ability of the parameter and minimize its instability to the discrimination object (i.e. patients) belonging to distinct classes (i.e. G1 and G3). Then, statistically robust discrete-valued variables and combinations thereof may be selected for further construction of class prediction algorithm. A sum of the statistically weighted discrete-valued variables and combinations thereof may be obtained based on the Weighted Voting Procedure procedure described in SWS method section. Finally, a predictive outcome (classification) scores of breast cancer subtypes based on the sum for sub-typing (re-classification) histologic G2 tumours may be obtained.

### APPLICATION TO GRADE 2 TUMOURS

In suitable embodiments, the method is applied to grade breast tumours which are traditionally graded as Grade 2 by conventional means, such as by histological grading as known in the art. Our method is capable of distinguishing the aggressiveness of tumours within the group of tumours in Grade 2 (which were hitherto thought to be homogenous) into Grade 1 like tumours (i.e., more aggressive) and Grade 3 like tumours (i.e., less aggressive). This is described in detail in the Examples.

Accordingly, we provide for a method of classifying a histological Grade 2 tumour into a low aggressiveness tumour or a high aggressiveness tumour. In other words, we provide a method for reassigning a more precise grading to a tumour which has been graded histologically as a Grade 2 tumour.

Such a method comprises assigning a grade to the histological Grade 2 tumour according to any of the methods described above. For example, the expression of any one or more genes, for example, all the genes, in any of the SWS Classifiers described here may be detected and a grade of 1 or 3 assigned using Columns 7, 8 or 9 individually or in combination, as described above.

Such a tumour which has been reassigned will suitably have one or more characteristics or features of the reassigned grade. The characteristics or features may include one or more histological or morphological features, susceptibility to treatment, rate of growth or proliferation, degree of differentiation, aggressiveness, etc. As an example, the characteristic or feature may comprise aggressiveness.

For example, a histological Grade 2 breast tumour which has been assigned a low aggressiveness grade by the gene expression detection methods described here may suitably have at least one feature of a histological Grade 1 breast tumour. Similarly, a breast tumour assigned a high aggressiveness grade may have at least one feature of a histological Grade 3 breast tumour.

Such a feature may comprise degree of differentiation (e.g., well-differentiated, moderately differentiated or poorly-differentiated). The feature may comprise rate of growth (e.g., slow-growing, fast-growing). The feature may comprise rate of proliferation (e.g., slow-proliferation, highly-proliferative). The feature may comprise likelihood of tumour recurrence post-surgery. The feature may comprise survival rate. The feature may comprise likelihood of tumour recurrence post-surgery and survival rate. The feature may comprise a disease free survival rate. The feature may comprise susceptibility to treatment.

Accordingly, application of the grading methods described here enables the classification of the histological Grade 2 tumour into a Grade 1 tumour or a Grade 3 tumour, so as to allow the clinician to treat the tumour accordingly in view of its aggressiveness, prognosis, etc.

Such regrading using our methods is suitably capable of classifying histological Grade 2 tumours into Grade 1 like and/ or Grade 3 like tumours with an accuracy of 70% or above, 80% or above, or 90% or above.

The histological grading may be performed by any means known in the art. For example, the breast tissue or tumour may be graded by the Nottingham Grading System (NGS) or the Elston-Ellis Modified Scarff, Bloom, Richardson Grading System, both methods being well known in the art.

The information obtained from the regrading may be used to predict any of the parameters which may be useful to the clinician. The parameter may include, for example, likelihood of tumour metastasis, prognosis of the patient, survival rate, possibility of recovery and recurrence, etc, depending on the grade of the tumour which has been reassigned to the histological Grade 2 tumour. We therefore describe a method of determining whether a breast tumour is a metastatic breast tumour, the method comprising assigning a grade to the breast tumour as described using gene expression data.

We describe a method of predicting a survival rate for an individual with a histological Grade 2 breast tumour, the method comprising assigning a grade to the breast tumour using gene expression data as described. A low aggressiveness grade may suitably indicate a high probability of survival and a high aggressiveness grade may suitably indicate a low probability of survival. We also provide for a method of prognosis of an individual with a breast tumour, the method comprising assigning a grade to the breast tumour by a method as described, and a method of diagnosis of aggressive breast cancer in an individual, the method comprising assigning a grade indicative of high aggressiveness to a breast tumour of the individual by a method as described.

The methods of gene expression analysis may be employed for determining the proliferative state of a cell. For example, such a method may comprise detecting the expression of a gene selected from the genes set out in Table D1 (SWS Classifier 0). Where a high level of expression of a gene which is annotated "3" in Column 7 is detected, this may indicate a highly proliferative cell. Similarly, where a high level of expression of a gene which is annotated "1" in Column 7 is detected, a non-proliferating cell or a slow-growing cell may be indicated. If a low level of expression of a gene which is annotated "3" in Column 8 is detected, this may indicate a highly proliferative cell and where a low level of expression of a gene which is annotated "1" in Column 8 is detected, this indicates a non-proliferating cell or a slow-growing cell.

The classifiers are described herein as combinations of probesets, and the skilled person will be aware that more than one probeset can correspond to one gene. Accordingly, the SWS Classifier 0 contains 264 probesets which represent 232 genes. It will be clear therefore that the invention encompasses detection of expression level of one or more genes, and/or one or more probesets within the relevant classifiers, or any combination of this.

### DIAGNOSIS AND TREATMENT

Suitably, the information obtained by the regarding may also be used by the clinician to recommend a suitable treatment, in line with the grade of the tumour which has been reassigned.

Thus, a tumour which has been reassigned to Grade 1 may require less aggressive treatment than a tumour which has been reassigned to Grade 3, for example. We therefore describe a method of choosing a therapy for an individual with breast cancer, the method comprising assigning a grade to the breast tumour by a method as described herein, and choosing an appropriate therapy based on the aggressiveness of the breast tumour. In general, the method may be employed for the treatment of an individual with breast cancer, by assigning a grade to the breast tumour and administering an appropriate therapy to the individual based on the aggressiveness of the breast tumour.

In general, we disclose a method of treatment of an individual suffering from breast cancer, the method comprising modulating the expression of a gene set out in Table D1 (SWS Classifier 0), Table D2 (SWS 1 Classifier), Table D3 (SWS Classifier 2), Table D4 (SWS Classifier 3) and/or Table D5 (SWS Classifier 4).

It will be evident that any of the diagnosis and treatment methods may suitably be combined with other methods of assessing the aggressiveness of the tumour, the patient's health and susceptibility to treatment, etc. For example, the diagnosis or choice of therapy may be determined by further assessing the size of the tumour, or the lymph node stage or both, optionally together or in combination with other risk factors

Specifically, the choice of therapy may be determined by assessing the Nottingham Prognostic Index (NPI). The NPI is described in detail in Haybittle, et al., 1982. In combination with the grading methods described here, the method is suitable for assigning a breast tumour patient into a prognostic group. Such a combined method comprises deriving a score which is the sum of the following: (a) (0.2 x tumour size in cm); (b) tumour grade in which the tumour grade is assigned by a method according to any of the gene expression detection methods described herein; and (c) lymph node stage; in which the tumour size and the lymph node stage are determined according to the Nottingham Prognostic Index, in which a patient with a score of 2.4 or less is categorised to a EPG (excellent prognostic group), a patient with a score of less than 3.4 is categorised to a GPG (good prognostic group), a patient with a score of between 3.4 and 5.4 is categorised to a MPG (moderate prognostic group), a patient with a score of greater than 5.4 is categorised to a PPG (poor prognostic group).

Alternatively, or in addition, a method of assigning a breast tumour patient into a prognostic group may comprise applying the Nottingham Prognostic Index to a breast tumour, but modified such that the histologic grade score of the breast tumour is replaced by a grade obtained by a gene expression detection method as described in this document.

Other factors which may of course be assessed for determining the choice of therapy may include receptor status, such as oestrogen receptor (ER) or progesterone receptor (PR) status, as known in the art. For example, the choice of therapy may be determined by further assessing the oestrogen receptor (ER) status of the breast tumour.

### GENE COMBINATIONS

We further provide for combinations of genes according to the various classifiers disclosed in this document. Such combinations may comprise mixtures of genes or corresponding probes, such as in a form which is suitable for detection of expression. For example, the combination may be provided in the form of DNA in solution.

In other embodiments, a microarray or chip is provided which comprises any combination of genes or probes, in the form of cDNA, genomic DNA, or RNA, within the classifiers. In some embodiments, the microarray or chip comprises all the genes or probes in SWS Classifier 0, SWS Classifier 1, SWS Classifier 2, SWS Classifier 3 or SWS Classifier 4. The genes may be synthesised or obtained by means known in the art, and attached on the microarray or chip by conventional means, as known in the art. Such microarrays or chips are useful in monitoring gene expression of any one or more of the genes comprised therein, and may be used for tumour grading or detection as described here.

We further describe a probe set consisting of a probe or probes having Affymetrix ID numbers as set out in Column 6 of Table D1, Table D2, Table D3, Table D4 or Table D5. Specifically, we describe an array, such as a microarray, comprising the probesets set out in Table D1 (SWS Classifier 0). We also describe an array such as a microarray comprising the genes or probesets set out in Table D2 (SWS 1 Classifier), an array such as a microarray comprising the genes or probesets set out in Table D3 (SWS Classifier 2), an array such as a microarray comprising the genes or probesets set out in Table D4 (SWS3 Classifier), and an array such as a microarray comprising the genes or probesets set out in Table D5 (SWS Classifier 4).

The probes or probe sets are suitably synthesised or made by means known in the art, for example by oligonucleotide synthesis, and may be attached to a microarray for easier carriage and storage. They may be used in a method of assigning a grade to a breast tumour as described herein.

We describe the use of Statistically Weighted Syndromes (SWS) on gene expression data which may comprise microarray gene expression data. We describe the use of SWS for gene discovery. We further describe such use in combination with Prediction Analysis of Microarrays (PAM). We describe the use of SWS to identify gene sets diagnostic of cancer status, such as breast cancer status or proliferative status.

### SCREENING

The methods and compositions described here may be used for identifying molecules capable of treating or preventing breast cancer, which may be used as drugs for cancer treatment. Such a method comprises: (a) grading a breast tumour as described using gene expression data; (b) exposing the breast tumour to a candidate molecule; and (c) detecting a change in tumour grade. The change in tumour grade is suitably determined by grading a breast tumour as described using gene expression data before and after exposure of the breast tumour to a candidate molecule. We provide molecule identified by such a method, for example for use in breast cancer treatment.

Particular screening applications relate to the testing of pharmaceutical compounds in drug research. The reader is referred generally to the standard textbook "In vitro Methods in Pharmaceutical Research", Academic Press, 1997, and U.S. Pat. No. 5,030,015). Assessment of the activity of candidate pharmaceutical compounds generally involves combining the breast cancer cells with the candidate compound, determining any change in the tumour grade, as determined by the gene expression detection methods described herein of the cells that is attributable to the compound (compared with untreated cells or cells treated with an inert compound), and then correlating the effect of the compound with the observed change.

The screening may be done, for example, either because the compound is designed to have a pharmacological effect on certain cell types such as tumour cells, or because a compound designed to have effects elsewhere may have unintended side effects. Two or more drugs can be tested in combination (by combining with the cells either simultaneously or sequentially), to detect possible drug-drug interaction effects. In some applications, compounds are screened initially for potential toxicity (Castell et al., pp. 375-410 in "In vitro Methods in Pharmaceutical Research," Academic Press, 1997). Cytotoxicity can be determined in the first instance by the effect on cell viability, survival, morphology, and expression or release of certain markers, receptors or enzymes. Effects of a drug on chromosomal DNA can be determined by measuring DNA synthesis or repair. [³H]thymidine or BrdU incorporation, especially at unscheduled times in the cell cycle, or above the level required for cell replication, is consistent with a drug effect. The reader is referred to A. Vickers (PP 375-410 in "In vitro Methods in Pharmaceutical Research," Academic Press, 1997) for further elaboration.

Candidate molecules subjected to the assay and which are found to be of interest may be isolated and further studied. Methods of isolation of molecules of interest will depend on the type of molecule employed, whether it is in the form of a library, how many candidate molecules are being tested at any one time, whether a batch procedure is being followed, etc.

The candidate molecules may be provided in the form of a library. In an embodiment, more than one candidate molecule is screened simultaneously. A library of candidate molecules may be generated, for example, a small molecule library, a polypeptide library, a nucleic acid library, a library of compounds (such as a combinatorial library), a library of antisense molecules such as antisense DNA or antisense RNA, an antibody library etc, by means known in the art. Such libraries are suitable for high-throughput screening. Tumour cells may be exposed to individual members of the library, and the effect on tumour grade, if any, cell determined. Array technology may be employed for this purpose. The cells may be spatially separated, for example, in wells of a microtitre plate.

In an embodiment, a small molecule library is employed. By a "small molecule", we refer to a molecule whose molecular weight may be less than about 50 kDa. In particular embodiments, a small molecule has a molecular weight may be less than about 30 kDa, such as less than about 15 kDa, or less than 10 kDa or so. Libraries of such small molecules, here referred to as "small molecule libraries" may contain polypeptides, small peptides, for example, peptides of 20 amino acids or fewer, for example, 15, 10 or 5 amino acids, simple compounds, etc.

Alternatively or in addition, a combinatorial library, as described in further detail below, may be screened for candidate modulators of tumour function.

### COMBINATORIAL LIBRARIES

Libraries, in particular, libraries of candidate molecules, may suitably be in the form of combinatorial libraries (also known as combinatorial chemical libraries).

A "combinatorial library", as the term is used in this document, is a collection of multiple species of chemical compounds that consist of randomly selected subunits. Combinatorial libraries may be screened for molecules which are capable of changing the choice by a stem cell between the pathways of self-renewal and differentiation.

Various combinatorial libraries of chemical compounds are currently available, including libraries active against proteolytic and non-proteolytic enzymes, libraries of agonists and antagonists of G-protein coupled receptors (GPCRs), libraries active against non-GPCR targets (e.g., integrins, ion channels, domain interactions, nuclear receptors, and transcription factors) and libraries of whole-cell oncology and anti-infective targets, among others. A comprehensive review of combinatorial libraries, in particular their construction and uses is provided in Dolle and Nelson (1999), Journal of Combinatorial Chemistry, Vol 1 No 4, 235-282. Reference is also made to *Comb inatorial peptide library protocols* (edited by Shmuel Cabilly, Totowa, N.J. : Humana Press, c1998. Methods in Molecular Biology ; v. 87). Specific combinatorial libraries and methods for their construction are disclosed in United States Patent 6,168,914 (Campbell, et al), as well as in Baldwin et al. (1995), "Synthesis of a Small Molecule Library Encoded with Molecular Tags," J. Am. Chem. Soc. 117:5588-5589, and in the references mentioned in those documents.

Further references describing chemical combinatorial libraries, their production and use include those available from the URL http://www.netsci.org/Science/Combichem/, including The Chemical Generation of Molecular Diversity. Michael R. Pavia, Sphinx Pharmaceuticals, A Division of Eli Lilly (Published July, 1995); Combinatorial Chemistry: A Strategy for the Future - MDL Information Systems discusses the role its Project Library plays in managing diversity libraries (Published July, 1995); Solid Support Combinatorial Chemistry in Lead Discovery and SAR Optimization, Adnan M. M. Mjalli and Barry E. Toyonaga, Ontogen Corporation (Published July, 1995); Non-Peptidic Bradykinin Receptor Antagonists From a Structurally Directed Non-Peptide Library. Sarvajit Chakravarty, Babu J. Mavunkel, Robin Andy, Donald J. Kyle*, Scios Nova Inc. (Published July, 1995); Combinatorial Chemistry Library Design using Pharmacophore Diversity Keith Davies and Clive Briant, Chemical Design Ltd. (Published July, 1995); A Database System for Combinatorial Synthesis Experiments - Craig James and David Weininger, Daylight Chemical Information Systems, Inc. (Published July, 1995); An Information Management Architecture for Combinatorial Chemistry, Keith Davies and Catherine White, Chemical Design Ltd. (Published July, 1995); Novel Software Tools for Addressing Chemical Diversity, R. S. Pearlman, Laboratory for Molecular Graphics and Theoretical Modeling, College of Pharmacy, University of Texas (Published June/July, 1996); Opportunities for Computational Chemists Afforded by the New Strategies in Drug Discovery: An Opinion, Yvonne Connolly Martin, Computer Assisted Molecular Design Project, Abbott Laboratories (Published June/July, 1996); Combinatorial Chemistry and Molecular Diversity Course at the University of Louisville: A Description, Arno F. Spatola, Department of Chemistry, University of Louisville (Published June/July, 1996); Chemically Generated Screening Libraries: Present and Future. Michael R. Pavia, Sphinx Pharmaceuticals, A Division of Eli Lilly (Published June/July, 1996); Chemical Strategies For Introducing Carbohydrate Molecular Diversity Into The Drug Discovery Process.. Michael J. Sofia, Transcell Technologies Inc. (Published June/July, 1996); Data Management for Combinatorial Chemistry. Maryjo Zaborowski, Chiron Corporation and Sheila H. DeWitt, Parke-Davis Pharmaceutical Research, Division of Warner-Lambert Company (Published November, 1995); and The Impact of High Throughput Organic Synthesis on R&D in Bio-Based Industries, John P. Devlin (Published March, 1996).

Techniques in combinatorial chemistry are gaining wide acceptance among modern methods for the generation of new pharmaceutical leads (Gallop, M. A. et al., 1994, J. Med. Chem. 37:1233-1251; Gordon, E. M. et al., 1994, J. Med. Chem. 37:1385-1401.). One combinatorial approach in use is based on a strategy involving the synthesis of libraries containing a different structure on each particle of the solid phase support, interaction of the library with a soluble receptor, identification of the 'bead' which interacts with the macromolecular target, and determination of the structure carried by the identified 'bead' (Lam, K. S. et al., 1991, Nature 354:82-84). An alternative to this approach is the sequential release of defined aliquots of the compounds from the solid support, with subsequent determination of activity in solution, identification of the particle from which the active compound was released, and elucidation of its structure by direct sequencing (Salmon, S. E. et al., 1993, Proc.Natl.Acad.Sci.USA 90:11708-11712), or by reading its code (Kerr, J. M. et al., 1993, J.Am.Chem.Soc. 115:2529-2531; Nikolaiev, V. et al., 1993, Pept. Res. 6:161-170; Ohlmeyer, M. H. J. et al., 1993, Proc.Natl.Acad.Sci.USA 90:10922-10926). '

Soluble random combinatorial libraries may be synthesized using a simple principle for the generation of equimolar mixtures of peptides which was first described by Furka (Furka, A. et al., 1988, Xth International Symposium on Medicinal Chemistry, Budapest 1988; Furka, A. et al., 1988, 14th International Congress of Biochemistry, Prague 1988; Furka, A. et al., 1991, Int. J. Peptide Protein Res. 37:487-493). The construction of soluble libraries for iterative screening has also been described (Houghten, R. A. et al. 1991, Nature 354:84-86). K. S. Lam disclosed the novel and unexpectedly powerful technique of using insoluble random combinatorial libraries. Lam synthesized random combinatorial libraries on solid phase supports, so that each support had a test compound of uniform molecular structure, and screened the libraries without prior removal of the test compounds from the support by solid phase binding protocols (Lam, K. S. et al., 1991, Nature 354:82-84).

Thus, a library of candidate molecules may be a synthetic combinatorial library (e.g., a combinatorial chemical library), a cellular extract, a bodily fluid (e.g., urine, blood, tears, sweat, or saliva), or other mixture of synthetic or natural products (e.g., a library of small molecules or a fermentation mixture).

A library of molecules may include, for example, amino acids, oligopeptides, polypeptides, proteins, or fragments of peptides or proteins; nucleic acids (e.g., antisense; DNA; RNA; or peptide nucleic acids, PNA); aptamers; or carbohydrates or polysaccharides. Each member of the library can be singular or can be a part of a mixture (e.g., a compressed library). The library may,contain purified compounds or can be "dirty" (i.e., containing a significant quantity of impurities).

Commercially available libraries (e.g., from Affymetrix, ArQule, Neose Technologies, Sarco, Ciddco, Oxford Asymmetry, Maybridge, Aldrich, Panlabs, Pharmacopoeia, Sigma, or Tripose) may also be used with the methods described here.

In addition to libraries as described above, special libraries called diversity files can be used to assess the specificity, reliability, or reproducibility of the new methods. Diversity files contain a large number of compounds (e.g., 1000 or more small molecules) representative of many classes of compounds that could potentially result in nonspecific detection in an assay. Diversity files are commercially available or can also be assembled from individual compounds commercially available from the vendors listed above.

### ANALYSIS METHOD - RNA PURIFICATION

The breast tumour is surgically resected, processed, and snap frozen. A frozen portion of the tumour is processed for total RNA extraction using the Qiagen RNeasy kit (Qiagen, Valencia, CA). Briefly, frozen tumours are cut into minute pieces, and pieces totalling ∼50-100 milligrams (mg) are homogenized for 40 seconds in RNeasy Lysis Buffer (RLT). Proteinase K is added, and the samples are incubated for 10 minutes at 55 degrees C, followed by centrifugation and the addition of ethanol. After transferring the supernatant into RNeasy columns, DNase is added. Collected RNA is then assessed for quality using an Agilent 2100 bioanalyzer (Agilent Technologies, Rockville, MD) or by agarose gel. The RNA is stored at minus -70 degrees C.

### MICROARRAY ANALYSIS

Labeled cRNA target is generated for microarray hybridization essentially according to the Affymetrix protocol (Affymetrix, Santa Clara, CA). Briefly, approximately 5 micrograms (µg) of total RNA are reversed transcribed into first-strand cDNA using a T7-linked oligo-dT primer, followed by second strand synthesis. A T7 RNA polymerase is then used to linearly amplify antisense RNA. This "cRNA" is biotinylated and chemically fragmented at 95°C. Ten µg of the fragmented, biotinylated cRNA is hybridized at 45°C for 16 hours to an Affymetrix high-density oligonucleotide GenChip array. The array is then washed and stained with streptavidin-phycoerythrin (10 µg/ml). Signal amplification is achieved using a biotinylated anti-streptavidin antibody. The scanned images are inspected for the presence of artifacts. In case of defects, the hybridization procedure is repeated. Expression values and detection calls are computed from raw data following the procedures outlined for the Affymetrix MAS 5.0 analysis software. Global mean normalization of the gene expression by hybridization signals across all arrays is used to control for differences in chip hybridization signal intensity values. To do that for a given array *j*(*j*=1,2,...*M*), we calculated normalization coefficients *kⱼ*(*j*=1,2,...,*n*), by the following formula: ${k}_{j} = n * ln \left(500\right) / {\sum }_{i = 1}^{n} ln \left({a}_{ij}\right) ,$ where *n* is the number of observed probe sets, *aᵢⱼ* is the signal intensity value of the i-th Affymetrix probesets representing a gene expression. Then the natural logarithm of the signal intensity value of the given array *j* was multiplied by this normalization coefficient. A normalisation coefficient of 500 is used in determining the cut-offs shown in the Tables in this document.

### SWS ANALYSIS

The microarray-derived normalized numerical expression values corresponding to the genetic grade signature genes are used as input for the SWS algorithm.

### OTHER METHODS

The RNA purification and microarray analysis methodologies above reflect only our "preffered methods", and that other variants exist that could be used in conjunction with our Process for Predicting Patient Outcome...For example, the starting material could be formalin-fixed paraffin-embedded tumour material instead of fresh frozen material, or the RNA might be extracted using a Cesium Chloride Gradient method, or the RNA could be analyzed by NimbleGen Microarrays that include DNA probes corresponding to our genes of interest. And it should also be noted that a microarray may not be necessary at all to determine the expression levels of our signature genes, but rather their expression could be quantitatively measured by PCR-based techniques such as real time-PCR.

### CLASSIFIERS, GENE SETS AND PROBE SETS

### SWS CLASSIFIER 0 (TABLE D1)

**Table D1: SWS Classifier 0: 264 Probesets.**

| **Order** | **UGID(build #177)** | **UnigeneName** | **Gene Symbol** | **Genbank Acc** | **Affi ID** | **Grade with Higher Expres sion** | **Grade with Lower Expressi on** | **Cut-Off** | **Chi-2** | **Instability indices** |
|---|---|---|---|---|---|---|---|---|---|---|
| 1 | Hs.528654 | Hypothetical protein FLJ11029 | FLJ11029 | BG165011 | B.228273_at | 3 | 1 | 7.7063 | 95.973 | 0.011 |
| 2 | acc_NM_00 3158.1 | | | NM_003158 | A.208079_s_at | 3 | 1 | 6.6526 | 95.599 | 0.002 |
| 3 | Hs.308045 | Barren homolog (Drosophila) | BRRN1 | D38553 | A.212949_at | 3 | 1 | 5.9167 | 92.640 | 0.006 |
| 4 | Hs.35962 | CDNA clone IMAGE:4452583, partial cds | | BG492359 | B.226936_at | 3 | 1 | 7.5619 | 92.601 | 0.003 |
| 5 | Hs.184339 | Maternal embryonic leucine zipper kinase | MELK | NM_014791 | A.204825_at | 3 | 1 | 7.1073 | 90.110 | 0.002 |
| 6 | Hs.250822 | Serine/threonine kinase 6 | *STK6* | NM_003600 | A.204092_s_at | 3 | 1 | 6.7266 | 88.639 | 0.003 |
| 7 | Hs.9329 | TPX2, microtubule-associated protein homolog (Xenopus laevis) | TPX2 | AF098158 | A.210052_s_at | 3 | 1 | 7.4051 | 86.239 | 0.001 |
| 8 | Hs.1594 | Centromere protein A, 17kDa | CENPA | NM_001809 | A.204962_s_at | 3 | 1 | 6.344 | 85.316 | 0.037 |
| 9 | Hs.198363 | MCM10 minichromosome maintenance deficient 10 (*S.* cerevisiae) | MCM10 | AB042719 | B.222962_s_at | 3 | 1 | 6.1328 | 85.176 | 0.001 |
| 10 | Hs.48855 | Cell division cycle associated 8 | CDCA8 | BC001651 | A.221520_s_at | 3 | 1 | 5.2189 | 85.152 | 0.018 |
| 11 | Hs.169840 | TTK protein kinase | TTK | NM_003318 | A.204822_at | 3 | 1 | 6.2397 | 82.242 | 0.017 |
| 12 | Hs.69360 | Kinesin family member 2C | KIF2C | U63743 | A.209408_at | 3 | 1 | 7.3717 | 82.105 | 0.006 |
| 13 | Hs.55028 | CDNA clone IMAGE:60A3059, partial cds | | BF111626 | B.228559_at | 3 | 1 | 7.2212 | 82.105 | 0.001 |
| 14 | Hs.511941 | Forkhead box M1 | FOXM1 | NM_021953 | A.202580_x_at | 3 | 1 | 6.5827 | 81.868 | 0.001 |
| 15 | Hs.3104 | Kinesin family member 14 | KIF14 | AW183154 | B.236641_at | 3 | 1 | 6.4175 | 81.868 | 0.023 |
| 16 | Hs.179718 | V-myb myeloblastosis viral oncogene homolog (avian)-like 2 | MYBL2 | NM_002466 | A.201710_at | 3 | 1 | 6.0661 | 79.208 | 0.017 |
| 17 | Hs.93002 | Ubiquitin-conjugating enzyme E2C | UBE2C | NM_007019 | A202954_at | 3 | 1 | 7.8431 | 79.208 | 0.064 |
| 18 | Hs.344037 | Protein regulator of cytokinesis 1 | PRC1 | NM_003981 | A.218009_s_at | 3 | 1 | 7.3376 | 79.208 | 0.003 |
| 19 | Hs.436187 | Thyroid hormone receptor interactor 13 | TRIP13 | NM_004237 | A.204033_at | 3 | 1 | 7.1768 | 78.981 | 0.091 |
| 20 | Hs.408658 | Cyclin E2 | CCNE2 | NM_004702 | A.205034_at | 3 | 1 | 6.2055 | 78.603 | 0.019 |
| 21 | Hs.30114 | Cell division cycle associated 3 | CDCA3 | BC002551 | B.223307_at | 3 | 1 | 7.8418 | 78.603 | 0.084 |
| 22 | Hs.84113 | Cyclin-dependent kinase inhibitor 3 (CDK2-associated dual specificity phosphatase) | CDKN3 | AF213033 | A.209714_s_at | 3 | 1 | 6.8414 | 78.554 | 0.005 |
| 23 | Hs.279766 | Kinesin family member 4A | KIF4A | NM_012310 | A.219355_at | 3 | 1 | 6.6174 | 78.212 | 0.013 |
| 24 | Hs.104859 | Hypothetical protein DKFZp762E1312 | DKFZp76 2E1312 | NM_018410 | A.218726_at | 3 | 1 | 6.3781 | 75.507 | 0.036 |
| 25 | Hs.444118 | MCM6 minichromosome maintenance deficient 6 (MIS5 homolog, S. pombe) (S. cerevisiae) | MCM6 | NM_005915 | A.201930_at | 3 | 1 | 7.9353 | 75.386 | 0.014 |
| 26 | acc_NM_01 8123.1 | | | NM_018123 | A.219918_s_at | 3 | 1 | 6.5958 | 75.386 | 0.002 |
| 27 | Hs.287472 | BUB1 budding uninhibited by benzimidazoles 1 homolog (yeast) | BUR1 | AF043294 | A.209642_at | 3 | 1 | 6.0118 | 74.136 | 0.058 |
| 28 | *Hs.36708* | BUB1 budding uninhibited by benzimidazoles 1 homolog beta (yeast) | BUB1B | NM_001211 | A.203755_at | 3 | 1 | 6.68 | 73.453 | 0.007 |
| 29 | Hs.77783 | Membrane-associated tyrosine- and threonine-specific cdc2-inhibitory kinase | PKMYT1 | NM_004203 | A.204267_x_at | 3 | 1 | 6.9229 | 73.441 | 0.002 |
| 30 | Hs.446554 | RAD51 homolog (RecA homolog, E. coli) (S. cerevisiae) | RAD51 | NM_002875 | A.205024_s_at | 3 | 1 | 6.3524 | 73.441 | 0.016 |
| 31 | Hs.82906 | CDC20 cell division cycle 20 homolog (S. cerevisiae) | CDC20 | NM_001255 | A.202870_s_at | 3 | 1 | 7.1291 | 72.984 | 0.108 |
| 32 | Hs.252712 | Karyopherin alpha 2 (RAG cohort 1, importin alpha 1) | KPNA2 | MM_002266 | A.201088_at | 3 | 1 | 8.4964 | 72.560 | 0.025 |
| 33 | Hs.3104 | | KIF14 | NM_014875 | A.206364_at | 3 | 1 | 6.1518 | 72.560 | 0.067 |
| 34 | Hs.103305 | Chromobox homolog 2 (Pc class homolog, Drosophila) | BE514414 | BES14414 | B.226473_at | 3 | 1 | 7.5588 | 72.560 | 0.014 |
| 35 | Hs.152759 | Activator of S phase kinase | ASK | NM_006716 | A.204244_s_at | 3 | 1 | 5.9825 | 72.294 | 0.018 |
| 36 | acc_AL1388 28 | | | AL138828 | B.228069_at | 3 | 1 | 7.0119 | 72.294 | 0.084 |
| 37 | Hs.226390 | Ribonucleotide reductase M2 polypeptide | RRM2 | NM_001034 | A.201890_at | 3 | 1 | 7.1014 | 70.961 | 0.002 |
| 38 | Hs.445890 | HSPC163 protein | HSPC163 | NM_014184 | A.218728_s_at | 3 | 1 | 7.6481 | 70.764 | 0.003 |
| 39 | Hs.194698 | Cyclin B2 | CCNB2 | NM_004701 | A.202705_at | 3 | 1 | 7.0096 | 70.698 | 0.001 |
| 40 | Hs.234545 | Cell division cycle associated 1 | CDCA1 | AF326731 | B.223381_at | 3 | 1 | 6.4921 | 70.698 | 0.008 |
| 41 | Hs.16244 | Sperm associated antigen 5 | SPAG5 | NM_006461 | A.203145_at | 3 | 1 | 6.4627 | 70.095 | 0.001 |
| 42 | Hs.62180 | Anillin, actin binding protein (scraps homolog, Drosophila) | ANLN | AK023208 | B.222608_s_at | 3 | 1 | 6.9556 | 69.641 | 0.013 |
| 43 | Hs.14559 | Chromosome 10 open reading frame 3 | C10orf3 | NM_018131 | A.218542_at | 3 | 1 | 6.4965 | 69.335 | 0.049 |
| 44 | Hs.122908 | DNA replication factor | CDT1 | AW075105 | B.228868_x_at | 3 | 1 | 7.0543 | 69.335 | 0.001 |
| 45 | Hs.8878 | Kinesin family member 11 | KIF11 | NM_004523 | A.204444_at | 3 | 1 | 6.4655 | 69.318 | 0.005 |
| 46 | Hs.83758 | CDC28 protein kinase regulatory subunit 2 | CKS2 | NM_001827 | A.204170_s_at | 3 | 1 | 7.8353 | 69.178 | 0.027 |
| 47 | Hs.112160 | Chromosome 15 open reading frame 20 | PIF1 | AF108138 | B.228252_at | 3 | 1 | 6.6518 | 69.178 | 0.039 |
| 48 | Hs.79078 | MAD2 mitotic arrest deficient-like 1 (yeast) | MAD2L1 | NM_002358 | A.203362_s_at | 3 | 1 | 6.4606 | 68.044 | 0.038 |
| 49 | Hs.226390 | Ribonucleotide reductase M2 polypeptide | RRM2 | BC001886 | A.209773_s_at | 3 | 1 | 7.2979 | 67.380 | 0.135 |
| 50 | Hs.462306 | Ubiquitin-conjugating enzyme E2S | UBE2S | NM_014501 | A.202779_s_at | 3 | 1 | 6.9165 | 67.359 | 0.013 |
| 51 | Hs.70704 | Chromosome 20 open reading frame 129 | C20orf12 9 | BC001068 | B.225687_at | 3 | 1 | 7.2322 | 67.359 | 0.039 |
| 52 | Hs.294088 | GAJ protein | GAJ | AY028916 | B.223700_at | 3 | 1 | 5.8432 | 67.299 | 0.005 |
| 53 | Hs.381225 | Kinetochore protein Spc24 | Spc24 | AI469788 | B.235572_at | 3 | 1 | 6.7839 | 67.299 | 0.002 |
| 54 | Hs.334562 | Cell division cycle 2, G1 to S and G2 to M | CDC2 | AL524035 | A.203213_at | 3 | 1 | 7.0152 | 66.861 | 0.024 |
| 55 | Hs.109706 | Hematological and neurological expressed 1 | HN1 | NM_016185 | A.217755_at | 3 | 1 | 7.9118 | 66.771 | 0.008 |
| 56 | Hs.23900 | Rac GTPase activating protein 1 | RACGAP 1 | AU153848 | A.222077_s_at | 3 | 1 | 7.1207 | 66.484 | 0.042 |
| 57 | Hs.77695 | Discs, large homolog 7 (Drosophila) | DLG7 | NM_014750 | A.203764_at | 3 | 1 | 6.3122 | 66.411 | 0.001 |
| 58 | Hs.46423 | Histone 1, H4c | HIST1H4 F | NM_003542 | A.205967_at | 3 | 1 | 8.3796 | 66.411 | 0.005 |
| 59 | Hs.20830 | Kinesin family member C1 | KIFC1 | BC000712 | A.209680_s_at | 3 | 1 | 6.9746 | 66.411 | 0.042 |
| 60 | Hs.339665 | Similar to Gastric cancer up-regulated-2 | | AL135396 | B.225834_at | 3 | 1 | 7.2467 | 66.411 | 0.020 |
| 61 | Hs.94292 | FLJ23311 protein | FLJ23311 | NM_024680 | A.219990_at | 3 | 1 | 5.0277 | 66.340 | 0.007 |
| 62 | Hs.73625 | Kinesin family member 20A | KIF20A | NM_005733 | A.218755_at | 3 | 1 | 7.2115 | 66.267 | 0.001 |
| 63 | Hs.315167 | Defective in sister chromatid cohesion homolog 1 (S. cerevisiae) | MGC552 8 | NM_024094 | A.219000_s_at | 3 | 1 | 6.2835 | 66.267 | 0.002 |
| 64 | Hs.85137 | Cyclin A2 | CCNA2 | NM_001237 | A.203418_at | 3 | 1 | 6.194 | 66.208 | 0.001 |
| 65 | Hs.528669 | Chromosome condensation protein G | HCAP-G | NM_022346 | A.218662_s_at | 3 | 1 | 6.0594 | 66.208 | 0.013 |
| 66 | Hs.75573 | Centromere protein E, 312kDa | CENPE | NM_001813 | A.205046_at | 3 | 1 | 5.1972 | 65.474 | 0.002 |
| 67 | acc_BE9661 46 | RAD51 associated protein 1 | | BE966146 | A.204146_at | 3 | 1 | 6.3049 | 65.318 | 0.007 |
| 68 | Hs.334562 | Cell division cycle 2, G1 to S and G2 to M | CDC2 | D88357 | A.210559_s_at | 3 | 1 | 7.0395 | 64.754 | 0.001 |
| 69 | Hs.108106 | Ubiquitin-like, containing PHD and RING finger domains, 1 | UHRF1 | AK025578 | B.225655_at | 3 | 1 | 7.7335 | 64.754 | 0.024 |
| 70 | Hs.1578 | Baculoviral IAP repeat-containing 5 (survivin) | BIRC5 | NM_001168 | A.202095_s_at | 3 | 1 | 6.8907 | 64.566 | 0.090 |
| 71 | acc_NM_02 1067.1 | | | NM_021067 | A.206102_at | 3 | 1 | 6.714 | 64.566 | 0.013 |
| 72 | Hs.244723 | Cyclin E1 | CCNE1 | AI671049 | A.213523_at | 3 | 1 | 6.082 | 64.566 | 0.001 |
| 73 | Hs.198363 | MCM10 minichromosome maintenance deficient 10 (S. cerevisiae) | MCM10 | NM_018518 | A.220651_s_at | 3 | 1 | 5.6784 | 64.175 | 0.081 |
| 74 | Hs.155223 | Stanniocalcin 2 | STC2 | AI435828 | A.203438_at | 1 | 3 | 7.5388 | 63.993 | 0.011 |
| 75 | Hs.25647 | V-fos FBJ murine osteosarcoma viral oncogene homolog | FOS | BC004490 | A.209189_at | 1 | 3 | 8.9921 | 63.898 | 0.162 |
| 76 | Hs.184601 | Solute carrier family 7 (cationic amino acid transporter, y+ system), member 5 | SLC7A5 | AB018009 | A.201195_s_at | 3 | 1 | 7.4931 | 63.584 | 0.011 |
| 77 | Hs.528669 | Chromosome condensation protein G | HCAP-G | NM_022346 | A.218663_at | 3 | 1 | 5.7831 | 63.584 | 0.007 |
| 78 | Hs.30114 | Cell division cycle associated 3 | CDCA3 | NM_031299 | A.221436_s_at | 3 | 1 | 6.1898 | 63.584 | 0.002 |
| 79 | Hs.296398 | Lysosomal associated protein transmembrane 4 beta | LAPTM4 B | T15777 | A.214039_s_at | 3 | 1 | 9.3209 | 63.330 | 0.001 |
| 80 | Hs.442658 | Aurora kinase B | AURKB | AB011446 | A.209464_at | 3 | 1 | 5.9611 | 63.256 | 0.005 |
| .81 | Hs.6879 | DC13 protein | DC13 | NM 020188 | A.218447_at | 3 | | 7.436 | 63.256 | 0.028 |
| 82 | Hs.78913 | Chemokine (C-X3-C motif) receptor 1 | CX3CR1 | U20350 | A.205898_at | 1 | 3 | 6.7764 | 63.223 | 0.014 |
| 83 | Hs.406684 | Sodium channel, voltage-gated, type VII, alpha | SCN7A | AI828648 | B.228504_at | 1 | 3 | 5.8248 | 63.223 | 0.004 |
| 84 | Hs.80976 | Antigen identified by monoclonal antibody Ki-67 | MKI67 | BF601806 | A.212022_s_at | 3 | 1 | 6.7255 | 62.415 | 0.125 |
| 85 | Hs.406639 | Hypothetical protein LOC146909 | LOC1469 09 | AA292789 | A.222039_at | 3 | 1 | 6.4591 | 62.214 | 0.018 |
| 86 | Hs.334562 | Cell division cycle 2, G1 to S and G2 to M | CDC2 | NM_001786 | A.203214_x_at | 3 | 1 | 6.588 | 61.528 | 0.002 |
| 87 | Hs.23960 | Cyclin B1 | CCNB1 | BE407516 | A.214710_s_at | 3 | 1 | 7.1555 | 60.835 | 0.014 |
| 88 | Hs.445098 | DEP domain containing 1 | SDP35 | AK000490 | B.222958_s_at | 3 | 1 | 6.8747 | 60.835 | 0.003 |
| 89 | Hs.58241 | Serine/threonine kinase 32B | HSA2508 39 | NM_018401 | A.219686_at | 1 | 3 | 4.5663 | 60.376 | 0.005 |
| 90 | Hs.5199 | HSPC150 protein similar to ubiquitin-conjugating enzyme | HSPC150 | AB032931 | B.223229_at | 3 | 1 | 7.3947 | 60.376 | 0.010 |
| 91 | acc_T58044 | | | T58044 | B.227232_at | 1 | 3 | 8.5021 | 60.376 | 0.003 |
| 92 | Hs.421337 | DEP domain containing 1B | XTP1 | AK001166 | B.226980_at | 3 | 1 | 5.4977 | 60.356 | 0.034 |
| 93 | Hs.238205 | Chromosome 6 open reading frame 115 | C6orf115 | AF116682 | B.223361_at | 3 | 1 | 8.7555 | 60.138 | 0.003 |
| 94 | Hs.27860 | Prostaglandin E receptor 3 (subtype EP3) | | AW242315 | A.213933_at | 1 | 3 | 7.3561 | 59.754 | 0.257 |
| 95 | Hs.292511 | Neuro-oncological ventral antigen 1 | NOVA1 | NM_002515 | A.205794_s_at | 1 | 3 | 6.7682 | 59.512 | 0.011 |
| 96 | Hs.276466 | Hypothetical protein FLJ21062 | FLJ21062 | NM_024788 | A.219455_at | 1 | 3 | 5.5257 | 59.307 | 0.003 |
| 97 | Hs.270845 | Kinesin family member 23 | KIF23 | NM_004856 | A.204704_s_at | 3 | 1 | 5.1731 | 59.307 | 0.154 |
| 98 | Hs.293257 | Epithelial cell transforming sequence 2 oncogene | ECT2 | NM_018098 | A.219787_s_at | 3 | 1 | 6.8052 | 59.307 | 0.000 |
| 99 | Hs.156346 | Topoisomerase (DNA) II alpha 170kDa | TOP2A | NM_001067 | A.201292_at | 3 | 1 | 7.2468 | 59.071 | 0.011 |
| 100 | Hs.31297 | Cytochrome b reductase 1 | CYBRD1 | AL136693 | B.222453_at | 1 | 3 | 9.3991 | 59.071 | 0.001 |
| 101 | Hs.414407 | Kinetochore associated 2 | KNTC2 | NM_006101 | A.204162_at | 3 | 1 | 6.017 | 58.653 | 0.076 |
| 102 | Hs.445098 | DEP domain containing 1 | SDP35 | AI810054 | B.235545_at | 3 | 1 | 6.2495 | 58.653 | 0.133 |
| 103 | Hs.301052 | Kinesin family member 18A | DKFZP43 4G2226 | NM_031217 | A.221258_s_at | 3 | 1 | 5.3649 | 58.160 | 0.158 |
| 104 | Hs.431762 | Tetratricopeptide repeat domain 18 | LOC1184 91 | AW024437 | B.229170_s_at | 1 | 3 | 6.2298 | 58.160 | 0.065 |
| 105 | Hs.24529 | CHK1 checkpoint homolog (S. pombe) | CHEK1 | NM_001274 | A.205394_at | 3 | 1 | 5.6217 | 58.087 | 0.017 |
| 106 | Hs.87507 | BRCA1 interacting protein C-terminal helicase 1 | BRIP1 | BF056791 | B.235609_at | 3 | 1 | 7.1489 | 58.087 | 0.011 |
| 107 | Hs.348920 | FSH primary response (LRPR1 homolog, rat) 1 | FSHPRH 1 | BF793446 | A.214804_at | 3 | 1 | 5.0105 | 57.817 | 0.057 |
| 108 | Hs.127797 | CDNA FLJ11381 fis, clone HEMBA1000501 | | AI807356 | B.227350_at | 3 | 1 | 6.8658 | 57.782 | 0.014 |
| 109 | Hs.92458 | G protein-coupled receptor 19 | GPR19 | NM_006143 | A.207183_at | 3 | 1 | 5.2568 | 57.642 | 0.002 |
| 110 | Hs.552 | Steroid-5-alpha-reductase, alpha polypeptide 1 (3-oxo-5 alpha-steroid delta 4-dehydrogenase alpha | SRDSA1 | BC006373 | A.211056_s_at | 3 | 1 | 6.7605 | 57.642 | 0.001 |
| 111 | Hs.435733 | Cell division cycle associated 7 | CDCA7 | AY029179 | B.224428_s_at | 3 | 1 | 7.6746 | 57.642 | 0.021 |
| 112 | Hs.101174 | Microtubule-associated protein tau | MAPT | NM_016835 | A.203929_s_at | 1 | 3 | 7.7914 | 57.600 | 0.003 |
| 113 | Hs.436376 | Synaptotagmin binding, cytoplasmic RNA interacting protein | SYNCRIP | NM_006372 | A.217834_s_at | 3 | 1 | 6.8123 | 57.600 | 0.001 |
| 114 | Hs.122552 | G-2 and S-phase expressed 1 | GTSE1 | NM_016426 | A.204315_s_at | 3 | 1 | 6.4166 | 57.542 | 0.036 |
| 115 | Hs.153704 | NIMA (never in mitosis gene a)-related kinase 2 | NEK2 | NM_002497 | A.204641_at | 3 | 1 | 7.0017 | 57.542 | 0.036 |
| 116 | Hs.208912 | Chromosome 22 open reading frame 18 | C22orf18 | NM_024053 | A.218741_at | 3 | 1 | 6.3488 | 56.776 | 0.006 |
| 117 | Hs.81892 | KIAA0101 | KIAA010 1 | NM_014736 | A.202503_s_at | 3 | 1 | 8.2054 | 56.644 | 0.029 |
| 118 | Hs.279905 | Nucleolar and spindle associated protein 1 | NUSAP1 | NM_016359 | A.218039_at | 3 | 1 | 7.542 | 56.644 | 0.006 |
| 119 | Hs.170915 | Hypothetical protein FLJ10948 | FLJ10948 | NM_018281 | A.218552_at | 1 | 3 | 7.9778 | 56.041 | 0.010 |
| 120 | Hs.144151 | Transcribed locus | | AI668620 | B.237339_at | 1 | 3 | 9.6693 | 56.041 | 0.029 |
| 121 | Hs.433180 | DNA replication complex GINS protein PSF2 | Pfs2 | BC003186 | A.221521_s_at | 3 | 1 | 6.3201 | 56.036 | 0.059 |
| 122 | Hs.47504 | Exonuclease 1 | EXO1 | NM_003686 | A.204603_at | 3 | 1 | 5.927 | 55.961 | 0.001 |
| 123 | Hs.293257 | Epithelial cell transforming sequence 2 oncogene | ECT2 | BG170335 | B.234992_x_at | 3 | 1 | 5.1653 | 55.559 | 0.002 |
| 124 | Hs.385913 | Acidic (leucine-rich) nuclear phosphoprotein 32 family, member E | ANP32E | NM_030920 | A.208103_s_at | 3 | 1 | 6.2989 | 55.557 | 0.001 |
| 125 | Hs.44380 | Transcribed locus, weakly similar to NP_060312.1 hypothetical protein FLJ20489 [Homo sapiens] | | AA938184 | B.236312_at | 3 | 1 | | 55.557 | 0.007 |
| 126 | Hs.19322 | Chromosome 9 open reading frame 140 | LOC8995 8 | AW250904 | B.225777_at | 3 | 1 | 7.8877 | 55.205 | 0.003 |
| 127 | Hs.188173 | Lymphoid nuclear protein related to AF4 | | AA572675 | B.232286_at | 1 | 3 | 7.169 | 55.205 | 0.008 |
| 128 | Hs.28264 | Chromosome 10 open reading frame 56 | FLJ90798 | AL049949 | A.212419_at | 1 | 3 | 7.6504 | 55.175 | 0.017 |
| 129 | Hs.387057 | Hypothetical protein FLJ13710 | FLJ13710 | AK024132 | B.232944_at | 1 | 3 | 6.1947 | 55.175 | 0.034 |
| 130 | acc_AL0316 58 | | | AL031658 | B.232357_at | 1 | 3 | 5.9761 | 54.950 | 0.033 |
| 131 | Hs.286049 | Phosphoserine aminotransferase 1 | PSAT1 | BC004863 | B.223062_s_at | 3 | 1 | 6.1035 | 54.930 | 0.003 |
| 132 | Hs.19173 | Nucleoporin 88kDa | | AI806781 | B.235786_at | 1 | 3 | 7.2856 | 54.930 | 0.037 |
| 133 | Hs.155223 | Stanniocalcin 2 | STC2 | BC000658 | A.203439_s_at | 1 | 3 | 7.6806 | 54.822 | 0.040 |
| 134 | acc_NM_03 0896.1 | | | NM_030896 | A.221275_s_at | 1 | 3 | 3.9611 | 54.822 | 0.002 |
| 135 | Hs.101174 | Microtubule-associated protein tau | MAPT | AA199717 | B.225379_at | 1 | 3 | 7.8574 | 54.814 | 0.021 |
| 136 | Hs.446680 | Retinoic acid induced 2 | RAI2 | NM_021785 | A.219440_at | 1 | 3 | 6.6594 | 54.307 | 0.057 |
| 137 | Hs.431762 | Tetratricopeptide repeat domain 18 | LOC1184 91 | AW024437 | B.229169_at | 1 | 3 | 5.8266 | 53.649 | 0.002 |
| 138 | acc_NM_00 5196.1 | | | NM_005196 | A.207828_s_at | 3 | 1 | 7.237 | 53.119 | 0.007 |
| 139 | acc_T90295 | Arsenic transactivated protein 1 | | T90295 | B.226661_at | 3 | 1 | 6.6825 | 52.825 | 0.002 |
| 140 | Hs.42650 | ZW10 interactor | ZWINT | NM_007057 | A.204026_s_at | 3 | 1 | 7.5055 | 52.716 | 0.034 |
| 141 | Hs.6641 | | KIF5C | NM_004522 | A.203130_s_at | 1 | 3 | 7.3214 | 52.703 | 0.013 |
| 142 | Hs.23960 | Cyclin B1 | CCNB1 | N90191 | B.228729_at | 3 | 1 | 6.8018 | 52.606 | 0.031 |
| 143 | Hs.72550 | Hyaluronan-mediated motility receptor (RHAMM) | HMMR | NM_012485 | A.207165_at | 3 | 1 | 6.5885 | 52.400 | 0.066 |
| 144 | Hs.73239 | Hypothetical protein FLJ10901 | FLJ10901 | NM_018265 | A.219010_at | 3 | 1 | 6.9429 | 52.323 | 0.020 |
| 145 | Hs.163533 | V-erb-a-erythroblastic leukemia viral oncogene homolog 4 (avian) | | AK024204 | B.233498_at | 1 | 3 | | 52.208 | 0.002 |
| 146 | Hs.109706 | Hematological and neurological expressed 1 | HN1 | AF060925 | B.222396_at | 3 | 1 | 8.4225 | 52.166 | 0.000 |
| 147 | Hs.165258 | Nuclear receptor subfamily 4, group A, member 2 | | AA523939 | B.235739_at | 1 | 3 | 7.1874 | 52.022 | 0.000 |
| 148 | Hs.20575 | Growth arrest-specific 2 like 3 | LOC2834 31 | H37811 | B.235709_at | 3 | 1 | 6.7278 | 51.899 | 0.010 |
| 149 | Hs.75678 | FBJ murine osteosarcoma viral oncogene homolog B | FOSB | NM_006732 | A.202768_at | 1 | 3 | 6.1922 | 51.899 | 0.059 |
| 150 | Hs.437351 | Cold inducible RNA binding protein | CIRBP | AL565767 | B.225191_at | 1 | 3 | 8.033 | 51.899 | 0.002 |
| 151 | Hs.57101 | MCM2 minichromosome maintenance deficient 2, mitotin (S. cerevisiae) | MCM2 | NM_004526 | A.202107_s_at | 3 | 1 | 7.861 | 51.655 | 0.273 |
| 152 | Hs.326736 | Ankyrin repeat domain 30A | NY-BR-1 | AF269087 | B.223864_at | 1 | 3 | 9.4144 | 51.336 | 0.042 |
| 153 | Hs.298646 | ATPase family, AAA domain containing 2 | PRO2000 | AI925583 | B.222740_at | 3 | 1 | 6.8416 | 50.763 | 0.130 |
| 154 | Hs.119192 | H2A histone family, member Z | H2AFZ | NM_002106 | A.200853_at | 3 | 1 | 8.5896 | 50.108 | 0.008 |
| 155 | Hs.119960 | PHD finger protein 19 | PHF19 | BE544837 | B.227211_at | 3 | | 6.3487 | 50.108 | 0.084 |
| 156 | Hs.78619 | Gamma-glutamyl hydrolase (conjugase, folylpolygammaglutamyl hydrolase) | GGH | NM_003878 | A.203560_at | 3 | 1 | 6.7708 | 49.945 | 0.006 |
| 157 | Hs.283532 | Uncharacterized bone marrow protein BM039 | BM039 | NM_018455 | A.219555_s_at | 3 | 1 | 4.1739 | 49.945 | 0.134 |
| 158 | Hs.221941 | Cytochrome b reductase 1 | | AI669804 | B.232459_at | 1 | 3 | 7.1171 | 49.945 | 0.015 |
| 159 | Hs.104019 | Transforming, acidic coiled-coil containing protein 3 | TACC3 | NM_006342 | A.218308_at | 3 | 1 | 6.1303 | 49.820 | 0.023 |
| 160 | acc_AK0022 03.1 | | | AK002203 | B.226992_at | 1 | 3 | 7.9091 | 49.696 | 0.037 |
| 161 | Hs.28625 | Transcribed locus | | AI693516 | B.228750_at | 1 | 3 | 7.1249 | 49.554 | 0.055 |
| 162 | Hs.206868 | B-cell CLL/lymphoma 2 | | AU146384 | B.232210_at | 1 | 3 | 8.0948 | 49.554 | 0.002 |
| 163 | Hs.75528 | Dynein, axonemal, light intermediate polypeptide 1 | HUMAU ANTIG | AW299538 | B.227081_at | 1 | 3 | 7.0851 | 49.549 | 0.003 |
| 164 | acc_AW271 106 | | | AW271106 | B.229490_s_at | 3 | 1 | 6.2222 | 49.544 | 0.017 |
| 165 | Hs.298646 | ATPase family, AAA domain containing 2 | PRO2000 | AI139629 | B.235266_at | 3 | 1 | 6.1913 | 49.544 | 0.009 |
| 166 | Hs.303090 | Protein phosphatase 1, regulatory (inhibitor) subunit 3C | PPP1R3C | N26005 | A.204284_at | 1 | 3 | 7.0275 | 49.520 | 0.011 |
| 167 | Hs.83169 | Matrix metalloproteinase 1 (interstitial collagenase) | MMP1 | NM_002421 | A.204475_at | 3 | 1 | 7.1705 | 49.410 | 0.028 |
| 168 | Hs.441708 | Leucine-rich repeat kinase 1 | MGC458 66 | AI638593 | B.230021_at | 3 | 1 | 6.424 | 49.410 | 0.005 |
| 169 | acc_AV7339 50 | | | AV733950 | A.201693_s_at | 1 | 3 | 7.9061 | 48.773 | 0.005 |
| 170 | Hs.171695 | Dual specificity phosphatase 1 | DUSP1 | NM_004417 | A.201041_s_at | 1 | | 9.7481 | 48.672 | 0.003 |
| 171 | Hs.87491 | Thymidylate synthetase | TYMS | NM_001071 | A.202589_at | 3 | 1 | 7.8242 | 48.672 | 0.041 |
| 172 | Hs.434886 | Cell division cycle associated 5 | CDCA5 | BE614410 | B.224753_at | 3 | 1 | 4.9821 | 48.488 | 0.106 |
| 173 | Hs.24395 | Chemokine (C-X-C motif) ligand 14 | CXCL14 | NM_004887 | A.218002_s_at | 1 | 3 | 8.2513 | 48.231 | 0.003 |
| 174 | Hs.104741 | T-LAK cell-originated protein kinase | TOPK | NM_018492 | A.219148_at | 3 | 1 | 6.4626 | 48.155 | 0.001 |
| 175 | Hs.272027 | F-box protein 5 | FBXO5 | AK026197 | B.234863_x_at | 3 | 1 | 6.935 | 48.155 | 0.037 |
| 176 | Hs.101174 | Microtubule-associated protein tau | MAPT | J03778 | A.206401_s_at | 1 | 3 | 6.4557 | 48.155 | 0.021 |
| 177 | Hs.7888 | V-erb-a erythroblastic leukemia viral oncogene homolog 4 (avian) | | AW772192 | A.214053_at | 1 | 3 | | 48.155 | 0.029 |
| 178 | Hs.372254 | Lymphoid nuclear protein related to AF4 | | AI033582 | B.244696_at | 1 | 3 | 7.4158 | 48.155 | 0.002 |
| 179 | Hs.435861 | Signal peptide, CUB domain, EGF-like 2 | SCUBE2 | AI424243 | A.219197_s_at | 1 | 3 | 8.3819 | 47.983 | 0.037 |
| 180 | Hs.385998 | WD repeat and HMG-box DNA binding protein 1 | WDHD1 | AK001538 | A.216228_s_at | 3 | 1 | 4.541 | 47.687 | 0.001 |
| 181 | Hs.306322 | Neuron navigator 3 | NAV3 | NM_014903 | A.204823_at | 1 | 3 | 5.8235 | 47.678 | 0.004 |
| 182 | Hs.21380 | CDNA FLJ36725 fis, clone UTERU2012230 | | AV709727 | B.225996_at | 1 | 3 | 7.5715 | 47.581 | 0.038 |
| 183 | Hs.89497 | Lamin B1 | LMNB1 | NM_005573 | A.203276_at | 3 | 1 | 7.11 | 47.281 | 0.004 |
| 184 | acc_NM_01 7669.1 | | | NM_017669 | A.219650_at | 3 | 1 | 5.0422 | 47.281 | 0.004 |
| 185 | Hs.12532 | Chromosome 1 open reading frame 21 | C1orf21 | NM_030806 | A.221272_s_at | 1 | 3 | 5.6228 | 47.104 | 0.066 |
| 186 | Hs.399966 | Calcium channel, voltage-dependent, L type, alpha 1D subunit | CACNA1 D | BE550599 | A.210108_at | 1 | 3 | 6.2612 | 46.990 | 0.063 |
| 187 | Hs. 159264 | Clone 23948 mRNA sequence | | U79293 | A.215304_at | 1 | 3 | 6.9317 | 46.990 | 0.066 |
| 188 | Hs.212787 | KIAA0303 protein | KIAA030 3 | AW971134 | A.222348_at | 1 | 3 | 4.964 | 46.984 | 0.002 |
| 189 | Hs.325650 | EH-domain containing 2 | EHD2 | AI417917 | A.221870_at | 1 | 3 | 6.4774 | 46.013 | 0.002 |
| 190 | Hs.388347 | Hypothetical protein LOC143381 | | AW242720 | B.227550_at | 1 | 3 | 7.657 | 45.314 | 0.001 |
| 191 | Hs.283853 | MRNA full length insert cDNA clone EUROIMAGE 980547 | | AL360204 | B.232855_at | 1 | 3 | 4.6288 | 45.314 | 0.006 |
| 192 | Hs.57301 | High mobility group AT-hook 1 | HMGA1 | NM_002131 | A.206074_s_at | 3 | 1 | 7.6723 | 44.940 | 0.001 |
| 193 | Hs.529285 | Solute carrier family 40 (iron-regulated transporter), member 1 | | AA588092 | B.239723_at | 1 | 3 | 6.9222 | 44.838 | 0.052 |
| 194 | Hs.252938 | Low density lipoprotein-related protein 2 | LRP2 | R73030 | B.230863_at | 1 | | 7.4648 | 44.706 | 0.003 |
| *195* | Hs.552 | Steroid-5-alpha-reductase, alpha polypeptide 1 (3-oxo-5 alpha-steroid delta 4-dehydrogenase alpha 1) | SRD5A1 | NM_001047 | A.204675_at | 3 | 1 | 7.1002 | 44.684 | 0.000 |
| 196 | Hs.156346 | Topoisomerase (DNA) II alpha 170kDa | TOP2A | NM_001067 | A.201291_s_at | 3 | 1 | 7.3566 | 44.552 | 0.110 |
| 197 | Hs.413924 | Chemokine (C-X-C motif) ligand 10 | CXCL10 | NM_001565 | A.204533_at | 3 | 1 | 7.9131 | 44.552 | 0.070 |
| 198 | Hs.287466 | CDNA FLJ11928 fis, clone HEMBB1000420 | | AK021990 | B.232699_at | 1 | 3 | 5.8675 | 44.552 | 0.002 |
| 199 | acc_X07868 | | | X07868 | A.202409_at | 1 | 3 | 7.9917 | 44.537 | 0.002 |
| 200 | Hs.101174 | Microtubule-associated protein tau | MAPT | NM_016835 | A.203928_x_at | 1 | 3 | 6.9103 | 44.537 | 0.005 |
| 201 | Hs.334828 | Hypothetical protein FLJ10719 | FLJ10719 | BG478677 | A.213008_at | 3 | 1 | 6.4461 | 44.494 | 0.009 |
| 202 | Hs.326035 | Early growth response 1 | EGR1 | NM_001964 | A.201694_s_at | 1 | 3 | 8.6202 | 44.199 | 0.025 |
| 203 | Hs.122552 | G-2 and S-phase expressed 1 | GTSE1 | BF973178 | A.215942_s_at | 3 | 1 | 5.4688 | 44.199 | 0.041 |
| 204 | Hs.24395 | Chemokine (C-X-C motif) ligand 14 | CXCL14 | AF144103 | B.222484_s_at | 1 | 3 | 9.3366 | 44.199 | 0.006 |
| 205 | Hs.102406 | Melanophilin | | AI810764 | B.229150_at | 1 | 3 | 8.078 | 44.199 | 0.031 |
| 206 | Hs.164018 | Leucine zipper protein FKSG14 | FKSG14 | BC005400 | B.222848_at | 3 | 1 | 6.6517 | 43.845 | 0.001 |
| 207 | Hs.19114 | High-mobility group box 3 | HMGB3 | NM_005342 | A.203744_at | 3 | 1 | 7.5502 | 43.661 | 0.007 |
| 208 | Hs.103982 | Chemokine (C-X-C motif) ligand 11 | CXCL11 | AF002985 | A.211122_s_at | 3 | 1 | 6.1001 | 43.014 | 0.003 |
| *209* | Hs.356349 | Transcribed locus | ZNF145 | AI492388 | B.228854_at | 1 | 3 | 6.8198 | 43.014 | 0.001 |
| 210 | Hs.1657 | Estrogen receptor 1 | ESR1 | NM_000125 | A.205225_at | 1 | 3 | 7.4943 | 42.966 | 0.188 |
| 211 | Hs.144479 | Transcribed locus | | BF433570 | B.237301_at | 1 | 3 | 6.3171 | 42.831 | 0.003 |
| 212 | acc_BF5080 74 | | | BF508074 | B.240465_at | 1 | 3 | 6.0041 | 42.720 | 0.002 |
| 213 | Hs.326391 | Phytanoyl-CoA dioxygenase domain containing 1 | PHYHD1 | AL545998 | B.226846_at | 1 | 3 | 7.2214 | 42.425 | 0.100 |
| 214 | Hs.338851 | FLJ41238 protein | FLJ41238 | AW629527 | B.229764_at | 1 | 3 | 6.5319 | 42.334 | 0.033 |
| 215 | Hs.65239 | Sodium channel, voltage-gated, type IV, beta | SCN4B | AW026241 | B.236359_at | 1 | 3 | 5.5526 | 42.084 | 0.106 |
| 216 | Hs.88417 | Sushi domain containing 3 | SUSD3 | AW966474 | B.227182_at | 1 | 3 | 8.195 | 41.808 | 0.015 |
| 217 | Hs.16530 | Chemokine (C-C motif) ligand 18 (pulmonary and activation-regulated) | CCL18 | Y13710 | A.32128_at | 3 | 1 | 6.2442 | 41.317 | 0.004 |
| 218 | Hs.384944 | Superoxide dismutase 2, mitochondrial | SOD2 | XI5132 | A.216841_s_at | 3 | 1 | 6.0027 | 41.317 | 0.115 |
| 219 | Hs.406050 | Dynein, axonemal, light intermediate polypeptide 1 | DNALI1 | NM_003462 | A.205186_at | 1 | 3 | 4.2997 | 40.911 | 0.009 |
| 220 | Hs.458430 | N-acetyltransferase 1 (arylamine N-acetyltransferase) | NAT1 | NM_000662 | A.214440_at | 1 | 3 | 7.7423 | 40.775 | 0.001 |
| 221 | Hs.437023 | Nucleoporin 62kDa | IL4I1 | AI859620 | B.230966_at | 3 | 1 | 6.4289 | 40.567 | 0.041 |
| 222 | Hs.279905 | Nucleolar and spindle associated protein 1 | NUSAP1 | NM_018454 | A.219978_s_at | 3 | 1 | 6.3357 | 40.119 | 0.011 |
| 223 | Hs.505337 | Claudin 5 (transmembrane protein deleted in velocardiofacial syndrome) | CLDN5 | NM_003277 | A.204482_at | 1 | 3 | 6.1516 | 40.053 | 0.001 |
| 224 | Hs.44227 | Heparanase | HPSE | NM_006665 | A.219403_s_at | 3 | 1 | 5.2989 | 40.005 | 0.253 |
| 225 | Hs.512555 | Collagen, type XIV, alpha 1 (undulin) | COL14A1 | BF449063 | A.212865_s_at | 1 | 3 | 7.2876 | 39.981 | 0.001 |
| 226 | Hs.511950 | Sirtuin (silent mating type information regulation 2 homolog) 3 (S. cerevisiae) | SIRT3 | AF083108 | A.221562_s_at | 1 | 3 | 5.9645 | 39.981 | 0.019 |
| 227 | Hs.371357 | RNA binding motif, single stranded interacting protein | | AW338699 | B.241789_at | 1 | 3 | 6.3656 | 39.981 | 0.009 |
| 228 | Hs.81131 | Guanidinoacetate N-methyltransferase | GAMT | NM_000156 | A.205354_at | 1 | 3 | 5.9474 | 39.852 | 0.005 |
| 229 | Hs.158992 | FLJ45983 protein | | AI631850 | B.240192_at | 1 | 3 | 5.2898 | 39.852 | 0.344 |
| 230 | Hs.104624 | Aquaporin 9 | AQP9 | NM_020980 | A.205568_at | 3 | 1 | 4.9519 | 39.848 | 0.010 |
| 231 | Hs.437867 | Homo sapiens, clone IMAGE:5759947, mRNA | | AW970881 | A.222314_x_at | 1 | 3 | 5.2505 | 39.816 | 0.042 |
| 232 | Hs.296049 | Microfibrillar-associated protein 4 | MFAP4 | R72286 | A.212713_at | 1 | 3 | 6.5149 | 39.749 | 0.001 |
| 233 | Hs.109439 | Osteoglycin (osteoinductive factor, mimecan) | OGN | NM_014057 | A.218730_s_at | 1 | 3 | 4.9325 | 39.749 | 0.015 |
| 234 | Hs.29190 | Hypothetical protein MGC24047 | MGC240 47 | AI732488 | B.229381_at | 1 | 3 | 7.2281 | 39.749 | 0.069 |
| 235 | Hs.252418 | Elastin (supravalvular aortic stenosis, Williams-Beuren syndrome) | ELN | AA479278 | A.212670_at | 1 | 3 | 6.8951 | 39.489 | 0.149 |
| 236 | Hs.252938 | Low density lipoprotein-related protein 2 | LRP2 | NM_004525 | A.205710_at | 1 | 3 | 5.9845 | 39.154 | 0.003 |
| 237 | Hs.32405 | MRNA; cDNA DKFZp586G0321 (from clone DKFZp586G0321) | | AL137566 | B.228554_at | 1 | 3 | 7.1124 | 38.597 | 0.015 |
| 238 | Hs.288720 | Leucine rich repeat containing 17 | LRRC17 | NM_005824 | A.205381_at | 1 | 3 | 7.217 | 38.493 | 0.279 |
| 239 | Hs.203963 | Helicase, lymphoid-specific | HELLS | NM_018063 | A.220085_at | 3 | 1 | 5.2886 | 38.493 | 0.001 |
| 240 | Hs.361171 | Placenta-specific 9 | PLAC9 | AW964972 | B.227419_x_at | 1 | 3 | 6.689 | 38.195 | 0.000 |
| 241 | Hs.396595 | Flavin containing monooxygenase 5 | FMO5 | AK022172 | A.215300_s_at | 1 | 3 | 4.1433 | 37.488 | 0.002 |
| 242 | Hs.105434 | Interferon stimulated gene 20kDa | ISG20 | NM_002201 | A.204698_at | 3 | 1 | 6.2999 | 37.448 | 0.003 |
| 243 | Hs.460184 | MCM4 minichromosome maintenance deficient 4 (S. cerevisiae) | MCM4 | X74794 | A.212141_at | 3 | 1 | 6.7292 | 36.577 | 0.176 |

| **Order** | **UGID(build #177)** | **UnigeneName** | **Gene Symbol** | **Genbank Acc** | **Affi ID** | **Grade with Higher Expres sion** | **Grade with Lower Expressi on** | **Cut-Off** | **Chi-2** | **Instabilit y indices** |
|---|---|---|---|---|---|---|---|---|---|---|
| 244 | Hs.169266 | Neuropeptide Y receptor Y1 | NPY1R | NM_000909 | A.205440_s_at | 1 | 3 | 5.8305 | 36.029 | 0.011 |
| 245 | acc_R38110 | | | R38110 | B.240112_at | 1 | 3 | 5.1631 | 35.441 | 0.021 |
| 246 | Hs.63931 | Dachshund homolog 1 (Drosophila) | DACH | AI650353 | B.228915_at | 1 | 3 | 7.6716 | 35.346 | 0.319 |
| 247 | Hs.102541 | Netrin 4 | NTN4 | AF278532 | B.223315_at | 1 | 3 | 8.2693 | 35.233 | 0.132 |
| 248 | Hs.418367 | Neuromedin U | NMU | NM_006681 | A.206023_at | 3 | 1 | 5.1017 | 34.589 | 0.035 |
| 249 | Hs.232127 | MRNA; cDNA DKFZp547P042 (from clone DKFZp547P042) | | AL512727 | A.215014_at | 1 | 3 | 4.8334 | 34.570 | 0.035 |
| 250 | Hs.212088 | Epoxide hydrolase 2, cytoplasmic | EPHX2 | AF233336 | A.209368_at | 1 | 3 | 6.4031 | 34.531 | 0.154 |
| 251 | Hs.439760 | Cytochrome P450, family 4, subfamily X, polypeptide 1 | CYP4X1 | AA557324 | B.227702_at | 1 | 3 | 8.5972 | 34.531 | 0.015 |
| 252 | acc_BF5134 68 | | | BF513468 | B.241505_at | 1 | 3 | 7.1517 | 34.140 | 0.001 |
| 253 | Hs.413078 | Nudix (nucleoside diphosphate linked moiety X)-type motif 1 | NUDT1 | NM_002452 | A.204766_s_at | 3 | 1 | 5.6705 | 33.955 | 0.069 |
| 254 | acc_AI49237 6 | | | AI492376 | B.231195_at | 3 | 1 | 5.1967 | 33.602 | 0.029 |
| 255 | acc_AW512 787 | | | AW512787 | B.238481_at | 1 | 3 | 8.5117 | 33.572 | 0.005 |
| 256 | Hs.74369 | Integrin, alpha 7 | ITGA7 | AK022548 | A.216331_at | 1 | 3 | 5.1535 | 33.290 | 0.003 |
| 257 | Hs.63931 | Dachshund homolog 1 (Drosophila) | DACH | NM_004392 | A.205472_s_at | 1 | 3 | 3.9246 | 33.177 | 0.002 |
| 258 | Hs.225952 | Protein tyrosine phosphatase, receptor type, T | PTPRT | NM_007050 | A.205948_at | 1 | 3 | 6.7634 | 32.152 | 0.190 |
| 259 | acc_BF7937 01 | Musculoskeletal, embryonic nuclear protein 1 | | BF793701 | B.226856_at | 1 | 3 | 5.5626 | 31.816 | 0.002 |
| 260 | Hs.283417 | Transcribed locus | | AI826437 | B.229975_at | 1 | 3 | 6.381 | 31.307 | 0.009 |
| 261 | Hs.21948 | Zinc finger protein 533 | | H15261 | B.243929_at | 1 | 3 | 4.7165 | 30.259 | 0.144 |
| 262 | Hs.31297 | Cytochrome b reductase 1 | CYBRD1 | NM_024843 | A.217889_s_at | 1 | 3 | 5.6427 | 27.628 | 0.056 |
| 263 | Hs.180142 | Calmodulin-like 5 | CALML5 | NM_017422 | A.220414_at | 3 | 1 | 5.994 | 27.417 | 0.009 |
| 264 | Hs.176588 | Cytochrome P450, family 4, subfamily Z, polypeptide 1 | CYP4Z1 | AV700083 | B.237395_at | 1 | 3 | 8.7505 | 24.383 | 0.400 |

For any particular gene, where the value of the cell in column 7 "Grade with Higher Expression" is 3, the value of the cell in column 8 "Grade with Lower Expression" is 1, and where the value of cell "Grade with Higher Expression" is 1, the value of column "Grade with Lower Expression" is 3. Colum 9 shows the cut off (Optimal Variance Cut-off) expressed as the natural log transform normalised signal intensity measurement for Affymetrix arrays (global mean normalisation with a scaling factor of 500).

### SWS CLASSIFIER 1 (TABLE D2)

**Table D2. SWS Classifier 1: 6 Probe Sets (5 Genes)**

| **No** | **UGID (build #183)** | **Unigene Name** | **Gene Symbol** | **Genbank Acc** | **Affi ID** | **Grade with Higher Expression** | **Grade with Lower Expression** | **Cut-off** |
|---|---|---|---|---|---|---|---|---|
| 1 | Hs.528654 | Hypothetical protein FLJ11029 | FLJ11029 | BG165011 | B.228273_at | 3 | 1 | 7.706303 |
| 2 | acc_NM_0031 58.1 | Serine/threonine kinase 6. transcript 1 | STK6 | NM_003158 | A.208079_s_at | 3 | 1 | 6.652593 |
| 3 | Hs.35962 | CDNA clone IMAGE:4452583, partial cds | | BG492359 | B.226936_at | 3 | 1 | 7.561905 |
| 4 | Hs.308045 | Barren homolog (Drosophila) | BRRN1 | D38553 | A.212949_at | 3 | 1 | 5.916703 |
| 5 | Hs.184339 | Maternal embryonic leucine zipper kinase | MELK | NM_014791 | A.204823_at | 3 | 1 | 7.107259 |
| 6 | Hs.250822 | Serine/threonine kinase 6, transcript 2 | STK6 | NM_003600 | A.204092_s_at | 3 | 1 | 6.726571 |

For any particular gene, where the value of the cell in column 7 "Grade with Higher Expression" is 3, the value of the cell in column 8 "Grade with Lower Expression" is 1, and where the value of cell "Grade with Higher Expression" is 1, the value of column "Grade with Lower Expression" is 3. Colum 9 shows the cut off (Optimal Variance Cut-off) expressed as the natural log transform normalised signal intensity measurement for Affymetrix arrays (global mean normalisation with a scaling factor of 500).

### SWS CLASSIFIER 2 (TABLE D3)

**Table D3. SWS Classifier 2: 18 Probe Sets (17 Genes)**

| **No** | **UGID (build #183)** | **UnigeneName** | **Gene Symbol** | **GenbankAcc** | **Affi ID** | **Grade with Higher Expression** | **Grade with Lower Expression** | **Cut-off** |
|---|---|---|---|---|---|---|---|---|
| 1 | Hs.184339 | Maternal embryonic leucine zipper kinase | MELK | NM_014791 | A.204825_at | 3 | 1 | 5.437105 |
| 2 | Hs.308045 | Barren homolog *(Drosophila)* | BRRN1 | D38553 | A.212949_at | 3 | 1 | 5.504552 |
| 3 | Hs.9329 | TPX2, microtubule-associated protein homolog (*Xenopus laevis*) | TPX2 | AF098158 | A.210052_s_at | 3 | 1 | 5.872187 |
| 4 | Hs.486401 | CDNA clone IMAGE:4452583, partial cds | | BG492359 | B.226936_at | 3 | 1 | 7.569926 |
| 5 | Hs.75573 | Centromere protein E, 312kDa | CENPE | NM_001813 | A.205046_at | 3 | 1 | 6.943423 |
| 6 | Hs.528654 | Hypothetical protein FLJ11029 | FLJ11029 | BG165011 | B.228273_at | 3 | 1 | 7.711138 |
| 7 | acc_NM_0031 58 | | | NM_003158 | A.208079_s_at | 3 | 1 | 6.571034 |
| 8 | Hs.524571 | Cell division cycle associated 8 | CDCA8 | BC001651 | A.221520_s_at | 3 | 1 | 6.894196 |
| 9 | Hs.239 | Forkhead box M1 | FOXM1 | NM_021953 | A.202580_x_at | 3 | 1 | 5.211513 |
| 10 | Hs.179718 | V-myb myeloblastosis viral oncogene homolog (avian)-like 2 | MYBL2 | NM_002466 | A.201710_at | 3 | 1 | 6.269081 |
| 11 | Hs.169840 | TTK protein kinase | TTK | NM_003318 | A.204822_at | 3 | 1 | 8.230804 |
| 12 | Hs.75678 | FBJ murine osteosarcoma viral oncogene homolog B | FOSB | NM_006732 | A.202768_at | 1 | 3 | 8.761579 |
| 13 | Hs.25647 | V-fos FBJ murine osteosarcoma viral oncogene homolog | FOS | BC004490 | A.209189_at | 1 | 3 | 7.085984 |
| 14 | Hs.524216 | Cell division cycle associated 3 | CDCA3 | NM_031299 | A.221436 s at | 3 | 1 | 6.29283 |
| 15 | Hs.381225 | Kinetochore protein Spc24 | Spc24 | AI469788 | B.235572_at | 3 | 1 | 6.340503 |
| 16 | Hs.62180 | Anillin, actin binding protein (scraps homolog, *Drosophilia*) | ANLN | AK023208 | B.222608_s_at | 3 | 1 | 6.84578 |
| 17 | Hs.434886 | Cell division cycle associated 5 | CDCA5 | BE614410 | B.224753_at | 3 | 1 | 5.290668 |
| 18 | Hs.523468 | Signal peptide, CUB domain, EGF-like 2 | SCUBE2 | AI424243 | A.219197_s_at | 3 | 1 | 5.792164 |

For any particular gene, where the value of the cell in column 7 "Grade with Higher Expression" is 3, the value of the cell in column 8 "Grade with Lower Expression" is 1, and where the value of cell "Grade with Higher Expression" is 1, the value of column "Grade with Lower Expression" is 3. Colum 9 shows the cut off (Optimal Variance Cut-off) expressed as the natural log transform normalised signal intensity measurement for Affymetrix arrays (global mean normalisation with a scaling factor of 500).

### SWS CLASSIFIER 3 (TABLE D4)

**Table D4. SWS Classifier 3: 7 Probe Sets (7 Genes)**

| **Or der** | **UGID(build #183)** | **Unigene Name** | **Gene Symbol** | **Genbank Acc** | **Affi ID** | **Grade with Higher Expression** | **Grade with Lower Expression** | **Cut-off** |
|---|---|---|---|---|---|---|---|---|
| 1 | Hs.9329 | TPX2, microtubule-associated protein homolog (Xenopus laevis) | TPX2 | AF098158 | A.210052_s_at | 3 | 1 | 8.7748 |
| 2 | Hs.344037 | Protein regulator of cytokinesis 1 | PRC1 | NM_003981 | A.218009_s_at | 3 | 1 | 8.2222 |
| 3 | Hs.292511 | Neuro-oncological ventral antigen 1 | NOVA1 | NM_002515 | A.205794_s_at | 1 | 3 | 6.7387 |
| 4 | Hs.155223 | Stanniocalcin 2 | STC2 | AI435828 | A.203438_at | 1 | 3 | 8.0766 |
| 5 | Hs.437351 | Cold inducible RNA binding protein | CIRBP | AL565767 | B.225191_at | 1 | 3 | 8.2308 |
| 6 | Hs.24395 | Chemokine (C-X-C motif) ligand 14 | CXCL14 | NM_004887 | A.218002_s_at | 1 | 3 | 7.086 |
| 7 | Hs.435861 | Signal peptide, CUB domain, EGF-like 2 | SCUBE2 | AI424243 | A.219197_s_at | 1 | 3 | 7.2545 |

For any particular gene, where the value of the cell in column 7 "Grade with Higher Expression" is 3, the value of the cell in column 8 "Grade with Lower Expression" is 1, and where the value of cell "Grade with Higher Expression" is 1, the value of column "Grade with Lower Expression" is 3. Colum 9 shows the cut off (Optimal Variance Cut-off) expressed as the natural log transform normalised signal intensity measurement for Affymetrix arrays (global mean normalisation with a scaling factor of 500).

### SWS CLASSIFIER 4 (TABLE D5)

**Table D5. SWS Classifier 4: 7 Probe Sets (7 Genes)**

| **Or der** | **UGID(build #183)** | **Unigene Name** | **Gene Symbol** | **GenbankAcc** | **Affi ID** | **Grade with Higher Expression** | **Grade with Lower Expression** | **Cut-off** |
|---|---|---|---|---|---|---|---|---|
| 1 | Hs.48855 | cell division cycle associated 8 | CDCA8 | BC001651 | A.221520_s_at | 3 | 1 | 5.5046 |
| 2 | Hs.75573 | centromere protein E, 312kDa | CENPE | NM_001813 | A.205046_at | 3 | 1 | 5.2115 |
| 3 | Hs.552 | steroid-5-alpha-reductase, alpha polypeptide 1 (3-oxo-5 alpha-steroid delta 4-dehydrogenase alpha 1) | SRD5A1 | BC006373 | A.211056_s_at | 3 | 1 | 6.9192 |
| 4 | Hs.101174 | microtubule-associated protein tau | MAPT | NM_016835 | A.203929_s_at | 1 | 3 | 4.8246 |
| 5 | Hs.164018 | leucine zipper protein FKSG14 | FKSG14 | BC005400 | B.222848_at | 3 | 1 | 6.1846 |
| 6 | acc_R38110 | N.A. | | R38110 | B.240112_at | 1 | 3 | 6.2557 |
| 7 | Hs.325650 | EH-domain containing 2 | EHD2 | AI417917 | A.221870_at | 1 | 3 | 7.6677 |

For any particular gene, where the value of the cell in column 7 "Grade with Higher Expression" is 3, the value of the cell in column 8 "Grade with Lower Expression" is 1, and where the value of cell "Grade with Higher Expression" is 1, the value of column "Grade with Lower Expression" is 3. Colum 9 shows the cut off (Optimal Variance Cut-off) expressed as the natural log transform normalised signal intensity measurement for Affymetrix arrays (global mean normalisation with a scaling factor of 500).

### EXAMPLES

### Example 1. Materials and Methods: Patients and Tumour Specimens

Clinical characteristics of patient and tumour samples of the Uppsala, Stockholm and Singapore cohorts are summarized in Table E1.

**Table E1. Distribution of patients and tumour characteristics**

| **Name of cohorts** | **Uppsala** | | | **Stockholm** | | | **Singapore** | | |
|---|---|---|---|---|---|---|---|---|---|
| | **n=254** | | | **n=147** | | | **n=98** | | |
| Patients, by grade | **G1** | **G2** | **G3** | **G1** | **G2** | **G3** | **G1** | **G2** | **G3** |
| | n=68 | n=126 | n=55 | n=28 | n=58 | n=61 | n=11 | n=40 | n=47 |
| Age, median yrs | 62 | 63 | 62 | 55 | 58 | 52 | 59 | 52 | 50 |
| <55 years, % | 26 | 25 | 44 | 50 | 41 | 56 | 37 | 60 | 68 |
| Tumour size, cm | 1.8 | 2.2 | 2.9 | 1.9 | 2.5 | 2.0 | 3.4 | 2.8 | 3.1 |
| Nodes, positive, % | 15 | 35 | 55 | 33 | 50 | 32 | 36 | 40 | 51 |
| ER negative tumours, % | 3 | 9 | 38 | 0 | 7 | 33 | 0 | 28 | 53 |
| Follow up, median yrs | 11 | 9 | 6 | 8 | 7 | 7 | - | - | - |
| All recurrences, % | 26 | 39 | 50 | 7 | 24 | 36 | - | - | - |
| Endocrine therapy, % | 18 | 37 | 36 | 75 | 62 | 49 | - | - | - |
| Chemotherapy, % | 4 | 6 | 22 | 4 | 5 | 13 | - | - | - |
| Combine therapy, % | 2 | 3 | 0 | 11 | | 10 | - | - | - |
| No systemic therapy, % | 77 | 54 | 45 | 11 | 17 | 28 | | | |

All cohorts are of unselected populations, and in each case, the original tumour material was collected at the time of surgery and freshly frozen on dry ice or in liquid nitrogen and stored under liquid nitrogen or at -70°C.

### Example 2. Methods: Details of Uppsala, Singapore and Stockholm Cohorts

### Uppsala Cohort

The Uppsala cohort originally comprised of 315 women representing 65% of all breast cancers resected in Uppsala County, Sweden from January 1, 1987 to December 31, 1989. Information pertaining to patient therapies, clinical follow up, and sample processing are described elsewhere (41).

### Histological Grading

For histological grading, new tumour sections are prepared from the original paraffin blocks, stained with eosin, and graded in a blinded fashion by H.N. according to the Nottingham grading system (6, Haybittle et al., 1982) as follows:
*Tubule Formation:* 3=poor, if < 10% of the tumour showed definite tubule formation, 2=moderate, if ≥10% but ≤75%, and 1=well, if >75%.
*Mitotic Index:* 1=low, if <10 mitoses, 2=medium, if 10-18 mitoses, and 3=high, if >18 mitoses (per 10 high-power fields). The field diameter was 0.57 mm.
*Nuclear Grade:* 1=low, if there was little variation in the size and shape of the nuclei, 2=medium for moderate variation, and 3=high for marked variation and large size.

Scores are then summed, and tumour samples with scores ranging from 3-5 are classified as Grade I; 6-7 as Grade II; and 8-9 as Grade III.

### Protein Assays

Protein levels of Estrogen Receptor (ER) and Progesterone Receptor (PgR) are assessed by immunoassay (monoclonal 6F11 anti-ER and monoclonal NCL-PGR, respectively, Novocastra Laboratories Ltd, Newcastle upon Tyne, UK) and deemed positive if >0.1 fmol/ug DNA. VEGF was measured in tumour cytosol by a quantitative immunoassay kit (Quantikine-human VEGF; R&D Systems, Minneapolis, MN, USA) as described (42). Protein levels of Ki-67 are analyzed using anti-Ki67 antibody (MIB-1) by the grid-graticula method with cut-offs: low=2, medium >2 and <6, high=6. Cyclin E was measured using the antibody HE12 (Santa Cruz Inc., USA) with cutoffs: low=0-4%, medium=5-49%, and high=50-100% stained tumour cells (43).

S-phase fraction was determined by flow cytometry and defined as high if >7% in diploid tumours, or >12% in aneuploid tumours. TP53 mutational status was determined by cDNA sequencing as previously described (41). The Uppsala tumour samples ar approved for microarray profiling by the ethical committee at the Karolinska Institute, Stockholm, Sweden.

### Stockholm Cohort

The Stockholm samples are derived from breast cancer patients that were operated on at the Karolinska Hospital from January 1, 1994 through December 31, 1996 and identified in the Stockholm-Gotland breast cancer registry.

Information on patient age, tumour size, number of metastatic axillary lymph nodes, hormonal receptor status, distant metastases, site and date of relapse, initial therapy, and date and cause of death are obtained from patient records filed with the Stockholm-Gotland registry.

Tumour sections are classified using the Nottingham grading system (Haybittle et al., 1982). The Stockholm tumour samples are approved for microarray profiling by the ethical committee at the Karolinska Hospital, Stockholm, Sweden.

### Singapore Cohort

The Singapore samples are derived from patients that were operated on at the National University Hospital (Singapore) from February 1, 2000 through January 31, 2002.

Information on patient age, tumour size, number of metastatic lymph nodes and hormonal receptor status are obtained from hospital records.

Tumour sections are graded in a blinded fashion according to the Nottingham grading system (Haybittle et al., 1982) as applied to the Uppsala and Stockholm cohorts, with the following exception: *Mitotic Index:* 1=low, if <8 mitoses, 2=medium, if 9-16 mitoses, and 3=high, if >16 mitoses (per 10 high-power fields). The field diameter is 0.55 mm. The Singapore tumour samples are approved for microarray profiling by the Singapore National University Hospital ethics board.

After exclusions based on tissue availability, RNA integrity, clinical annotation and microarray quality control, expression profiles of 249, 147, and 98 tumours from the Uppsala, Stockholm and Singapore cohorts, respectively, were deemed suitable for further analysis.

### Example 3. Materials and Methods: Microarray Expression Profiling and Processing

All tumour samples are profiled on the Affymetrix U133A and B genechips. Microarray analysis of the Uppsala and Singapore samples was carried out at the Genome Institute of Singapore (44). The Stockholm samples are analyzed by microarray at Bristol-Myers Squibb, Princeton, New Jersey, USA. RNA processing and microarray hybridizations are carried out essentially as described (44).

Microarray data processing: all microarray data are processed as previously described (44).

### Example 4. Materials and Methods: Statistical Analysis of Gene Ontology (GO) Terms

GO analysis is facilitated by PANTHER software (https://panther.appliedbiosystems.com/) (46). Selected gene lists are statistically compared (Mann-Whitney) with a reference list (ie, NCBI Build 35) comprised of all genes represented on the microarray to identify significantly over- and under-represented GO terms.

### Example 5. Materials and Methods: Survival Analysis

The Kaplan Meier estimate is used to compute survival curves, and the p-value of the likelihood-ratio test is used to assess the statistical significance of the resultant hazard ratios. For standardization, events occurring beyond 10 years are censored. All cases of contralateral disease are censored. Disease-free survival (DFS) is defined as the time interval from surgery until the first recurrence or last follow-up.

Multivariate analysis by Cox proportional hazard regression, including a stepwise model selection procedure based on the Akaike information criterion, and all survival statistics are performed in the R survival package. Remaining predictors in the Cox models are assessed by Likelihood-ratio test p-values.

### Example 6. Methods: Scoring by the Nottingham Prognostic Index (NPI)

NPI scores (Haybittle et al., 1982) are calculated according to the following formula: $NPI score = \left(0.2\times tumour size \left(cm\right)\right) + grade \left(1,2 or 3\right) + LN stage \left(1,2 or 3\right)$

Tumour size is defined as the longest diameter of the resected tumour. LN stage is 1, if lymph node negative, 2, if 3 or fewer nodes involved, and 3, if >3 nodes involved (47). As the number of cancerous lymph nodes are not available for the Uppsala cohort, a LN stage score of 2 is assigned if 1 or more nodes are involved, and a score of 3 is assigned if nodal involvement showed evidence of periglandular growth. For ggNPI calculations, grade scores (1,2 or 3) are replaced by genetic grade predictions (1 or 3).

NPI scores <3.4 = GPG (good prognostic group); scores of 3.4 to 5.4 = MPG (moderate prognostic group); scores >5.4 = PPG (poor prognostic group). Scores of 2.4 or less = EPG (excellent prognostic group).

### Example 7. Methods: Descriptive Statistics

For inter-group comparisons using the clinicopathological measurements, non-parametric Mann-Whitney U-test statistics are used for continuous variables and one-sided Fisher's exact test used for categorical variables. This work is facilitated by the Statistica-6 and StatXact-6 software packages.

### Example 8. Materials and Methods: Details of Genetic Reclassification Algorithm of Grade 2 Tumours Based on SWS Approach

In simplified terms, the algorithm of genetic re-classification of Grade 2 tumour, based on SWS approach can be described as follows.

A *training set* consisting of samples of known classes (eg, histologic Grade I (G1) and histologic Grade III (G3) tumours) is used to select the *variables* (ie, gene expression measurements; *probesets* or *predictors*)*,* that'allow the most accurate discrimination (or prediction) of the samples in the training set. Once the SWS algorithm is *trained* on the optimal set of variables, it is then applied to an independent *exam set* (ie, a new set of samples not used in training) to validate it's prediction accuracy. More details are given below.

Briefly, for constructing the class prediction function, the SWS method uses the training set *S̃*₀ (comprised of Gland G3 tumour samples) to evaluate statistically the weight of the graduated "informative" variables (predictors), and all possible pairs of these predictors. The predictors are automatically selected by SWS from n (*n*=44,500) probe sets (which represents the gene expression measurements) on U133A and U133B Affymetrix Genechips.

The description of each patient includes *n* (potential) prognostic variables *X*₁,...,*Xₙ* (signals from probe sets of the U133A and U133B chips) and information about class to which a patient belongs. In particular, the predictors might be able to discriminate G1 and G3 tumours with minimum "a posteriori probability". Reliability of the SWS class prediction function is based on the standard "leave-one-out procedure" and on an additional *exam* of the class prediction ability on one or more independent sample populations (ie, patient cohorts). In this application of SWS, the G2 tumour samples of the Uppsala cohort and two other cohorts (NUH and Stockholm cohorts; see Methods) have been used as exam datasets to test the SWS class prediction function.

Let us consider the available *n*-dimension domain of the variables (the probesets) *X*₁*,...,Xₙ* as prognostic variable space. The SWS algorithm is based on calculating the a posteriori probabilities of the tumours belonging to one of two classes using a weighted voting scheme involving the sets of so called "syndromes". A syndrome is the sub-region of prognostic variable space. For a syndrome to be useful in the algorithm, within the syndrome, one class of samples (for instance, G3 tumours) must be significantly highly represented than another class (for instance, G1s), and in other sub-region(s) the inverse relationship should be observed. In the present version of the SWS method, one-dimensional and two-dimensional sub-regions (syndromes) are used.

Let *b'ᵢ* and *b*"*ᵢ* denote the boundaries of the sub-region for the variable *Xᵢ* (the *i-th* probe set); ${b}_{i}^{′} \geq {X}_{i} > {b}_{i}^{"} .$ One-dimensional syndrome for the variable *Xᵢ* is defined as the set of points in variable space for which inequalities ${b}_{i}^{′} \geq {X}_{i} > {b}_{i}^{"} .$ are satisfied. Two-dimensional syndrome for variables *X_{i'}* and *X_{i"}* is defined as a set of points in variable space for which inequalities ${b}_{i}^{′} ′ \geq {X}_{i ′} > {b}_{i ′}^{"} .$ and ${b}_{i "}^{′} \geq {X}_{i "} > {b}_{i "}^{"} .$ are satisfied. The syndromes are constructed at the initial stage of training using the optimal partitioning (OP) algorithm described below.

### SWS Training Algorithm

SWS training algorithm is based on three major steps:
1) optimal recoding (partitioning) of the given covariates (signal intensity values) to obtain discrete-valued variables with low and high gradation;
2) selection of the most informative and robust of these discrete-valued variables and their paired combinations (termed syndromes) that together best characterize the classes of interest;
3) tallying the statistically weighted votes of these syndromes to allow us to compute the value of the outcome prediction function.

### Optimal Partitioning (OP)

The OP method is used for constructing the optimal syndromes for each class (G1 and G3) using the training set *S̃*₀. The OP is based on the optimal partitioning of some potential prognostic variable *Xᵢ* range that allows the best separation of the samples belonging to different classes. To evaluate the separating ability of partition R (see below) in the training set *S̃*₀ the chi-2 functional is used (Kuznetsov et al, 1998). The optimal partitions are searched inside observed variable domain that contain partitions with cut-off values not greater than a fixed threshold (defined below). The partition with the maximal value of the chi-2 functional is considered optimal for the given variable.

### Stability of partitioning

Another important characteristic that allows evaluation the prognostic ability of partitioning model for specific variables is the index of *boundary instability.* Let *Rₒ, R*₁*,..., Rₘ* be optimal partitions of variable *Xᵢ* ranges that is calculated by training set *S̃*₀,*S̃*₁*,... ,S̃ₘ,* where *S̃ₖ* is the training set without description of the *k^{th}* sample. Let *Kⱼ* denote the different classes (*j*=*1,2*). Let ${b}_{1}^{k} , … , {b}_{r - 1}^{k}$ be boundary points of optimal partition *Rₖ* found by training set *S̃ₖ ; Dᵢ* is the variance of variable *Xᵢ*. The boundary instability index *κ*(*S̃*₀*,Kⱼ,r*) for partitioning with *r* elements is calculated as the ratio (Kuznetsov et al, 1996): $κ \left({{S}^{˜}}_{0}, {K}_{j}, r\right) = \frac{1}{{D}_{i} \left(r-1\right)} \left[{\sum }_{k = 1}^{m} {\sum }_{l = 1}^{r - 1} {\left({b}_{l}^{k}-{b}_{l}^{0}\right)}^{2}\right] .$

### Selecting of Optimal Variables Set

The OP can be used at the initial stage of training for reducing the dimension of the prognostic variables set. Selection of the optimal set of prognostic variables depends on a sufficiently high partition value determined by the Chi-2 function. The additional criterion of selection of prognostic variables is the instability index *κ*(*S̃*₀*,Kⱼ, r*)*.* The variable is used if value *κ*(*S̃₀ Kⱼ, r*) is less than threshold *κ*₀, defined *a priori* by the user. When the partition of the given variable is instable (*κ*(*S̃*₀*, Kⱼ, r)* < *κ*₀)*,* the variable is removed from the final optimal set of prognostic variables. Finally, the optimal set of prognostic variables is defined if both selection criteria are fulfilled.

### The Weighted Voting Procedure

Let ${{Q}^{˜}}_{j}^{0}$ denote the set of constructed syndromes for class *Kⱼ.* Let **x*** denote the point of parametric space. The SWS estimates a posteriori probability ${P}_{j}^{sv} \left({x}^{*}\right)$ of the class *Kⱼ* at the point x***** that belongs to the intersection of syndromes *q*₁*,..., qᵣ* from ${{Q}^{˜}}_{j}^{0}$ as follows: ${P}_{j}^{sv} \left({x}^{*}\right) = \frac{{\sum }_{i = 1}^{r} {w}_{i}^{j} {v}_{i}^{j}}{{\sum }_{i = 1}^{r} {w}_{i}^{j}} ,$ where ${v}_{i}^{j}$ is the fraction of class *Kⱼ* among objects with prognostic variables vectors belonging to syndrome *qᵢ*, *wᵢ* is the so-called "weight" of syndrome *qᵢ*. The weight *wᵢ* is calculated by the formula, ${w}_{i} = \frac{{m}_{i}}{{m}_{i} + 1} \frac{1}{{d̑}_{i}} ,$ where ${d̑}_{i} = \left(1-{v}_{i}^{i}\right) {v}_{i}^{i} + \frac{1}{{m}_{i}} \left(1-{v}_{0}^{j}\right) {v}_{0}^{j}$ (Kuznetsov, 1996). The estimate of fraction ${v}_{i}^{j}$ variance has the second term $\frac{1}{{m}_{j}} \left(1-{v}_{0}^{j}\right) {v}_{0}^{j} ,$ which is used to avoid a value 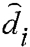 equal to zero in cases when the given syndrome is associated only with objects of one class from the training set.

The results of testing applied and simulated tasks have demonstrated that formula (1) gives too low of estimates of conditional probabilities for classes that are of smaller fraction in the training set. So the additional correction of estimates in (1) has been implemented. The final estimates of conditional probability at point **x*** are calculated as ${P}_{j}^{sws} \left({x}^{*}\right) = {P}_{j}^{sv} \left({x}^{*}\right) χ \left({{S}^{˜}}_{0}, {K}_{j}\right) ,$ where $χ \left({{S}^{˜}}_{0}, {K}_{j}\right) = \frac{1}{{\sum }_{k = 1}^{m} {P}_{j}^{sv} \left({x}_{i}\right)}$ and **x***ₖ* is the vector of prognostic variables for the *k*-th samples from the training set.

### Example 9. Derivation of a Classifier Comprising 264 Probe Sets (SWS Classifier 0)

### Schema of the SWS-Based Discovery Method of Novel Classes of Tumours

Our methodology is based on the schema presented in Figure 1.

Beginning with the Uppsala dataset comprised of 68 G1 and 55 G3 tumours, we used SWS optimal partitioning (OP) at the initial stage of training to reduce the dimension of the prognostic variables set. SWS rank orders the set of probes according to specific algorithmic criteria for assessing differential expression between classes.

Based on this two-criteria (chi-2 and instability index) selection algorithm, we used SWS chi-2 values bigger than 24.38 (at p-value less then 0.00001); in combination with low boundary instability index criteria (*κ*₀ <0.1 for 90% of the selected informative variables and *κ*₀ <0.4 for 10% of the other informative variables). Visual presentation on scatchard plot (log *κ*₀, chi-2) distribution of probesets, these two cut-off values discriminated the relatively small and compact group of probesets. We observed that this group of probesets provide a local minima on the Class Error Rate (CER) function and provide an optimal selection of 264 probesets classifier of G1 and G3. Using these 264 probe sets, the both SWS and PAM methods provide a small misclassification error (4.5% for G1, and 5.5% for G3, respectively) when the leave-one-out cross-validation procedure is used. We also used the U-test with critical value p=0.05 (with Bonferroni correction) and all 264 probesets follow this cut-off value.

Based on our selection criteria, we selected a classifier comprising 264 probe sets, which we term the "SWS Classifier 0". See Table D1 in section "SWS Classifier 0" of the Description as well as Appendix 1.

Details are shown in Appendix 1A, Appendix 2, Appendix 3 and Appendix 4.

### Example 10. A Posteriori Probability for SWS Classifier 0 (264 Probe Sets) G1 and G3 Estimated by SWS Classifier 2

A posterior probability for G1 and G3 was also estimated by SWS Classifier 2 for each tumour sample by the classical leave-one-out cross-validation procedure.

We estimated the class error rate based on the misclassification error rate plot (Tibshirani et al, 2002) and found that for the 264 selected probe sets, CER consists of 5% for G1, and 6% for G3, respectively. Similar discrimination was obtained with SWS methods (see above).

Based on consistency between SWS and U-tests and PAM CER validation of the selection procedure, we further considered the classification results using the 264 variables. In two-group comparisons, high CER were observed in the G1-G2 and G2-G3 predictions (data not shown), while G1-G3 classification accuracy was high (<5% errors). Complementary to SWS classification method, the PAM method confirms that G2 tumours are not molecularly distinct from either low or high grade tumours, possibly owing to substantial molecular heterogeneity within the G2 class.

### Example 11. Derivation of Classifiers of 6 Genes (SWS Classifier 1)

To extract the smallest possible classifier from the 264 variables, we varied the initial parameters of the SWS algorithm to minimize the number of predictors in training set providing the maximum correlation coefficient between posteriori probabilities and true class indicators (specifically, 1 was the indicator of G1 tumours, and 3 was the indicator of G3 tumours in the G1-G3 comparison). The predictive power of the predictor set was estimated using standard leave-one-out procedure and counting the numbers of errors of class predictions.

We derived a classifier comprising 6 gene probe sets (5 genes) which we term the "SWS Classifier 1". 4.4% for class G1; and 5.5% for class G3 CERs were obtained with the SWS Classifier 1. See Figure 1 and Table D2 in section "SWS Classifier 1" of the Description.

Appendix 5A, Appendix 5B and Appendix 5C show detailed information about selected gene probe sets, optimal partition boundaries, true classes, posterior probabilities and clinical significance of the SWS Classifier 1 predictor (estimated by patient survival analysis).

### Example 12. Derivation of Classifiers of 18 Genes (SWS Classifier 2)

By SWS, for the G1-G3 comparisons, maximal prediction accuracies are obtained with 18 probe sets (17 genes). We refer to this 18 probe set as the "SWS Classifier 2". See Table D3 in section "SWS Classifier 2" of the Description. This classifier includes all five genes represented by SWS Classifier 1.

Appendix 6A, Appendix 6B and Appendix 6C show detailed information about selected gene probe sets, optimal partition boundaries, true classes, posterior probabilities and clinical significance of the SWS Classifier 2 (estimated by patient survival analysis).

With the 18 probe sets, both the SWS Classifier 2 and PAM correctly classify ∼96% (65/68) of the G1s and ∼95% (52/55) of the G3s (by leave one-out method).

The smaller number of probes sets required by SWS Classifier 1 (6 probe sets) compared to PAM (18 probe sets, data not presented) may reflect the ability of SWS to use more diverse interaction and/or co-expression patterns during variable selection.

The posterior probability (Pr) is an estimate of the likelihood that a sample from the exam group of tumours belongs to one class (termed "G1-like") or the other (ie, "G3-like"). Both 18 probesets SWS and PAM classifiers scored the vast majority of G1 and G3 tumours with high probabilities of class membership.

### Example 13. The SWS Classifier 0 (264 Gene Probe Set) Contains Many Small Subsets Which Can Provide Equally High Discrimination Ability of the Genetic G2a and G2b Tumours

Due to the highly informative and stable nature of each gene (represented by Affymetrix probe-sets) of the 264 predictor set we hypothesized that there are many small alternative gene sub-sets that could be used to classify tumours with high accuracy (and therefore classify patients according to outcome with high prognostic significance). For example, high Pr scores for the class assignments of G1 and G3 by SWS classifier 1 (6 probesets, as shown in Table D2 in section "SWS classifier 1" of the Description and Appendix 5A) and SWS class assignments of G1-like and G3-like classes within G2 class were observed.

Notably, 95% of the tumours of the Uppsala cohort showed >75% probability of belonging to either the G1-like or G3-like class, indicating a highly discriminant statistical basis for the class prediction function of the SWS classifier 1 for the G2 class.

### Example 14. SWS Classifier 3 and SWS Classifier 4

To find other classifiers, we excluded the best 6 probe sets (SWS classifier 1) from the 264 probe sets, and randomly selected two non-overlapping subsets (each of 40 probe sets) from the remaining 258 probe sets and applied the SWS algorithm to each subset.

In this way, we selected two additional classifiers: SWS classifier 3 (6-probe sets; Table D4 in section "SWS Classifier 3" of the Description and Appendix 7A) and SWS classifier 4 (7-probe sets; Table D5 in section "SWS Classifier 4" of the Description and Appendix 8A).

Tables D4 and D5 are organized as Table D3. For Uppsala, Stockholm and Singapore cohorts, each of three SWS classifiers provide similar high accuracy of classification in G1-G3 comparisons (Tables D3-D5). SWS also provided high and reproducible levels of separation of G2a and G2b sub-groups for different cohorts and highly significant differences in G2a-G2b comparison based on survival analysis (Tables D3-D5).

These tables show the values of parameters of SWS algorithm for selected classifies, predicted individual probabilities of belonging to the given class, and gene annotation, clinical significance etc.

Thus, we could consider the 264 probe sets as a general genetic classifier of the G2a (G1-like) and G2b (G3-like) tumour types.

### Example 15. Dichotomy of G2 Tumours by 264 Probe Sets Gene Grade Classifier

We next applied our grade classifiers directly to the 126 G2 tumours of the Uppsala cohort to ask if these genetic determinants of low and high grade might resolve moderately differentiated G2 tumours into separable classes. Using SWS for the 264 predictor set, we observed that the G2 tumours could be separated into G1-like (n=83) and G3-like (n=43) classes with few tumours exhibiting intermediate Pr scores (Appendix 2).

The probabilities of the SWS class assignments are shown in Figure 2B (Figure 2, Panel B) and more detailed information in Appendix 2.

We found 96% of the G2 tumours were assigned by the SWS classifier (and 94% by the PAM classifier, data not shown) to either the G1-like or G3-like classes with >75% probability, indicating that almost all G2 tumours can be molecularly well separated into distinct low- and high-grade-like classes (henceforth referred to as "G2a" and "G2b" genetic grades) (Appendix 2).

We validated the separation ability of G1a and G2b based on individual predictors and showed that all of them are statistically significant by U-test and t-test (Appendix 3).

Clinical validation (survival analysis) of G2a and G2b tumour subtypes based on the predictor set (or genetic classifier), showed a highly significant difference between survival curves of the G1a and G2b patients (Appendix 4).

### Example 16. Genetic Grade is Prognostic of Tumour Recurrence

To determine if the genetic grade classification correlates with patient outcome, we compared the disease-free survival (DFS) of patients with histologic G2 tumours classified as G2a or G2b by the SWS algorithm. (Due to space limitations and high concordance between the SWS and PAM classifiers, only data for the SWS classifier are presented hereafter.)

The Kaplan-Meier survival curves for these patients are shown in Figure 3 (green and red curves) superimposed on the survival curves of histologic G1, G2 and G3 patients (black curves) for comparison. Patients with G2a tumours showed a significantly better disease-free survival than those with G2b disease, regardless of therapeutic background (p=0.001; Figure 3A).

This finding is consistent in specific therapeutic contexts including untreated patients (Figure 3B), systemic therapy (Figure 3C), and hormone therapy only (Figure 3D) with survival differences significant at p=0.019, p=0.10 and p=0.022, respectively. These findings demonstrate a robust prognostic power of the genetic grade classifier in moderately differentiated tumours independent of therapeutic effects.

### Example 17. External Validation of the Genetic Grade Signature on the Stockholm and Singapore Cohorts

For external validation, we directly applied the SWS classifier to two large independent cohorts of primary breast cancer cases that are also graded according to the NGS guidelines and profiled on the Affymetrix platform (albeit at different times and in different laboratories). The results of the grade classifications are shown in Figure 2.

In both the Stockholm and Singapore cohorts, the G1 tumours are correctly classified with high accuracies similar to that observed in the training set: 96% (27/28) for Stockholm and 91% (10/11) for Singapore (Figure 2C and Figure 2E). However, both cohorts showed less accuracy in classifying the G3 tumours: 75% (46/61) for Stockholm and 72% (34/47) for Singapore. Despite this, the classifier remained capable of dividing the vast majority of the tumour samples into G1-like and G3-like classes with high Pr scores, and this remained true for the G2 tumours of both the Stockholm and Singapore cohorts (Figure 2D and Figure 2F).

As clinical histories are available on the Stockholm patients, we tested the prognostic performance of the classifier on this new G2 population of which 79% (46/58) of tumours are classified as G2a and 21% (12/58) are classified as G2b. Though this set is considerably smaller than the Uppsala G2 set, similar survival associations are observed.

As Figure 3E and Figure 3F show, patients with the G2a subtype are significantly less likely to relapse than those with tumours of the G2b subtype, indicating that the prognostic performance of the genetic grade classifier is reproducible in a second, independent population of G2 patients.

### Example 18. The Prognostic Power of Genetic Grade is Independent of Other Risk Factors

To assess the prognostic novelty of the classifier, we used multivariate Cox regression models to compare its performance to that of other conventional prognostic indicators assessed in the Uppsala cohort including lymph node status, tumour size, patient age, and estrogen (ER) and progesterone (PgR) receptor status. See Table E3 below.

**Table E3. The genetic grade signature is a strong independent indicator of disease-free survival in a multivariate analysis with conventional risk factors.**

| | **All patients** | | **Untreated patients** | | **Systemic therapy-treated patients** | | **ER+, Tamoxifen-treated patients** | |
|---|---|---|---|---|---|---|---|---|
| **Variables** | **p-value** | **Hazard ratio (95% CI)** | **p-value** | **Hazard ratio (95% CI)** | **p-value** | **Hazard ratio (95% CI)** | **p-value** | **Hazard ratio (95% CI)** |
| genetic grade signature | **0.001** | 1.50-5.09 | **0.046** | 1.02-7.77 | **0.038** | 1.49-8.01 | **0.009** | 1.39-9.99 |
| LN status | **0.031** | 0.27-0.94 | 0.700 | 0.01-23.53 | **0.091** | 0.13-1.16 | **0.096** | 0.11-1.20 |
| Tumour size | **0.054** | 0.99-1.07 | 0.950 | 0.91-1.10 | **0.016** | 1.01-1.11 | 0.250 | 0.97-1.09 |
| Age | 0.500 | 0.97-1.06 | 0.820 | 0.96-1.03 | 0.440 | 0.96-1.02 | 0.450 | 0.94-1.02 |
| ER status | 0.061 | 0.46-1.06 | 0.640 | 0.15-3.18 | 0.110 | 0.01-1.55 | - | - |
| PgR status | 0.300 | 0.57-6.10 | - | - | 0.270 | 0.56-7.76 | 0.990 | 0.10-9.50 |

As Table E3 shows, the genetic grade signature remained significantly associated with outcome in the different therapeutic contexts independent of the classical predictors, and is superior to both LN status and tumour size in all four treatment subgroups with the exception of systemic therapy where only tumour size is more significant.

This finding is further substantiated by a robust model selection approach (the Akaike Information Criterion) whereby the genetic grade classifier remained more significant than LN status and tumour size in all therapeutic subgroups (data not shown). These results demonstrate a powerful and additive contribution of the genetic grade classifier to patient prognosis.

### Example 19. G2a and G2b Subtypes Are Molecularly and Pathologically Distinct

The prognostic performance of the classifier suggests that G2a and G2b genetic grades may in fact represent distinct pathological entities previously unrecognized. We investigated this possibility by several approaches.

First we examined the histopathological composition of the G2a and G2b tumours and found that the predominant histologic subtypes - ductal, lobular and tubular - are equally distributed within the two classes and therefore not correlated with genetic grade (data not shown). Next, we analyzed the expression levels of the selected 264 probesets (i.e., representing ∼232 genes) as the *maximum* number of probesets capable of recapitulating a high G1/G3 classification accuracy (see Methods). These genes represent the top most significantly differentially expressed genes between G1 and G3 tumours after correcting for false discovery (see Table D1 above).

As shown in Figure 4, hierarchical cluster analysis using this set of genes shows a striking separation of the G2 population into two primary tumour profiles highly resembling the G1 and G3 profiles and that separate well into the G2a and G2b classes. Indeed, all but 11 of these 264 gene probesets are also differentially expressed (at p<0.05, Wilcoxon rank-sum test) between the G2a and G2b tumours.

This finding shows that extensive molecular heterogeneity exists within the G2 tumour population, and this heterogeneity is robustly defined by the major determinants of G1 and G3 cancer. It also demonstrates that a much larger and pervasive transcriptional program underlies the genetic grade predictions of the SWS signature - despite its composition of a mere 5 genes. Furthermore, statistical analysis of the gene ontology (GO) terms associated with the G2a-G2b differentially expressed genes revealed the significant enrichment of numerous biological processes and molecular functions.

Table E4 displays a selected set of significantly enriched GO categories which includes cell cycle, inhibition of apoptosis, cell motility and stress response, suggesting an imbalance of these cellular processes between the G2a- and G2b-type tumour cells.

**Table E4. Gene ontology analysis of differentially expressed genes. Selected terms are shown with corresponding p- values that reflect significance of term enrichment**

| **Biological Process** | **G1 vs G2a** | **G2a vs G2b** | **G2b vs G3** |
|---|---|---|---|
| Cell cycle | 6.2E-06 | 5.7E-28 | 2.5E-06 |
| Chromatin packaging and remodeling | 1.3E-02 | 2.5E-02 | |
| Mitosis | 2.7E-02 | 6.8E-15 | 1.1E-03 |
| Inhibition of apoptosis | | 4.4E-03 | 4.9E-03 |
| Oncogenesis | 1.6E-02 | 5.5E-04 | 5.5E-03 |
| Cell motility | | 3.6E-02 | 4.4E-02 |
| Stress response | | 5.0E-03 | |

| **Molecular Function** | | | |
|---|---|---|---|
| Kinase activator | 1.1E-03 | 7.2E-06 | |
| Histone | 3.5E-03 | 5.0E-02 | |
| Nucleic acid binding | 1.3E-02 | | |
| Microtubule family cytoskeletal protein | | 7.6E-07 | 4.2E-04 |
| Chemokine | | | 7.5E-03 |
| Non-receptor serine/threonine protein kinase | | 7.8E-04 | |
| Extracellular matrix linker protein | | 1.9E-02 | |

| **Pathway** | | | |
|---|---|---|---|
| Insulin/IGF pathway-MAPKK/MAPK cascade | 4.9E-02 | | |
| Apoptosis signaling pathway | | | 4.9E-02 |
| Ubiquitin proteasome pathway | | 3.0E-02 | |

Table S2 below shows the complete list of GO categories and their p values.

**Table S2. Comprehensive table of significant gene ontology terms identified in the different tumours group comparisons.**

| ***G2a vs. G2b tumours*** | | | | |
|---|---|---|---|---|
| **Biological Process** | **NCBI REFLIST (23481)** | **expected ratio** | **observed ratio** | **P value** |
| Cell cycle | 853 | 7.08 | 50 | 5.69E-28 |
| Mitosis | 287 | 2.38 | 22 | 6.78E-15 |
| Cell proliferation and differentiation | 751 | 6.24 | 32 | 4.21E-14 |
| Cell cycle control | 390 | 3.24 | 23 | 3.50E-13 |
| Chromosome segregation | 102 | 0.85 | 10 | 2.00E-08 |
| Cell structure | 624 | 5.18 | 17 | 2.16E-05 |
| Protein targeting and localization | 225 | 1.87 | 10 | 2.27E-05 |
| Cell structure and motility | 1021 | 8.48 | 22 | 4.73E-05 |
| DNA metabolism | 305 | 2.53 | 11 | 5.82E-05 |
| Oncogenesis | 600 | 4.98 | 14 | 5.52E-04 |
| DNA replication | 89 | 0.74 | 5 | 9.62E-04 |
| Protein phosphorylation | 592 | 4.92 | 13 | 1.49E-03 |
| Meiosis | 68 | 0.56 | 4 | 2.65E-03 |
| Inhibition of apoptosis | 127 | 1.05 | 5 | 4.43E-03 |
| Stress response | 187 | 1.55 | 6 | 5.03E-03 |
| Biological process unclassified | 9457 | 78.54 | 61 | 5.89E-03 |
| Protein biosynthesis | 598 | 4.97 | 0 | 6.54E-03 |
| Carbohydrate metabolism | 512 | 4.25 | 0 | 1.36E-02 |
| Cytokinesis | 116 | 0.96 | 4 | 1.65E-02 |
| Protein modification | 1013 | 8.41 | 15 | 2.27E-02 |
| Chromatin packaging and remodeling | 196 | 1.63 | 5 | 2.47E-02 |
| Sensory perception | 642 | 5.33 | 1 | 2.91E-02 |
| Cytokine/chemokine mediated immunity | 83 | 0.69 | 3 | 3.26E-02 |
| Other cell cycle process | 4 | 0.03 | 1 | 3.27E-02 |
| Proteolysis | 813 | 6.75 | 2 | 3.35E-02 |
| Chemosensory perception | 399 | 3.31 | 0 | 3.54E-02 |
| Cell motility | 291 | 2.42 | 6 | 3.57E-02 |
| Apoptosis | 459 | 3.81 | 8 | 3.91E-02 |
| DNA recombination | 38 | 0.32 | 2 | 4.03E-02 |
| Olfaction | 364 | 3.02 | 0 | 4.75E-02 |

| **Molecular Function** | **NCBI REFLIST (23481)** | **expected ratio** | **observed ratio** | **P value** |
|---|---|---|---|---|
| Microtubule binding motor protein | 74 | 0.61 | 10 | 9.86E-10 |
| Microtubule family cytoskeletal protein | 233 | 1.93 | 12 | 7.63E-07 |
| Kinase activator | 54 | 0.45 | 6 | 7.21E-06 |
| Kinase modulator | 126 | 1.05 | 8 | 1.27E-05 |
| Replication origin binding protein | 19 | 0.16 | 4 | 2.2it-05 |
| Non-receptor serine/threonine protein kinase | 289 | 2.4 | 9 | 7.79E-04 |
| Protein kinase | 526 | 4.37 | 12 | 1.64E-03 |
| Voltage-gated sodium channel | 14 | 0.12 | 2 | 6.23E-03 |
| Cytoskeletal protein | 824 | 6.84 | 14 | 9.42E-03 |
| Kinase | 692 | 5.75 | 12 | 1.36E-02 |
| Extracellular matrix linker protein | 25 | 0.21 | 2 | 1.87E-02 |
| Ribosomal protein | 431 | 3.58 | 0 | 2.70E-02 |
| KRAB box transcription factor | 640 | 5.31 | 1 | 2.95E-02 |
| DNA strand-pairing protein | 6 | 0.05 | 1 | 4.86E-02 |
| Histone | 99 | 0.82 | 3 | 5.03E-02 |

| **Pathway** | **NCBI REFLIST (23481)** | **expected ratio** | **observed ratio** | **P value** |
|---|---|---|---|---|
| Cell cycle | 22 | 0.18 | 3 | 8.75E-04 |
| Ubiquitin proteasome pathway | 80 | 0.66 | 3 | 2.97E-02 |
| DNA replication | 43 | 0.36 | 2 | 5.03E-02 |

| ***G1 vs. G2a tumours*** | | | | |
|---|---|---|---|---|
| **Biological Process** | **NCBI REFLIST (23481)** | **expected ratio** | **observed ratio** | **P value** |
| Cell cycle control | 390 | 0.35 | 6 | 9.19E-07 |
| Cell cycle | 853 | 0.76 | 7 | 6.19E-06 |
| Chromatin packag ing and remodeling | 196 | 0.18 | 2 | 1.32E-02 |
| Oncogenesis | 600 | 0.54 | 3 | 1.57E-02 |
| Nucleoside, nucleotide and nucleic acid metabolism | 3372 | 3.02 | 7 | 2.31E-02 |
| Mitosis | 287 | 0.26 | 2 | 2.69E-02 |
| Calcium ion homeostasis | 32 | 0.03 | 1 | 2.82E-02 |
| Developmental processes | 2150 | 1.92 | 5 | 3.77E-02 |
| mRNA transcription regulation | 1553 | 1.39 | 4 | 4.63E-02 |

| **Molecular Function** | **NCBI REFLIST (23481)** | **expected ratio** | **observed ratio** | **P value** |
|---|---|---|---|---|
| Kinase activator | 54 | 0.05 | 2 | 1.08E-03 |
| Histone | 99 | 0.09 | 2 | 3.54E-03 |
| Kinase modulator | 126 | 0.11 | 2 | 5.65E-03 |
| Select regulatory molecule | 979 | 0.88 | 4 | 1.02E-02 |
| Nucleic acid binding | 3014 | 2.7 | 7 | 1.29E-02 |
| Nuclear hormone receptor | 48. | 0.04 | 1 | 4.21E-02 |
| Other transcription factor | 387 | 0.35 | 2 | 4.64E-02 |

| **Pathway** | **NCBI REFLIST (23481)** | **expected ratio** | **observed ratio** | **P value** |
|---|---|---|---|---|
| Axon guidance mediated by semaphorins | 50 | 0.04 | 1 | 4.38E-02 |
| Insulin/IGF pathway-mitogen activated protein kinase kinase/MAP kinase cascade | 56 | 0.05 | 1 | 4.89E-02 |

| ***G2b vs. G3 tumours*** | | | | |
|---|---|---|---|---|
| **Biological Process** | **NCBI REFLIST (23481)** | **expected ratio** | **observed ratio** | **P value** |
| Cell cycle | 853 | 2.29 | 12 | 2.50E-06 |
| Cell proliferation and differentiation | 751 | 2.01 | 10 | 3.03E-05 |
| Cell cycle control | 390 | 1.05 | 7 | 8.55E-05 |
| Mitosis | 287 | 0.77 | 5 | 1.06E-03 |
| Chromosome segregation | 102 | 0.27 | 3 | 2.68E-03 |
| Inhibition of apoptosis | 127 | 0.34 | 3 | 4.93E-03 |
| Oncogenesis | 600 | 1.61 | 6 | 5.45E-03 |
| Apoptosis | 459 | 1.23 | 5 | 7.85E-03 |
| Meiosis | 68 | 0.18 | 2 | 1.46E-02 |
| Chromatin packaging and remodeling | 196 | 0.53 | 3 | 1.59E-02 |
| Protein targeting and localization | 225 | 0.6 | 3 | 2.28E-02 |
| Developmental processes | 2150 | 5.77 | 11 | 2.69E-02 |
| Oncogene | 98 | 0.26 | 2 | 2.88E-02 |
| Skeletal development | 108 | 0.29 | 2 | 3.43E-02 |
| Determination of dorsal/ventral axis | 14 | 0.04 | 1 | 3.69E-02 |
| Cytokinesis | 116 | 0.31 | 2 | 3.91E-02 |
| Cell motility | 291 | 0.78 | 3 | 4.36E-02 |
| Embryogenesis | 131 | 0.35 | 2 | 4.86E-02 |

| **Molecular Function** | **NCBI REFLIST (23481)** | **expected ratio** | **observed ratio** | **P value** |
|---|---|---|---|---|
| Microtubule family cytoskeletal protein | 233 | 0.63 | 5 | 4.19E-04 |
| Chromatin/chromatin-binding protein | 132 | 0.35 | 3 | 5.49E-03 |
| Chemokine | 48 | 0.13 | 2 | 7.51E-03 |
| Non-motor microtubule binding protein | 52 | 0.14 | 2 | 8.76E-03 |
| Microtubule binding motor protein | 74 | 0.2 | 2 | 1.71E-02 |
| Other transcription factor | 387 | 1.04 | 4 | 2.04E-02 |
| Cytoskeletal protein | 824 | 2.21 | 6 | 2.31E-02 |
| Reductase | 108 | 0.29 | 2 | 3.43E-02 |

| **Pathway** | **NCBI REFLIST (23481)** | **expected ratio** | **observed ratio** | **P value** |
|---|---|---|---|---|
| Apoptosis signaling pathway | 131 | 0.35 | 2 | 4.86E-02 |

To extend our analysis beyond the transcript level, we investigated the differences between G2a and G2b tumours using conventional clinicopathological markers.

Of the three histologic grading criteria, both mitotic count and nuclear pleomorphism are found to significantly vary between the G2a and G2b tumours (p=0.007 and p=0.05; Figure 5A and Figure 5I). Protein levels of the proliferation marker Ki67 are also found to be significantly different between the G2a and G2b tumours (p<0.0001; Figure 5B).

These findings, together with those of the gene ontology analysis, suggest that the genetic grade classifier may largely mirror cell proliferation and thus reflect the replicative potential of the breast tumour cells. However, proliferation is not the only oncogenic factor found to be associated with genetic grade. In the G2b tumours, protein levels of VEGF (Figure 5C), a major inducer of angiogenesis, and the degree of vascular growth (Figure 5D) are both found to be significantly higher compared to the G2a samples (p=0.015 and p=0.002, respectively) suggesting that a difference in angiogenic potential also distinguishes the two genetic grade classes.

Further analysis of bio-markers revealed yet more oncogenic differences. P53 mutations are found in only 6% of the G2a tumours, whereas 44% of the G2b tumours are p53 mutants (p<0.0001; Figure 5E) consistent with their higher replicative potential, and likely conferring a further survival advantage to these tumours via decreased apoptotic potential. We also observed higher levels of cyclin E1 protein (p=0.04; Figure 5F) in the G2b tumours which, in addition to contributing to enhanced proliferation (20), may also confer greater genomic instability (21, 22).

Finally, we observed a significant difference in hormonal status between the G2a and G2b tumours, with an increasing fraction of ER negative (7% versus 19%; p=0.06) and PgR negative (8.5% versus 23%; p=0.02) tumours in the G2b class, indicating differences in hormome sensitivity and dependence.

Taken together, these results show that multiple tumourigenic properties measured at the RNA, DNA, protein, and cellular levels can subdivide the G2a and G2b tumour subtypes - a finding that may explain, in part, the different patient survival outcomes observed between these two genetic classes.

### Example 20. The Grade Signature is More Than a Proliferative Marker

The genetic and clinicopathological evidence suggests that the genetic grade signature reflects, among other properties, the proliferative capacity of tumour cells. That proliferation rate is positively correlated with poor outcome in breast cancer (23) could explain the prognostic capacity of the genetic grade signature.

To further investigate this possibility, we analyzed the major proliferation markers, Ki67, S-phase fraction and mitotic index, together with the genetic grade signature, for survival correlations in Cox regression models (Table S3).

**Table S3. Multivariate analysis of proliferation markers and the genetic grade signature for disease-free survival correlations among patients with Grade II tumours.**

| | **Uppsala G2 patients** | |
|---|---|---|
| **Variables** | **p-value** | **Hazard ratio (95% CI)** |
| Genetic grade signature | **0.0075** | 1.28-4.88 |
| Ki67 | 0.9300 | 0.92-1.08 |
| S-phase fraction | 0.9200 | 0.50-1.86 |
| Mitotic index | 0.6900 | 0.56-2.40 |

Multivariate analysis showed that the genetic grade signature remained a significant independent predictor of recurrence (p=0.0075) in the presence of these proliferation markers, suggesting that the prognostic power of the grade signature derives from more than just and association with cell proliferation.

### Example 21. G2a and G2b Tumours Are Not Identical to Histologic G1 and G3 Cancers

In the survival analysis (Figure 3), we observed no significant survival differences between patients with G1 and G2a tumours, nor those with G3 and G2b tumours. This observation, together with the transcriptional analysis in Figure 4, suggests that the G2a and G2b classes may be clinically and molecularly indistinguishable from histologic G1 and G3 tumours, respectively.

To address this, we further analyzed the expression patterns of the 264 grade-associated probesets described in Figure 4. We discovered 14 genes and 57 genes significantly differentially expressed (p<0.01, Mann-Whitney U-test) between the G1 and G2a tumours and G3 and G2b tumours, respectively.

Notably, FOS and FOSB, central components of the AP-1 transcription factor complex, are expressed at higher levels in the G1 tumours, while genes involved in cell cycle progression such as CCNE2, MAD2L1, ASK and ECT2 are expressed at higher levels in the G2a tumours. In a similar fashion, the G3 tumours showed higher expression of cell cycle genes such as CDC20, BRRN1 and TTK as well as proliferative genes with oncogenic potential including MYBL2, ECT2 and CCNE1 when compared to the G2b tumours, while the anti-apoptotic gene, BCL2, is expressed at higher levels in the G2b tumours.

GO analysis of these differentially expressed genes indicated larger biological differences. In the G1-G2a comparison, the differentially expressed genes pointed to differences primarily in cell cycle-related processes and oncogenesis, while differences between the G2a and G3 tumours included cell cycle-related processes, inhibition of apoptosis, oncogenesis and cell motility (Table E4, Table S2).

Statistical analysis of conventional clinicopathological markers revealed further distinctions in the G1-G2a and the G2b-G3 tumour comparisons. As shown in Figure 5, G2a tumours showed significant increases in tumour size (K), lymph node positivity (L), cellular mitoses (A), tubule formation (J) and Ki67 levels (B) compared to histologic G1 tumours, and the G3 population showed significant increases in tumour size (K), vascular growth (D), mitoses (A), tubule formation (J), cyclin E1 (F) and ER negative status (G) when compared to the G2b tumours.

Taken together, these data indicate that the G2a and G2b populations, though highly similar to G1 and G3 tumours in terms of survival and transcriptional configuration, remain separable at multiple molecular and clinicopathological levels.

### Example 22. Prognostic Potential of the Genetic Grade Signature in G3 Tumours

The prognostic performance of the genetic grade signature in the G2 population suggests that the molecular "misclassifications" in the G1-G3 comparisons might correlate with survival differences. Of the 68 Uppsala and 28 Stockholm G1 tumours, too few are classified as G3-like (ie, 4 in total) for a reliable Kaplan-Meier estimate.

However, among the 55 Uppsala and 61 Stockholm G3 tumours, a total of 18 are classified as G1-like. Kaplan-Meier analysis could not confirm a significant disease-free-survival advantage for these patients, though a trend is observed (Figure 7A). Interestingly, scaling of the SWS probability (Pr) score to a threshold of Pr>0.8 (for G1-like) resulted in the selection of 12 G1-like G3 tumours associated with only two relapse events (one being a local recurrence only), thus having a survival curve moderately different from that of the remaining G3 population (p=0.077; Figure 7A).

This finding suggests that the prognostic significance of the classifier may extend also to the poorly differentiated G3 tumours, and that scaling based on the classifier Pr score may allow the fine tuning of prognostic sensitivity and/or specificity, depending on the clinical application.

### Example 23. Genetic Grade Improves Prognosis by the Nottingham Prognostic Index

The Nottingham Prognostic Index (NPI) is a widely accepted method of stratifying patients into prognostic groups (good (GPG), moderate (MPG) and poor (PPG)) based on lymph node stage, tumour size, and histologic grade (24). It is described in detail in Haybittle et al., 1982. We investigated whether incorporating genetic grade into the NPI could improve patient stratification. A simplified substitution method was explored.

For all tumours of the Uppsala and Stockholm cohorts for which NPI scores and survival information could be obtained (n=382), histologic grade (1, 2 or 3) is replaced by the genetic grade prediction (1 or 3) and new NPI (ie, *ggNPI*) scores are computed (see Methods). The survival of patients stratified into risk groups is then compared between classic NPI and ggNPI.

Though the survival curves of the NPI and ggNPI prognostic groups are comparable (Figure 6A and Figure 6B), the ggNPI reclassified 96 patients into different prognostic groups (ie, 46 into GPG, 36 into MPG, and 13 into PPG). The survival curves of these reclassified patients are highly similar to the GPG, MPG and PPG of the classic NPI (Figure 6C) indicating that reclassification by genetic grade improves prognosis of patient risk.

Practical guidelines that use the NPI in therapeutic decision making often recognize an excellent prognostic group (EPG) comprised of patients with NPI scores </= 2.4 (25, 26). Untreated patients in this group with lymph node negative disease have a 95% 10-year survival probability - equivalent to that of an age-matched female population without breast cancer (26). Thus, patients in this group are routinely not recommended for post-operative adjuvant therapy (25-27).

We compared the NPI and ggNPI stratifications on a subset of 161 lymph-node-negative patients who received no adjuvant systemic therapy. Forty-three and 87 patients are classified into the EPG by the classic NPI and ggNPI, respectively. Of the 43 patients classified into the EPG by the classic NPI, only one was considered different by the ggNPI; whereas, of those classified as needing adjuvant therapy by the classic NPI (ie, scores >2.4), 45 are reclassified by the ggNPI into the EPG.

When examined for outcome, the survival curves of the 43 and 87 EPG patients by NPI and ggNPI, respectively, are statistically indistinguishable, both showing ∼94% survival at 10 years (Figure 6D).

Thus, twice as many patients could be accurately classified into the EPG by the ggNPI, suggesting that the use of genetic grade can improve prediction of which patients should be spared systemic adjuvant therapy.

### Example 24. Discussion

The clinical subtyping of cancer directly impacts disease management. Subtypes indicative of tumour recurrence or drug resistance indicate the need for more aggressive or specific therapeutic strategies, while those that suggest less aggressive disease may specify milder therapeutic options. While clinical subtyping has historically been based primarily on the phenotypic properties of cancer, comprehensive genomic and transcriptomic analyses are beginning to reveal robust genotypic determinants of tumour subtype. In this context, we have studied the transcriptomes of primary invasive breast cancers using expression microarray technology to elucidate the genetic underpinnings of histologic grade, and to use this information to resolve the clinical heterogeneity associated with histologic grade.

Using two different supervised learning algorithms, SWS and PAM, we identified small gene subsets capable of classifying histologic Grade I and Grade III tumours with high accuracy. The smallest gene signature (SWS), comprised of a mere 5 genes (6 probesets), partitioned the large majority of G2 tumours into two highly distinguishable subclasses with G1-like and G3-like properties (G2a and G2b, respectively). Not only are the G2a and G2b tumours molecularly similar to those of histologic G1 and G3, respectively, but the disease-free survival curves of G2a and G2b patients are also highly resemblant of those of G1 and G3 patients. Moreover, these observations are confirmed in a large independent breast cancer cohort. Further analysis revealed that extensive genetic differences between the G2a and G2b classes are accompanied by a host of biological and tumourigenic differences know to separate low and high grade cancer (28) including proliferation rate (mitotic index, Ki67), angiogenic potential (VEGF, vascular growth), p53 mutational status, and estrogen and progesterone dependence, to name a few. Together, these findings demonstrate that the genetic grade signature recognizes and delineates two novel grade-related clinical subtypes among moderately differentiated G2 tumours.

Ma et. al. (2003) were the first to report a histologic grade signature capable of distinguishing low and high grade breast tumours. Using 12K cDNA microarrays to analyse material from 10 G1, 11G2 and 10 G3 microdissected tumours, they identified from a list of 1,940 variably expressed, well-measured genes (the top 200 differentially expressed between G1 and G3 tumours (p<0.01 after false discovery correction) (29). Using these genes to cluster their graded tumours, they observed that the majority of G2 tumours possessed a hybrid signature intermediate to that of G1 and G3 with few exceptions (see Figure 3 in Ma et. al., PNAS, 2003). Notably, this finding is in contrast with our discovery that the majority of G2 tumours do not display hybrid signatures (Figure 4; profiles of the top 264 gene probesets), but rather possess clear G1-like or G3-like gene features. According to our SWS classifiers, only a small percentage (6%) of the Grade 2 tumours has intermediate grade measurements (i.e. Pr score <0.75 for G1-like and G3-like).

To address this discrepancy, we cross-compared their list of 200 grade-associated genes to our list of 232 and observed a significant overlap of 35 genes (p<1.0x10-7; Monte Carlo simulation) including 2 of our 5 SWS signature genes, MELK and STK6. However, this overlap, despite its significance, represents only a small percentage of either gene list. That the two lists are mostly dissimilar in composition, and that the Ma et. al. study included both invasive (IDC) and noninvasive (DCIS) tumours could explain, to some degree, the variable results observed. Nevertheless, our finding that G2 tumours are predominantly G1-like or G3-like is clinically substantiated by the significant and reproducible survival differences observed between the G2a and G2b classes. It is also possible that differences in sample size (we have much larger number of patients than in Ma etc work), sample preparation, sample size, RNA purification, data normalization could have contributed to the variable results.

To better understand the prognostic value of the genetic grade signature, we compared its performance to other major indicators of outcome in multivariate Cox regression models. In G2 tumours, not only did the classifier remain an independent predictor of disease recurrence, but it is consistently a more powerful predictor than lymph node status and tumour size, underscoring its value as a new prognostic indicator. When incorporated into the Nottingham Prognostic Index (Haybittle et al., 1982), the genetic grade signature improved risk stratification for 25% of patients (compared to the classic NPI) and more than doubled the fraction of lymph node negative patients that should be classified into the excellent prognostic group and thus spared adjuvant treatment.

Breast cancer is thought to progress from a hyperplastic state, to a noninvasive malignant form (carcinoma in situ), to invasive carcinoma and, ultimately, to metastatic disease (30-32). Both the noninvasive and invasive forms can be stratified according to histologic grade. Whether grade is a continuum through which breast cancer progresses, or whether it is merely the endpoint of distinct genetic pathways has been debated (33-38). Studies comparing primary tumours to their subsequent metastases have supported the grade progression model, particularly when multiple metachronous recurrences are analyzed (38, 39). However, comparative genomic studies have identified reproducible chromosomal alterations that distinguish low and high grade disease including a 16q deletion unique to G1 carcinomas (36, 37, 40). These studies argue against the progression model and point to genetic origins of histologic grade. In our study of 494 invasive primary tumours, 94% could be molecularly classified with high probability of being G1-like or G3-like, while only 6% showed intermediate Pr scores (ie, <0.75 for G1-like or G3-like). Notably, we observed these same percentages in the G2 population of 224 tumours. These findings support the genetic pathways'ₘodel of grade origin, as they suggest that the large majority of breast cancers fundamentally exist in one of two predominant forms marked by the molecular and clinical essence of low or high grade. Whether these forms correlate with the grade-specific genomic alterations previously reported (36, 37, 40) remains to be elucidated.

It should also be noted that although a small percentage (∼6%) of the tumours in our study had intermediate genetic grade measurements (ie, analogous to the hybrid signature observed in Ma et. al. (2003)), too few were discovered to determine the clinical relevance of this intermediate genotype. Furthermore, it is unclear whether these intermediates arise as homogeneous cells that truly borderline low and high grade, or rather represent heterogeneous tumours comprised of distinct low and high grade cell types, such as that observed in tubular mixed carcinoma (38). Alternatively, that we observed the same percentage of intermediacy in tumour classification of all grades and across cohorts, suggests that this class represent a baseline level of uncertainty owing the technical noise.

In conclusion, our results show that the genetic essence of histologic grade can be distilled down to the expression patterns of a mere 5 genes with powerful prognostic implications, particularly in the Grade II setting and in the context of the NPI. The results indicate that G2 invasive breast cancer, at least in genetic terms, does not exist as a significant clinical entity. Indeed, our genetic grade signature dichotomized G2 tumours into two biologically and clinically distinct subtypes that could further be distinguished from G1 and G3 populations. Thus histologic grading, together with measurements of genetic grade, provide a rational basis for the refinement of the G2 subtype into subgrades "2a" and "2b" with immediate clinical ramifications.

Furthermore, our finding that the genetic grade signature could further resolve outcome prediction in G3 tumours, and in a manner dependent on Pr score thresholding, suggests that the genetic grade classifier, viewed as a scalable continuous variable, may have robust prognostic benefit in the diagnosis of all breast tumours. How to optimally weight the genetic grade measurement in combination with other risk factors for greatest prognostic return is a clinical challenge that must next be addressed.

### REFERENCES

1. Alizadeh, A.A., Eisen, M.B., Davis, R.E., Ma, C., Lossos, I.S., Rosenwald, A., Boldrick, J.C., Sabet, H., Tran, T., Yu, X., et al. 2000. Distinct types of diffuse large B-cell lymphoma identified by gene expression profiling. Nature 403:503-511.
2. Sorlie, T., Perou, C.M., Tibshirani, R., Aas, T., Geisler, S., Johnsen, H., Hastie, T., Eisen, M.B., van de Rijn, M., Jeffrey, S.S., et al. 2001. Gene expression patterns of breast carcinomas distinguish tumour subclasses with clinical implications. Proc Natl Acad Sci U S A 98:10869-10874.
3. van't Veer, L.J., Dai, H., van de Vijver, M.J., He, Y.D., Hart, A.A., Mao, M., Peterse, H.L., van der Kooy, K., Marton, M.J., Witteveen, A.T., et al. 2002. Gene expression profiling predicts clinical outcome of breast cancer. Nature 415:530-536.
4. Bullinger, L., Dohner, K., Bair, E., Frohling, S., Schlenk, R.F., Tibshirani, R., Dohner, H., and Pollack, J.R. 2004. Use of gene-expression profiling to identify prognostic subclasses in adult acute myeloid leukemia. N Engl J Med 350:1605-1616.
5. Bloom, H.J., and Richardson, W.W. 1957. Histological grading and prognosis in breast cancer; a study of 1409 cases of which 359 have been followed for 15 years. Br J Cancer 11:359-377.
6. Elston, C.W., and Ellis, I.O. 1991. Pathological prognostic factors in breast cancer. I. The value of histological grade in breast cancer: experience from a large study with long-term follow-up. Histopathology 19:403-410.
7. Schumacher, M., Schmoor, C., Sauerbrei, W., Schauer, A., Ummenhofer, L., Gatzemeier, W., and Rauschecker, H. 1993. The prognostic effect of histological tumour grade in node-negative breast cancer patients. Breast Cancer Res Treat 25:235-245.
8. Roberti, N.E. 1997. The role of histologic grading in the prognosis of patients with carcinoma of the breast: is this a neglected opportunity? Cancer 80:1708-1716.
9. Lundin, J., Lundin, M., Holli, K., Kataja, V., Elomaa, L., Pylkkanen, L., Turpeenniemi-Hujanen, T., and Joensuu, H. 2001. Omission of histologic grading from clinical decision making may result in overuse of adjuvant therapies in breast cancer: results from a nationwide study. J Clin Oncol 19:28-36.
10. Harvey, J.M., de Klerk, N.H., and Sterrett, G.F. 1992. Histological grading in breast cancer: interobserver agreement, and relation to other prognostic factors including ploidy. Pathology 24:63-68.
11. Frierson, H.F., Jr., Wolber, R.A., Berean, K.W., Franquemont, D.W., Gaffey, M.J., Boyd, J.C., and Wilbur, D.C. 1995. Interobserver reproducibility of the Nottingham modification of the Bloom and Richardson histologic grading scheme for infiltrating ductal carcinoma. Am J Clin Pathol 103:195-198.
12. Robbins, P., Pinder, S., de Klerk, N., Dawkins, H., Harvey, J., Sterrett, G., Ellis, I., and Elston, C. 1995. Histological grading of breast carcinomas: a study of interobserver agreement. Hum Pathol 26:873-879.
13. Dalton, L.W., Pinder, S.E., Elston, C.E., Ellis, I.O., Page, D.L., Dupont, W.D., and Blarney, R.W. 2000. Histologic grading of breast cancer: linkage of patient outcome with level of pathologist agreement. Mod Pathol 13:730-735.
14. Younes, M., and Laucirica, R. 1997. Lack of prognostic significance of histological grade in node-negative invasive breast carcinoma. Clin Cancer Res 3:601-604.
15. Hayes, D.F., Isaacs, C., and Stearns, V. 2001. Prognostic factors in breast cancer: current and new predictors of metastasis. J Mammary Gland Biol Neoplasia 6:375-392.
16. Trudeau, M.E., Pritchard, K.I., Chapman, J.A., Hanna, W.M., Kahn, H.J., Murray, D., Sawka, C.A., Mobbs, B.G., Andrulis, I., McCready, D.R., et al. 2005. Prognostic factors affecting the natural history of node-negative breast cancer. Breast Cancer Res Treat 89:35-45.
17. Tibshirani, R., Hastie, T., Narasimhan, B., and Chu, G. 2002. Diagnosis of multiple cancer types by shrunken centroids of gene expression. Proc Natl Acad Sci U S A 99:6567-6572.
18. Kuznetsov, V.A., Ivshina, A.V., Sen'ko, O.V., Kuznetsova, A.V. 1996. Syndrome approach for computer recognition of fuzzy systems and its application to immunological diagnostics and prognosis of human cancer. Math. Comput. Modeling 23:92-112.
19. Jackson, A.M., Ivshina, A.V., Senko, O., Kuznetsova, A., Sundan, A., O'Donnell, M.A., Clinton, S., Alexandroff, A.B., Selby, P.J., James, K., et al. 1998. Prognosis of intravesical bacillus Calmette-Guerin therapy for superficial bladder cancer by immunological urinary measurements: statistically weighted syndrome analysis. J Urol 159:1054-1063.
20. Lukas, J., Herzinger, T., Hansen, K., Moroni, M.C., Resnitzky, D., Helin, K., Reed, S.I., and Bartek, J. 1997. Cyclin E-induced S phase without activation of the pRb/E2F pathway. Genes Dev 11:1479-1492.
21. Spruck, C.H., Won, K.A., and Reed, S.I. 1999. Deregulated cyclin E induces chromosome instability. Nature 401:.297-300.
22. Minella, A.C., Swanger, J., Bryant, E., Welcker, M., Hwang, H., and Clurman, B.E. 2002. p53 and p21 form an inducible barrier that protects cells against cyclin E-cdk2 deregulation. Curr Biol 12:1817-1827.
23. van Diest, P.J., van der Wall, E., and Baak, J.P. 2004. Prognostic value of proliferation in invasive breast cancer: a review. J Clin Pathol 57:675-681.
24. Haybittle, J.L., Blamey, R.W., Elston, C.W., Johnson, J., Doyle, P.J., Campbell, F.C., Nicholson, R.I., and Griffiths, K. 1982. A prognostic index in primary breast cancer. Br J Cancer 45:361-366.
25. Blarney, R.W. 1996. The design and clinical use of the Nottingham Prognostic Index in breast cancer. Breast 5:156-157.
26. Stotter, A. 1999. A prognostic table to guide practitioners advising patients on adjuvant systemic therapy in early breast cancer. Eur J Surg Oncol 25:341-343.
27. Feldman, M., Stanford, R., Catcheside, A., and Stotter, A. 2002. The use of a prognostic table to aid decision making on adjuvant therapy for women with early breast cancer. Eur J Surg Oncol 28:615-619.
28. Lacroix, M., Toillon, R.A., and Leclercq, G. 2004. Stable 'portrait' of breast tumours during progression: data from biology, pathology and genetics. Endocr Relat Cancer 11:497-522.
29. Ma, X.J., Salunga, R., Tuggle, J.T., Gaudet, J., Enright, E., McQuary, P., Payette, T., Pistone, M., Stecker, K., Zhang, B.M., et al. 2003. Gene expression profiles of human breast cancer progression. Proc Natl Acad Sci U S A 100:5974-5979.
30. Lakhani, S.R. 1999. The transition from hyperplasia to invasive carcinoma of the breast. J Pathol 187:272-278.
31. Shackney, S.E., and Silverman, J.F. 2003. Molecular evolutionary patterns in breast cancer. Adv Anat Pathol 10:278-290.
32. Simpson, P.T., Reis-Filho, J.S., Gale, T., and Lakhani, S.R. 2005. Molecular evolution of breast cancer. J Pathol 205:248-254.
33. Tubiana, M., and Koscielny, S. 1991. Natural history of human breast cancer: recent data and clinical implications. Breast Cancer Res Treat 18:125-140.
34. Tabar, L., Fagerberg, G., Chen, H.H., Duffy, S.W., and Gad, A. 1996. Tumour development, histology and grade of breast cancers: prognosis and progression. Int J Cancer 66:413-419.
35. Millis, R.R., Barnes, D.M., Lampejo, O.T., Egan, M.K., and Smith, P. 1998. Tumour grade does not change between primary and recurrent mammary carcinoma. Eur J Cancer 34:548-553.
36. Roylance, R., Gorman, P., Harris, W., Liebmann, R., Barnes, D., Hanby, A., and Sheer, D. 1999. Comparative genomic hybridization of breast tumours stratified by histological grade reveals new insights into the biological progression of breast cancer. Cancer Res 59:1433-1436.
37. Buerger, H., Otterbach, F., Simon, R., Schafer, K.L., Poremba, C., Diallo, R., Brinkschmidt, C., Dockhorn-Dworniczak, B., and Boecker, W. 1999. Different genetic pathways in the evolution of invasive breast cancer are associated with distinct morphological subtypes. J Pathol 189:521-526.
38. Cserni, G. 2002. Tumour histological grade may progress between primary and recurrent invasive mammary carcinoma. J Clin Pathol 55:293-297.
39. Hitchcock, A., Ellis, I.O., Robertson, J.F., Gilmour, A., Bell, J., Elston, C.W., and Blarney, R.W. 1989. An observation of DNA ploidy, histological grade, and immunoreactivity for tumour-related antigens in primary and metastatic breast carcinoma. J Pathol 159:129-134.
40. Buerger, H., Mommers, E.C., Littmann, R., Simon, R., Diallo, R., Poremba, C., Dockhorn-Dworniczak, B., van Diest, P.J., and Boecker, W. 2001. Ductal invasive G2 and G3 carcinomas of the breast are the end stages of at least two different lines of genetic evolution. J Pathol 194:165-170.
41. Bergh, J., Norberg, T., Sjogren, S., Lindgren, A., and Holmberg, L. 1995. Complete sequencing of the p53 gene provides prognostic information in breast cancer patients, particularly in relation to adjuvant systemic therapy and radiotherapy. Nat Med 1:1029-1034.
42. Linderholm, B.K., Lindahl, T., Holmberg, L., Klaar, S., Lennerstrand, J., Henriksson, R., and Bergh, J. 2001. The expression of vascular endothelial growth factor correlates with mutant p53 and poor prognosis in human breast cancer. Cancer Res 61:2256-2260.
43. Lindahl, T., Landberg, G., Ahlgren, J., Nordgren, H., Norberg, T., Klaar, S., Holmberg, L., and Bergh, J. 2004. Overexpression of cyclin E protein is associated with specific mutation types in the p53 gene and poor survival in human breast cancer. Carcinogenesis 25:375-380.
44. Miller, L.D., Smeds, J., George, J., Vega, V.B., Vergara, L., Ploner, A., Pawitan, Y., Hall, P., Klaar, S., Liu, E.T., et al. 2005. An expression signature for p53 status in human breast cancer predicts mutation status, transcriptional effects, and patient survival. Proc Natl Acad Sci USA.
45. Kuznetsov, V.A., Knott, G.D., Ivshina, A.V. 1998. Artificial immune system based on syndromes-response approach: Theory and their application to recognition of the patterns of immune response and prognosis of therapy outcome. In Proc. of IEEE Intern. Conf. on Systems, Man, and Cybernetics. San Diego, CA, USA. 3804-3809.
46. Mi, H., Lazareva-Ulitsky, B., Loo, R., Kejariwal, A., Vandergriff, J., Rabkin, S., Guo, N., Muruganujan, A., Doremieux, O., Campbell, M.J., et al. 2005. The PANTHER database of protein families, subfamilies, functions and pathways. Nucleic Acids Res 33:D284-288.
47. 1996. Randomized trial of two versus five years of adjuvant tamoxifen for postmenopausal early stage breast cancer. Swedish Breast Cancer Cooperative Group. J Natl Cancer Inst 88:1543-1549.

Sotiriou, T., Perou, C.M., Tibshirani, R., Aas, T., Geisler, S., Johnsen, H., Hastie, T., Eisen, M.B., van de Rijn, M., Jeffrey, S.S., et al. 2001. Gene expression patterns of breast carcinomas distinguish tumour subclasses with clinical implications. Proc Natl Acad Sci USA 98:10869-10874.
van't Veer, L.J., Dai, H., van de Vijver, M.J., He, Y.D., Hart, A.A., Mao, M., Peterse, H.L., van der Kooy, K., Marton, M.J., Witteveen, A.T., et al. 2002. Gene expression profiling predicts clinical outcome of breast cancer. Nature 415:530-536.
Ma, X.J., Salunga, R., Tuggle, J.T., Gaudet, J., Enright, E., McQuary, P., Payette, T., Pistone, M., Stecker, K., Zhang, B.M., et al. 2003. Gene expression profiles of human breast cancer progression. Proc Natl Acad Sci USA 100:5974-5979.
Miller, L.D., Smeds, J., George, J., Vega, V.B., Vergara, L., Ploner, A., Pawitan, Y., Hall, P., Klaar, S., Liu, E.T., et al. 2005. An expression signature for p53 status in human breast cancer predicts mutation status, transcriptional effects, and patient survival. Proc Natl Acad Sci U S A.
Kuznetsov, V.A., Ivshina, A.V., Sen'ko, O.V., Kuznetsova, A.V. 1996. Syndrome approach for computer recognition of fuzzy systems and its application to immunological diagnostics and prognosis of human cancer. Math. Comput. Modeling 23:92-112.
Kuznetsov, V.A., Knott, G.D., Ivshina, A.V. 1998. Artificial immune system based on syndromes-response approach: Theory and their application to recognition of the patterns of immune response and prognosis of therapy outcome. In Proc. of IEEE Intern. Conf. on Systems, Man, and Cybernetics. San Diego, CA, USA. 3804-3809.
Jackson, A.M., Ivshina, A.V., Senko, O., Kuznetsova, A., Sundan, A., O'Donnell, M.A., Clinton, S., Alexandroff, A.B., Selby, P.J., James, K., Kuznetsov,V.A. 1998. Prognosis of intravesical bacillus Calmette-Guerin therapy for superficial bladder cancer by immunological urinary measurements: statistically weighted syndrome analysis. J Urol 159:1054-1063.
Mueller, B.U. , Zeichner, S.L. , Kuznetsov, V.A., Heath-Chiozzi,M., Pizzo P.A., and Dimitrov, D.S. Individual prognoses of long-term responses to antiretroviral treatment based on virological, immunological and pharmacological parameters measured during the first week under therapy. AIDS, 13, 1998, pp. f191-f196.
Tibshirani, R., Hastie, T., Narasimhan, B., and Chu, G. 2002. Diagnosis of multiple cancer types by shrunken centroids of gene expression. Proc Natl Acad Sci U S A 99:6567-6572.
Haybittle JL, Blamey RW, Elston CW, Johnson J, Doyle PJ, Campbell FC, Nicholson RI, Griffiths K. et al. A prognostic index in primary breast cancer. Br J Cancer 1982; 45 (3):361-6

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments and that many modifications and additions thereto may be made within the scope of the invention. Indeed, various modifications of the described modes for carrying out the invention which are obvious to those skilled in molecular biology or related fields are intended to be within the scope of the claims. Furthermore, various combinations of the features of the following dependent claims can be made with the features of the independent claims without departing from the scope of the present invention.

### APPENDIX 1

**SWS Classifier 0**

| **Order** | **UGID(build #177)** | **UnigeneName** | **Gene Symbol** | **Genbank Acc** | **Affi ID** | **Cut-off** | **SWS: Chi-2** | **Instability indices** |
|---|---|---|---|---|---|---|---|---|
| 1 | Hs.528654 | Hypothetical protein FLJ11029 | FLJ11029 | BG165011 | B.228273_at | 7.7063 | 96.0 | 0.01139 |
| 2 | acc_NM_00315 8.1 | | | NM_003158 | A.208079_s_at | 6.6526 | 95.6 | 0.002087 |
| 3 | Hs.308045 | Barren homolog (Drosophila) | BRRN1 | D38553 | A.212949_at | 5.9167 | 92.6 | 0.005697 |
| 4 | Hs.35962 | CDNA clone IMAGE:4452583, partial cds | | BG492359 | B.226936_at | 7.5619 | 92.6 | 0.003179 |
| 5 | Hs.184339 | Maternal embryonic leucine zipper kinase | MELK | NM_014791 | A.204825_at | 7.1073 | 90.1 | 0.002296 |
| 6 | Hs.250822 | Serine/threonine kinase 6 | STK6 | NM_003600 | A.204092_s_at | 6.7266 | 88.6 | 0.003041 |
| 7 | Hs.9329 | TPX2, microtubule-associated protein homolog (Xenopus laevis) | TPX2 | AF098158 | A.210052_s_at | 7.4051 | 86.2 | 0.000788 |
| 8 | Hs.1594 | Centromere protein A, 17kDa | CENPA | NMU_001809 | A.204962_s_at | 6.344 | 85.3 | 0.037328 |
| 9 | Hs.198363 | MCM10 minichromosome maintenance deficient 10 (S. cerevisiae) | MCM10 | AB042719 | B.222962_s_at | 6.1328 | 85.2 | 0.001132 |
| 10 | Hs.48855 | Cell division cycle associated 8 | CDCA8 | BC001651 | A.221520_s_at | 5.2189 | 85.2 | 0.018247 |
| 11 | Hs.169840 | TTK protein kinase | TTK | NM_003318 | A.204822_at | 6.2397 | 82.2 | 0.017014 |
| 12 | Hs.69360 | Kinesin family member 2C | KIF2C | U63743 | A.209408_at | 7.3717 | 82.1 | 0.006487 |
| 13 | Hs.55028 | CDNA clone IMAGE:6043059, partial cds | | BF111626 | B.228559_at | 7.2212 | 82.1 | 0.000785 |
| 14 | Hs.511941 | Forkhead box M1 | FOXM1 | NM_021953 | A.202580_x_at | 6.5827 | 81.9 | 0.001279 |
| 15 | Hs.3104 | Kinesin family member 14 | KIF14 | AW183154 | B.236641_at | 6.4175 | 81.9 | 0.02267 |
| 16 | Hs.179718 | V-myb myeloblastosis viral oncogene homolog (avian)-like 2 | MYBL2 | NM_002466 | A.201710_at | 6.0661 | 79.2 | 0.017019 |
| 17 | Hs.93002 | Ubiquitin-conjugating enzyme E2C | UBE2C | NM_007019 | A.202954_at | 7.8431 | 79.2 | 0.06442 |
| 18 | Hs.344037 | Protein regulator of cytokinesis 1 | PRC1 | NM_003981 | A.218009_s_at | 7.3376 | 79.2 | 0.002774 |
| 19 | Hs.436187 | Thyroid hormone receptor interactor 13 | TRIP13 | NM_004237 | A.204033_at | 7.1768 | 79.0 | 0.090947 |
| 20 | Hs.408658 | Cyclin E2 | CCNE2 | NM_004702 | A.205034_at | 6.2055. | 78.6 | 0.018747 |
| 21 | Hs.30114 | Cell division cycle associated 3 | CDCA3 | BC002551 | 8.223307_at | 7.8418 | 78.6 | 0.083659 |
| 22 | Hs.84113 | Cyclin-dependent kinase inhibitor 3 (CDK2-associated dual specificity phosphatase) | CDKN3 | AF213033 | A.209714_s_at | 6.8414 | 78.6 | 0.005037 |
| 23 | Hs.279766 | Kinesin family member 4A | KIF4A | NM_012310 | A.218355_at | 6.6174 | 78.2 | 0.013173 |
| 24 | Hs.104859 | Hypothetical protein DKFZp762E1312 | DKFZp762E 1312 | NM_018410 | A.218726_at | 6.3781 | 75.5 | 0.035806 |
| 25 | Hs.444118 | MCM6 minichromosome maintenance deficient 6 (MIS5 homolog, S. pombe) (S. cerevisiae) | MCM6 | NM_005915 | A.201930_at | 7.9353 | 75.4 | 0.013732 |
| 26 | acc_NM_01812 3.1 | | | NM_018123 | A.219918_s_at | 6.5958 | 75.4 | 0.001536 |
| 27 | Hs.287472 | BUB1 budding uninhibited by benzimidazoles 1 homolog (yeast) | BUB1 | AF043294 | A.209642_at | 6.0118 | 74.1 | 0.057721 |
| 28 | Hs.36708 | BUB1 budding uninhibited by benzimidazoles 1 homolog beta (yeast) | BUB1B | NM_001211 | A.203755_at | 6.68 | 73.5 | 0.006753 |
| 29 | Hs.77783 | Membrane-associated tyrosine- and threonine-specific cdc2-inhibitory kinase | PKMYT1 | NM_004203 | A.204.267_x_at | 6.9229 | 73.4 | 0.001777 |
| 30 | Hs.446554 | RAD51 homolog (RecA homolog, E. coli) (S. cerevisiae) | RAD51 | NM_002875 | A.205024_s_at | 6.3524 | 73.4 | 0.016246 |
| 31 | Hs.82906 | CDC20 cell division cycle 20 homolog (S. cerevisiae) | CDC20 | NM_001255 | A.202870_s_at | 7.1291 | 73.0 | 0.108453 |
| 32 | Hs.252712 | Karyopherin alpha 2 (RAG cohort 1, importin alpha 1) | KPNA2 | NM_002266 | A.201088_at | 8.4964 | 72.6 | 0.025069 |
| 33 | Hs.3104 | | KIF14 | NM_014875 | A.206364_at | 6.1518 | 72.6 | 0.066755 |
| 34 | Hs.103305 | Chromobox homolog 2 (Pc class homolog, Drosophila) | | BE514414 | B.226473_at | 7.5588 | 72.6 | 0.013762 |
| 35 | Hs.152759 | Activator of S phase kinase | ASK | NM_006716 | A.204244_s_at | 5.9825 | 72.3 | 0.018258 |
| 36 | acc_AL138828 | | | AL138828 | B.228069_at | 7.0119 | 72.3 | 0.084119 |
| 37 | Hs.226390 | Ribonucleotide reductase M2 polypeptide | RRM2 | NM_001034 | A.201890_at | 7.1014 | 71.0 | 0.00223 |
| 38 | Hs.445890 | HSPC163 protein | HSPC163 | NM_014184 | A.218728_s_at | 7.6481 | 70.8 | 0.003156 |
| 39 | Hs.194698 | Cyclin B2 | CCNB2 | NM_004701 | A.202705_at | 7.0096 | 70.7 | 0.000753 |
| 40 | Hs.234545 | Cell division cycle associated 1 | CDCA1 | AF326731 | B.223381_at | 6.4921 | 70.7 | 0.008259 |
| 41 | Hs.16244 | Sperm associated antigen 5 | SPAG5 | NM_006461 | A.203145_at | 6.4627 | 70.1 | 0.000806 |
| 42 | Hs.62180 | Anillin, actin binding protein (scraps homolog, Drosophila) | ANLN | AK023208 | B.222608_s_at | 6.9556 | 69.6 | 0.012886 |
| 43 | Hs.14559 | Chromosome 10 open reading frame 3 | C10orf3 | NM_018131 | A.218542_at | 6.4965 | 69.3 | 0.048726 |
| 44 | Hs.122908 | DNA replication factor | CDT1 | AW075105 | B.228868_x_at | 7.0543 | 69.3 | 0.001059 |
| 45 | Hs.8878 | Kinesin family member 11 | KIF11 | NM_004523 | A.204444_at | 6.4655 | 69.3 | 0.005297 |
| 46 | Hs.83758 | CDC28 protein kinase regulatory subunit 2 | CKS2 | NM_001827 | A.204170_s_at | 7.8353 | 69.2 | 0.027378 |
| 47 | Hs.112160 | Chromosome 15 open reading frame 20 | PIF1 | AF108138 | B.228252_at | 6.6518 | 69.2 | 0.038767 |
| 48 | Hs.79078 | MAD2 mitotic arrest deficient-like 1 (yeast) | MAD2L1 | NM_002358 | A.203362_s_at | 6.4606 | 68.0 | 0.038039 |
| 49 | Hs.226390 | Ribonucleotide reductase M2 polypeptide | RRM2 | BC001886 | A.209773_s_at | 7.2979 | 67.4 | 0.135043 |
| 50 | Hs.462306 | Ubiquitin-conjugating enzyme E2S | UBE2S | NM_014501 | A.202779_s_at | 6.9165 | 67.4 | 0.01343 |
| 51 | Hs.70704 | Chromosome 20 open reading frame 129 | C20orf129 | BC001068 | B.225687_at | 7.2322 | 67.4 | 0.038884 |
| 52 | Hs.294088 | GAJ protein | GAJ | AY028916 | B.223700_at | 5.8432 | 67.3 | 0.00478 |
| 53 | Hs.381225 | Kinetochore protein Spc24 | Spc24 | AI469788 | B.235572_at | 6.7839 | 67.3 | 0.002404 |
| 54 | Hs.334562 | Cell division cycle 2, G1 to S and G2 to M | CDC2 | AL524035 | A.203213_at | 7.0152 | 66.9 | 0.024298 |
| 55 | Hs.109706 | Hematological and neurological expressed 1 | HN1 | NM_016185 | A.217755_at | 7.9118 | 66.8 | 0.008041 |
| 56 | Hs.23900 | Rac GTPase activating protein 1 | RACGAP1 | AU153848 | A.222077_s_at | 7.1207 | 66.5 | 0.042338 |
| 57 | Hs.77695 | Discs, large homolog 7 (Drosophila) | DLG7 | NM_014750 | A.203764_at | 6.3122 | 66.4 | 0.001011 |
| 58 | Hs.46423 | Histone 1, H4c | HIST1H4F | NM_003542 | A.205967_at | 8.3796 | 66.4 | 0.00.462 |
| 59 | Hs.20830 | Kinesin family member C1 | KIFC1 | BC000712 | A.209680_s_at | 6.9746 | 66.4 | 0.041639 |
| 60 | Hs.339665 | Similar to Gastric cancer up-regulated-2 | | AL135396 | B.225834_at | 7.2467 | 66.4 | 0.019861 |
| 61 | Hs.94292 | FLJ23311 protein | FLJ23311 | NM_024680 | A.219990_at | 5.0277 | 66.3 | 0.006891 |
| 62 | Hs.73625 | Kinesin family member 20A | KIF20A | NM_005733 | A.218755_at | 7.2115 | 66.3 | 0.000671 |
| 63 | Hs.315167 | Defective in sister chromatid cohesion homolog 1 (S. cerevisiae) | MGC5528. | NM_024094 | A.219000_s_at | 6.2835 | 66.3 | 0.001518 |
| 64 | Hs.85137 | Cyclin A2 | CCNA2 | NM_001237 | A.203418_at | 6.194 | 66.2 | 0.00117 |
| 65 | Hs.528669 | Chromosome condensation protein G | HCAP-G | NM_022346 | A.218662_s_at | 6.0594 | 66.2 | 0.01287 |
| 66 | Hs.75573 | Centromere protein E, 312kDa | CENPE | NM_001813 | A.205046_at | 5.1972 | 65.5 | 0.002372 |
| 67 | acc_BE966146 | RAD51 associated protein 1 | | BE966146 | A.204146_at | 6.3049 | 65.3 | 0.006989 |
| 68 | Hs.334562 | Cell division cycle 2, G1 to S and G2 to M | CDC2 | D88357 | A210559_s_at | 7.0395 | 64.8 | 0.000887 |
| 69 | Hs.108106 | Ubiquitin-like containing PHD and RING finger domains, 1 | UHRF1 | AK025578 | B.225655_at | 7.7335 | 64.8 | 0.024133 |
| 70 | Hs.1578 | Baculoviral IAP repeat-containing 5 (survivin) | BIRC5 | NMU_001168 | A.202095_s_at | 6.8907 | 64.6 | 0.090038 |
| 71 | acc_N M_02106 7.1 | | | NM_021067 | A.206102_at | 6.714 | 64.6 | 0.01255 |
| 72 | Hs.244723 | Cyclin E1 | CCNE1 | AI671049 | A.213523_at | 6.082 | 64.6 | 0.000547 |
| 73 | Hs.198363 | MCM10 minichromosome maintenance deficient 10 (S. cerevisiae) | MCM10 | NM_018518 | A.220651_s_at | 5.6784 | 64.2 | 0.0809977 |
| 74 | Hs.155223 | Stanniocalcin 2 | STC2 | AI435828 | A.203438_at | 7.5388 | 64.0 | 0.011227 |
| 75 | Hs.25647 | V-fos FBJ murine osteosarcoma viral oncogene homolog | FOS | BC004490 | A.209189_at | 8.9921 | 63.9 | 0.162153 |
| 76 | Hs.184601 | Solute carrier family 7 (cationic amino acid transporter, y+ system), member 5 | SLC7A5 | AB018009 | A.201195_s_at | 7.4931 | 63.6 | 0.010677 |
| 77 | Hs.528669 | Chromosome condensation protein G | HCAP-G | NM_022346 | A.218663_at | 5.7831 | 63.6 | 0.0072 |
| 78 | Hs.30114 | Cell division cycle associated 3 | CDCA3 | NM_031299 | A.221436_s_at | 6.1898 | 63.6 | 0.001853 |
| 79 | Hs.296398 | Lysosomal associated protein transmembrane 4 beta | LAPTM4B | T15777 | A.214039_s_at | 9.3209 | 63.3 | 0.001249 |
| 80 | Hs.442658 | Aurora kinase B | AURKB | AB011446 | A.209464_at | 5.9611 | 63.3 | 0.005453 |
| 81 | Hs.6879 | DC13 protein | DC13 | NM_020188 | A.218447_at | 7.436 | 63.3 | 0.027988 |
| 82 | Hs.78913 | Chemokine (C-X3-C motif) receptor 1 | CX3CR1 | U20350 | A.205898_at | 6.7764 | 63.2 | 0.014155 |
| 83 | Hs.406684 | Sodium channel, voltage-gated, type VII, alpha | SCN7A | AI828648 | B.228504_at | 5.8248 | 63.2 | 0.003803 |
| 84 | Hs.80976 | Antigen identified by monoclonal antibody Ki-67 | MKI67 | BF001806 | A.212022_s_at | 6.7255 | 62.4 | 0.124758 |
| 85 | Hs.406639 | Hypothetical protein LOC146909 | LOC146909 | AA292789 | A.222039_at | 6.4591 | 62.2 | 0.017876 |
| 86 | Hs.334562 | Cell division cycle 2, G1 to S and G2 to M | CDC2 | NM_001786 | A.203214_x_at | 6.588 | 61.5 | 0.001897 |
| 87 | Hs.23960 | Cyclin B1 | CCNB1 | BE407516 | A.214710_s_at | 7.1555 | 60.8 | 0.01353 |
| 88 | Hs.445098 | DEP domain containing 1 | SDP35 | AK000490 | B.222958_s_at | 6.8747 | 60.8 | 0.003156 |
| 89 | Hs.58241 | Serine/threonine kinase 32B | HSA250839 | NM_018401 | A.219686_at | 4.5663 | 60.4 | 0.005019 |
| 90 | Hs.5199 | HSPC150 protein similar to ubiquitin-conjugating enzyme | HSPC150 | AB032931 | B.223229_at | 7.3947 | 60.4 | 0.010211 |
| 91 | acc_T58044 | | | T58044 | B.227232_at | 8.5021 | 60.4 | 0.00327 |
| 92 | Hs.421337 | DEP domain containing 1B | XTP1 | AK001166 | B.226980_at | 5.4977 | 60.4 | 0.033734 |
| 93 | Hs.238205 | Chromosome 6 open reading frame 115 | C6orf115 | AF116682 | B.223361_at | 8.7555 | 60.1 | 0.003347 |
| 94 | Hs.27860 | Prostaglandin E receptor 3 (subtype EP3) | | AW242315 | A.213933_at | 7.3561 | 59.8 | 0.256699 |
| 95 | Hs.292511 | Neuro-oncological ventral antigen 1 | NOVA1 | NM_002515 | A.205794_s_at | 6.7682 | 59.5 | 0.010617 |
| 96 | Hs.276466 | Hypothetical protein FLJ21062 | FLJ21062 | NM_024788 | A.219455_at | 5.5257 | 59.3 | 0.003021 |
| 97 | Hs.270845 | Kinesin family member 23 | KIF23 | NM_004856 | A.204709_s_at | 5.1731 | 59.3 | 0.15391 |
| 98 | Hs.293257 | Epithelial cell transforming sequence 2 oncogene | ECT2 | NM_018098 | A.219787_s_at | 6.8052 | 59.3 | 0.000246 |
| 99 | Hs.156346 | Topoisomerase (DNA) II alpha 170kDa | TOP2A | NM_001067 | A.201292_at | 7.2468 | 59.1 | 0.011073 |
| 100 | Hs.31297 | Cytochrome b reductase 1 | CYBRD1 | AL136693 | B.222453_at | 9.3991 | 59.1 | 0.001036 |
| 101 | Hs.414407 | Kinetochore associated 2 | KNTC2 | NM_006101 | A.204162_at | 6.017 | 58.7 | 0.076227 |
| 102 | Hs.445098 | DEP domain containing 1 | SDP35 | AI810054 | B.235545_at | 6.2495 | 58.7 | 0.133208 |
| 103 | Hs.301052 | Kinesin family member 18A | DKFZP434 G2226 | NM_031217 | A.221258_s_at | 5.3649 | 58.2 | 0.157731 |
| 104 | Hs.431762 | Tetratricopeptide repeat domain 18 | LOC118491 | AW024437 | B.229170_s_at | 6.2298 | 58.2 | 0.065188 |
| 105 | Hs.24529 | CHK1 checkpoint homolog (S. pombe) | CHEK1 | NM_001274 | A.205394_at | 5.6217 | 58.1 | 0.016515 |
| 106 | Hs.87507 | BRCA1 interacting protein C-terminal helicase 1 | BRIP1 | BF056791 | B.235609_at | 7.1489 | 58.1 | 0.010814 |
| 107 | Hs.348920 | FSH primary response (LRPR1 homolog, rat) 1 | FSHPRH1 | BF793446 | A.214804_at | 5.0105 | 57.8 | 0.056646 |
| 108 | Hs.127797 | CDNA FLJ11381 fis, clone HEMBA1000501 | | AI807356 | B.227350_at | 6.8658 | 57.8 | 0.014086 |
| 109 | Hs.92458 | G protein-coupled receptor 19 | GPR19 | NM_006143 | A.207183_at | 5.2568 | 57.6 | 0.001708 |
| 110 | Hs.552 | Steroid-5-alpha-reductase, alpha polypeptide 1 (3-oxo-5 alpha-steroid delta 4-dehydrogenase alpha 1) | SRD5A1 | BC006373 | A.211056_s_at | 6.7605 | 57.6 | 0.00075 |
| 111 | Hs.435733 | Cell division cycle associated 7 | CDCA7 | AY029179 | B.224428_s_at | 7.6746 | 57.6 | 0.020822 |
| 112 | Hs.101174 | Microtubule-associated protein tau | MAPT | NM_016835 | A.203929_s_at | 7.7914 | 57.6 | 0.003067 |
| 113 | Hs.436376 | Synaptotagmin binding, cytoplasmic RNA interacting protein | SYNCRIP | NM_006372 | A.217834_s_at | 6.8123 | 57.6 | 0.000586 |
| 114 | Hs.122552 | G-2 and S-phase expressed 1 | GTSE1 | NM_016426 | A.204315_s_at | 6.4166 | 57.5 | 0.036289 |
| 115 | Hs.153704 | NIMA (never in mitosis gene a)-related kinase 2 | NEK2 | NM_002497 | A.204641_at | 7.0017 | 57.5 | 0.03551 |
| 116 | Hs.208912 | Chromosome 22 open reading frame 18 | C22orf18 | NM_024053 | A.218741_at | 6.3488 | 56.8 | 0.006304 |
| 117 | Hs.81892 | KIAA0101 | KIAA0101 | NM_014736 | A.202503_s_at | 8.2054 | 56.6 | 0.029102 |
| 118 | Hs.279905 | Nucleolar and spindle associated protein 1 | NUSAP1 | NM_016359 | A.218039_at | 7.542 | 56.6 | 0.005918 |
| 119 | Hs.170915 | Hypothetical protein FLJ10948 | FLJ10948 | NM_018281 | A.218552_at | 7.9778 | 56.0 | 0.00983 |
| 120 | Hs.144151 | Transcribed locus | | AI668620 | B.237339_at | 9.6693 | 56.0 | 0.028527 |
| 121 | Hs.433180 | DNA replication complex GINS protein PSF2 | Pfs2 | BC003186 | A.221521_s_at | 6.3209 | 56.0 | 0.058903 |
| 122 | Hs.47504 | Exonuclease 1 | EXO1 | NM_003686 | A.204603_at | 5.927 | 56.0 | 0.001031 |
| 123 | Hs.293257 | Epithelial cell transforming sequence 2 oncogene | ECT2 | BG170335 | B.234992_x_at | 5.1653 | 55.6 | 0.001881 |
| 124 | Hs.385913 | Acidic (leucine-rich) nuclear phosphoprotein 32 family, member E | ANP32E | NM_030920 | A.208103_s_at | 6.2989 | 55.6 | 0.001331 |
| 125 | Hs.44380 | Transcribed locus, weakly similar to NP_060312.1 hypothetical protein FLJ20489 [Homo sapiens] | | AA938184 | B.236312_at | 5.7016 | 55.6 | 0.007219 |
| 126 | Hs.19322 | Chromosome 9 open reading frame 140 | LOC89958 | AW250904 | B.225777_at | 7.8877 | 55.2 | 0.003266 |
| 127 | Hs.188173 | Lymphoid nuclear protein related to AF4 | | AA572675 | B.232286_at | 7.169 | 55.2 | 0.008402 |
| 128 | Hs.28264 | Chromosome 10 open reading frame 56 | FLJ90798 | AL049949 | A.212419_at | 7.6504 | 55.2 | 0.017182 |
| 129 | Hs.387057 | Hypothetical protein FLJ13710 | FLJ13710 | AK024132 | B.232944_at | 6.1947 | 55.2 | 0.03374 |
| 130 | acc_AL031658 | | | AL031658 | B.232357_at | 5.9761 | 54.9 | 0.032742 |
| 131 132 | Hs.286049 | Phosphoserine aminotransferase 1 | PSAT1 | BC004863 | B.223062_s_at | 6.1035 | 54.9 | 0.003426 |
| | Hs.19173 | Nucleoporin 88kDa | | AI806781 | B.235786_at | 7.2856 | 54.9 | 0.036867 |
| 133 | Hs.155223 | Stanniocalcin 2 | STC2 | BC000658 | A.203439_s_at | 7.6806 | 54.8 | 0.039627 |
| 134 | acc_NM_03089 6.1 | | | NM_030896 | A.221275_s_at | 3.9611 | 54.8 | 0.001787 |
| 135 | Hs.101174 | Microtubule-associated protein tau | MAPT | AA199717 | B.225379_at | 7.8574 | 54.8 | 0.021421 |
| 136 | Hs.446680 | Retinoic acid induced 2 | RAI2 | NM_021785 | A.219440_at | 6.6594 | 54.3 | 0.057037 |
| 137 | Hs.431762 | Tetratricopeptide repeat domain 18 | LOC118491 | AW024437 | B.229169_at | 5.8266 | 53.6 | 0.002367 |
| 138 | acc_NM_00519 6.1 | | | NM_005196 | A.207828_s_at | 7.237 | 53.1 | 0.007336 |
| 139 | acc_T90295 | Arsenic transactivated protein 1 | | T90295 | B.226661_at | 6.6825 | 52.8 | 0.001873 |
| 140 | Hs.42650 | ZW10 interactor | ZWINT | NM_007057 | A.204026_s_at | 7.5055 | 52.7 | 0.033812 |
| 141 | Hs.6641 | | KIF5C | NM_004522 | A.203130_s_at | 7.3214 | 52.7 | 0.012878 |
| 142 | Hs.23960 | Cyclin B1 | CCNB1 | N90191 | B.228729_at | 6.8018 | 52.6 | 0.031361 |
| 143 | Hs.72550 | Hyaluronan-mediated motility receptor (RHAMM) | HMMR | NM_012485 | A.207165_at | 6.5885 | 52.4 | 0.065936 |
| 144 | Hs.73239 | Hypothetical protein FLJ10901 | FLJ10901 | NM_018265 | A.219010_at | 6.9429 | 52.3 | 0.020279 |
| 145 | Hs.163533 | V-erb-a erythroblastic leukemia viral oncogene homolog 4 (avian) | | AK024204 | B.233498_at | 7.5435 | 52.2 | 0.002319 |
| 146 | Hs.109706 | Hematological and neurological expressed 1 | HN1 | AF060925 | B.222396_at | 8.4225 | 52.2 | 0.000387 |
| 147 | Hs.165258 | Nuclear receptor subfamily 4, group A, member 2 | | AA523939 | B.235739_at | 7.1874 | 52.0 | 0.000444 |
| 148 | Hs.20575 | Growth arrest-specific 2 like 3 | LOC283431 | H37811 | B.235709_at | 6.7278 | 51.9 | 0.009763 |
| 149 | Hs.75678 | FBJ murine osteosarcoma viral oncogene homolog B | FOSB | NM_006732 | A.202768_at | 6.1922 | 51.9 | 0.059132 |
| 150 | Hs.437351 | Cold inducible RNA binding protein | CIRBP | AL565767 | B.225191_at | 8.033 | 51.9 | 0.00158 |
| 151 | Hs.57101 | MCM2 minichromosome maintenance deficient 2, mitotin (S. cerevisiae) | MCM2 | NM_004526 | A.202107_s_at | 7.861 | 51.7 | 0.27277 |
| 152 | Hs.326736 | Ankyrin repeat domain 30A | NY-BR-1 | AF269087 | B.223864_at | 9.4144 | 51.3 | 0.042111 |
| 153 | Hs.298646 | ATPase family, AAA domain containing 2 | PRO2000 | AI925583 | B.222740_at | 6.8416 | 50.8 | 0.130085 |
| 154 | Hs.119192 | H2A histone family, member Z | H2AFZ | NM_002106 | A.200853_at | 8.5896 | 50.1 | 0.007836 |
| 155 | Hs.119960 | PHD finger protein 19 | PHF19 | BE544837 | B.227211_at | 6.3487 | 50.1 | 0.084007 |
| 156 | Hs.78619 | Gamma-glutamyl hydrolase (conjugase, folylpolygammaglutamyl | hydrolase) GGH | NM_003878 | A.203560_at | 6.7708 | 49.9 | 0.006283 |
| 157 | Hs.283532 | Uncharacterized bone marrow protein BM039 | BM039 | NM_018455 | A.219555_s_at | 4.1739 | 49.9 | 0.13406 |
| 158 | Hs.221941 | Cytochrome b reductase 1 | | A1669804 | B.232459_at | 7.1171 | 49.9 | 0.01473 |
| 159 | Hs.104019 | Transforming, acidic coiled-coil containing protein 3 | TACC3 | NM_006342 | A.218308_at | 6.1303 | 49.8 | 0.022905 |
| 160 | acc_AK002203. 1 | | | AK002203 | B.226992_at | 7.9091 | 49.7 | 0.036845 |
| 161 | Hs.28625 | Transcribed locus | | AI693516 | B.228750_at | 7.1249 | 49.6 | 0.055282 |
| 162 | Hs.206868 | B-cell CLL/lymphoma 2 | | AU146384 | B.232210_at | 8.0948 | 49.6 | 0.002178 |
| 163 | Hs.75528 | Dynein, axonemal, light intermediate polypeptide 1 | HUMAUAN TIG | AW299538 | B.227081_at | 7.0851 | 49.5 | 0.003326 |
| 164 | acc_AW271106 | | | AW271106 | B.229490_s_at | 6.2222 | 49.5 | 0.017341 |
| 165 | Hs.298646 | ATPase family, AAA domain containing 2 | PRO2000 | AI139629 | B.235266_at | 6.1913 | 49.5 | 0.009434 |
| 166 | Hs.303090 | Protein phosphatase 1, regulatory (inhibitor) subunit 3C | PPP1R3C | N26005 | A.204284_at | 7.0275 | 49.5 | 0.011239 |
| 167 | Hs.83169 | Matrix metalloproteinase 1 (interstitial collagenase) | MMP1 | NM_002421 | A.204475_at | 7.1705 | 49.4 | 0.027959 |
| 168 | Hs.441708 | Leucine-rich repeat kinase 1 | MGC45866 | AI638593 | B.230021_at | 6.424 | 49.4 | 0.005067 |
| 169 | acc_AV733950 | | | AV733950 | A.201693_s_at | 7.9061 | 48.8 | 0.004273 |
| 170 | Hs.171695 | Dual specificity phosphatase 1 | DUSP1 | NM_004417 | A.201041_s_at | 9.7481 | 48.7 | 0.002971 |
| 171 | Hs.87491 | Thymidylate synthetase | TYMS | NM_001071 | A.202589_at | 7.8242 | 48.7 | 0.040774 |
| 172 | Hs.434886 | Cell division cycle associated 5 | CDCA5 | BE614410 | B.224753_at | 4.9821 | 48.5 | 0.106362 |
| 173 | Hs.24395 | Chemokine (C-X-C motif) ligand 14 | CXCL14 | NM_004887 | A.218002_s_at | 8.2513 | 48.2 | 0.002571 |
| 174 | Hs.104741 | T-LAK cell-originated protein kinase | TOPK | NM_018492 | A.219148_at | 6.4626 | 48.2 | 0.001405 |
| 175 | Hs.272027 | F-box protein 5 | FBXO5 | AK026197 | B.234863_x_at | 6.935 | 48.2 | 0.036746 |
| 176 | Hs.101174 | Microtubule-associated protein tau | MAPT | J03778 | A.206401_s_at | 6.4557 | 48.2 | 0.020545 |
| 177 | Hs.7888 | V-erb-a erythroblastic leukemia viral oncogene homolog 4 (avian) | | AW772192 | A.214053_at | 7.0744 | 48.2 | 0.028848 |
| 178 | Hs.372254 | Lymphoid nuclear protein related to AF4 | | AI033582 | B.244696_at | 7.4158 | 48.2 | 0.001898 |
| 179 | Hs.435861 | Signal peptide, CUB domain, EGF-like 2 | SCUBE2 | AI424243 | A.219197_s_at | 8.3819 | 48.0 | 0.037351 |
| 180 | Hs.385998 | WD repeat and HMG-box DNA binding protein 1 | WDHD1 | AK001538 | A.216228_s_at | 4.541 | 47.7 | 0.000561 |
| 181 | Hs.306322 | Neuron navigator 3 | NAV3 | NM_014903 | A.204823_at | 5.8235 | 47.7 | 0.003778 |
| 182 | Hs.21380 | CDNA FLJ36725 fis, clone UTERU2012230 | | AV709727 | B.225996_at | 7.5715 | 47.6 | 0.038219 |
| 183 | Hs.89497 | Lamin B1 | LMNB1 | NM_005573 | A.203276_at | 7.11 | 47.3 | 0.003693 |
| 184 | acc_NM_01766 9.1 | | | NM_017669 | A.219650_at | 5.0422 | 47.3 | 0.003906 |
| 185 | Hs.12532 | Chromosome 1 open reading frame 21 | C1orf21 | NM_030806 | A.221272_s_at | 5.6228 | 47.1 | 0.06632 |
| 186 | Hs.399966 | Calcium channel, voltage-dependent, L type, alpha 1D subunit | CACNA1D | BE550599 | A.210108_at | 6.2612 | 47.0 | 0.063467 |
| 187 | Hs.159264 | Clone 23948 mRNA sequence | | U79293 | A.215304_at | 6.9317 | 47.0 | 0.066157 |
| 188 | Hs.212787 | KIAA0303 protein | KIAA0303 | AW971134 | A.222348_at | 4.964 | 47.0 | 0.002269 |
| 189 | Hs.325650 | EH-domain containing 2 | EHD2 | AI417917 | A.221870_at | 6.4774 | 46.0 | 0.001916 |
| 190 | Hs.388347 | Hypothetical protein LOC143381 | | AW242720 | B.227550_at | 7.657 | 45.3 | 0.001238 |
| 191 | Hs.283853 | MRNA full length insert cDNA clone EUROIMAGE 980547 | | AL360204 | B.232855_at | 4.6288 | 45.3 | 0.00605 |
| 192 | Hs.57301 | High mobility group AT-hook 1 | HMGA1 | NM_002131 | A.206074_s_at | 7.6723 | 44.9 | 0.001416 |
| 193 | Hs.529285 | Solute carrier family 40 (iron-regulated transporter), member | | AA588092 | B.239723_at | 6.9222 | 44.8 | 0.051707 |
| 194 | Hs.252938 | Low density lipoprotein-related protein 2 | LRP2 | R73030 | B.230863_at | 7.4648 | 44.7 | 0.003167 |
| 195 | Hs.552 | Steroid-5-alpha-reductase, alpha polypeptide 1 (3-oxo-5 alpha-steroid delta 4-dehydrogenase alpha 1) | SRD5A1 | NM_001047 | A.204675_at | 7.1002 | 44.7 | 0.000327 |
| 196 | Hs.156346 | Topoisomerase (DNA) II alpha 170kDa | TOP2A | NM_001067 | A.201291_s_at | 7.3566 | 44.6 | 0.110228 |
| 197 | Hs.413924 | Chemokine (C-X-C motif) ligand 10 | CXCL10 | NMU_001565 | A.204533_at | 7.9131 | 44.6 | 0.06956 |
| 198 | Hs.287466 | CDNA FLJ11928 fis, clone HEMBB1000420 | | AK021990 | B.232699_at | 5.8675 | 44.6 | 0.001646 |
| 199 | acc_X07868 | | | X07868 | A.202409_at | 7.9917 | 44.5 | 0.001984 |
| 200 | Hs.101174 | Microtubule-associated protein tau | MAPT | NM_016835 | A.203928_x_at | 6.9103 | 44.5 | 0.005431 |
| 201 | Hs.334828 | Hypothetical protein FLJ10719 | FLJ10719 | BG478677 | A.213008_at | 6.4461 | 44.5 | 0.009488 |
| 202 | Hs.326035 | Early growth response 1 | EGR1 | NM_001964 | A.201694_s_at | 8.6202 | 44.2 | 0.024935 |
| 203 | Hs.122552 | G-2 and S-phase expressed 1 | GTSE1 | BF973178 | A.215942_s_at | 5.4688 | 44.2 | 0.041015 |
| 204 | Hs.24395 | Chemokine (C-X-C motif) ligand 14 | CXCL14 | AF144103 | B.222484_s_at | 9.3366 | 44.2 | 0.005525 |
| 205 | Hs.102406 | Melanophilin | | AI810764 | B.229150_at | 8.078 | 44.2 | 0.030939 |
| 206 | Hs.164018 | Leucine zipper protein FKSG14 | FKSG14 | BC005400 | B.222848_at | 6.6517 | 43.8 | 0.001146 |
| 207 | Hs.19114 | High-mobility group box 3 | HMGB3 | NM_005342 | A.203744_at | 7.5502 | 43.7 | 0.007416 |
| 208 | Hs.103982 | Chemokine (C-X-C motif) ligand 11 | CXCL11 | AF002985 | A.211122_s_at | 6.1001 | 43.0 | 0.003299 |
| 209 | Hs.356349 | Transcribed locus | ZNF145 | AI492388 | B.228854_at | 6.8198 | 43.0 | 0.001352 |
| 210 | Hs.1657 | Estrogen receptor 1 | ESR1 | NM_000125 | A.205225_at | 7.4943 | 43.0 | 0.188092 |
| 211 | Hs.144479 | Transcribed locus | | BF433570 | B.237301_at | 6.3171 | 42.8 | 0.003359 |
| 212 | acc_BF508074 | | | BF508074 | B.240465_at | 6.0041 | 42.7 | 0.001555 |
| 213 | Hs.326391 | Phytanoyl-CoA dioxygenase domain containing 1 | PHYHD1 | AL545998 | B.226846_at | 7.2214 | 42.4 | 0.100092 |
| 214 | Hs.338851 | FLJ41238 protein | FLJ41238 | AW629527 | B.229764_at | 6.5319 | 42.3 | 0.032903 |
| 215 | Hs.65239 | Sodium channel, voltage-gated, type IV, beta | SCN4B | AW026241 | B.236359_at | 5.5526 | 42.1 | 0.106317 |
| 216 | Hs.88417 | Sushi domain containing 3 | SUSD3 | AW966474 | B.227182_at | 8.195 | 41.8 | 0.015261 |
| 217 | Hs.16530 | Chemokine (C-C motif) ligand 18 (pulmonary and activation-regulated) | CCL18 | Y13710 | A.32128_at | 6.2442 | 41.3 | 0.003608 |
| 218 | Hs.384944 | Superoxide dismutase 2, mitochondrial | SOD2 | X15132 | A.216841_s_at | 6.0027 | 41.3 | 0.115014 |
| 219 | Hs.406050 | Dynein, axonemal, light intermediate polypeptide 1 | DNALI1 | NM_003462 | A.205186_at | 4.2997 | 40.9 | 0.008737 |
| 220 | Hs.458430 | N-acetyltransferase 1 (arylamine N-acetyltransferase) | NAT1 | NM_000662 | A.214440_at | 7.7423 | 40.8 | 0.001176 |
| 221 | Hs.437023 | Nucleoporin 62kDa | IL4I1 | AI859620 | B.230966_at | 6.4289 | 40.6 | 0.041224 |
| 222 | Hs.279905 | Nucleolar and spindle associated protein 1 | NUSAP1 | NM_018454 | A.219978_s_at | 6.3357 | 40.1 | 0.011365 |
| 223 | Hs.505337 | Claudin 5 (transmembrane protein deleted in velocardiofacial syndrome) | CLDN5 | NM_003277 | A.204482_at | 6.1516 | 40.1 | 0.00138 |
| 224 | Hs.44227 | Heparanase | HPSE | NM_006665 | A.219403_s_at | 5.2989 | 40.0 | 0.252507 |
| 225 | Hs.512555 | Collagen, type XIV, alpha 1 (undulin) | COL14A1 | BF449063 | A.212865_s_at | 7.2876 | 40.0 | 0.00.117 |
| 226 | Hs.511950 | Sirtuin (silent mating type information regulation 2 homolog) 3 (S. cerevisiae) | SIRT3 | AF083108 | A.221562_s_at | 5.9645 | 40.0 | 0.018847 |
| 227 | Hs.371357 | RNA binding motif, single stranded interacting protein | | AW338699 | B.241789_at | 6.3656 | 40.0 | 0.009148 |
| 228 | Hs.81131 | Guanidinoacetate N-methyltransferase | GAMT | NM_000156 | A.205354_at | 5.9474 | 39.9 | 0.005094 |
| 229 | Hs.158992 | FLJ45983 protein | | AI631850 | B.240192_at | 5.2898 | 39.9 | 0.344219 |
| 230 | Hs.104624 | Aquaporin 9 | AQP9 | NM_020980 | A.205568_at | 4.9519 | 39.8 | 0.010084 |
| 231 | Hs.437867 | Homo sapiens, clone IMAGE:5759947, mRNA | | AW970881 | A.222314_x_at | 5.2505 | 39.8 | 0.042065 |
| 232 | Hs.296049 | Microfibrillar-associated protein 4 | MFAP4 | R72286 | A.212713_at | 6.5149 | 39.7 | 0.001482 |
| 233 | Hs.109439 | Osteoglycin (osteoinductive factor, mimecan) | OGN | NM_014057 | A.218730_s_at | 4.9325 | 39.7 | 0.014665 |
| 234 | Hs.29190 | Hypothetical protein MGC24047 | MGC24047 | AI732488 | B.229381_at | 7.2281 | 39.7 | 0.068574 |
| 235 | Hs.252418 | Elastin (supravalvular aortic stenosis, Williams-Beuren syndrome) | ELN | AA479278 | A.212670_at | 6.8951 | 39.5 | 0.148698 |
| 236 | Hs.252938 | Low density lipoprotein-related protein 2 | LRP2 | NM_004525 | A.205710_at | 5.9845 | 39.2 | 0.003389 |
| 237 | Hs.32405 | MRNA; cDNA DKFZp586G0321 (from clone DKFZp586G0321) | | AL137566 | B.228554_at | 7.1124 | 38.6 | 0.014875 |
| 238 | Hs.288720 | Leucine rich repeat containing 17 | LRRC17 | NM_005824 | A.205381_at | 7.217 | 38.5 | 0.278881 |
| 239 | Hs.203963 | Helicase, lymphoid-specific | HELLS | NM_018063 | A.220085_at | 5.2886 | 38.5 | 0.001189 |
| 240 | Hs.361171 | Placenta-specific 9 | PLAC9 | AW964972 | B.227419_x_at | 6.689 | 38.2 | 0.000231 |
| 241 | Hs.396595 | Flavin containing monooxygenase 5 | FMO5 | AK022172 | A.215300_s_at | 4.1433 | 37.5 | 0.00184 |
| 242 | Hs.105434 | Interferon stimulated gene 20kDa | ISG20 | NM_002201 | A.204698_at | 6.2999 | 37.4 | 0.002793 |
| 243 | Hs.460184 | MCM4 minichromosome maintenance deficient 4 (S. cerevisiae) | MCM4 | X74794 | A.212141_at | 6.7292 | 36.6 | 0.175849 |
| 244 | Hs.169266 | Neuropeptide Y receptor Y1 | NPY1R | NM_000909 | A.205440_s_at | 5.8305 | 36.0 | 0.011114 |
| 245 | acc_R38110 | | | R38110 | B.240112_at | 5.1631 | 35.4 | 0.020648 |
| 246 | Hs.63931 | Dachshund homolog 1 (Drosophila) | DACH | AI650353 | B.228915_at | 7.6716 | 35.3 | 0.318902 |
| 247 | Hs.102541 | Netrin 4 | NTN4 | AF278532 | B.223315_at | 8.2693 | 35.2 | 0.132405 |
| 248 | Hs.418367 | Neuromedin U | NMU | NM_006681 | A.206023_at | 5.1017 | 34.6 | 0.03508 |
| 249 | Hs.232127 | MRNA; cDNA DKFZp547P042 (from clone DKFZp547P042) | | AL512727 | A.215014_at | 4.8334 | 34.6 | 0.035434 |
| 250 | Hs.212088 | Epoxide hydrolase 2, cytoplasmic | EPHX2 | AF233336 | A.209368_at | 6.4031 | 34.5 | 0.153812 |
| 251 | Hs.439760 | Cytochrome P450, family 4, subfamily X, polypeptide 1 | CYP4X1 | AA557324 | B.227702_at | 8.5972 | 34.5 | 0.015323 |
| 252 | acc_BF513468 | | | BF513468 | B.241505_at | 7.1517 | 34.1 | 0.001404 |
| 253 | Hs.413078 | Nudix (nucleoside diphosphate linked moiety X)-type motif 1 | NUDT1 | NM_002452 | A.204766_s_at | 5.6705 | 34.0 | 0.069005 |
| 254 | acc_AI492376 | | | AI492376 | B.231195_at | 5.1967 | 33.6 | 0.029021 |
| 255 | acc_AW512787 | | | AW512787 | B.238481_at | 8.5117 | 33.6 | 0.004714 |
| 256 | Hs.74369 | Integrin, alpha 7 | ITGA7 | AK022548 | A.216331_at | 5.1535 | 33.3 | 0.003271 |
| 257 | Hs.63931 | Dachshund homolog 1 (Drosophila) | DACH | NM_004392 | A.205472_s_at | 3.9246 | 33.2 | 0.001985 |
| 258 | Hs.225952 | Protein tyrosine phosphatase, receptor type, T | PTPRT | NM_007050 | A.205948_at | 6.7634 | 32.2 | 0.190046 |
| 259 | acc_BF793701 | Musculoskeletal, embryonic nuclear protein 1 | | BF793701 | B.226856_at | 5.5626 | 31.8 | 0.002068 |
| 260 | Hs.283417 | Transcribed locus | | AI826437 | B.229975_at | 6.381 | 31.3 | 0.008528 |
| 261 | Hs.21948 | Zinc finger protein 533 | | H15261 | B.243929_at | 4.7165 | 30.3 | 0.14416 |
| 262 | Hs.31297 | Cytochrome b reductase 1 | CYBRD1 | NM_024843 | A.217889_s_at | 5.6427 | 27.6 | 0.055739 |
| 263 | Hs.180142 | Calmodulin-like 5 | CALML5 | NM_017422 | A.220414_at | 5.994 | 27.4 | 0.008616 |
| 264 | Hs.176588 | Cytochrome P450, family 4, subfamily Z, polypeptide 1 | CYP4Z1 | AV700083 | B.237395_at | 8.7505 | 24.4 | 0.399969 |

### APPENDIX 1A

### SWS Classifier 0 Accuracy G1 vs G3

**Accuracy: G1 vs**
**G3**
G1=63/68 (92.6%)
G3=51/55 (92.7%)

| Number | Patient ID | Histolgic grade | Probability for G1 | Probability for G3 | Predicted grade |
|---|---|---|---|---|---|
| 1 | X100B08 | 1 | **0.956** | 0.044 | 1 |
| 2 | X209C10 | 1 | **0.930** | 0.070 | 1 |
| 3 | X21C28 | 1 | **0.941** | 0.059 | 1 |
| 4 | X220C70 | 1 | **0.941** | 0.059 | 1 |
| 5 | X224C93 | 1 | **0.834** | 0.166 | 1 |
| 6 | X227C50 | 1 | **0.950** | 0.050 | 1 |
| 7 | X229C44 | 1 | **0.917** | 0.083 | 1 |
| 8 | X231C80 | 1 | **0.860** | 0.140 | 1 |
| 9 | X233C91 | 1 | **0.958** | 0.042 | 1 |
| 10 | X235C20 | 1 | 0.231 | **0.769** | 3 |
| 11 | X236C55 | 1 | **0.955** | 0.045 | 1 |
| 12 | X114B68 | 1 | **0:502** | 0.498 | 1 |
| 13 | X243C70 | 1 | **0.951** | 0.049 | 1 |
| 14 | X246C75 | 1 | **0.950** | 0.050 | 1 |
| 15 | X248C91 | 1 | **0.956** | 0.044 | 1 |
| 16 | X253C20 | 1 | **0.948** | 0.052 | 1 |
| 17 | X259C74 | 1 | **0.949** | 0.051 | 1 |
| 18 | X261C94 | 1 | **0.952** | 0.048 | 1 |
| 19 | X262C85 | 1 | **0.924** | 0.076 | 1 |
| 20 | X263C82 | 1 | **0.955** | 0.045 | 1 |
| 21 | X266C51 | 1 | **0.950** | 0.050 | 1 |
| 22 | X267C04 | 1 | **0.628** | 0.372 | 1 |
| 23 | X282C51 | 1 | **0.942** | 0.058 | 1 |
| 24 | X284C63 | 1 | **0.923** | 0.077 | 1 |
| 25 | X289C75 | 1 | **0.958** | 0.042 | 1 |
| 26 | X28C76 | 1 | **0.927** | 0.073 | 1 |
| 27 | X294C04 | 1 | 0.310 | **0.690** | 3 |
| 28 | X309C49 | 1 | 0.013 | **0.987** | 3 |
| 29 | X316C65 | 1 | **0:952** | 0.048 | 1 |
| 30 | X128B48 | 1 | **0.962** | 0.038 | 1 |
| 31 | X33C30 | 1 | **0.945** | 0.055 | 1 |
| 32 | X39C24 | 1 | **0.935** | 0.065 | 1 |
| 33 | X42C57 | 1 | **0.912** | 0.088 | 1 |
| 34 | X45A96 | 1 | **0.844** | 0.156 | 1 |
| 35 | X48A46 | 1 | **0.942** | 0.058 | 1 |
| 36 | X49A07 | 1 | **0.886** | 0.114 | 1 |
| 37 | X52A90 | 1 | **0.954** | 0.046 | 1 |
| 38 | X61A53 | 1 | **0.878** | 0.122 | 1 |
| 39 | X65A68 | 1 | **0.888** | 0.112 | 1 |
| 40 | X6B85 | 1 | 0.212 | **0.788** | 3 |
| 41 | X72A92 | 1 | **0.867** | 0.133 | 1 |
| 42 | X135B40 | 1 | **0.901** | 0.099 | 1 |
| 43 | X74A63 | 1 | **0.635** | 0.365 | 1 |
| 44 | X83A37 | 1 | **0:779** | 0.221 | 1 |
| 45 | X8B87 | 1 | **0.949** | 0.051 | 1 |
| 46 | X99A50 | 1 | **0.767** | 0.233 | 1 |
| 47 | X138B34 | 1 | **0.956** | 0.044 | 1 |
| 48 | X155B52 | 1 | **0.961** | 0.039 | 1 |
| 49 | X156B01 | 1 | **0.962** | 0.038 | 1 |
| 50 | X160B16 | 1 | **0.956** | 0.044 | 1 |
| 51 | X163B27 | 1 | **0.945** | 0.055 | 1 |
| 52 | X105B13 | 1 | **0.877** | 0.123 | 1 |
| 53 | X173B43 | 1 | **0.959** | 0.041 | 1 |
| 54 | X174B41 | 1 | **0.910** | 0.090 | 1 |
| 55 | X177B67 | 1 | **0.958** | 0.042 | 1 |
| 56 | X106B55 | 1 | **0.940** | 0.060 | 1 |
| 57 | X180B38 | 1 | **0.948** | 0.052 | 1 |
| 58 | X181B70 | 1 | **0.834** | 0.166 | 1 |
| 59 | X184B38 | 1 | **0.936** | 0.064 | 1 |
| 60 | X185B44 | 1 | **0.943** | 0.057 | 1 |
| 61 | X10B88 | 1 | 0:444 | **0.556** | 3 |
| 62 | X192B69 | 1 | **0.960** | 0.040 | 1 |
| 63 | X195B75 | 1 | **0.916** | 0.084 | 1 |
| 64 | X196B81 | 1 | **0.868** | 0.132 | 1 |
| 65 | X19C33 | 1 | **0.690** | 0.310 | 1 |
| 66 | X204B85 | 1 | **0.948** | 0.052 | 1 |
| 67 | X205B99 | 1 | **0.570** | 0.430 | 1 |
| 68 | X207C08 | 1 | **0.921** | 0.079 | 1 |
| 69 | X111B51 | 3 | 0,043 | **0.957** | 3 |
| 70 | X222C26 | 3 | **0.680** | 0.320 | 1 |
| 71 | X226C06 | 3 | 0.013 | **0.987** | 3 |
| 72 | X113B11 | 3 | 0.077 | **0.923** | 3 |
| 73 | X232C58 | 3 | 0.040 | **0.960** | 3 |
| 74 | X234C15 | 3 | 0.086 | **0.914** | 3 |
| 75 | X238C87 | 3 | 0.153 | **0.847** | 3 |
| 76 | X241C01 | 3 | 0.035 | **0.965** | 3 |
| 77 | X249C42 | 3 | 0.036 | **0.964** | 3 |
| 78 | X250C78 | 3 | 0:039 | **0.961** | 3 |
| 79 | X252C64 | 3 | 0.033 | **0.967** | 3 |
| 80 | X269C68 | 3 | 0.015 | **0.985** | 3 |
| 81 | X26C23 | 3 | 0.250 | **0.750** | 3 |
| 82 | X270C93 | 3 | 0.028 | **0.972** | 3 |
| 83 | X271C71 | 3 | 0.065 | **0.935** | 3 |
| 84 | X279C61 | 3 | 0.024 | **0.976** | 3 |
| 85 | X287C67 | 3 | 0.045 | **0.955** | 3 |
| 86 | X291C17 | 3 | 0.015 | **0.985** | 3 |
| 87 | X127B00 | 3 | 0.026 | **0.974** | 3 |
| 88 | X303C36 | 3 | 0.017 | **0.983** | 3 |
| 89 | X304C89 | 3 | **0.961** | 0.039 | 1 |
| 90 | X311A27 | 3 | 0.041 | **0.959** | 3 |
| 91 | X313A87 | 3 | 0.024 | **0.976** | 3 |
| 92 | X314B55 | 3 | 0.016 | **0.984** | 3 |
| 93 | X101B88 | 3 | 0.014 | **0.986** | 3 |
| 94 | X37C06 | 3 | 0.030 | **0.970** | 3 |
| 95 | X46A25 | 3 | 0:044 | **0.956** | 3 |
| 96 | X131B79 | 3 | 0.151 | **0.849** | 3 |
| 97 | X54A09 | 3 | 0.013 | **0.987** | 3 |
| 98 | X55A79 | 3 | 0.075 | **0.925** | 3 |
| 99 | X62A02 | 3 | 0.018 | **0.982** | 3 |
| 100 | X66A84 | 3 | 0.019 | **0.981** | 3 |
| 101 | X67A43 | 3 | 0.020 | **0.980** | 3 |
| 102 | X69A93 | 3 | 0.084 | **0.916** | 3 |
| 103 | X70A79 | 3 | 0.016 | **0.984** | 3 |
| 104 | X73A01 | 3 | 0.324 | **0.676** | 3 |
| 105 | X76A44 | 3 | 0.123 | **0.877** | 3 |
| 106 | X79A35 | 3 | 0.048 | **0.952** | 3 |
| 107 | X82A83 | 3 | 0.235 | **0.765** | 3 |
| 108 | X89A64 | 3 | 0.015 | **0.985** | 3 |
| 109 | X90A63 | 3 | 0.031 | **0.969** | 3 |
| 110 | X139B03 | 3 | 0.133 | **0.867** | 3 |
| 111 | X102B06 | 3 | 0.034 | **0.966** | 3 |
| 112 | X142B05 | 3 | 0.037 | **0.963** | 3 |
| 113 | X143B81 | 3 | 0.073 | **0.927** | 3 |
| 114 | X146B39 | 3 | 0.015 | **0.985** | 3 |
| 115 | X147B19 | 3 | 0.037 | **0.963** | 3 |
| 116 | X103B41 | 3 | 0.016 | **0.984** | 3 |
| 117 | X153B09 | 3 | 0.023 | **0.977** | 3 |
| 118 | X104B91 | 3 | 0.104 | **0.896** | 3 |
| 119 | X162B98 | 3 | **0:503** | 0.497 | 1 |
| 120 | X172B19 | 3 | 0.079 | **0.921** | 3 |
| 121 | X182B43 | 3 | 0.014 | **0.986** | 3 |
| 122 | X194B60 | 3 | 0.030 | **0.970** | 3 |
| 123 | X200B47 | 3 | **0.951** | 0.049 | 1 |

### APPENDIX 2

SWS Classifier 0: Prediction of genetic G2a and G2b tumour sub-types based on 264 gene classifier

| **Order** | **Patient ID** | **Histologic grade** | **Probability for G2a** | **Probability for G2b** | **Predicted grade** |
|---|---|---|---|---|---|
| 1 | X210C72 | 2 | 0.404 | **0.596** | 2b |
| 2 | X211C88 | 2 | 0.445 | 0.555 | 2b |
| 3 | X212C21 | 2 | **0.959** | 0.041 | 2a |
| 4 | X213C36 | 2 | 0.333 | **0.667** | 2b |
| 5 | X216C61 | 2 | **0.856** | 0.144 | 2a |
| 6 | X217C79 | 2 | **0.943** | 0.057 | 2a |
| 7 | X218C29 | 2 | **0.805** | 0.195 | 2a |
| 8 | X112B55 | 2 | 0.337 | **0.663** | 2b |
| 9 | X221C14 | 2 | **0.612** | 0.388 | 2a |
| 10 | X223C51 | 2 | **0.818** | 0.182 | 2a |
| 11 | X225C52 | 2 | 0.055 | **0.945** | 2b |
| 12 | X22C62 | 2 | **0.82** | 0.18 | 2a |
| 13 | X230C47 | 2 | 0.042 | **0.958** | 2b |
| 14 | X237C56 | 2 | 0.046 | **0.954** | 2b |
| 15 | X23C52 | 2 | 0.095 | **0.905** | 2b |
| 16 | X240C54 | 2 | 0.157 | **0.843** | 2b |
| 17 | X242C21 | 2 | 0.287 | **0.713** | 2b |
| 18 | X244C89 | 2 | 0.104 | **0.896** | 2b |
| 19 | X245C22 | 2 | 0.142 | **0.858** | 2b |
| 20 | X247C76 | 2 | **0.501** | 0.499 | 2a |
| 21 | X11B47 | 2 | **0.941** | 0.059 | 2a |
| 22 | X24C30 | 2 | **0.924** | 0.076 | 2a |
| 23 | X251C14 | 2 | **0.95** | 0.05 | 2a |
| 24 | X254C80 | 2 | **0.949** | 0.051 | 2a |
| 25 | X255C06 | 2 | **0.905** | 0.095 | 2a |
| 26 | X256C45 | 2 | 0.025 | **0.975** | 2b |
| 27 | X120B73 | 2 | 0.032 | **0.968** | 2b |
| 28 | X257C87 | 2 | **0.931** | 0.069 | 2a |
| 29 | X258C21 | 2 | **0.958** | 0.042 | 2a |
| 30 | X260C91 | 2 | **0.643** | 0.357 | 2a |
| 31 | X265C40 | 2 | 0.253 | **0.747** | 2b |
| 32 | X122B81 | 2 | **0.933** | 0.067 | 2a |
| 33 | X268C87 | 2 | 0.013 | **0.987** | 2b |
| 34 | X272C88 | 2 | **0.939** | 0.061 | 2a |
| 35 | X274C81 | 2 | **0.918** | 0.082 | 2a |
| 36 | X275C70 | 2 | **0.933** | 0.067 | 2a |
| 37 | X277C64 | 2 | **0.957** | 0.043 | 2a |
| 38 | X124B25 | 2 | **0.921** | 0.079 | 2a |
| 39 | X278C80 | 2 | 0.219 | **0.781** | 2b |
| 40 | X27C82 | 2 | **0.892** | 0.108 | 2a |
| 41 | X280C43 | 2 | **0.957** | 0.043 | 2a |
| 42 | X286C91 | 2 | **0.959** | 0.041 | 2a |
| 43 | X288C57 | 2 | **0.943** | 0.057 | 2a |
| 44 | X290C91 | 2 | **0.945** | 0.055 | 2a |
| 45 | X292C66 | 2 | **0.914** | 0.086 | 2a |
| 46 | X296C95 | 2 | **0.932** | 0.068 | 2a |
| 47 | X297C26 | 2 | **0.945** | 0.055 | 2a |
| 48 | X298C47 | 2 | **0.609** | 0.391 | 2a |
| 49 | X301C66 | 2 | 0.372 | **0.628** | 2b |
| 50 | X307C50 | 2 | **0.752** | 0.248 | 2a |
| 51 | X308C93 | 2 | 0.044 | **0.956** | 2b |
| 52 | X34C80 | 2 | **0.931** | 0.069 | 2a |
| 53 | X35C29 | 2 | **0.872** | 0.128 | 2a |
| 54 | X36C17 | 2 | **0.933** | 0.067 | 2a |
| 55 | X40C57 | 2 | **0.814** | 0.186 | 2a |
| 56 | X41C65 | 2 | **0.859** | 0.141 | 2a |
| 57 | X130B92 | 2 | **0.954** | 0.046 | 2a |
| 58 | X43C47 | 2 | **0.564** | 0.436 | 2a |
| 59 | X44A53 | 2 | **0.696** | 0.304 | 2a |
| 60 | X47A87 | 2 | 0.025 | **0.975** | 2b |
| 61 | X50A91 | 2 | **0.779** | 0.221 | 2a |
| 62 | X51A98 | 2 | 0.386 | **0.614** | 2b |
| 63 | X53A06 | 2 | 0.336 | **0.664** | 2b |
| 64 | X56A94 | 2 | **0.853** | 0.147 | 2a |
| 65 | X58A50 | 2 | 0.017 | **0.983** | 2b |
| 66 | X5B97 | 2 | 0.049 | **0.951** | 2b |
| 67 | X60A05 | 2 | **0.9** | 0.1 | 2a |
| 68 | X134B33 | 2 | 0.197 | **0.803** | 2b |
| 69 | X63A62 | 2 | **0.919** | 0.081 | 2a |
| 70 | X64A59 | 2 | 0.186 | **0.814** | 2b |
| 71 | X75A01 | 2 | **0.506** | 0.494 | 2a |
| 72 | X77A50 | 2 | **0.593** | 0.407 | 2a |
| 73 | X7B96 | 2 | 0.461 | **0.539** | 2b |
| 74 | X84A44 | 2 | 0.127 | **0.873** | 2b |
| 75 | X136B04 | 2 | **0.74** | 0.26 | 2a |
| 76 | X85A03 | 2 | 0.364 | **0.636** | 2b |
| 77 | X86A40 | 2 | 0.02 | **0.98** | 2b |
| 78 | X87A79 | 2 | **0.817** | 0.183 | 2a |
| 79 | X88A67 | 2 | 0.262 | **0.738** | 2b |
| 80 | X94A16 | 2 | **0.957** | 0.043 | 2a |
| 81 | X96A21 | 2 | **0.817** | 0.183 | 2a |
| 82 | X137B88 | 2 | **0.579** | 0.421 | 2a |
| 83 | X9B52 | 2 | **0.712** | 0.288 | 2a |
| 84 | X13B79 | 2 | **0.955** | 0.045 | 2a |
| 85 | X140B91 | 2 | **0.958** | 0.042 | 2a |
| 86 | X144B49 | 2 | **0.87** | 0.13 | 2a |
| 87 | X145B10 | 2 | 0.056 | **0.944** | 2b |
| 88 | X14B98 | 2 | **0.754** | 0.246 | 2a |
| 89 | X150B81 | 2 | **0.914** | 0.086 | 2a |
| 90 | X151B84 | 2 | **0.926** | 0.074 | 2a |
| 91 | X152B99 | 2 | **0.934** | 0.066 | 2a |
| 92 | X154B42 | 2 | 0.07 | **0.93** | 2b |
| 93 | X158B84 | 2 | **0.922** | 0.078 | 2a |
| 94 | X159B47 | 2 | 0.14 | **0.86** | 2b |
| 95 | X15C94 | 2 | **0.944** | 0.056 | 2a |
| 96 | X161B31 | 2 | **0.949** | 0.051 | 2a |
| 97 | X164B81 | 2 | 0.024 | **0.976** | 2b |
| 98 | X165B72 | 2 | 0.384 | **0.616** | 2b |
| 99 | X166B79 | 2 | 0.399 | **0.601** | 2b |
| 100 | X168B51 | 2 | **0.889** | 0.111 | 2a |
| 101 | X169B79 | 2 | **0.751** | 0.249 | 2a |
| 102 | X16C97 | 2 | **0.946** | 0.054 | 2a |
| 103 | X170B15 | 2 | **0.867** | 0.133 | 2a |
| 104 | X171B77 | 2 | 0.05 | **0.95** | 2b |
| 105 | X175B72 | 2 | **0.762** | 0.238 | 2a |
| 106 | X176B74 | 2 | **0.955** | 0.045 | 2a |
| 107 | X178B74 | 2 | **0.814** | 0.186 | 2a |
| 108 | X179B28 | 2 | **0.793** | 0.207 | 2a |
| 109 | X17C40 | 2 | **0.909** | 0.091 | 2a |
| 110 | X183B75 | 2 | **0.834** | 0.166 | 2a |
| 111 | X186B22 | 2 | 0.216 | **0.784** | 2b |
| 112 | X187B36 | 2 | 0.017 | **0.983** | 2b |
| 113 | X188B13 | 2 | 0.384 | **0.616** | 2b |
| 114 | X189B83 | 2 | 0.035 | **0.965** | 2b |
| 115 | X18C56 | 2 | **0.747** | 0.253 | 2a |
| 116 | X191B79 | 2 | 0.038 | **0.962** | 2b |
| 117 | X193B72 | 2 | 0.218 | **0.782** | 2b |
| 118 | X197B95 | 2 | 0.247 | **0.753** | 2b |
| 119 | X198B90 | 2 | **0.943** | 0.057 | 2a |
| 120 | X199B55 | 2 | **0.668** | 0.332 | 2a |
| 121 | X110B34 | 2 | 0.016 | **0.984** | 2b |
| 122 | X201B68 | 2 | **0.884** | 0.116 | 2a |
| 123 | X202B44 | 2 | **0.944** | 0.056 | 2a |
| 124 | X203B49 | 2 | **0.961** | 0.039 | 2a |
| 125 | X206C05 | 2 | **0**.**675** | 0.325 | 2a |
| 126 | X208C06 | 2 | 0.07 | **0.93** | 2b |

### APPENDIX 3

SWS Classifier 0: Tests of differences G2a and G2b by 264 gene classifier

| **Nn** | **GeneSymbol** | **Genbank AccNo** | **Affy ID** | **SWS Cut-off** | **G2a-G2b: U-test, p-value** | **G2a-G2b:t-test, p value** | **hazard ratio** | **survival p value** |
|---|---|---|---|---|---|---|---|---|
| 11 | | BG492359 | B.226936_at | **7.561905** | 8.79E-17 | 1.69E-16 | 1.134468229 | 0.003878804 |
| 77 | FLJ11029 | BG165011 | B.228273_at | **7.706303** | 1.00E-16 | 8.50E-17 | 0.670107381 | 0.076512816 |
| 108 | KIF2C | U63743 | A.209408_at | **7.371746** | 2.81E-16 | 1.78E-16 | 0.567505306 | 0.139342988 |
| 59 | CDC20 | NM_001255 | A.202870_s_at | **7.129081** | 3.34E-16 | 1.12E-16 | 0.763919106 | 0.050953165 |
| 19 | BRRN1 | D38553 | A.212949_at | **5**.**916703** | 3.07E-15 | 3.10E-19 | 0.979515664 | 0.009067157 |
| 30 | LOC146909 | AA292789 | A.222039_at | **6.459052** | 5.69E-15 | 3.50E-16 | 0.883296839 | 0.019272796 |
| 70 | BIRC5 | NM_001168 | A.202095_s_at | **6.890672** | 5.69E-15 | 4.44E-17 | 0.708102857 | 0.064775046 |
| 36 | TRIP13 | NM_004237 | A.204033_at | **7.176822** | 6.43E-15 | 3.00E-15 | 0.850126178 | 0.022566954 |
| 129 | KNTC2 | NM_006101 | A.204162_at | **6.017032** | 7.57E-15 | 1.06E-18 | 0.490312356 | 0.194120238 |
| 110 | TPX2 | AF098158 | A.210052_s_at | **7.405101** | 1.05E-14 | 1.42E-17 | 0.573617337 | 0.142253402 |
| 79 | CDCA8 | BC001651 | A.221520_s_at | **5.218868** | 1.09E-14 | 1.33E-14 | 0.658701923 | 0.078806541 |
| 204 | MCM10 | NM_018518 | A.220651_s_at | **5.678376** | 1.09E-14 | 6.18E-17 | 0.241978243 | 0.517878593 |
| 123 . | MELK | NM_014791 | A.204825_at | **7.107259** | 1.13E-14 | 5.94E-12 | 0.564480902 | 0.182369797 |
| 181 | UBE2C | NM_007019 | A.202954 at | **7.84307** | 1.18E-14 | 1.13E-15 | 0.330908338 | 0.37930096 |
| 71 | DLG7 | NM_014750 | A.203764_at | **6.312237** | 1.91E-14 | 1.00E-16 | 0.690085276 | 0.067402271 |
| 189 | BUB1 | AF043294 | A.209642_at | **6.011844** | 2.16E-14 | 1.92E-16 | 0.307317212 | 0.412526477 |
| 45 | KIF11 | NM_004523 | A.204444 at | **6.4655** | 2.85E-14 | 9.97E-17 | 0.79912997 | 0.033774349 |
| 92 | NUSAP1 | NM_016359 | A.218039 at | **7.542048** | 2.85E-14 | 2.99E-17 | 0.637335706 | 0.097019049 |
| 81 | CCNB2 | NM_004701 | A.202705 at | **7.009613** | 3.77E-14 | 3.42E-14 | 0.657262238 | 0.080389152 |
| 65 | CENPA | NM_001809 | A.204962_s_at | **6.344048** | 4.08E-14 | 3.68E-15 | 0.704184519 | 0.059400118 |
| 153 | TACC3 | NM_006342 | A.218308_at | **6.130286** | 7.36E-14 | 3.10E-18 | 0.412032354 | 0.281085866 |
| 149 | C10orf3 | NM_018131 | A.218542 at | **6.496495** | 1.17E-13 | 9.06E-14 | 0.420069588 | 0.270728962 |
| 1 | TTK | NM_003318 | A.204822 at | **6.239673** | 1.22E-13 | 2.13E-11 | 1.171238059 | 0.001762406 |
| 121 | BUB1B | NM_001211 | A.203755_at | **6.680032** | 1.22E-13 | 1.02E-15 | 0.516583867 | 0.174775842 |
| 87 | KIFC1 | BC000712 | A.209680_s_at | **6.974641** | 1.27E-13 | 1.21E-17 | 0.666825849 | 0.082783088 |
| 57 | PRC1 | NM_003981 | A.218009_s_at | **7.337561** | 1.37E-13 | 2.10E-15 | 0.739645377 | 0.049096515 |
| 113 | RRM2 | NM_001034 | A.201890_at | **7.101362** | 1.43E-13 | 8.41E-17 | 0.546002522 | 0.149339996 |
| 80 | | AI807356 | B.227350_at | **6.865844** | 1.48E-13 | 2.74E-15 | 0.67252443 | 0.080294447 |
| 98 | CENPE | NM_001813 | A.205046_at | **5.197169** | 1.60E-13 | 1.08E-17 | 0.599151091 | 0.113911695 |
| 72 | | AL138828 | B.228069_at | **7.011902** | 1.94E-13 | 5.85E-13 | 0.688832061 | 0.067813492 |
| 35 | RRM2 | BC001886 | A.209773_s_at | **7.297867** | 2.35E-13 | 1.38E-14 | 0.872228422 | 0.021541003 |
| 88 | MCM10 | AB042719 | B.222962_s_at | **6.132775** | 2.35E-13 | 6.93E-133 | 0.654527529 | 0.082868968 |
| 131 | FOXM1 | NM_021953 | A.202580_x_at | **6.582712** | 3.20E-13 | 1.17E-11 | 0.474709695 | 0.205857802 |
| 48 | HMMR | NM_012485 | A.207165_at | **6.588466** | 3.87E-13 | 1.27E-15 | 0.779819603 | 0.035980677 |
| 135 | C15orf20 | AF108138 | B.228252_at | **6.651787** | 5.24E-13 | 1.78E-14 | 0.46154664 | 0.218296542 |
| 224 | | NM_018123 | A.219918_s_at | **6.595823** | 5.65E-13 | 1.99E-14 | 0.187818441 | 0.619909548 |
| 120 | CDKN3 | AF213033 | A.209714_s_at | **6.841428** | 6.09E-13 | 8.44E-14 | 0.515982924 | 0.173720857 |
| 147 | KIAA0101 | NM_014736 | A.202503_s_at | **8.205376** | 7.09E-13 | 2.32E-15 | 0.419483056 | 0.265960679 |
| 103 | TOP2A | NM_001067 | A.201292_at | **7.246792** | 7.93E-13 | 1.91E-12 | 0.580536011 | 0.127083337 |
| 244 | CCNA2 | NM_001237 | A.203418_at | **6.194046** | 9.57E-13 | 7.68E-13 | 0.145722581 | 0.709744105 |
| 260 | MCM6 | NM_005915 | A.201930_at | **7.935338** | 1.07E-12 | 1.16E-11 | 0.052604412 | 0.888772119 |
| 144 | | NM_003158 | A.208079_s_at | **6.652593** | 1.11E-12 | 3.25E-12 | 0.433825107 | 0.249984002 |
| 228 | CDCA3 | BC002551 | B.223307_at | **7.841831** | 1.20E-12 | 5.50E-12 | 0.179511584 | 0.640656915 |
| 32 | RACGAP1 | AU153848 | A.222077_s_at | **7.120661** | 1.24E-12 | 2.34E-14 | 0.913401129 | 0.020315096 |
| 63 | CDC2 | AL524035 | A.203213_at | **7.015218** | 1.34E-12 | 9.22E-15 | 0.735324772 | 0.055865092 |
| 200 | TYMS | NM_001071 | A.202589_at | **7.824209** | 1.39E-12 | 1.51E-13 | 0.263662339 | 0.502055144 |
| 107 | SPAG5 | NM_006461 | A.203145_at | **6.462682** | 1.44E-12 | 2.15E-10 | 0.558587857 | 0.135557314 |
| 105 | | AL135396 | B.225834_at | **7.24667** | 1.67E-12 | 8.09E-13 | 0.564178077 | 0.129238859 |
| 82 | HCAP-G | NM_022346 | A.218663_at | **5.783124** | 1.94E-12 | 1.35E-13 | 0.656424847 | 0.080453666 |
| 28 | KIF20A | NM_005733 | A.218755_at | **7.211537** | 2.33E-12 | 3.05E-12 | 1.045743941 | 0.01613823 |
| 21 | FLJ10719 | BG478677 | A.213008_at | **6.446077** | 2.42E-12 | 3.00E-13 | 0.965117941 | 0.01033584 |
| 245 | LMNB1 | NM_005573 | A.203276_at | **7.110038** | 3.36E-12 | 1.50E-12 | -0.13973266 | 0.719231757 |
| 215 | AURKB | AB011446 | A.209464_at | **5.961137** | 4.18E-12 | 1.56E-12 | 0.221725785 | 0.555668954 |
| 138 | STK6 | NM_003600 | A.204092_s_at | **6.726571** | 4.84E-12 | 5.72E-12 | 0.442828835 | 0.235837295 |
| 33 | CCNB1 | BE407516 | A.214710_s_at | **7.155461** | 5.20E-12 | 5.40E-12 | 0.864913582 | 0.021007755 |
| 119 | ZWINT | NM_007057 | A.204026_s_at | **7.505467** | 6.01E-12 | 1.15E-12 | 0.55129017 | 0.171799897 |
| 226 | HSPC150 | AB032931 | B.223229_at | **7.394742** | 6.95E-12 | 3.63E-13. | 0.183095635 | 0.629346456 |
| 50 | DKFZp762E1312 | NM_018410 | A.218726_at | **6.378121** | 9.59E-12 | 1.09E-12 | 0.773287213 | 0.038624799 |
| 199 | KIF14 | AW183154 | B.236641_at | **6.417492** | 1.07E-11 | 2.06E-13 | 0.255996821 | 0.501112791 |
| 139 | CDC2 | NM_001786 | A.203214_x_at | **6.588012** | 1.19E-11 | 6.90E-12 | 0.474661236 | 0.239070992 |
| 66 | CDC2 | D88357 | A.210559_s_at | **7**.**039539** | 1.42E-11 | 4.29E-13 | 0.738161604 | 0.059693607 |
| 173 | MAD2L1 | NM_002358 | A.203362_s_at | **6.460559** | 1.42E-11 | 1.47E-12 | 0.351480911 | 0.351833246 |
| 46 | HCAP-G | NM_022346 | A.218662_s_at | **6.059402** | 1.47E-11 | 1.20E-11 | 0.794011776 | 0.033909771 |
| 180 | | NM_005196 | A.207828_s_at | **7.236993** | 1.52E-11 | 1.01E-11 | 0.331918842 | 0.374266884 |
| 208 | KIF4A | NM_012310 | A.218355_at | **6.617376** | 1.64E-11 | 3.36E-10 | 0.249364706 | 0.538318296 |
| 95 | C6orf115 | AF116682 | B.223361_at | **8.755507** | 1.70E-11 | 1.02E-12 | 0.681679019 | 0.104802269 |
| 104 | DEPDC1 | AK000490 | B.222958_s_at | **6.874692** | 1.82E-11 | 1.69E-11 | 0.589562887 | 0.127107203 |
| 38 | FKSG14 | BC005400 | B.222848_at | **6.651721** | 1.88E-11 | 1.30E-12 | 0.884636483 | 0.024726016 |
| 89 | CKS2 | NM_001827 | A.204170_s_at | **7**.**835274** | 1.88E-11 | 2.57E-13 | 0.663167842 | 0.083465644 |
| 155 | CDCA1 | AF326731 | B.223381_at | **6.49209** | 3.80E-11 | 5.13E-13 | 0.388889256 | 0.296165769 |
| 94 | DEPDC1 | AI810054 | B.235545_at | **6**.**249524** | 3.93E-11 | 5.99E-11 | 0.627093597 | 0.104698657 |
| 220 | ANLN | AK023208 | B.222608_s_at | **6.955614** | 4.68E-11 | 1.12E-11 | 0.198482286 | 0.602004883 |
| 213 | HN1 | AF060925 | B.222396_at | **8.422507** | 4.84E-11 | 6.28E-11 | -0.230083835 | 0.550728055 |
| 85 | NEK2 | NM_002497 | A.204641_at | **7.001719** | 5.19E-11 | 1.15E-12 | 0.647731608 | 0.081742332 |
| 150 | PKMYT1 | NM_004203 | A.204267_x_at | **6.922908** | 5.37E-11 | 1.32E-10 | 0.411866565 | 0.277601663 |
| 231 | BRIP1 | BF056791 | B.235609_at | **7**.**148933** | 5.75E-11 | 9.99E-12 | 0.16413055 | 0.666683251 |
| 263 | DEPDC1B | AK001166 | B.226980_at | **5.497689** | 5.75E-11 | 2.26E-09 | -0.024099105 | 0.95106539 |
| 17 | Spc24 | AI469788 | B.235572_at | **6.783946** | 6.38E-11 | 6.44E-12 | 0.992685847 | 0.007915906 |
| 115 | CCNB1 | N90191 | B.228729_at | **6.801847** | 6.38E-11 | 1.14E-11 | 0.528115076 | 0.166575624 |
| 61 | GAJ | AY028916 | B.223700_at | **5.843192** | 6.60E-11 | 6.67E-12 | 0.741255524 | 0.055223051 |
| 91 | C9orf140 | AW250904 | B.225777_at | **7.887661** | 6.83E-11 | 4.69E-10 | 0.679522784 | 0.08594282 |
| 125 | KPNA2 | NM_002266 | A.201088_at | **8.496449** | 7.07E-11 | 7.68E-11 | 0.519228058 | 0.185275145 |
| 86 | | NM_021067 | A.206102_at | **6.71395** | 7.57E-11 | 2.09E-11 | 0.646830568 | 0.081940926 |
| 165 | TOPK | NM_018492 | A.219148_at | **6.462595** | 7.84E-11 | 4.16E-11 | 0.3730149 | 0.327935025 |
| 15 | GAS2L3 | H37811 | B.235709_at | **6.727849** | 8.11E-11 | 3.33E-12 | 1.034666753 | 0.00553654 |
| 20 | C22orf18 | NM_024053 | A.218741_at | **6.348817** | 8.11E-11 | 2.63E-10 | 0.960849718 | 0.010156324 |
| 163 | MKI67 | BF001806 | A.212022_s_at | **6.725468** | 8.11E-11 | 4.78E-11 | 0.429593931 | 0.323243491 |
| 111 | MYBL2 | NM_002466 | A.201710_at | **6.06614** | 8.98E-11 | 5.62E-11 | 0.550044743 | 0.143391526 |
| 214 | UHRF1 | AK025578 | B.225655_at | **7.733479** | 9.62E-11 | 5.34E-12 | 0.224764258 | 0.552775395 |
| 248 | ANP32E | NM_030920 | A.208103_s_at | **6**.**298887** | 1.07E-10 | 1.11E-08 | 0.103382105 | 0.797118551 |
| 236 | GTSE1 | BF973178 | A.215942_s_at | **5**.**468846** | 1.22E-10 | 3.81E-12 | 0.162445666 | 0.691025332 |
| 13 | RAD51 | NM_002875 | A.205024_s_at | **6**.**352379** | 1.26E-10 | 1.00E-12 | 1.114959663 | 0.004430713 |
| 178 | UBE2S | NM_014501 | A.202779_s_at | **6**.**916494** | 1.31E-10 | 3.36E-10 | 0.363864456 | 0.368883213 |
| 74 | GTSE1 | NM_016426 | A.204315_s_at | **6.416579** | 1.65E-10 | 7.20E-12 | 0.678223538 | 0.069012359 |
| 101 | TOP2A | NM_001067 | A.201291_s_at | **7**.**356644** | 2.24E-10 | 5.61E-11 | 0.578232509 | 0.125387811 |
| 172 | CDCA7 | AY029179 | B.224428_s_at | **7.674613** | 3.56E-10 | 1.86E-08 | 0.429731941 | 0.350206624 |
| 122 | CDCA3 | NM_031299 | A.221436_s_at | **6.189773** | 3.93E-10 | 1.33E-09 | 0.511019556 | 0.176038534 |
| 93 | | NM_014875 | A.206364_at | **6.151827** | 5.11E-10 | 7.01E-11 | 0.614988939 | 0.103135349 |
| 183 | | T90295 | B.226661_at | **6**.**682487** | 6.64E-10 | 5.62E-09 | 0.346703445 | 0.401640846 |
| 166 | MGC45866 | AI638593 | B.230021_at | **6.42395** | 7.32E-10 | 2.47E-11 | 0.446135442 | 0.332297655 |
| 205 | MCM2 | NM_004526 | A.202107_s_at | **7.860975** | 8.89E-10 | 8.26E-10 | 0.274006856 | 0.528409926 |
| 78 | | AW271106 | B.229490_s_at | **6.222193** | 9.18E-10 | 3.24E-10 | 0.677333915 | 0.077888591 |
| 198 | C20orf129 | BC001068 | B.225687_at | **7.232237** | 1.08E-09 | 5.23E-10 | 0.257721719 | 0.500092255 |
| 40 | RAD51AP1 | BE966146 | A.204146_at | **6**.**304944** | 1.11E-09 | 3.76E-08 | 0.865618275 | 0.026849949 |
| 207 | CCNE2 | NM_004702 | A.205034_at | **6.205506** | 1.64E-09 | 1.51E-08 | 0.231488922 | 0.536273359 |
| 185 | NUDT1 | NM_002452 | A.209.766_s_at | **5.670523** | 2.04E-09 | 3.43E-11 | 0.336279873 | 0.404064878 |
| 34 | GPR19 | NM_006143 | A.207183_at | **5.256843** | 3.83E-09 | 1.26E-08 | 0.929389932 | 0.021115848 |
| 247 | | NM_017669 | A.219650_at | **5**.**042153** | 3.95E-09 | 1.21E-08 | 0.116316954 | 0.762199631 |
| 140 | HN1 | NM_016185 | A.217755_at | **7.911819** | 5.22E-09 | 4.13E-08 | 0.44433103 | 0.239189026 |
| 237 | HIST1H4C | NM_003542 | A.205967_at | **8.379597** | 5.55E-09 | 3.41E-08 | 0.155454713 | 0.692380424 |
| 102 | HMGA1 | NM_002131 | A.206074_s_at | **7.672253** | 6.68E-09 | 2.90E-08 | 0.57340264. | 0.126796719 |
| 141 | H2AFZ | NM_002106 | A.200853_at | **8.589569** | 6.68E-09 | 1.57E-09 | 0.438942866 | 0.241203655 |
| 168 | WDHD1 | AK001538 | A.216228_s_at | **4.541043** | 6.68E-09 | 3.23E-09 | 0.362835144 | 0.336253542 |
| 2 | KIF18A | NM_031217 | A.221258_s_at | **5.364945** | 6.89E-09 | 7.41E-10 | 1.170250756 | 0.001940291 |
| 39 | | X07868 | A.202409_at | **7**.**991737** | 8.27E-09 | 1.54E-08 | -0.856276422 | 0.025419272 |
| 174 | ATAD2 | AI925583 | B.222740_at | **6.841603** | 8.53E-09 | 2.90E-08 | 0.349834975 | 0.351965862 |
| 37 | | BF111626 | B.228559_at | **7.221195** | 1.16E-08 | 1.63E-07 | 0.89220144 | 0.022622085 |
| 22 | FLJ23311 | NM_024680 | A.219990_at | **5.027727** | 1.81E-08 | 7.53E-10 | 1.11499904 | 0.010526137 |
| 212 | ASK | NM_006716 | A.204244_s_at | **5.982485** | 2.04E-08 | 8.87E-08 | 0.22517382 | 0.547726962 |
| 127 | DC13 | NM_020188 | A.218447_at | **7**.**435987** | 2.59E-08 | 3.28E-08 | 0.49836629 | 0.192923434 |
| 146 | FLJ10948 | NM_018281 | A.218552_at | **7.977808** | 2.59E-08 | 7.07E-08 | -0.420287158 | 0.265366947 |
| 187 | CHEEK1 | NM_001274 | A.205394_at | **5.621699** | 2.67E-08 | 1.47E-07 | 0.313136396 | 0.408533969 |
| 84 | FBXO5 | AK026197 | B.234863_x_at | **6.934979** | 3.37E-08 | 8.76E-09 | 0.655530277 | 0.08133619 |
| 221 | NUP62 | AI859620 | B.230966_at | **6.428907** | 5.37E-08 | 9.30E-08 | 0.194175581 | 0.602079507 |
| 191 | CDCA5 | BE614410 | B.224753_at | **4.982139** | 5.85E-08 | 1.79E-07 | 0.29495453 | 0.433517741 |
| 56 | DCC1 | NM_024094 | A.219000_s_at | **6.283528** | 8.74E-08 | 6.85E-06 | 0.768011092 | 0.045286733 |
| 69 | HELLS | NM_018063 | A.220085_at | **5.288593** | 8.74E-08 | 3.52E-07 | 0.713632416 | 0.06333745 |
| 83 | CDT1 | AW075105 | B.228868_x_at | **7.054331** | 9.79E-08 | 5.55E-07 | 0.648477951 | 0.081174122 |
| 203 | Pfs2 | BC003186 | A.221521_s_at | **6.320114** | 1.45E-07 | 9.94E-08 | 0.246497936 | 0.516223881 |
| 255 | | AA938184 | B.236312_at | **5.701626** | 1.62E-07 | 2.80E-08 | -0.07481093 | 0.857385605 |
| 192 | | T58044 | B.227232_at | **8.502082** | 1.67E-07 | 8.48E-08 | -0.297539293 | 0.446463222 |
| 229 | FLJ13710 | AK024132 | B.232944_at | **6.19474** | 1.67E-07 | 2.60E-07 | -0.186238076 | 0.642824579 |
| 223 | PHF19 | BE544837 | B.227211_at | **6.348665** | 2.03E-07 | 3.74E-07 | -0.223589203 | 0.606554898 |
| 206 | KIF23 | NM_004856 | A.204709_s_at | **5.173124** | 2.74E-07 | 2.81E-08 | 0.274556227 | 0.529893874 |
| 243 | EXO1 | NM_003686 | A.204603_at | **5.927018** | 3.60E-07 | 1.00E-07 | 0.141097415 | 0.709U73685 |
| 170 | CXCL10 | NM_001565 | A.204533_at | **7.91312** | 6.01E-07 | 1.24E-06 | 0.354258493 | 0.340498438 |
| 256 | MLPH | AI810764 | B.229150_at | **8.078007** | 7.23E-07 | 1.23E-05 | -0.076268477 | 0.86056146 |
| 29 | LAPTM4B | T15777 | A.214039_s_at | **9.320913** | 7.83E-07 | 5.35E-06 | 0.889471325 | 0.016767645 |
| 42 | NUSAP1 | NM_018454 | A.219978_s_at | **6.335678** | 1.07E-06 | 2.59E-06 | 0.903401222 | 0.029991418 |
| 44 | EHD2 | AI417917 | A.221870_at | **6.477374** | 1.22E-06 | 3.36E-06 | -0.893991178 | 0.032844532 |
| 148 | C10orf56 | AL049949 | A.212419_at | **7.650367** | 1.25E-06 | 2.36E-06 | -0.426019275 | 0.266863651 |
| 145 | FSHPRH1 | BF793446 | A.214804_at | **5.010521** | 1.32E-06 | 1.57E-05 | 0.422634781 | 0.264823066 |
| 134 | ECT2 | NM_018098 | A.219787_s_at | **6.80516** | 1.43E-06 | 1.69E-06 | 0.486045036 | 0.213892061 |
| 116 | SLC7A5 | AB018009 | A.201195_s_at | **7.493131** | 1.46E-06 | 7.74E-06 | 0.540964485 | 0.166626703 |
| 26 | NUP88 | AI806781 | B.235786_at | **7.285647** | 1.62E-06 | 6.36E-07 | -0.911250522 | 0.014788954 |
| 136 | SCN7A | AI828648 | B.228504_at | **5.824759** | 1.89E-06 | 1.44E-06 | -0.453703343 | 0.222310621 |
| 171 | HPSE | NM_006665 | A.219403_s_at | **5.298862** | 1.99E-06 | 1.28E-06 | 0.394569194 | 0.343049791 |
| 25 | FLJ21062 | NM_024788 | A.219455_at | **5.525652** | 2.15E-06 | 5.20E-06 | -0.941228426 | 0.014095722 |
| 259 | CLDN5 | NM_003277 | A.204482_at | **6.151636** | 2.32E-06 | 6.44E-06 | -0.055268705 | 0.883493297 |
| 218 | SRD5A1 | NM_001047 | A.204675_at | **7.100171** | 2.70E-06 | 4.78E-05 | 0.219783486 | 0.596970945 |
| 142 | SOD2 | X15132 | A.216841_s_at | **6.002653** | 3.14E-06 | 3.73E-06 | 0.444778653 | 0.246622419 |
| 210 | | AI668620 | B.237339_at | **9.669306** | 3.22E-06 | 1.88E-05 | -0.226029013 | 0.54306855 |
| 157 | ANKRD30A | AF269087 | B.223864_at | **9.414368** | 3.30E-06 | 9.96E-05 | -0.387746621 | 0.299216824 |
| 58 | COL14A1 | BF449063 | A.212865_s_at | **7.287585** | 4.02E-06 | 1.36E-05 | -0.749700525 | 0.05022335 |
| 230 | C1orf21 | NM_030806 | A.221272_s_at | **5.622823** | 4.55E-06 | 1.14E-05 | -0.1682899 | 0.656466607 |
| 55 | CX3CR1 | U20350 | A.205898_at | **6.776389** | 5.27E-06 | 1.23E-04 | -0.749645527 | 0.043720315 |
| 151 | EGR1 | NM_001964 | A.201694_s_at | **8.620234** | 5.81E-06 | 3.60E-06 | -0.423112634 | 0.279987351 |
| 222 | | U79293 | A.215304_at | **6.931746** | 5.96E-06 | 2.81E-05 | -0.201281803 | 0.606462487 |
| 3 | CCL18 | Y13710 | A.32128_at | **6.244174** | 6.41E-06 | 2.75E-05 | 1.14221045 | 0.002597504 |
| 12 | CBX2 | BE514414 | B.226473_at | **7.558812** | 6.41E-06 | 1.13E-04 | 1.07504449 | 0.004054863 |
| 109 | ISG20 | NM_002201 | A.204698_at | **6.299944** | 6.73E-06 | 4.62E-06 | 0.5459336 | 0.14211529 |
| 118 | | AL360204 | B.232855_at | **4.628799** | 6.89E-06 | 9.05E-06 | -0.535303385 | 0.171221041 |
| 219 | DACH1 | NM_004392 | A.205472_s_at | **3.924559** | 6.89E-06 | 1.02E-05 | -0.212822165 | 0.597050977 |
| 132 | HSPC163 | NM_014184 | A.218728_s_at | **7.648067** | 7.41E-06 | 3.15E-06 | 0.507545115 | 0.210023483 |
| 152 | CIRBP | AL565767 | B.225191_at | **8.032986** | 8.16E-06 | 2.52E-06 | -0.469803635 | 0.280312337 |
| 158 | CYBRD1 | AI669804 | B.232459_at | **7.117116** | 8.36E-06 | 3.94E-05 | -0.388568867 | 0.310287696 |
| 160 | MCM4 | X74794 | A.212141_at | **6.729237** | 8.36E-06 | 1.02E-05 | 0.406623286 | 0.316436679 |
| 49 | FOS | BC004490 | A.209189_at | **8.992075** | 8.98E-06 | 4.05E-05 | -0.911746653 | 0.036012408 |
| 143 | CCNE1 | AI671049 | A.213523_at | **6.08195** | 1.04E-05 | 5.87E-05 | 0.463724353 | 0.248407611 |
| 137 | RBMS3 | AW338699 | B.241789_at | **6.365561** | 1.14E-05 | 2.42E-04 | -0.454436208 | 0.224664187 |
| 112 | ITGA7 | AK022548 | A.216331_at | **5.153545** | 1.62E-05 | 1.32E-05 | -0.541433612 | 0.145348566 |
| 232 | CXCL11 | AF002985 | A.211122_s_at | **6.1001** | 1.66E-05 | 1.05E-05 | -0.1728883 | 0.666951268 |
| 76 | BM039 | NM_018455 | A.219555_s_at | **4.173851** | 2.14E-05 | 9.20E-06 | 0.673164666 | 0.074344562 |
| 62 | ATAD2 | AI139629 | B.235266_at | **6.191308** | 2.34E-05 | 1.39E-04 | 0.748127999 | 0.055689556 |
| 193 | GGH | NM 003878 | A.203560_at | **6.77081** | 2.75E-05 | 2.09E-05 | -0.293893248 | 0.453096633 |
| 14 | | AI693516 | B.228750_at | **7**.**124873** | 2.94E-05 | 2.85E-04 | -1.073910408 | 0.00444517 |
| 179 | ELN | AA479278 | A.212670_at | **6.895109** | 3.08E-05 | 1.86E-04 | -0.334047514 | 0.369570896 |
| 133 | NOVA1 | NM 002515 | A.205794_s_at | **6.768152** | 3.68E-05 | 3.98E-04 | -0.489575159 | 0.211015726 |
| 90 | CACNA1D | BE550599 | A.210108_at | **6.26118** | 4.21E-05 | 5.08E-05 | -0.642967417 | 0.084876377 |
| 234 | | AK002203 | B.226992_at | **7.90914** | 5.25E-05 | 2.56E-04 | -0.154632796 | 0.678987899 |
| 67 | NR4A2 | AA523939 | B.235739_at | **7**.**187449** | 5.73E-05 | 3.92E-06 | -0.731391224 | 0.062004634 |
| 190 | | AL512727 | A.215014_at | **4.833426** | 5.99E-05 | 1.73E-04 | -0.295736426 | 0.432032039 |
| 73 | DUSP1 | NM_004417 | A.201041_s_at | **9.748091** | 6.12E-05 | 4.18E-05 | -0.758479385 | 0.068505145 |
| 262 | | R38110 | B.240112_at | **5.163128** | 6.53E-05 | 2.48E-04 | -0.036764785 | 0.921615167 |
| 7 | STC2 | BC000658 | A.203439_s_at | **7.680632** | 6.82E-05 | 2.05E-04 | -1.191837167 | 0.003010392 |
| 52 | PLAC9 | AW964972 | B.227419_x_at | **6.688968** | 7.76E-05 | 2.06E-04 | -0.786290483 | 0.040004936 |
| 211 | | BF508074 | B.240465_at | **6.004131** | 8.10E-05 | 5.21E-05 | 0.233504153 | 0.545194111 |
| 254 | KIAA0303 | AW971134 | A.222348_at | **4**.**963999** | 8.10E-05 | 3.04E-04 | -0.080778342 | 0.833005228 |
| 97 | PSAT1 | BC004863 | B.223062_s_at | **6**.**103481** | 9.21E-05 | 5.37E-05 | 0.595123345 | 0.109627082 |
| 68 | LRP2 | R73030 | B.230863_at | **7**.**464817** | 1.00E-04 | 6.57E-05 | -0.69766747 | 0.062336219 |
| 161 | | AL137566 | B.228554_at | **7.112413** | 1.05E-04 | 1.18E-04 | -0.40109127 | 0.318261339 |
| 162 | | BF513468 | B.241505_at | **7.15166** | 1.05E-04 | 1.53E-04 | 0.374700717 | 0.32253637 |
| 252 | MGC24047 | AI732488 | B.229381_at | **7.228131** | 1.07E-04 | 1.17E-04 | -0.082087159 | 0.83034645 |
| 195 | NPY1R | NM_000909 | A.205440_s_at | **5.830472** | 1.11E-04 | 4.10E-04 | 0.337889908 | 0.461696619 |
| 27 | SIRT3 | AF083108 | A.221562_s_at | **5.964518** | 1.16E-04 | 6.45E-04 | -0.927132823 | 0.01545353 |
| 128 | LRP2 | NM_004525 | A.205710_at | **5.984454** | 1.19E-04 | 1.06E-04 | -0.492675347 | 0.193865955 |
| 235 | | AI492376 | B.231195_at | **5.196657** | 1.21E-04 | 2.67E-04 | -0.161302941 | 0.680051165 |
| 246 | NTN4 | AF278532 | B.223315_at | **8.269299** | 1.24E-04 | 1.70E-04 | -0.132354027 | 0.725835139 |
| 43 | STC2 | AI435828 | A.203438_at | **7.538814** | 1.32E-04 | 1.57E-04 | -0.797860709 | 0.031924561 |
| 175 | | AV733950 | A.201693_s_at | **7.906065** | 1.37E-04 | 9.86E-06 | -0.347314523 | 0.355018177 |
| 8 | RAI2 | NM_021785 | A.219440_at | **6.659438** | 1.99E-04 | 2.01E-04 | -1.108174776 | 0.003077111 |
| 196 | NMU | NM 006681 | A.206023_at | **5.10173** | 2.49E-04 | 1.99E-04 | 0.298272606 | 0.461878171 |
| 24 | | AI492388 | B.228854_at | **6.819756** | 2.70E-04 | 7.97E-04 | -0.950969041 | 0.013149939 |
| 5 | PTGER3 | AW242315 | A.213933_at | **7.356099** | 2.98E-04 | 1.25E-03 | -1.295337189 | 0.002908446 |
| 117 | FLJ10901 | NM 018265 | A.219010_at | **6.942924** | 3.29E-04 | 5.19E-04 | 0.519806366 | 0.168424663 |
| 41 | FOSB | NM 006732 | A.202768_at | **6.19218** | 3.35E-04 | 1.36E-04 | -0.815647159 | 0.028388157 |
| 177 | ERBB4 | AK024204 | B.233498_at | **7.543523** | 3.77E-04 | 6.61E-04 | -0.336800577 | 0.367457847 |
| 106 | LAF4 | AI033582 | B.244696_at | **7.41577** | 4.24E-04 | 4.62E-04 | -0.572783549 | 0.134590614 |
| 6 | MAPT | NM 016835 | A.203928_x_at | **6.910278** | 4.41E-04 | 1.10E-03 | -1.114016712 | 0.002947734 |
| 124 | | AW970881 | A.222314_x_at | **5.250506** | 4.67E-04 | 3.33E-04 | -0.49598679 | 0.183685062 |
| 240 | SRD5A1 | BC006373 | A.211056_s_at | **6.760491** | 4.95E-04 | 1.14E-03 | -0.177760256 | 0.69950506 |
| 176 | FMO5 | AK022172 | A.215300_s_at | **4.143345** | 5.24E-04 | 2.15E-04 | -0.338873235 | 0.365924454. |
| 186 | ZNF533 | H15261 | B.243929_at | **4.716503** | 5.77E-04 | 7.17E-05 | -0.312801434 | 0.408005813 |
| 169 | TTC18 | AW024437 | B.229170_s_at | **6.229818** | 6.11E-04 | 1.99E-03 | -0.373029504 | 0.339898261 |
| 54 | BCL2 | AU146384 | B.232210_at | **8.094828** | 6.71E-04 | 1.23E-03 | -0.760752368 | 0.043704125 |
| 47 | CYBRD1 | NM 024843 | A.217889_s_at | **5.642724** | 6.97E-04 | 6.69E-04 | -0.79117731 | 0.035959897 |
| 201 | SLC40A1 | AA588092 | B.239723_at | **6.922208** | 6.97E-04 | 2.68E-04 | 0.246250082 | 0.508863506 |
| 253 | MUSTN1 | BF793701 | B.226856_at | **5.562608** | 7.51E-04 | 1.01E-03 | 0.096986779 | 0.832624185 |
| 9 | MFAP4 | R72286 | A.212713_at | **6.51492** | 8.09E-04 | 1.76E-03 | -1.113082042 | 0.003213842 |
| 99 | LRRC17 | NM_005824 | A.205381_at | **7.216997** | 8.24E-04 | 1.34E-03 | -0.571472856 | 0.124800311 |
| 239 | STK32B | NM_018401 | A.219686_at | **4.566312** | 8.88E-04 | 1.45E-03 | -0.157335553 | 0.695207523 |
| 164 | | BF433570 | B.237301_at | **6.317098** | 1.09E-03 | 1.13E-03 | -0.408773136 | 0.325727984 |
| 114 | | AW512787 | B.238481_at | **8.511705** | 1.17E-03 | 1.56E-03 | -0.558216466 | 0.164426983 |
| 242 | NAT1 | NM_000662 | A.214440_at | **7.742309** | 1.19E-03 | 1.86E-03 | 0.171562865 | 0.708554521 |
| 60 | EPHX2 | AF233336 | A.209368_at | **6.403114** | 1.21E-03 | 1.87E-04 | -0.760554602 | 0.052355087 |
| 167 | PHYHD1 | AL545998 | B.226846_at | **7.221441** | 1.25E-03 | 1.72E-03 | -0.359283155 | 0.333823065 |
| 159 | | NM_030896 | A.221275_s_at | **3.961128** | 1.28E-03 | 5.94E-04 | -0.376502717 | 0.314482067 |
| 130 | CYBRD1 | AL136693 | B.222453_at | **9.399092** | 1.30E-03 | 1.48E-03 | -0.48333705 | 0.195588312 |
| 238 | NAV3 | NM_014903 | A.204823_at | **5.823519** | 1.47E-03 | 1.61E-03 | -0.158076864 | 0.693777462 |
| 53 | OGN | NM_014057 | A.218730_s_at | **4.932506** | 1.64E-03 | 5.08E-03 | -0.757516472 | 0.042394291 |
| 100 | SYNCRIP | NM_006372 | A.217834_s_at | **6.812321** | 1.85E-03 | 1.62E-03 | 0.587077047 | 0.125280752 |
| 154 | | AK021990 | B.232699_at | **5.867527** | 1.95E-03 | 1.28E-03 | -0.393427065 | 0.289892653 |
| 184 | ERBB4 | AW772192 | A.214053_at | **7.07437** | 2.09E-03 | 8.91E-04 | -0.336719007 | 0.401781194 |
| 216 | | NM_004522 | A.203130_s_at | **7.321429** | 2.28E-03 | 1.57E-02 | 0.231698726 | 0.564239609 |
| 4 | MAPT | J03778 | A.206401_s_at | **6.455705** | 2.36E-03 | 5.08E-03 | -1.13042772 | 0.002820509 |
| 64 | HMGB3 | NM_005342 | A.203744_at | **7.550192** | 3.25E-03 | 4.04E-03 | 0.738482321 | 0.05884424 |
| 251 | LAF4 | AA572675 | B.232286_at | **7.169029** | 3.25E-03 | 3.10E-03 | 0.108992215 | 0.812211511 |
| 31 | AQP9 | NM_020980 | A.205568_at | **4.951949** | 3.53E-03 | 1.59E-03 | 0.895190406 | 0.019505478 |
| 188 | DACH1 | AI650353 | B.228915_at | **7.671623** | 3.53E-03 | 1.64E-03 | -0.311012322 | 0.411423928 |
| 75 | SCN4B | AW026241 | B.236359_at | **5.552642** | 3.89E-03 | 6.07E-03 | -0.677852485 | 0.073197783 |
| 233 | FLJ41238 | AW629527 | B.229764_at | **6.531923** | 4.16E-03 | 5.68E-03 | -0.176712594 | 0.671030052 |
| 156 | SCUBE2 | AI424243 | A.219197_s_at | **8.381941** | 5.04E-03 | 5.90E-03 | -0.386317867 | 0.298631944 |
| 227 | CYP4Z1 | AV700083 | B.237395_at | **8.750525** | 5.04E-03 | 3.96E-03 | 0.18037008 | 0.631134131 |
| 217 | ESR1 | NM_000125 | A.205225_at | **7.494275** | 5.12E-03 | 4.39E-04 | 0.416453493 | 0.570106612 |
| 225 | CYP4X1 | AA557324 | B.227702_at | **8.597239** | 5.29E-03 | 5.71E-03 | -0.187667891 | 0.625691687 |
| 202 | TTC18 | AW024437 | B.229169_at | **5.826554** | 5.55E-03 | 6.63E-03 | -0.242354326 | 0.51485792 |
| 16 | MAPT | NM_016835 | A.203929_s_at | **7.791403** | 5.73E-03 | 3.01E-03 | -1.029153262 | 0.00579453 |
| 182 | ECT2 | BG170335 | B.234992_x_at | **5.165319** | 6.91E-03 | 9.85E-03 | 0.329010815 | 0.379594706 |
| 261 | | AV709727 | B.225996_at | **7.571507** | 7.58E-03 | 5.58E-04 | 0.044547315 | 0.905089266 |
| 250 | PTPRT | NM_007050 | A.205948_at | **6.763414** | 8.18E-03 | 7.66E-03 | -0.089691431 | 0.810363802 |
| 209 | CALML5 | NM_017422 | A.220414_at | **5.994003** | 8.56E-03 | 3.32E-03 | 0.267191443 | 0.540775453 |
| 18 | SUSD3 | AW966474 | B.227182_at | **8.195015** | 1.04E-02 | 8.78E-03 | -1.297832347 | 0.008305284 |
| 10 | STH | AA199717 | B.225379_at | **7.857365** | 2.30E-02 | 7.91E-03 | -1.097446295 | 0.003735657 |
| 197 | FLJ45983 | AI631850 | B.240192_at | **5.289779** | 4.94E-02 | 4.10E-02 | 0.314861713 | 0.468985395 |
| 241 | | AL031658 | B.232357_at | **5.976136** | 5.06E-02 | **4.81E-02** | -0.145562103 | 0.700546776 |
| 96 | | AI826437 | B.229975_at | **6.381037** | 5.90E-02 | 5.75E-02 | 0.78769613 | 0.109281577 |
| 249 | LOC143381 | AW242720 | B.227550_at | **7.656959** | 9.35E-02 | 2.86E-02 | -0.106502567 | 0.798016237 |
| 258 | DNALI1 | AW299538 | B.227081_at | **7.085104** | 1.03E-01 | 5.27E-03 | -0.068369896 | 0.881511542 |
| 194 | GAMT | NM_000156 | A.205354_at | **5.947354** | 1.53E-01 | 2.91E-02 | -0.284372326 | 0.457600609 |
| 257 | DNALI1 | NM_003462 | A.205186_at | **4.299739** | 1.54E-01 | 2.58E-02 | -0.08851533 | 0.869483818 |
| 23 | MMP1 | NM_002421 | A.204475_at | **7.170495** | 2.04E-01 | 2.26E-01 | 1.047070923 | 0.01186788 |
| 264 | PPP1R3C | N26005 | A.204284_at | **7.027458** | 2.85E-01 | 6.40E-01 | -0.006752502 | 0.987063337 |
| 126 | CXCL14 | NM_004887 | A.218002_s_at | **8.251287** | 4.49E-01 | 5.03E-01 | -0.502169588 | 0.190758302 |
| 51 | CXCL14 | AF144103 | B.222484_s_at | **9.336584** | 6.54E-01 | 5.00E-01 | -0.777835445 | 0.03993233 |

### APPENDIX 4

SWS Classifier 0: Clinical validation (survival analysis) of G2a and G2b tumour subtypes (264 classifier).

| **# Cox PH test summary (Baseline group 1)** | | | | | | |
|---|---|---|---|---|---|---|
| | coef | exp(coef) | se(coef) | z | p | |
| group2b | 0.795 | 2.21 | 0.292 | 2.72 | 0.0066 | |
| | | | | | | |

| **Likelihood ratio test=7.25 on 1 df, p=0.00711 n = 126** | | | | | | |
|---|---|---|---|---|---|---|
| | n events | rmean | se(rmean) | median | 0.95LCL | 0.95UCL |
| group 2a= 79 | 23 | 9.97 | 0.507 | Inf | Inf | Inf |
| group 2b= 47 | 24 | 7.35 | 0.793 | 8.5 | 2.58 | Inf |

### APPENDIX 5A

**SWS Classifier 1**

| **Order** | **UGID(build #183)** | **Unigen eName** | **GeneSym bol** | **Genbank Acc** | **Affi ID** | **Cut-off** |
|---|---|---|---|---|---|---|
| 1 | Hs.528654 | Hypothe tical protein FLJ110 29 | FLJ11029 | BG16501 1 | B.228273_a t | **7.706303** |
| 2 | acc_NM_0031 58.1 | Serine/t hreonin e kinase 6. transcri pt 1 | STK6 | NM_0031 58 | A.208079_s _at | **6.652593** |
| 3 | Hs.35962 | CDNA clone IMAGE:4452583, partial cds | | BG49235 9 | B.226936_a t | **7.561905** |
| 4 | Hs.308045 | Barren homolo 9 (Drosop hila) | BRRN1 | D38553 | A.212949_a t | **5.916703** |
| 5 | Hs.184339 | Maternal I embryo nic leucine zipper kinase | MELK | NM_0147 91 | A.204825_a t | **7.107259** |
| 6 | Hs.250822 | Serine/t hreonin e kinase 6, transcri pt 2 | STK6 | NM_0036 00 ' | A.204092_s _at | **6.726571** |

### APPENDIX 5B

### SWS Classifier 1: Classifier Accuracy

Accuracy
G1 = 65/68
(95.6%)
G3 = 51/55 ?
(94.5%)

| Number | Patient ID | Histologic grade | Probability for G 1 | Probability for G3 | Predicted grade |
|---|---|---|---|---|---|
| 1 | X100B08 | 1 | **0.959** | 0.041 | 1 |
| 2 | X209C10 | 1 | **0.959** | 0.041 | 1 |
| 3 | X21C28 | 1 | **0.959** | 0.041 | 1 |
| 4 | X220C70 | 1 | **0.959** | 0.041 | 1 |
| 5 | X224C93 | 1 | **0.959** | 0.041 | 1 |
| 6 | X227C50 | 1 | **0.959** | 0.041 | 1 |
| 7 | X229C44 | 1 | **0.959** | 0.041 | 1 |
| 8 | X231C80 | 1 | **0.959** | 0.041 | 1 |
| 9 | X233C91 | 1 | **0.959** | 0.041 | 1 |
| 10 | X235C20 | 1 | 0.287 | **0.713** | 3 |
| 11 | X236C55 | 1 | **0.959** | 0.041 | 1 |
| 12 | X114B68 | 1 | **0.782** | 0.218 | 1 |
| 13 | X243C70 | 1 | **0.959** | 0.041 | 1 |
| 14 | X246C75 | 1 | **0.959** | 0.041 | 1 |
| 15 | X248C91 | 1 | **0.959** | 0.041 | 1 |
| 16 | X253C20 | 1 | **0.959** | 0.041 | 1 |
| 17 | X259C74 | 1 | **0.959** | 0.041 | 1 |
| 18 | X261C94 | 1 | **0.959** | 0.041 | 1 |
| 19 | X262C85 | 1 | **0.959** | 0.041 | 1 |
| 20 | X263C82 | 1 | **0.959** | 0.041 | 1 |
| 21 | X266C51 | 1 | **0.959** | 0.041 | 1 |
| 22 | X267C04 | 1 | **0.959** | 0.041 | 1 |
| 23 | X282C51 | 1 | **0.959** | 0.041 | 1 |
| 24 | X284C63 | 1 | **0.959** | 0.041 | 1 |
| 25 | X289C75 | 1 | **0.959** | 0.041 | 1 |
| 26 | X28C76 | 1 | **0.959** | 0.041 | 1 |
| 27 | X294C04 | 1 | **0.887** | 0.113 | 1 |
| 28 | X309C49 | 1 | 0.01 | **0.99** | 3 |
| 29 | X316C65 | 1 | **0.959** | 0.041 | 1 |
| 30 | X128B48 | 1 | **0.959** | 0.041 | 1 |
| 31 | X33C30 | 1 | **0.959** | 0.041 | 1 |
| 32 | X39C24 | 1 | **0.959** | 0.041 | 1 |
| 33 | X42C57 | 1 | **0.959** | 0.041 | 1 |
| 34 | X45A96 | 1 | **0.959** | 0.041 | 1 |
| 35 | X48A46 | 1 | **0.959** | 0.041 | 1 |
| 36 | X49A07 | 1 | **0.959** | 0.041 | 1 |
| 37 | X52A90 | 1 | **0.959** | 0.041 | 1 |
| 38 | X61A53 | 1 | **0.959** | 0.041 | 1 |
| 39 | X65A68 | 1 | **0.959** | 0.041 | 1 |
| 40 | X6B85 | 1 | **0.733** | 0.267 | 1 |
| 41 | X72A92 | 1 | 0.489 | **0.511** | 3 |
| 42 | X135B40 | 1 | **0.959** | 0.041 | 1 |
| 43 | X74A63 | 1 | **0.894** | 0.106 | 1 |
| 44 | X83A37 | 1 | **0.733** | 0.267 | 1 |
| 45 | X8B87 | 1 | **0.959** | 0.041 | 1 |
| 46 | X99A50 | 1 | **0.959** | 0.041 | 1 |
| 47 | X138B34 | 1 | **0.959** | 0.041 | 1 |
| 48 | X155B52 | 1 | **0.959** | 0.041 | 1 |
| 49 | X156B01 | 1 | **0.959** | 0.041 | 1 |
| 50 | X160B16 | 1. | **0.959** | 0.041 | 1 |
| 51 | X163B27 | 1 | **0.959** | 0.041 | 1 |
| 52 | X105B13 | 1 | **0.959** | 0.041 | 1 |
| 53 | X173B43 | 1 | **0.959** | 0.041 | 1 |
| 54 | X174B41 | 1 | **0.959** | 0.041 | 1 |
| 55 | X177B67 | 1 | **0.959** | 0.041 | 1 |
| 56 | X106B55 | 1 | **0.959** | 0.041 | 1 |
| 57 | X180B38 | 1 | **0.959** | 0.041 | 1 |
| 58 | X181B70 | 1 | **0.887** | 0.113 | 1 |
| 59 | X184B38 | 1 | **0.959** | 0.041 | 1 |
| 60 | X185B44 | 1 | **0.959** | 0.041 | 1 |
| 61 | X10B88 | 1 | **0.678** | 0.322 | 1 |
| 62 | X192B69 | 1 | **0.959** | 0.041 | 1 |
| 63 | X195B75 | 1 | **0.959** | 0.041 | 1 |
| 64 | X196B81 | 1 | **0.887** | 0.113 | 1 |
| 65 | X19C33 | 1 | **0.959** | 0.041 | 1 |
| 66 | X204B85 | 1 | **0.959** | 0.041 | 1 |
| 67 | X205B99 | 1 | **0.915** | 0.085 | 1 |
| 68 | X207C08 | 1 | **0.959** | 0.041 | 1 |
| 69 | X111B51 | 3 | 0.001 | **0.999** | 3 |
| 70 | X222C26 | 3 | 0.036 | **0.974** | 3 |
| 71 | X226C06 | 3 | 0.001 | **0.999** | 3 |
| 72 | X113B11 | 3 | 0.001 | **0.999** | 3 |
| 73 | X232C58 | 3 | 0.001 | **0.999** | 3 |
| 74 | X234C15 | 3 | 0.003 | **0.997** | 3 |
| 75 | X238C87 | 3 | 0.163 | **0.837** | 3 |
| 76 | X241C01 | 3 | 0.001 | **0.999** | 3 |
| 77 | X249C42 | 3 | 0.001 | **0.999** | 3 |
| 78 | X250C78 | 3 | 0.001 | **0.999** | 3 |
| 79 | X252C64 | 3 | 0.001 | **0.999** | 3 |
| 80 | X269C68 | 3 | 0.001 | **0.999** | 3 |
| 81 | X26C23 | 3 | 0.047 | **0.953** | 3 |
| 82 | X270C93 | 3 | 0.001 | **0.999** | 3 |
| 83 | X271C71 | 3 | 0.001 | **0.999** | 3 |
| 84 | X279C61 | 3 | 0.001 | **0.999** | 3 |
| 85 | X287C67 | 3 | 0.001 | **0.999** | 3 |
| 86 | X291C17 | 3 | 0.001 | **0.999** | 3 |
| 87 | X127B00 | 3 | 0.001 | **0.999** | 3 |
| 88 | X303C36 | 3 | 0.001 | **0.999** | 3 |
| 89 | X304C89 | 3 | **0.996** | 0.004 | 1 |
| 90 | X311A27 | 3 | 0.001 | **0.999** | 3 |
| 91 | X313A87 | 3 | 0.001 | **0.999** | 3 |
| 92 | X314B55 | 3 | 0.001 | **0.999** | 3 |
| 93 | X101B88 | 3 | 0.001 | **0.999** | 3 |
| 94 | X37C06 | 3 | 0.001 | **0.999** | 3 |
| 95 | X46A25 | 3 | 0.001 | **0.999** | 3 |
| 96 | X131 B79 | 3 | **0.597** | 0.403 | 1 |
| 97 | X54A09 | 3 | 0.001 | **0.999** | 3 |
| 98 | X55A79 | 3 | 0.001 | **0.999** | 3 |
| 99 | X62A02 | 3 | 0.001 | **0.999** | 3 |
| 100 | X66A84 | 3 | 0.001 | **0.999** | 3 |
| 101 | X67A43 | 3 | 0.001 | **0.999** | 3 |
| 102 | X69A93 | 3 | 0.001 | **0.999** | 3 |
| 103 | X70A79 | 3 | 0.001 | **0.999** | 3 |
| 104 | X73A01 | 3 | 0.034 | **0.966** | 3 |
| 105 | X76A44 | 3 | 0.005 | **0.995** | 3 |
| 106 | X79A35 | 3 | 0.005 | **0.995** | 3 |
| 107 | X82A83 | 3 | 0.005 | **0.995** | 3 |
| 108 | X89A64 | 3 | 0.001 | **0.999** | 3 |
| 109 | X90A63 | 3 | 0.001 | **0.999** | 3 |
| 110 | X139B03 | 3 | 0.001 | **0.999** | 3 |
| 111 | X102B06 | 3 | 0.001 | **0.999** | 3 |
| 112 | X142B05 | 3 | 0.003 | **0.998** | 3 |
| 113 | X143B81 | 3 | 0.016 | **0.984** | 3 |
| 114 | X146B39 | 3 | 0.001 | **0.999** | 3 |
| 115 | X147B19 | 3 | 0.001 | **0.999** | 3 |
| 116 | X103B41 | 3 | 0.001 | **0.999** | 3 |
| 117 | X153B09 | 3 | 0.001 | **0.999** | 3 |
| 118 | X104B91 | 3 | 0.001 | **0.999** | 3 |
| 119 | X162B98 | 3 | 0.033 | **0.977** | 3 |
| 120 | X172B19 | 3 | 0.004 | **0.996** | 3 |
| 121 | X182B43 | 3 | 0.001 | **0.999** | 3 |
| 122 | X194B60 | 3 | 0.005 | **0.995** | 3 |
| 123 | X200B47 | 3 | **0.931** | 0.069 | 1 |

### APPENDIX 5C

**SWS Classifier 1: Prediction validation**

| # Cox PH test summary (Baseline group 1) | | | | | | |
|---|---|---|---|---|---|---|
| | coef | exp(coef) | se(coef) | z | p | |
| group3 | 0.921 | 2.51 | 0.292 | 3.15 | 0.0016 | |
| | | | | | | |

| Likelihood ratio test=9.66 on 1 df, p=0.00189 n= 126 | | | | | | |
|---|---|---|---|---|---|---|
| | n events | rmean | se(rmean) | median | 0.95LCL | 0.95UCL |
| group 2a=83 | 23 | 10.0 | 0.489 | Inf | Inf | Inf |
| group 2b=43 | 24 | 7.0 | 0.820 | 6.5 | 2.58 | Inf |

| | | | | | | |
|---|---|---|---|---|---|---|
| * DFS Event defined as any type of recurrence or death because of breast cancer, whichever comes first | | | | | | |

| Number | Patient ID | Probability for G2a | Probability for G2b | Predicted grade | DFS TIME | DFS EVENT* |
|---|---|---|---|---|---|---|
| 1 | X210C72 | **0.894** | 0.106 | 2a | 0.5 | 1 |
| 2 | X211C88 | **0.777** | 0.223 | 2a | 1.5 | 0 |
| 3 | X212C21 | **0.959** | 0.041 | 2a | 3.75 | 1 |
| 4 | X213C36 | 0.005 | **0.995** | 2b | 10.08 | 0 |
| 5 | X216C61 | **0.959** | 0.041 | 2a | 10.75 | 0 |
| 6 | X217C79 | **0.959** | 0.041 | 2a | 10.75 | 0 |
| 7 | X218C29 | **0.894** | 0.106 | 2a | 10.75 | 0 |
| 8 | X112B55 | 0.007 | **0.993** | 2b | 0.92 | 1 |
| 9 | X221C14 | 0.143 | **0.857** | 2b | 3 | 1 |
| 10 | X223C51 | **0.894** | 0.106 | 2a | 8.42 | 0 |
| 11 | X225C52 | 0.001 | **0.999** | 2b | 10.75 | 0 |
| 12 | X22C62 | **0.959** | 0.041 | 2a | 4.83 | 0 |
| 13 | X230C47 | 0.001 | **0.999** | 2b | 0.5 | 1 |
| 14 | X237C56 | 0.143 | **0.857** | 2b | 10.67 | 0 |
| 15 | X23C52 | 0.005 | **0.995** | 2b | 8.5 | 1 |
| 16 | X240C54 | 0.005 | **0.995** | 2b | 2.42 | 1 |
| 17 | X242C21 | 0.209 | **0.791** | 2b | 2.17 | 1 |
| 18 | X244C89 | **0.777** | 0.223 | 2a | 7.25 | 1 |
| 19 | X245C22 | 0.143 | **0.857** | 2b | 0 | 1 |
| 20 | X247C76 | **0.959** | 0.041 | 2a | 10.5 | 0 |
| 21 | X11B47 | **0.959** | 0.041 | 2a | 7.42 | 0 |
| 22 | X24C30 | **0.959** | 0.041 | 2a | 10.67 | 0 |
| 23 | X251C14 | **0.959** | 0.041 | 2a | 10.5 | 0 |
| 24 | X254C80 | **0.959** | 0.041 | 2a | 10.5 | 0 |
| 25 | X255C06 | **0.959** | 0.041 | 2a | 10.5 | 0 |
| 26 | X256C45 | 0.001 | **0.999** | 2b | 1.25 | 1 |
| 27 | X120B73 | 0.001 | **0.999** | 2b | 11.58 | 0 |
| 28 | X257C87 | **0.959** | 0.041 | 2a | 10.5 | 0 |
| 29 | X258C21 | **0.959** | 0.041 | 2a | 5.75 | 1 |
| 30 | X260C91 | 0.09 | **0.91** | 2b | 10.42 | 0 |
| 31 | X265C40 | **0.777** | 0.223 | 2a | 10.42 | 0 |
| 32 | X122B81 | **0.959** | 0.041 | 2a | 11.17 | 0 |
| 33 | X268C87 | 0.001 | **0.999** | 2b | 10.33 | 0 |
| 34 | X272C88 | **0.959** | 0.041 | 2a | 10.33 | 0 |
| 35 | X274C81 | **0.959** | 0.041 | 2a | 10.33 | 0 |
| 36 | X275C70 | **0.959** | 0.041 | 2a | 10.25 | 0 |
| 37 | X277C64 | **0.959** | 0.041 | 2a | 8.58 | 0 |
| 38 | X124B25 | **0.959** | 0.041 | 2a | 5 | 1 |
| 39 | X278C80 | 0.351 | **0.649** | 2b | 10.25 | 0 |
| 40 | X27C82 | **0.959** | 0.041 | 2a | 6.83 | 0 |
| 41 | X280C43 | **0.959** | 0.041 | 2a | 1 | 1 |
| 42 | X286C91 | **0.959** | 0.041 | 2a | 10 | 0 |
| 43 | X288C57 | **0.959** | 0.041 | 2a | 10 | 0 |
| 44 | X290C91 | **0.959** | 0.041 | 2a | 10 | 0 |
| 45 | X292C66 | **0.959** | 0.041 | 2a | 10 | 0 |
| 46 | X296C95 | **0.959** | 0.041 | 2a | 9.92 | 0 |
| 47 | X297C26 | **0.959** | 0.041 | 2a | 9.92 | 0 |
| 48 | X298C47 | **0.959** | 0.041 | 2a | 6.5 | 1 |
| 49 | X301C66 | **0.959** | 0.041 | 2a | 9.92 | 0 |
| 50 | X307C50 | **0.777** | 0.223 | 2a | 9.83 | 0 |
| 51 | X308C93 | 0.005 | **0.995** | 2b | 2.25 | 1 |
| 52 | X34C80 | **0.959** | 0.041 | 2a | 10.17 | 0 |
| 53 | X35C29 | 0.202 | **0.798** | 2b | 2.42 | 1 |
| 54 | X36C17 | **0.959** | 0.041 | 2a | 10.08 | 0 |
| 55 | X40C57 | **0.877** | 0.123 | 2a | 10 | 0 |
| 56 | X41C65 | **0.959** | 0.041 | 2a | 9.92 | 0 |
| 57 | X130B92 | **0.959** | 0.041 | 2a | 4.42 | 1 |
| 58 | X43C47 | **0.877** | 0.123 | 2a | 9.92 | 0 |
| 59 | X44A53 | 0.123 | **0.877** | 2b | 12.75 | 0 |
| 60 | X47A87 | 0.001 | **0.999** | 2b | 9.58 | 1 |
| 61 | X50A91 | **0.777** | 0.223 | 2a | 9.08 | 1 |
| 62 | X51A98 | **0.959** | 0.041 | 2a | 12.67 | 0 |
| 63 | X53A06 | 0.202 | **0.798** | 2b | 2.58 | 1 |
| 64 | X56A94 | **0.959** | 0.041 | 2a | 1.08 | 1 |
| 65 | X58A50 | 0.001 | **0.999** | 2b | 0.42 | 1 |
| 66 | X5B97 | 0.001 | **0.999** | 2b | 0.75 | 1 |
| 67 | X60A05 | **0.959** | 0.041 | 2a | 0.67 | 1 |
| 68 | X134B33 | 0.015 | **0.985** | 2b | 2 | 1 |
| 69 | X63A62 | **0.959** | 0.041 | 2a | 0.17 | 1 |
| 70 | X64A59 | 0.046 | **0.954** | 2b | 12.42 | 0 |
| 71 | X75A01 | 0.202 | **0.798** | 2b | 3.58 | 1 |
| 72 | X77A50 | **0.662** | 0.338 | 2a | 1.08 | 1 |
| 73 | X7B96 | **0.959** | 0.041 | 2a | 2.42 | 1 |
| 74 | X84A44 | 0.017 | **0.983** | 2b | 12.17 | 0 |
| 75 | X136B04 | **0.959** | 0.041 | 2a | 2.42 | 1 |
| 76 | X85A03 | **0.777** | 0.223 | 2a | 2.08 | 0 |
| 77 | X86A40 | 0.001 | **0.999** | 2b | 12.17 | 0 |
| 78 | X87A79 | **0.662** | 0.338 | 2a | 12.08 | 0 |
| 79 | X88A67 | 0.029 | **0.971** | 2b | 4.25 | 1 |
| 80 | X94A16 | **0.959** | 0.041 | 2a | 11.08 | 0 |
| 81 | X96A21 | **0.959** | 0.041 | 2a | 0.08 | 1 |
| 82 | X137B88 | **0.894** | 0.106 | 2a | 10.5 | 1 |
| 83 | X9B52 | **0.877** | 0.123 | 2a | 11.33 | 0 |
| 84 | X13B79 | **0.959** | 0.041 | 2a | 10.83 | 0 |
| 85 | X140B91 | **0.959** | 0.041 | 2a | 11.5 | 0 |
| 86 | X144B49 | **0.959** | 0.041 | 2a | 11.5 | 0 |
| 87 | X145B10 | 0.003 | **0.997** | 2b | 11.42 | 0 |
| 88 | X14B98 | **0.924** | 0.076 | 2a | 10.83 | 0 |
| 89 | X150B81 | **0.777** | 0.223 | 2a | 11.42 | 0 |
| 90 | X151B84 | **0.894** | 0.106 | 2a | 11.42 | 0 |
| 91 | X152B99 | **0.959** | 0.041 | 2a | 2.08 | 0 |
| 92 | X154B42 | 0.005 | **0.995** | 2b | 3.42 | 1 |
| 93 | X158B84 | **0.959** | 0.041 | 2a | 4.67 | 1 |
| 94 | X159B47 | 0.001 | **0.999** | 2b | 6.5 | 1 |
| 95 | X15C94 | **0.959** | 0.041 | 2a | 4.42 | 0 |
| 96 | X161B31 | **0.959** | 0.041 | 2a | 11.42 | 0 |
| 97 | X164B81 | 0.001 | **0.999** | 2b | 11.33 | 0 |
| 98 | X165B72 | 0.046 | **0.954** | 2b | 1.5 | 1 |
| 99 | X166B79 | 0.025 | **0.975** | 2b | 11.33 | 0 |
| 100 | X168B51 | **0.959** | 0.041 | 2a | 5.33 | 0 |
| 101 | X169B79 | **0.959** | 0.041 | 2a | 11.33 | 0 |
| 102 | X16C97 | **0.877** | 0.123 | 2a | 3.58 | 1 |
| 103 | X170B15 | **0.894** | 0.106 | 2a | 4.08 | 1 |
| 104 | X171B77 | 0.005 | **0.995** | 2b | 1.75 | 1 |
| 105 | X175B72 | **0.894** | 0.106 | 2a | 0 | 1 |
| 106 | X176B74 | **0.959** | 0.041 | 2a | 6 | 0 |
| 107 | X178B74 | **0.761** | 0.239 | 2a | 7.42 | 0 |
| 108 | X179B28 | **0.959** | 0.041 | 2a | 2.33 | 1 |
| 109 | X17C40 | **0.959** | 0.041 | 2a | 1.92 | 0 |
| 110 | X183B75 | **0.894** | 0.106 | 2a | 7 | 1 |
| 111 | X186B22 | 0.029 | **0.971** | 2b | 0.17 | 1 |
| 112 | X187B36 | 0.001 | **0.999** | 2b | 0 | 1 |
| 113 | X188B13 | 0.469 | **0.531** | 2b | 11 | 0 |
| 114 | X189B83 | 0.005 | **0.995** | 2b | 11 | 0 |
| 115 | X18C56 | **0.777** | 0.223 | 2a | 10.75 | 0 |
| 116 | X191B79 | 0.001 | **0.999** | 2b | 4.42 | 1 |
| 117 | X193B72 | 0.469 | **0.531** | 2b | 10.92 | 0 |
| 118 | X197B95 | **0.777** | 0.223 | 2a | 10.92 | 0 |
| 119 | X198B90 | **0.959** | 0.041 | 2a | 10.92 | 0 |
| 120 | X199B55 | **0.894** | 0.106 | 2a | 10.92 | 0 |
| 121 | X110B34 | 0.001 | **0.999** | 2b | 11.67 | 0 |
| 122 | X201B68 | **0.959** | 0.041 | 2a | 10.92 | 0 |
| 123 | X202B44 | **0.959** | 0.041 | 2a | 10.83 | 0 |
| 124 | X203B49 | **0.959** | 0.041 | 2a | 10.83 | 0 |
| 125 | X206C05 | **0.924** | 0.076 | 2a | 6.42 | 0 |
| 126 | X208C06 | 0.001 | **0.999** | 2b | 0.08 | 0 |

### APPENDIX 6A

**SWS Classifier 2**

| Order | UGID (build #177) | Unigene Name | Gene Symbol | Genbank Acc | AffyID | cut-off |
|---|---|---|---|---|---|---|
| 1 | Hs.184339 | Maternal embryonic leucine zipper kinase | MELK | NM_0147 91 | A.204825 _at | 5.43711 |
| 2 | Hs.308045 | Barren homolog (Drosophila) | BRRN1 | D38553 | A.212949 _at | 5.50455 |
| 3 | Hs.244580 | TPX2, microtubule-associated protein homolog (Xenopus laevis) | TPX2 | AF098158 | A.210052 _s_at | 5.87219 |
| 4 | Hs.486401 | CDNA clone IMAGE:4452583, partial cds | | BG49235 9 | B.226936 _at | 7.56993 |
| 5 | Hs.75573 | Centromere protein E, 312kDa | CENPE | NM_0018 13 | A.205046 _at | 6.94342 |
| 6 | Hs.528654 | Hypothetical protein FLJ11029 | FLJ11029 | BG16501 1 | B.228273 at | 7.71114 |
| 7 | acc_NM_003 158 | | | NM_0031 58 | A.208079 _s_at | 6.57103 |
| 8 | Hs.524571 | Cell division cycle associated 8 | CDCA8 | BC001651 | A.221520 _s_at | 6.8942 |
| 9 | Hs.239 | Forkhead box M1 | FOXM1 | NM_0219 53 | A.202580 _x_at | 5.21151 |
| 10 | Hs.179718 | V-myb myeloblastosis viral oncogene homolog (avian)-like 2 | MYBL2 | NM_0024 66 | A.201710 _at | 6.26908 |
| 11 | Hs.169840 | TTK protein kinase | TTK | NM_0033 18 | A.204822 _at | 8.2308 |
| 12 | Hs.75678 | FBJ murine osteosarcoma viral oncogene homolog B | FOSB | NM_0067 32 | A.202768 _at | 8.76158 |
| 13 | Hs.25647 | V-fos FBJ murine osteosarcoma viral oncogene homolog | FOS | BC004490 | A.209189 _at | 7.08598 |
| 14 | Hs.524216 | Cell division cycle associated 3 | CDCA3 | NM_0312 99 | A.221436 _s_at | 6.29283 |
| 15 | Hs.381225 | Kinetochore protein Spc24 | Spc24 | AI469788 | B.235572 _at | 6.3405 |
| 16 | Hs.62180 | Anillin, actin binding protein (scraps homolog, Drosophila) | ANLN | AK023208 | B.222608 _s_at | 6.84578 |
| 17 | Hs.434886 | Cell division cycle associated 5 | CDCA5 | BE614410 | B.224753 _at | 5.29067 |
| 18 | Hs.523468 | Signal peptide, CUB domain, EGF-like 2 | SCUBE2 | AI424243 | A.219197 _s_at | 5.79216 |

### APPENDIX 6B

### SWS Classifier 2: Accuracy

Accuracy
G1=65/68
(95.6%)
G3=53/55
(96.4%)

| Number | Patients ID | Histologic grade | Probability for G1 | Probability for G3 | Predicted grade G1 or G3 |
|---|---|---|---|---|---|
| 1 | X100B08 | 1 | 0.993 | 0.007 | 1 |
| 2 | X209C10 | 1 | 0.982 | 0.018 | 1 |
| 3 | X21C28 | 1 | 0.993 | 0.007 | 1 |
| 4 | X220C70 | 1 | 0.993 | 0.007 | 1 |
| 5 | X224C93 | 1 | 0.991 | 0.009 | 1 |
| 6 | X227C50 | 1 | 0.995 | 0.005 | 1 |
| 7 | X229C44 | 1 | 0.987 | 0.013 | 1 |
| 8 | X231C80 | 1 | 0.978 | 0.022 | 1 |
| 9 | X233C91 | 1 | 0.993 | 0.007 | 1 |
| 10 | X235C20 | 1 | 0.120 | 0.880 | 3 |
| 11 | X236C55 | 1 | 0.995 | 0.005 | 1 |
| 12 | X114B68 | 1 | 0.684 | 0.316 | 1 |
| 13 | X243C70 | 1 | 0.993 | 0.007 | 1 |
| 14 | X246C75 | 1 | 0.993 | 0.007 | 1 |
| 15 | X248C91 | 1 | 0.995 | 0.005 | 1 |
| 16 | X253C20 | 1 | 0.995 | 0.005 | 1 |
| 17 | X259C74 | 1 | 0.991 | 0.009 | 1 |
| 18 | X261C94 | 1 | 0.995 | 0.005 | 1 |
| 19 | X262C85 | 1 | 0.995 | 0.005 | 1 |
| 20 | X263C82 | 1 | 0.995 | 0.005 | 1 |
| 21 | X266C51 | 1 | 0.976 | 0.024 | 1 |
| 22 | X267C04 | 1 | 0.812 | 0.188 | 1 |
| 23 | X282C51 | 1 | 0.995 | 0.005 | 1 |
| 24 | X284C63 | 1 | 0.989 | 0.011 | 1 |
| 25 | X289C75 | 1 | 0.995 | 0.005 | 1 |
| 26 | X28C76 | 1 | 0.995 | 0.005 | 1 |
| 27 | X294C04 | 1 | 0.859 | 0.141 | 1 |
| 28 | X309C49 | 1 | 0.086 | 0.914 | 3 |
| 29 | X316C65 | 1 | 0.993 | 0.007 | 1 |
| 30 | X128B48 | 1 | 0.995 | 0.005 | 1 |
| 31 | X33C30 | 1 | 0.995 | 0.005 | 1 |
| 32 | X39C24 | 1 | 0.989 | 0.011 | 1 |
| 33 | X42C57 | 1 | 0.995 | 0.005 | 1 |
| 34 | X45A96 | 1 | 0.995 | 0.005 | 1 |
| 35 | X48A46 | 1 | 0.995 | 0.005 | 1 |
| 36 | X49A07 | 1 | 0.993 | 0.007 | 1 |
| 37 | X52A90 | 1 | 0.985 | 0.015 | 1 |
| 38 | X61A53 | 1 | 0.968 | 0.032 | 1 |
| 39 | X65A68 | 1 | 0.991 | 0.009 | 1 |
| 40 | X6B85 | 1 | 0.035 | 0.965 | 3 |
| 41 | X72A92 | 1 | 0.855 | 0.145 | 1 |
| 42 | X135B40 | 1 | 0.995 | 0.005 | 1 |
| 43 | X74A63 | 1 | 0.927. | 0.073 | 1 |
| 44 | X83A37 | 1 | 0.833 | 0.167 | 1 |
| 45 | X8B87 | 1 | 0.995 | 0.005 | 1 |
| 46 | X99A50 | 1 | 0.759 | 0.241 | 1 |
| 47 | X138B34 | 1 | 0.995 | 0.005 | 1 |
| 48 | X155B52 | 1 | 0.995 | 0.005 | 1 |
| 49 | X156B01 | 1 | 0.995 | 0.005 | 1 |
| 50 | X160B16 | 1 | 0.993 | 0.007 | 1 |
| 51 | X163B27 | 1 | 0.995 | 0.005 | 1 |
| 52 | X105B13 | 1 | 0.870 | 0.130 | 1 |
| 53 | X173B43 | 1 | 0.995 | 0.005 | 1 |
| 54 | X174B41 | 1 | 0.990 | 0.010 | 1 |
| 55 | X177B67 | 1 | 0.993 | 0.007 | 1 |
| 56 | X106B55 | 1 | 0.993 | 0.007 | 1 |
| 57 | X180B38 | 1 | 0.993 | 0.007 | 1 |
| 58 | X181B70 | 1 | 0.969 | 0.031 | 1 |
| 59 | X184B38 | 1 | 0.983 | 0.017 | 1 |
| 60 | X185B44 | 1 | 0.995 | 0.005 | 1 |
| 61 | X10B88 | 1 | 0.892 | 0.108 | 1 |
| 62 | X192B69 | 1 | 0.995 | 0.005 | 1 |
| 63 | X195B75 | 1 | 0.993 | 0.007 | 1 |
| 64 | X196B81 | 1 | 0.644 | 0.356 | 1 |
| 65 | X19C33 | 1 | 0.986 | 0.014 | 1 |
| 66 | X204B85 | 1 | 0.995 | 0.005 | 1 |
| 67 | X205B99 | 1 | 0.837 | 0.163 | 1 |
| 68 | X207C08 | 1 | 0.993 | 0.007 | 1 |
| 69 | X111B51 | 3 | 0.001 | 0.999 | 3 |
| 70 | X222C26 | 3 | 0.240 | 0.760 | 3 |
| 71 | X226C06 | 3 | 0.001 | 0.999 | 3 |
| 72 | X113B11 | 3 | 0.005 | 0.995 | 3 |
| 73 | X232C58 | 3 | 0.001 | 0.999 | 3 |
| 74 | X234C15 | 3 | 0.014 | 0.986 | 3 |
| 75 | X238C87 | 3 | 0.293 | 0.707 | 3 |
| 76 | X241C01 | 3 | 0.001 | 0.999 | 3 |
| 77 | X249C42 | 3 | 0.002 | 0.998 | 3 |
| 78 | X250C78 | 3 | 0.004 | 0.996 | 3 |
| 79 | X252C64 | 3 | 0.002 | 0.998 | 3 |
| 80 | X269C68 | 3 | 0.001 | 0.999 | 3 |
| 81 | X26C23 | 3 | 0.444 | 0.556 | 3 |
| 82 | X270C93 | 3 | 0.018 | 0.982 | 3 |
| 83 | X271C71 | 3 | 0.005 | 0.995 | 3 |
| 84 | X279C61 | 3 | 0.001 | 0.999 | 3 |
| 85 | X287C67 | 3 | 0.005 | 0.995 | 3 |
| 86 | X291C17 | 3 | 0.001 | 0.999 | 3 |
| 87 | X127B00 | 3 | 0.001 | 0.999 | 3 |
| 88 | X303C36 | 3 | 0.001 | 0.999 | 3 |
| 89 | X304C89 | 3 | 0.999 | 0.001 | 1 |
| 90 | X311A27 | 3 | 0.004 | 0.996 | 3 |
| 91 | X313A87 | 3 | 0.001 | 0.999 | 3 |
| 92 | X314B55 | 3 | 0.002 | 0.998 | 3 |
| 93 | X101B88 | 3 | 0.001 | 0.999 | 3 |
| 94 | X37C06 | 3 | 0.003 | 0.997 | 3 |
| 95 | X46A25 | 3 | 0.002 | 0.998 | 3 |
| 96 | X131B79 | 3 | 0.241 | 0.759 | 3 |
| 97 | X54A09 | 3 | 0.001 | 0.999 | 3 |
| 98 | X55A79 | 3 | 0.002 | 0.998 | 3 |
| 99 | X62A02 | 3 | 0.001 | 0.999 | 3 |
| 100 | X66A84 | 3 | 0.001 | 0.999 | 3 |
| 101 | X67A43 | 3 | 0.001 | 0.999 | 3 |
| 102 | X69A93 | 3 | 0.043 | 0.957 | 3 |
| 103 | X70A79 | 3 | 0.001 | 0.999 | 3 |
| 104 | X73A01 | 3 | 0.145 | 0.855 | 3 |
| 105 | X76A44 | 3 | 0.018 | 0.982 | 3 |
| 106 | X79A35 | 3 | 0.004 | 0.996 | 3 |
| 107 | X82A83 | 3 | 0.012 | 0.988 | 3 |
| 108 | X89A64 | 3 | 0.000 | 1.000 | 3 |
| 109 | X90A63 | 3 | 0.001 | 0.999 | 3 |
| 110 | X139B03 | 3 | 0.003 | 0.997 | 3 |
| 111 | X102B06 | 3 | 0.001 | 0.999 | 3 |
| 112 | X142B05 | 3 | 0.006 | 0.994 | 3 |
| 113 | X143B81 | 3 | 0.009 | 0.991 | 3 |
| 114 | X146B39 | 3 | 0.001 | 0.999 | 3 |
| 115 | X147B19 | 3 | 0.003 | 0.997 | 3 |
| 116 | X103B41 | 3 | 0.001 | 0.999 | 3 |
| 117 | X153B09 | 3 | 0.001 | 0.999 | 3 |
| 118 | X104B91 | 3 | 0.023 | 0.977 | 3 |
| 119 | X162B98 | 3 | 0.134 | 0.866 | 3 |
| 120 | X172B19 | 3 | 0.051 | 0.949 | 3 |
| 121 | X182B43 | 3 | 0.001 | 0.999 | 3 |
| 122 | X194B60 | 3 | 0.004 | 0.996 | 3 |
| 123 | X200B47 | 3 | 1.000 | 0.000 | 1 |

### APPENDIX 6C

**SWS Classifier 2: G2a-G2b Prediction and Survival**

| # Cox PH test summary (Baseline group 1) | | | | | | |
|---|---|---|---|---|---|---|
| | coef | exp(coef) | se(coef) | z | p | |
| group2b | 1.06 | 2.87 | 0.298 | 3.54 | 4e-04 | |
| | | | | | | |

| Likelihood ratio test=12.8 on 1 df, p=0.000341 n= 126 | | | | | | |
|---|---|---|---|---|---|---|
| | n events | rmean | se(rmean) | median | 0.95LCL0 | 95UCL |
| group 2a= 77 | 19 | 10.33 | 0.499 | Inf | Inf | Inf |
| group 2b=49 | 28 | 6.98 | 0.750 | 7 | 3 | Inf |

| | | | | | | |
|---|---|---|---|---|---|---|
| * DFS Event defined as any type of recurrence or death because of breast cancer, whichever comes first | | | | | | |

| Num ber | Patient ID | Histologic grade | Probability for G2a | Probability for G2b | Predicted grade (2a-G2a, 2b-G2b) | DFS TIME | DFS EVENT* |
|---|---|---|---|---|---|---|---|
| 1 | X210C72 | 2 | 0.017 | 0.983 | 2b | 0.5 | 1 |
| 2 | X211C88 | 2 | 0.673 | 0.327 | 2a | 1.5 | 0 |
| 3 | X212C21 | 2 | 1.000 | 0.000 | 2a | 3.75 | 1 |
| 4 | X216C61 | 2 | 0.999 | 0.001 | 2a | 10.75 | 0 |
| 5 | X217C79 | 2 | 0.999 | 0.001 | 2a | 10.75 | 0 |
| 6 | X218C29 | 2 | 0.999 | 0.001 | 2a | 10.75 | 0 |
| 7 | X223C51 | 2 | 0.997 | 0.003 | 2a | 8.42 | 0 |
| 8 | X22C62 | 2 | 0.999 | 0.001 | 2a | 4.83 | 0 |
| 9 | X244C89 | 2 | 0.059 | 0.941 | 2b | 7.25 | 1 |
| 10 | X247C76 | 2 | 0.894 | 0.106 | 2a | 10.5 | 0 |
| 11 | X11B47 | 2 | 0.999 | 0.001 | 2a | 7.42 | 0 |
| 12 | X24C30 | 2 | 1.000 | 0.000 | 2a | 10.67 | 0 |
| 13 | X251C14 | 2 | 1.000 | 0.000 | 2a | 10.5 | 0 |
| 14 | X254C80 | 2 | 1.000 | 0.000 | 2a | 10.5 | 0 |
| 15 | X255C06 | 2 | 0.999 | 0.001 | 2a | 10.5 | 0 |
| 16 | X257C87 | 2 | 1.000 | 0.000 | 2a | 10.5 | 0 |
| 17 | X258C21 | 2 | 1.000 | 0.000 | 2a | 5.75. | 1 |
| 18 | X265C40 | 2 | 0.934 | 0.066 | 2a | 10.42 | 0 |
| 19 | X122B81 | 2 | 0.999 | 0.001 | 2a | 11.17 | 0 |
| 20 | X272C88 | 2 | 1.000 | 0.000 | 2a | 10.33 | 0 |
| 21 | X274C81 | 2 | 1.000 | 0.000 | 2a | 10.33 | 0 |
| 22 | X275C70 | 2 | 0.999 | 0.001 | 2a | 10.25 | 0 |
| 23 | X277C64 | 2 | 1.000 | 0.000 | 2a | 8.58 | 0 |
| 24 | X124B25 | 2 | 0.999 | 0.001 | 2a | 5 | 1 |
| 25 | X27C82 | 2 | 1.000 | 0.000 | 2a | 6.83 | 0 |
| 26 | X280C43 | 2 | 1.000 | 0.000 | 2a | 1 | 1 |
| 27 | X286C91 | 2 | 1.000 | 0.000 | 2a | 10 | 0 |
| 28 | X288C57 | 2 | 0.999 | 0.001 | 2a | 10 | 0 |
| 29 | X290C91 | 2 | 1.000 | 0.000 | 2a | 10 | 0 |
| 30 | X292C66 | 2 | 0.961 | 0.039 | 2a | 10 | 0 |
| 31 | X296C95 | 2 | 1.000 | 0.000 | 2a | 9.92 | 0 |
| 32 | X297C26 | 2 | 1.000 | 0.000 | 2a | 9.92 | 0 |
| 33 | X298C47 | 2 | 0.998 | 0.002 | 2a | 6.5 | 1 |
| 34 | X301 C66 | 2 | 0.902 | 0.098 | 2a | 9.92 | 0 |
| 35 | X307C50 | 2 | 0.406 | 0.594 | 2b | 9.83 | 0 |
| 36 | X34C80 | 2 | 0.999 | 0.001 | 2a | 10.17 | 0 |
| 37 | X36C17 | 2 | 1.000 | 0.000 | 2a | 10.08 | 0 |
| 38 | X40C57 | 2 | 0.805 | 0.195 | 2a | 10 | 0 |
| 39 | X41C65 | 2 | 0.999 | 0.001 | 2a | 9.92 | 0 |
| 40 | X130B92 | 2 | 1.000 | 0.000 | 2a | 4.42 | 1 |
| 41 | X43C47 | 2 | 0.539 | 0.461 | 2a | 9.92 | 0 |
| 42 | X50A91 | 2 | 0.998 | 0.002 | 2a | 9.08 | 1 |
| 43 | X51A98 | 2 | 0.155 | 0.845 | 2b | 12.67 | 0 |
| 44 | X56A94 | 2 | 0.999 | 0.001 | 2a | 1.08 | 1 |
| 45 | X60A05 | 2 | 0.999 | 0.001 | 2a | 0.67 | 1 |
| 46 | X63A62 | 2 | 0.999 | 0.001 | 2a | 0.17 | 1 |
| 47 | X7B96 | 2 | 0.081 | 0.919 | 2b | 2.42 | 1 |
| 48 | X136B04 | 2 | 0.999 | 0.001 | 2a | 2.42 | 1 |
| 49 | X85A03 | 2 | 0.939 | 0.061 | 2a | 2.08 | 0 |
| 50 | X94A16 | 2 | 1.000 | 0.000 | 2a | 11.08 | 0 |
| 51 | X96A21 | 2 | 0.999 | 0.001 | 2a | 0.08 | 1 |
| 52 | X137B88 | 2 | 0.992 | 0.008 | 2a | 10.5 | 1 |
| 53 | X9B52 | 2 | 0.134 | 0.866 | 2b | 11.33 | 0 |
| 54 | X13879 | 2 | 1.000 | 0.000 | 2a | 10.83 | 0 |
| 55 | X140B91 | 2 | 1.000 | 0.000 | 2a | 11.5 | 0 |
| 56 | X144B49 | 2 | 1.000 | 0.000 | 2a | 11.5 | 0 |
| 57 | X14B98 | 2 | 0.997 | 0.003 | 2a | 10.83 | 0 |
| 58 | X150B81 | 2 | 0.995 | 0.005 | 2a | 11.42 | 0 |
| 59 | X151B84 | 2 | 0.998 | 0.002 | 2a | 11.42 | 0 |
| 60 | X152B99 | 2 | 1.000 | 0.000 | 2a | 2.08 | 0 |
| 61 | X158B84 | 2 | 1.000 | 0.000 | 2a | 4.67 | 1 |
| 62 | X15C94 | 2 | 1.000 | 0.000 | 2a | 4.42 | 0 |
| 63 | X161B31 | 2 | 0.999 | 0.001 | 2a | 11.42 | 0 |
| 64 | X168B51 | 2 | 1.000 | 0.000 | 2a | 5.33 | 0 |
| 65 | X169B79 | 2 | 0.996 | 0.004 | 2a | 11.33 | 0 |
| 66 | X16C97 | 2 | 0.997 | 0.003 | 2a | 3.58 | 1 |
| 67 | X170B15 | 2 | 0.913 | 0.087 | 2a | 4.08 | 1 |
| 68 | X175B72 | 2 | 0.760 | 0.240 | 2a | 0 | 1 |
| 69 | X176B74 | 2 | 1.000 | 0.000 | 2a | 6 | 0 |
| 70 | X178B74 | 2 | 0.996 | 0.004 | 2a | 7.42 | 0 |
| 71 | X179B28 | 2 | 0.999 | 0.001 | 2a | 2.33 | 1 |
| 72 | X17C40 | 2 | 0.999 | 0.001 | 2a | 1.92 | 0 |
| 73 | X183B75 | 2 | 0.045 | 0.955 | 2b | 7 | 1 |
| 74 | X18C56 | 2 | 0.997 | 0.003 | 2a | 10.75 | 0 |
| 75 | X197B95 | 2 | 0.072 | 0.928 | 2b | 10.92 | 0 |
| 76 | X198B90 | 2 | 0.999 | 0.001 | 2a | 10.92 | 0 |
| 77 | X199B55 | 2 | 0.074 | 0.926 | 2b | 10.92 | 0 |
| 78 | X201B68 | 2 | 0.998 | 0.002 | 2a | 10.92 | 0 |
| 79 | X202B44 | 2 | 1.000 | 0.000 | 2a | 10.83 | 0 |
| 80 | X203B49 | 2 | 1.000 | 0.000 | 2a | 10.83 | 0 |
| 81 | X206C05 | 2 | 0.994 | 0.006 | 2a | 6.42 | 0 |
| 82 | X278C80 | 2 | 0.990 | 0.010 | 2a | 10.25 | 0 |
| 83 | X77A50 | 2 | 0.989 | 0.011 | 2a | 1.08 | 1 |
| 84 | X87A79 | 2 | 0.927 | 0.073 | 2a | 12.08 | 0 |
| 85 | X188B13 | 2 | 0.934 | 0.066 | 2a | 11 | 0 |
| 86 | X193B72 | 2 | 0.400 | 0.600 | 2b | 10.92 | 0 |
| 87 | X213C36 | 2 | 0.041 | 0.959 | 2b | 10.08 | 0 |
| 88 | X112B55 | 2 | 0.000 | 1.000 | 2b | 0.92 | 1 |
| 89 | X221C14 | 2 | 0.363 | 0.637 | 2b | 3 | 1 |
| 90 | X225C52 | 2 | 0.000 | 1.000 | 2b | 10.75 | 0 |
| 91 | X230C47 | 2 | 0.000 | 1.000 | 2b | 0.5 | 1 |
| 92 | X237C56 | 2 | 0.001 | 0.999 | 2b | 10.67 | 0 |
| 93 | X23C52 | 2 | 0.000 | 1.000 | 2b | 8.5 | 1 |
| 94 | X240C54 | 2 | 0.050 | 0.950 | 2b | 2.42 | 1 |
| 95 | X242C21 | 2 | 0.099 | 0.901 | 2b | 2.17 | 1 |
| 96 | X245C22 | 2 | 0.005 | 0.995 | 2b | 0 | 1 |
| 97 | X256C45 | 2 | 0.000 | 1.000 | 2b | 1.25 | 1 |
| 98 | X120B73 | 2 | 0.000 | 1.000 | 2b | 11.58 | 0 |
| 99 | X260C91 | 2 | 0.005 | 0.995 | 2b | 10.42 | 0 |
| 100 | X268C87 | 2 | 0.000 | 1.000 | 2b | 10.33 | 0 |
| 101 | X308C93 | 2 | 0.000 | 1.000 | 2b | 2.25 | 1 |
| 102 | X35C29 | 2 | 0.003 | 0.997 | 2b | 2.42 | 1 |
| 103 | X44A53 | 2 | 0.996 | 0.004 | 2a | 12.75 | 0 |
| 104 | X47A87 | 2 | 0.000 | 1.000 | 2b | 9.58 | 1 |
| 105 | X53A06 | 2 | 0.038 | 0.962 | 2b | 2.58 | 1 |
| 106 | X58A50 | 2 | 0.000 | 1.000 | 2b | 0.42 | 1 |
| 107 | X5B97 | 2 | 0.000 | 1.000 | 2b | 0.75 | 1 |
| 108 | X134B33 | 2 | 0.000 | 1.000 | 2b | 2 | 1 |
| 109 | X64A59 | 2 | 0.001 | 0.999 | 2b | 12.42 | 0 |
| 110 | X75A01 | 2 | 0.001 | 0.999 | 2b | 3.58 | 1 |
| 111 | X84A44 | 2 | 0.000 | 1.000 | 2b | 12.17 | 0 |
| 112 | X86A40 | 2 | 0.000 | 1.000 | 2b | 12.17 | 0 |
| 113 | X88A67 | 2 | 0.000 | 1.000 | 2b | 4.25 | 1 |
| 114 | X145B10 | 2 | 0.000 | 1.000 | 2b | 11.42 | 0 |
| 115 | X154B42 | 2 | 0.000 | 1.000 | 2b | 3.42 | 1 |
| 116 | X159B47 | 2 | 0.010 | 0.990 | 2b | 6.5 | 1 |
| 117 | X164B81 | 2 | 0.000 | 1.000 | 2b | 11.33 | 0 |
| 118 | X165B72 | 2 | 0.304 | 0.696 | 2b | 1.5 | 1 |
| 119 | X166B79 | 2 | 0.064 | 0.936 | 2b | 11.33 | 0 |
| *120* | X171B77 | 2 | 0.000 | 1.000 | | 1.75 | 1 |
| 121 | X186B22 | 2 | 0.002 | 0.998 | 2b | 0.17 | 1 |
| 122 | X187B36 | 2 | 0.000 | 1.000 | 2b | 0 | 1 |
| 123 | X189B83 | 2 | 0.000 | 1.000 | 2b | 11 | 0 |
| 124 | X191B79 | 2 | 0.000 | 1.000 | 2b | 4.42 | 1 |
| 125 | X110B34 | 2 | 0.000 | 1.000 | 2b | 11.67 | 0 |
| 126 | X208C06 | 2 | 0.000 | 1.000 | 2b | 0.08 | 0 |

### APPENDIX 7A

**SWS Classifier 3**

| **Order** | **UGID(build #183)** | **UnigeneName** | **GeneSy mbol** | **GenbankAcc** | **Affi ID** | **Cut-off** |
|---|---|---|---|---|---|---|
| 1 | Hs.9329 | TPX2, microtubule-associated protein homolog (Xenopus laevis) | TPX2 | AF098158 | A.210052_s_at | 8.7748 |
| 2 | Hs.344037 | Protein regulator of cytokinesis 1 | PRC1 | NM_003981 | A.218009_s_at | 8.2222 |
| 3 | Hs.292511 | Neuro-oncological ventral antigen 1 | NOVA1 | NM_002515 | A.205794_s_at | 6.7387 |
| 4 | Hs.155223 | Stanniocalcin 2 | STC2 | AI435828 | A.203438_at | 8.0766 |
| 5 | Hs.437351 | Cold inducible RNA binding protein | CIRBP | AL565767 | B.225191_at | 8.2308 |
| 6 | Hs.24395 | Chemokine (C-X-C motif) ligand | CXCL14 | NM_004887 | A.218002_s_at | 7.086 |
| 7 | Hs.435861 | Signal peptide, CUB domain, EGF-like | SCUBE2 | AI424243 | A.219197_s_at | 7.2545 |

### APPENDIX 7B

### SWS Classifier 3: Classifier Accuracy

Accuracy
G1 = 67/68 (98.5%)
G3 = 51/55 (92.7%)

| Numb er | Patients ID | Histol ogic grade | Probability for G1 | Probability for G3 | Predicted grade |
|---|---|---|---|---|---|
| 1 | X100B08 | 1 | 0.990 | 0.010 | 1 |
| 2 | X209C10 | 1 | 0.818 | 0.182 | 1 |
| 3 | X21C28 | 1 | 0.964 | 0.036 | 1 |
| 4 | X220C70 | 1 | 0.990 | 0.010 | 1 |
| 5 | X224C93 | 1 | 0.587 | 0:413 | 1 |
| 6 | X227C50 | 1 | 1.000 | 0.000 | 1 |
| 7 | X229C44 | 1 | 0.981 | 0.019 | 1 |
| 8 | X231C80 | 1 | 1.000 | 0.000 | 1 |
| 9 | X233C91 | 1 | 0.990 | 0.010 | 1 |
| 10 | X235C20 | 1 | 0.976 | 0.024 | 1 |
| 11 | X236C55 | 1 | 1.000 | 0.000 | 1 |
| 12 | X114B68 | 1 | 0.990 | 0.010 | 1 |
| 13 | X243C70 | 1 | 0.818 | 0.182 | 1 |
| 14 | X246C75 | 1 | 0.990 | 0.010 | 1 |
| 15 | X248C91 | 1 | 0.907 | 0.093 | 1 |
| 16 | X253C20 | 1 | 1.000 | 0.000 | 1 |
| 17 | X259C74 | 1 | 0.990 | 0.010 | 1 |
| 18 | X261C94 | 1 | 1.000 | 0.000 | 1 |
| 19 | X262C85 | 1 | 1.000 | 0.000 | 1 |
| 20 | X263C82 | 1 | 1.000 | 0.000 | 1 |
| 21 | X266C51 | 1 | 1.000 | 0.000 | 1 |
| 22 | X267C04 | 1 | 0.907 | 0.093 | 1 |
| 23 | X282C51 | 1 | 0.907 | 0.093 | 1 |
| 24 | X284C63 | 1 | 1.000 | 0.000 | 1 |
| 25 | X289C75 | 1 | 1.000 | 0.000 | 1 |
| 26 | X28C76 | 1 | 1.000 | 0.000 | 1 |
| 27 | X294C04 | 1 | 0.587 | 0.413 | 1 |
| 28 | X309C49 | 1 | 0.015 | 0.985 | 3 |
| 29 | X316C65 | 1 | 0.990 | 0.010 | 1 |
| 30 | X128B48 | 1 | 1.000 | 0:000 | 1 |
| 31 | X33C30 | 1 | 1.000 | 0.000 | 1 |
| 32 | X39C24 | 1 | 0.907 | 0.093 | 1 |
| 33 | X42C57 | 1 | 0.983 | 0.017 | 1 |
| 34 | X45A96 | 1 | 0.765 | 0.235 | 1 |
| 35 | X48A46 | 1 | 1.000 | 0.000 | 1 |
| 36 | X49A07 | 1 | 0.990 | 0.010 | 1 |
| 37 | X52A90 | 1 | 0.990 | 0.010 | 1 |
| 38 | X61A53 | 1 | 1.000 | 0.000 | 1 |
| 39 | X65A68 | 1 | 0.827 | 0.173 | 1 |
| 40 | X6B85 | 1 | 0.529 | 0.471 | 1 |
| 41 | X72A92 | 1 | 0.907 | 0.093 | 1 |
| 42 | X135B40 | 1 | 0.907 | 0.093 | 1 |
| 43 | X74A63 | 1 | 0.529 | 0.471 | 1 |
| 44 | X83A37 | 1 | 0.976 | 0.024 | 1 |
| 45 | X8B87 | 1 | 0.910 | 0.090 | 1 |
| 46 | X99A50 | 1 | 0.531 | 0.469 | 1 |
| 47 | X138B34 | 1 | 1.000 | 0.000 | 1 |
| 48 | X155B52 | 1 | 1.000 | 0.000 | 1 |
| 49 | X156B01 | 1 | 1.000 | 0.000 | 1 |
| 50 | X160B16 | 1 | 1.000 | 0.000 | 1 |
| 51 | X163B27 | 1 | 1.000 | 0.000 | 1 |
| 52 | X105B13 | 1 | 0.907 | 0.093 | 1 |
| 53 | X173B43 | 1 | 0.910 | 0.090 | 1 |
| 54 | X174B41 | 1 | 1.000 | 0.000 | 1 |
| 55 | X177B67 | 1 | 0.990 | 0.010 | 1 |
| 56 | X106B55 | 1 | 0.990 | 0.010 | 1 |
| 57 | X180B38 | 1 | 0.990 | 0.010 | 1 |
| 58 | X181B70 | 1 | 0.990 | 0.010 | 1 |
| 59 | X184B38 | 1 | 0.907 | 0.093 | 1 |
| 60 | X185B44 | 1 | 1.000 | 0:000 | 1 |
| 61 | X10B88 | 1 | 0.739 | 0.261 | 1 |
| 62 | X192B69 | 1 | 1.000 | 0.000 | 1 |
| 63 | X195B75 | 1 | 1.000 | 0.000 | 1 |
| 64 | X196B81 | 1 | 1.000 | 0.000 | 1 |
| 65 | X19C33 | 1 | 0.587 | 0.413 | 1 |
| 66 | X204B85 | 1 | 1.000 | 0.000 | 1 |
| 67 | X205B99 | 1 | 0.827 | 0.173 | 1 |
| 68 | X207C08 | 1 | 1.000 | 0.000 | 1 |
| 69 | X111B51 | 3 | 0.006 | 0.994 | 3 |
| 70 | X222C26 | 3 | 0.623 | 0.377 | 1 |
| 71 | X226C06 | 3 | 0.005 | 0.995 | 3 |
| 72 | X113B11 | 3 | 0.093 | 0.907 | 3 |
| 73 | X232C58 | 3 | 0.016 | 0.984 | 3 |
| 74 | X234C15 | 3 | 0.005 | 0.995 | 3 |
| 75 | X238C87 | 3 | 0.205 | 0.795 | 3 |
| 76 | X241C01 | 3 | 0.009 | 0,991 | 3 |
| 77 | X249C42 | 3 | 0.002 | 0.998 | 3 |
| 78 | X250C78 | 3 | 0.016 | 0.984 | 3 |
| 79 | X252C64 | 3 | 0.016 | 0.984 | 3 |
| 80 | X269C68 | 3 | 0.002 | 0.998 | 3 |
| 81 | X26C23 | 3 | 0.129 | 0.871 | 3 |
| 82 | X270C93 | 3 | 0.000 | 1.000 | 3 |
| 83 | X271C71 | 3 | 0.002 | 0.998 | 3 |
| 84 | X279C61 | 3 | 0.002 | 0.998 | 3 |
| 85 | X287C67 | 3 | 0.005 | 0.995 | 3 |
| 86 | X291C17 | 3 | 0.006 | 0.994 | 3 |
| 87 | X127B00 | 3 | 0.016 | 0.984 | 3 |
| 88 | X303C36 | 3 | 0.005 | 0.995 | 3 |
| 89 | X304C89 | 3 | 0.899 | 0.101 | 1 |
| 90 | X311A27 | 3 | 0.045 | 0.955 | 3 |
| 91 | X313A87 | 3 | 0.002 | 0.998 | 3 |
| 92 | X314B55 | 3 | 0.002 | 0.998 | 3 |
| 93 | X101B88 | 3 | 0.009 | 0.991 | 3 |
| 94 | X37C06 | 3 | 0.006 | 0.994 | 3 |
| 95 | X46A25 | 3 | 0.057 | 0.943 | 3 |
| 96 | X131B79 | 3 | 0.075 | 0.925 | 3 |
| 97 | X54A09 | 3 | 0.000 | 1.000 | 3 |
| 98 | X55A79 | 3 | 0.028 | 0.972 | 3 |
| 99 | X62A02 | 3 | 0.006 | 0.994 | 3 |
| 100 | X66A84 | 3 | 0.002 | 0.998 | 3 |
| 101 | X67A43 | 3 | 0.002 | 0.998 | 3 |
| 102 | X69A93 | 3 | 0.136 | 0.864 | 3 |
| 103 | X70A79 | 3 | 0.005 | 0.995 | 3 |
| 104 | X73A01 | 3 | 0.194 | 0.806 | 3 |
| 105 | X76A44 | 3 | 0.022 | 0.978 | 3 |
| 106 | X79A35 | 3 | 0.006 | 0.994 | 3 |
| 107 | X82A83 | 3 | 0.062 | 0.938 | 3 |
| 108 | X89A64 | 3 | 0.005 | 0.995 | 3 |
| 109 | X90A63 | 3 | 0.002 | 0.998 | 3 |
| 110 | X139B03 | 3 | 0.022 | 0.978 | 3 |
| 111 | X102B06 | 3 | 0.006 | 0.994 | 3 |
| 112 | X142B05 | 3 | 0.005 | 0.995 | 3 |
| 113 | X143B81 | 3 | 0.002 | 0.998 | 3 |
| 114 | X146B39 | 3 | 0.002 | 0.998 | 3 |
| 115 | X147B19 | 3 | 0.016 | 0:984 | 3 |
| 116 | X103B41 | 3 | 0.002 | 0.998 | 3 |
| 117 | X153B09 | 3 | 0.002 | 0.998 | 3 |
| 118 | X104B91 | 3 | 0.119 | 0.881 | 3 |
| 119 | X162B98 | 3 | 0.623 | 0.377 | 1 |
| 120 | X172B19 | 3 | 0.055 | 0.945 | 3 |
| 121 | X182B43 | 3 | 0.002 | 0.998 | 3 |
| 122 | X194B60 | 3 | 0.002 | 0.998 | 3 |
| 123 | X200B47 | 3 | 0.979 | 0.021 | 1 |

### APPENDIX 7C

**SWS Classifier 3: G2a-G2b Prediction Validation**

| # Cox PH test summary (Baseline group 1) | | | | | | | |
|---|---|---|---|---|---|---|---|
| | coef | | exp(coef) | se(coef) | z | p | |
| group2b | 1.05 | | 2.85 | 0.292 | 3.58 | 0.00035 | |
| | | | | | | | |
| **Likelihood ratio test=12.2 on 1 df, p=0.000485 n=126** | | | | | | | |
| | | | | | | | |

| # Survival fit summaries | | | | | | | |
|---|---|---|---|---|---|---|---|
| | n | events | rmean | se(rmean) | median | 0.95LCL | 0.95UCL |
| group2a= | 87 | 24 | 10.05 | 0.482 | Inf | Inf | Inf |
| group2b= | 39 | 23 | 6.61 | 0.844 | 6.5 | 2.42 | Inf |

| | | | | | | | |
|---|---|---|---|---|---|---|---|
| * DFS Event defined as any type of recurrence or death because of breast cancer, whichever comes first | | | | | | | |

| Number | Patient ID | Histolo gic grade | Probability for G2a | Probability for G2b | Predicted grade (2a-G2a, 2b-G2b) | DFS TIME | DFS Event |
|---|---|---|---|---|---|---|---|
| 1 | X210C72 | 2 | 0.012 | 0.988 | 2b | 0.5 | 1 |
| 2 | X211C88 | 2 | 0.999 | 0.001 | 2a | 1.5 | 0 |
| 3 | X212C21 | 2 | 1.000 | 0.000 | 2a | 3.75 | 1 |
| 4 | X213C36 | 2 | 0.001 | 0.999 | 2b | 10.08 | 0 |
| 5 | X216C61 | 2 | 0.820 | 0.180 | 2a | 10.75 | 0 |
| 6 | X217C79 | 2 | 0.999 | 0.001 | 2a | 10.75 | 0 |
| 7 | X218C29 | 2 | 0.996 | 0.004 | 2a | 10.75 | 0 |
| 8 | X112B55 | 2 | 0.418 | 0.582 | 2b | 0.92 | 1 |
| 9 | X221C14 | 2 | 0.901 | 0.099 | 2a | 3 | 1 |
| 10 | X223C51 | 2 | 0.999 | 0.001 | 2a | 8.42 | 0 |
| 11 | X225C52 | 2 | 0.001 | 0.999 | 2b | 10.75 | 0 |
| 12 | X22C62 | 2 | 0.901 | 0.099 | 2a | 4.83 | 0 |
| 13 | X230C47 | 2 | 0.000 | 1.000 | 2b | 0.5 | 1 |
| 14 | X237C56 | 2 | 0.000 | 1.000 | 2b | 10.67 | 0 |
| 15 | X23C52 | 2 | 0.001 | 0.999 | 2b | 8.5 | 1 |
| 16 | X240C54 | 2 | 0.001 | 0.999 | 2b | 2.42 | 1 |
| 17 | X242C21 | 2 | 0.634 | 0.366 | 2a | 2.17 | 1 |
| 18 | X244C89 | 2 | 0.001 | 0.999 | 2b | 7.25 | 1 |
| 19 | X245C22 | 2 | 0.004 | 0.996 | 2b | 0 | 1 |
| 20 | X247C76 | 2 | 0.996 | 0.004 | 2a | 10.5 | 0 |
| 21 | X11B47 | 2 | 0.640 | 0.360 | 2a | 7.42 | 0 |
| 22 | X24C30 | 2 | 0.999 | 0.001 | 2a | 10.67 | 0 |
| 23 | X251C14 | 2 | 0.999 | 0.001 | 2a | 10.5 | 0 |
| 24 | X254C80 | 2 | 0.999 | 0.001 | 2a | 10.5 | 0 |
| 25 | X255C06 | 2 | 0.744 | 0.256 | 2a | 10.5 | 0 |
| 26 | X256C45 | 2 | 0.000 | 1.000 | 2b | 1.25 | 1 |
| 27 | X120B73 | 2 | 0.000 | 1.000 | 2b | 11.58 | 0 |
| 28 | X257C87 | 2 | 0.901 | 0.099 | 2a | 10.5 | 0 |
| 29 | X258C21 | 2 | 0.999 | 0.001 | 2a | 5.75 | 1 |
| 30 | X260C91 | 2 | 0.640 | 0.360 | 2a | 10.42 | 0 |
| 31 | X265C40 | 2 | 0.578 | 0.422 | 2a | 10.42 | 0 |
| 32 | X122B81 | 2 | 0.999 | 0.001 | 2a | 11.17 | 0 |
| 33 | X268C87 | 2 | 0.000 | 1.000 | 2b | 10.33 | 0 |
| 34 | X272C88 | 2 | 0.998 | 0.002 | 2a | 10.33 | 0 |
| 35 | X274C81 | 2 | 0.820 | 0.180 | 2a | 10.33 | 0 |
| 36 | X275C70 | 2 | 0.999 | 0.001 | 2a | 10.25 | 0 |
| 37 | X277C64 | 2 | 0.999 | 0.001 | 2a | 8.58 | 0 |
| 38 | X124B25 | 2 | 0.640 | 0.360 | 2a | 5 | 1 |
| 39 | X278C80 | 2 | 0.002 | 0.998 | 2b | 10.25 | 0 |
| 40 | X27C82 | 2 | 0.550 | 0.450 | 2a | 6.83 | 0 |
| 41 | X280C43 | 2 | 1.000 | 0.000 | 2a | 1 | 1 |
| 42 | X286C91 | 2 | 1.000 | 0.000 | 2a | 10 | 0 |
| 43 | X288C57 | 2 | 0.820 | 0.180 | 2a | 10 | 0 |
| 44 | X290C91 | 2 | 1.000 | 0.000 | 2a | 10 | 0 |
| 45 | X292C66 | 2 | 0.999 | 0.001 | 2a | 10 | 0 |
| 46 | X296C95 | 2 | 1.000 | 0.000 | 2a | 9.92 | 0 |
| 47 | X297C26 | 2 | 0.820 | 0.180 | 2a | 9.92 | 0 |
| 48 | X298C47 | 2 | 0.999 | 0.001 | 2a | 6.5 | 1 |
| 49 | X301C66 | 2 | 0.640 | 0.360 | 2a | 9.92 | 0 |
| 50 | X307C50 | 2 | 0.744 | 0.256 | 2a | 9.83 | 0 |
| 51 | X308C93 | 2 | 0.000 | 1.000 | 2b | 2.25 | 1 |
| 52 | X34C80 | 2 | 0.820 | 0.180 | 2a | 10.17 | 0 |
| 53 | X35C29 | 2 | 0.999 | 0.001 | 2a | 2.42 | 1 |
| 54 | X36C17 | 2 | 0.901 | 0.099 | 2a | 10.08 | 0 |
| 55 | X40C57 | 2 | 0.999 | 0.001 | 2a | 10 | 0 |
| 56 | X41C65 | 2 | 1.000 | 0.000 | 2a | 9.92 | 0 |
| 57 | X130B92 | 2 | 1.000 | 0.000 | 2a | 4.42 | 1 |
| 58 | X43C47 | 2 | 0.574 | 0.426 | 2a | 9.92 | 0 |
| 59 | X44A53 | 2 | 1.000 | 0.000 | 2a | 12.75 | 0 |
| 60 | X47A87 | 2 | 0.000 | 1.000 | 2b | 9.58 | 1 |
| 61 | X50A91 | 2 | 0.012 | 0.988 | 2b | 9.08 | 1 |
| 62 | X51A98 | 2 | 0.998 | 0.002 | 2a | 12.67 | 0 |
| 63 | X53A06 | 2 | 1.000 | 0.000 | 2a | 2.58 | 1 |
| 64 | X56A94 | 2 | 0.998 | 0.002 | 2a | 1.08 | 1 |
| 65 | X58A50 | 2 | 0.000 | 1.000 | 2b | 0.42 | 1 |
| 66 | X5B97 | 2 | 0.000 | 1.000 | 2b | 0.75 | 1 |
| 67 | X60A05 | 2 | 1.000 | 0.000 | 2a | 0.67 | 1 |
| 68 | X134B33 | 2 | 0.001 | 0.999 | 2b | 2 | 1 |
| 69 | X63A62 | 2 | 0.999 | 0.001 | 2a | 0.17 | 1 |
| 70 | X64A59 | 2 | 0.001 | 0.999 | 2b | 12.42 | 0 |
| 71 | X75A01 | 2 | 0.999 | 0.001 | 2a | 3.58 | 1 |
| 72 | X77A50 | 2 | 0.391 | 0.609 | 2b | 1.08 | 1 |
| 73 | X7B96 | 2 | 0.391 | 0.609 | 2b | 2.42 | 1 |
| 74 | X84A44 | 2 | 0.002 | 0.998 | 2b | 12.17 | 0 |
| 75 | X136B04 | 2 | 0.012 | 0.988 | 2b | 2.42 | 1 |
| 76 | X85A03 | 2 | 0.012 | 0.988 | 2b | 2.08 | 0 |
| 77 | X86A40 | 2 | 0.000 | 1.000 | 2b | 12.17 | 0 |
| 78 | X87A79 | 2 | 0.820 | 0.180 | 2a | 12.08 | 0 |
| 79 | X88A67 | 2 | 0.574 | 0.426 | 2a | 4.25 | 1 |
| 80 | X94A16 | 2 | 0.999 | 0.001 | 2a | 11.08 | 0 |
| 81 | X96A21 | 2 | 0.020 | 0.980 | 2b | 0.08 | 1 |
| 82 | X137B88 | 2 | 0.640 | 0.360 | 2a | 10.5 | 1 |
| 83 | X9B52 | 2 | 0.999 | 0.001 | 2a | 11.33 | 0 |
| 84 | X13B79 | 2 | 0.999 | 0.001 | 2a | 10.83 | 0 |
| 85 | X140B91 | 2 | 0.901 | 0.099 | 2a | 11.5 | 0 |
| 86 | X144B49 | 2 | 0.796 | 0.204 | 2a | 11.5 | 0 |
| 87 | X145B10 | 2 | 0.000 | 1.000 | 2b | 11.42 | 0 |
| 88 | X14B98 | 2 | 0.999 | 0.001 | 2a | 10.83 | 0 |
| 89 | X150B81 | 2 | 1.000 | 0.000 | 2a | 11.42 | 0 |
| 90 | X151B84 | 2 | 1.000 | 0.000 | 2a | 11.42 | 0 |
| 91 | X152B99 | 2 | 1.000 | 0.000 | 2a | 2.08 | 0 |
| 92 | X154B42 | 2 | 0.099 | 0.901 | 2b | 3.42 | 1 |
| 93 | X158B84 | 2 | 0.999 | 0.001 | 2a | 4.67 | 1 |
| 94 | X159B47 | 2 | 0.002 | 0.998 | 2b | 6.5 | 1 |
| 95 | X15C94 | 2 | 1.000 | 0.000 | 2a | 4.42 | 0 |
| 96 | X161B31 | 2 | 1.000 | 0.000 | 2a | 11.42 | 0 |
| 97 | X164B81 | 2 | 0.000 | 1.000 | 2b | 11.33 | 0 |
| 98 | X165B72 | 2 | 0.944 | 0.056 | 2a | 1.5 | 1 |
| 99 | X166B79 | 2 | 0.980 | 0.020 | 2a | 11.33 | 0 |
| 100 | X168B51 | 2 | 0.800 | 0.200 | 2a | 5.33 | 0 |
| 101 | X169B79 | 2 | 0.995 | 0.005 | 2a | 11.33 | 0 |
| 102 | X16C97 | 2 | 1.000 | 0.000 | 2a | 3.58 | 1 |
| 103 | X170B15 | 2 | 0.999 | 0.001 | 2a | 4.08 | 1 |
| 104 | X171B77 | 2 | 0.000 | 1.000 | 2b | 1.75 | 1 |
| 105 | X175B72 | 2 | 0.901 | 0.099 | 2a | 0 | 1 |
| 106 | X176B74 | 2 | 1.000 | 0.000 | 2a | 6 | 0 |
| 107 | X178B74 | 2 | 1.000 | 0.000 | 2a | 7.42 | 0 |
| 108 | X179B28 | 2 | 0.999 | 0.001 | 2a | 2.33 | 1 |
| 109 | X17C40 | 2 | 0.999 | 0.001 | 2a | 1.92 | 0 |
| 110 | X183B75 | 2 | 0.820 | 0.180 | 2a | 7 | 1 |
| 111 | X186B22 | 2 | 0.786 | 0.214 | 2a | 0.17 | 1 |
| 112 | X187B36 | 2 | 0.000 | 1.000 | 2b | 0 | 1 |
| 113 | X188B13 | 2 | 0.999 | 0.001 | 2a | 11 | 0 |
| 114 | X189B83 | 2 | 0.000 | 1.000 | 2b | 11 | 0 |
| 115 | X18C56 | 2 | 1.000 | 0.000 | 2a | 10.75 | 0 |
| 116 | X191B79 | 2 | 0.099 | 0.901 | 2b | 4.42 | 1 |
| 117 | X193B72 | 2 | 0.640 | 0.360 | 2a | 10.92 | 0 |
| 118 | X197B95 | 2 | 0.297 | 0.703 | 2b | 10.92 | 0 |
| 119 | X198B90 | 2 | 0.901 | 0.099 | 2a | 10.92 | 0 |
| 120 | X199B55 | 2 | 0.820 | 0.180 | 2a | 10.92 | 0 |
| 121 | X110B34 | 2 | 0.000 | 1.000 | 2b | 11.67 | 0 |
| 122 | X201B68 | 2 | 0.999 | 0.001 | 2a | 10.92 | 0 |
| 123 | X202B44 | 2 | 0.999 | 0.001 | 2a | 10.83 | 0 |
| 124 | X203B49 | 2 | 1.000 | 0.000 | 2a | 10.83 | 0 |
| 125 | X206C05 | 2 | 1.000 | 0.000 | 2a | 6.42 | 0 |
| 126 | X208C06 | 2 | 0.136 | 0.864 | 2b | 0.08 | 0 |

### APPENDIX 8A

**SWS Classifier 4**

| **Order** | **UGID(build #183)** | **UnigeneName** | **GeneSymbol** | **GenbankAcc** | **Affi ID** | **Cut-off** |
|---|---|---|---|---|---|---|
| 1 | Hs.48855 | cell division cycle associated 8 | CDCA8 | BC001651 | A.221520_s_at | **5.5046** |
| 2 | Hs.75573 | centromere protein E, 312kDa | CENPE | NM_001813 | A.205046_at | **5.2115** |
| 3 | Hs.552 | steroid-5-alpha-reductase, alpha polypeptide 1 (3-oxo-5 alpha-steroid delta 4-dehydrogenase alpha 1) | SRD5A1 | BC006373 | A.211056_s_at | **6.9192** |
| 4 | Hs.101174 | microtubule-associated protein tau | MAPT | NM_016835 | A.203929_s_at | **4.8246** |
| 5 | Hs.164018 | leucine zipper protein FKSG14 | FKSG14 | BC005400 | B.222848_at | **6.1846** |
| 6 | acc_R38110 | N.A. | | R38110 | B.240112_at | **6.2557** |
| 7 | Hs.325650 | EH-domain containing_2 | EHD2 | AI417917 | A.221870_at | **7.6677** |

### APPENDIX 8B

### SWS Classifier 4: Classifier Accuracy

Accuracy
G1= 67/68
(98.5%)
G3= 52/55
(94.5%)

| Number | Patients ID | Histologic grade | Probability for G1 | Probability for G3 | Predicted grade (G1 or G3) |
|---|---|---|---|---|---|
| 1 | X100B08 | 1 | 1.000 | 0 | 1 |
| 2 | X209C10 | 1 | 0.992 | 0.008 | 1 |
| 3 | X21C28 | 1 | 0.992 | 0.008 | 1 |
| 4 | X220C70 | 1 | 1.000 | 0.000 | 1 |
| 5 | X224C93 | 1 | 0.962 | 0.038 | 1 |
| 6 | X227C50 | 1 | 1.000 | 0.000 | 1 |
| 7 | X229C44 | 1 | 0.962 | 0.038 | 1 |
| 8 | X231C80 | 1 | 0.742 | 0.258 | 1 |
| 9 | X233C91 | 1 | 1.000 | 0.000 | 1 |
| 10 | X235C20 | 1 | 0.633 | 0.367 | 1 |
| 11 | X236C55 | 1 | 0.986 | 0.014 | 1 |
| 12 | X114B68 | 1 | 0.852 | 0.148 | 1 |
| 13 | X243C70 | 1 | 1.000 | 0.000 | 1 |
| 14 | X246C75 | 1 | 1.000 | 0.000 | 1 |
| 15 | X248C91 | 1 | 1.000 | 0.000 | 1 |
| 16 | X253C20 | 1 | 1.000 | 0.000 | 1 |
| 17 | X259C74 | 1 | 1.000 | 0.000 | 1 |
| 18 | X261C94 | 1 | 1.000 | 0.000 | 1 |
| 19 | X262C85 | 1 | 0.992 | 0.008 | 1 |
| 20 | X263C82 | 1 | 1.000 | 0.000 | 1 |
| 21 | X266C51 | 1 | 1.000 | 0.000 | 1 |
| 22 | X267C04 | 1 | 0.633 | 0.367 | 1 |
| 23 | X282C51 | 1 | 0.962 | 0.038 | 1 |
| 24 | X284C63 | 1 | 0.992 | 0.008 | 1 |
| 25 | X289C75 | 1 | 0.969 | 0.031 | 1 |
| 26 | X28C76 | 1 | 0.992 | 0.008 | 1 |
| 27 | X294C04 | 1 | 0.667 | 0.333 | 1 |
| 28 | X309C49 | 1 | 0.531 | 0.469 | 1 |
| 29 | X316C65 | 1 | 1.000 | 0.000 | 1 |
| 30 | X128B48 | 1 | 1.000 | 0.000 | 1 |
| 31 | X33C30 | 1 | 0.992 | 0.008 | 1 |
| 32 | X39C24 | 1 | 0.992 | 0.008 | 1 |
| 33 | X42C57 | 1 | 1.000 | 0.000 | 1 |
| 34 | X45A96 | 1 | 0.703 | 0.297 | 1 |
| 35 | X48A46 | 1 | 1.000 | 0.000 | 1 |
| 36 | X49A07 | 1 | 0.992 | 0.008 | 1 |
| 37 | X52A90 | 1 | 0.992 | 0.008 | 1 |
| 38 | X61A53 | 1 | 0.742 | 0.258 | 1 |
| 39 | X65A68 | 1 | 0.975 | 0.025 | 1 |
| 40 | X6B85 | 1 | 0.633 | 0.367 | 1 |
| 41 | X72A92 | 1 | 0.992 | 0.008 | 1 |
| 42 | X135B40 | 1 | 1.000 | 0.000 | 1 |
| 43 | X74A63 | 1 | 0.852 | 0.148 | 1 |
| 44 | X83A37 | 1 | 0.852 | 0.148 | 1 |
| 45 | X8B87 | 1 | 1.000 | 0.000 | 1 |
| 46 | X99A50 | 1 | 0.738 | 0.262 | 1 |
| 47 | X138B34 | 1 | 0.992 | 0.008 | 1 |
| 48 | X155B52 | 1 | 1.000 | 0.000 | 1 |
| 49 | X156B01 | 1 | 1.000 | 0.000 | 1 |
| 50 | X160B16 | 1 | 0.992 | 0.008 | 1 |
| 51 | X163B27 | 1 | 0.992 | 0.008 | 1 |
| 52 | X105B13 | 1 | 0.939 | 0.061 | 1 |
| 53 | X173B43 | 1 | 1.000 | 0.000 | 1 |
| 54 | X174B41 | 1 | 0.986 | 0.014 | 1 |
| 55 | X177B67 | 1 | 1.000 | 0.000 | 1 |
| 56 | X106B55 | 1 | 1.000 | 0.000 | 1 |
| 57 | X180B38 | 1 | 1.000 | 0.000 | 1 |
| 58 | X181B70 | 1 | 0.947 | 0.053 | 1 |
| 59 | X184B38 | 1 | 0.852 | 0.148 | 1 |
| 60 | X185B44 | 1 | 0.992 | 0.008 | 1 |
| 61 | X10B88 | 1 | 0.463 | 0.537 | 3 |
| 62 | X192B69 | 1 | 0.992 | 0.008 | 1 |
| 63 | X195B75 | 1 | 1.000 | 0.000 | 1 |
| 64 | X196B81 | 1 | 0.742 | 0.258 | 1 |
| 65 | X19C33 | 1 | 0.962 | 0.038 | 1 |
| 66 | X204B85 | 1 | 1.000 | 0.000 | 1 |
| 67 | X205B99 | 1 | 0.633 | 0.367 | 1 |
| 68 | X207C08 | 1 | 1.000 | 0.000 | 1 |
| 69 | X111B51 | 3 | 0.027 | 0.973 | 3 |
| 70 | X222C26 | 3 | 0.105 | 0.895 | 3 |
| 71 | X226C06 | 3 | 0.003 | 0.997 | 3 |
| 72 | X113B11 | 3 | 0.320 | 0.680 | 3 |
| 73 | X232C58 | 3 | 0.020 | 0.980 | 3 |
| 74 | X234C15 | 3 | 0.028 | 0.972 | 3 |
| 75 | X238C87 | 3 | 0.062 | 0.938 | 3 |
| 76 | X241C01 | 3 | 0.009 | 0.991 | 3 |
| 77 | X249C42 | 3 | 0.003 | 0.997 | 3 |
| 78 | X250C78 | 3 | 0.007 | 0.993 | 3 |
| 79 | X252C64 | 3 | 0.020 | 0.980 | 3 |
| 80 | X269C68 | 3 | 0.003 | 0.997 | 3 |
| 81 | X26C23 | 3 | 0.078 | 0.922 | 3 |
| 82 | X270C93 | 3 | 0.105 | 0.895 | 3 |
| 83 | X271C71 | 3 | 0.009 | 0.991 | 3 |
| 84 | X279C61 | 3 | 0.009 | 0.991 | 3 |
| 85 | X287C67 | 3 | 0.079 | 0.921 | 3 |
| 86 | X291C17 | 3 | 0.008 | 0.992 | 3 |
| 87 | X127B00 | 3 | 0.003 | 0.997 | 3 |
| 88 | X303C36 | 3 | 0.003 | 0.997 | 3 |
| 89 | X304C89 | 3 | 0.888 | 0.112 | 1 |
| 90 | X311A27 | 3 | 0.010 | 0.990 | 3 |
| 91 | X313A87 | 3 | 0.059 | 0.941 | 3 |
| 92 | X314B55 | 3 | 0.010 | 0.990 | 3 |
| 93 | X101B88 | 3 | 0.007 | 0.993 | 3 |
| 94 | X37C06 | 3 | 0.003 | 0.997 | 3 |
| 95 | X46A25 | 3 | 0.064 | 0.936 | 3 |
| 96 | X131B79 | 3 | 0.078 | 0.922 | 3 |
| 97 | X54A09 | 3 | 0.007 | 0.993 | 3 |
| 98 | X55A79 | 3 | 0.322 | 0.678 | 3 |
| 99 | X62A02 | 3 | 0.007 | 0.993 | 3 |
| 100 | X66A84 | 3 | 0.003 | 0.997 | 3 |
| 101 | X67A43 | 3 | 0.003 | ' 0.997 | 3 |
| 102 | X69A93 | 3 | 0.007 | 0.993 | 3 |
| 103 | X70A79 | 3 | 0.003 | 0.997 | 3 |
| 104 | X73A01 | 3 | 0.643 | 0.357 | 1 |
| 105 | X76A44 | 3 | 0.064 | 0.936 | 3 |
| 106 | X79A35 | 3 | 0.007 | 0.993 | 3 |
| 107 | X82A83 | 3 | 0.147 | 0.853 | 3 |
| 108 | X89A64 | 3 | 0.003 | 0.997 | 3 |
| 109 | X90A63 | 3 | 0.009 | 0.991 | 3 |
| 110 | X139B03 | 3 | 0.067 | 0.933 | 3 |
| 111 | X102B06 | 3 | 0.003 | 0.997 | 3 |
| 112 | X142B05 | 3 | 0.010 | 0.990 | 3 |
| 113 | X143B81 | 3 | 0.020 | 0.980 | 3 |
| 114 | X146B39 | 3 | 0.007 | 0.993 | 3 |
| 115 | X147B19 | 3 | 0.020 | 0.980 | 3 |
| 116 | X103B41 | 3 | 0.009 | 0.991 | 3 |
| 117 | X153B09 | 3 | 0.007 | 0.993 | 3 |
| 118 | X104B91 | 3 | 0.052 | 0.948 | 3 |
| 119 | X162B98 | 3 | 0.439 | 0.561 | 3 |
| 120 | X172B19 | 3 | 0.007 | 0.993 | 3 |
| 121 | X182B43 | 3 | 0.003 | 0.997 | 3 |
| 122 | X194B60 | 3 | 0.009 | 0.991 | 3 |
| 123 | X200B47 | 3 | 0.795 | 0.205 | 1 |

### APPENDIX 8C

**SWS Classifier 4: G2a-G2b Prediction Validation**

| # Cox PH test summary (Baseline group 1) | | | | | | |
|---|---|---|---|---|---|---|
| | coef | exp(coef) | se(coef) | z | p | |
| group2b | 0.789 | 2.2 | 0.293 | 2.69 | 0.007 | |
| | | | | | | |
| Likelihood ratio test=7.2 on 1 df, p=0.0073 n= 126 | | | | | | |
| | | | | | | |

| | n events | rmean | se(rmean) | median | 0.95LCL | 0.95UCL |
|---|---|---|---|---|---|---|
| Grade 2a=77 | 22 | 10.0 | 0.508 | Inf | Inf | Inf |
| Grade 2b=49 | 25 | 7.4 | 0.777 | 8.5 | 3 | Inf |

| | | | | | | |
|---|---|---|---|---|---|---|
| * DFS Event defined as any type of recurrence or death because of breast cancer, whichever comes first | | | | | | |

| Probability for G2a | Probability for G2b | Predicted grade (2a-G2a, 2b-G2b) | DFS TIME | DFS Event * |
|---|---|---|---|---|
| 0.001 | 0.999 | 2b | 0.5 | 1 |
| 0.001 | 0.999 | 2b | 1.5 | 0 |
| 0.999 | 0.001 | 2a | 3.75 | 1 |
| 0.003 | 0.997 | 2b | 10.08 | 0 |
| 0.999 | 0.001 | 2a | 10.75 | 0 |
| 1.000 | 0.000 | 2a | 10.75 | 0 |
| 1.000 | 0.000 | 2a | 10.75 | 0 |
| 0.024 | 0.976 | 2b | 0.92 | 1 |
| 0.024 | 0.976 | 2b | 3 | 1 |
| 0.998 | 0.002 | 2a | 8.42 | 0 |
| 0.001 | 0.999 | 2b | 10.75 | 0 |
| 1.000 | 0.000 | 2a | 4.83 | 0 |
| 0.001 | 0.999 | 2b | 0.5 | 1 |
| 0.000 | 1.000 | 2b | 10.67 | 0 |
| 0.001 | 0.999 | 2b | 8.5 | 1 |
| 0.002 | 0.998 | 2b | 2.42 | 1 |
| 0.670 | 0.330 | 2a | 2.17 | 1 |
| 0.007 | 0.993 | 2b | 7.25 | 1 |
| 0.002 | 0.998 | 2b | 0 | 1 |
| 0.525 | 0.475 | 2a | 10.5 | 0 |
| 1.000 | 0.000 | 2a | 7.42 | 0 |
| 1.000 | 0.000 | 2a | 10.67 | 0 |
| 0.999 | 0.001 | 2a | 10.5 | 0 |
| 1.000 | 0.000 | 2a | 10.5 | 0 |
| 1.000 | 0.000 | 2a | 10.5 | 0 |
| 0.000 | 1.000 | 2b | 1.25 | 1 |
| 0.000 | 1.000 | 2b | 11.58 | 0 |
| 1.000 | 0.000 | 2a | 10.5 | 0 |
| 1.000 | 0.000 | 2a | 5.75 | 1 |
| 0.025 | 0.975 | 2b | 10.42 | 0 |
| 0.008 | 0.992 | 2b | 10.42 | 0 |
| 1.000 | 0.000 | 2a | 11.17 | 0 |
| 0.000 | 1.000 | 2b | 10.33 | 0 |
| 1.000 | 0.000 | 2a | 10.33 | 0 |
| 1.000 | 0.000 | 2a | 10.33 | 0 |
| 0.999 | 0.001 | 2a | 10.25 | 0 |
| 1.000 | 0.000 | 2a | 8.58 | 0 |
| 0.999 | 0.001 | 2a | 5 | 1 |
| 0.997 | 0.003 | 2a | 10.25 | 0 |
| 1.000 | 0.000 | 2a | 6.83 | 0 |
| 0.999 | 0.001 | 2a | 1 | 1 |
| 1.000 | 0.000 | 2a | 10 | 0 |
| 1.000 | 0.000 | 2a | 10 | 0 |
| 1.000 | 0.000 | 2a | 10 | 0 |
| 1.000 | 0.000 | 2a | 10 | 0 |
| 1.000 | 0.000 | 2a | 9.92 | 0 |
| 1.000 | 0.000 | 2a | 9.92 | 0 |
| 1.000 | 0.000 | 2a | 6.5 | 1 |
| 0.007 | 0.993 | 2b | 9.92 | 0 |
| 0.754 | 0.246 | 2a | 9.83 | 0 |
| 0.001 | 0.999 | 2b | 2.25 | 1 |
| 1.000 | 0.000 | 2a | 10.17 | 0 |
| 0.003 | 0.997 | 2b | 2.42 | 1 |
| 1.000 | 0.000 | 2a | 10.08 | 0 |
| 1.000 | 0.000 | 2a | 10 | 0 |
| 0.999 | 0.001 | 2a | 9.92 | 0 |
| 1.000 | 0.000 | 2a | 4.42 | 1 |
| 0.727 | 0.273 | 2a | 9.92 | 0 |
| 0.525 | 0.475 | 2a | 12.75 | 0 |
| 0.000 | 1.000 | 2b | 9.58 | 1 |
| 0.999 | 0.001 | 2a | 9.08 | 1 |
| 0.007 | 0.993 | 2b | 12.67 | 0 |
| 0.001 | 0.999 | 2b | 2.58 | 1 |
| 1.000 | 0.000 | 2a | 1.08 | 1 |
| 0.000 | 1.000 | 2b | 0.42 | 1 |
| 0.001 | 0.999 | 2b | 0.75 | 1 |
| 0.999 | 0.001 | 2a | 0.67 | 1 |
| 0.007 | 0.993 | 2b | 2 | 1 |
| 1.000 | 0.000 | 2a | 0.17 | 1 |
| 0.001 | 0.999 | 2b | 12.42 | 0 |
| 0.848 | 0.152 | 2a | 3.58 | 1 |
| 0.719 | 0.281 | 2a | 1.08 | 1 |
| 0.719 | 0.281 | 2a | 2.42 | 1 |
| 0.001 | 0.999 | 2b | 12.17 | 0 |
| 0.693 | 0.307 | 2a | 2.42 | 1 |
| 0.999 | 0.001 | 2a | 2.08 | 0 |
| 0.001 | 0.999 | 2b | 12.17 | 0 |
| 1.000 | 0.000 | 2a | 12.08 | 0 |
| 0.001 | 0.999 | 2b | 4.25 | 1 |
| 1.000 | 0.000 | 2a | 11.08 | 0 |
| 0.999 | 0.001 | 2a | 0.08 | 1 |
| 0.999 | 0.001 | 2a | 10.5 | 1 |
| 0.754 | 0.246 | 2a | 11.33 | 0 |
| 1.000 | 0.000 | 2a | 10.83 | 0 |
| 1.000 | 0.000 | 2a | 11.5 | 0 |
| 1.000 | 0.000 | 2a | 11.5 | 0 |
| 0.000 | 1.000 | 2b | 11.42 | 0 |
| 0.848 | 0.152 | 2a | 10.83 | 0 |
| 1.000 | 0.000 | 2a | 11.42 | 0 |
| 1.000 | 0.000 | 2a | 11.42 | 0 |
| 0.999 | 0.001 | 2a | 2.08 | 0 |
| 0.002 | 0.998 | 2b | 3.42 | 1 |
| 1.000 | 0.000 | 2a | 4.67 | 1 |
| 0.001 | 0.999 | 2b | 6.5 | 1 |
| 1.000 | 0.000 | 2a | 4.42 | 0 |
| 1.000 | 0.000 | 2a | 11.42 | 0 |
| 0.000 | 1.000 | 2b | 11.33 | 0 |
| 0.001 | 0.999 | 2b | 1.5 | 1 |
| 0.001 | 0.999 | 2b | 11.33 | 0 |
| 1.000 | 0.000 | 2a | 5.33 | 0 |
| 0.525 | 0.475 | 2a | 11.33 | 0 |
| 1.000 | 0.000 | 2a | 3.58 | 1 |
| 1.000 | 0.000 | 2a | 4.08 | 1 |
| 0.001 | 0.999 | 2b | 1.75 | 1 |
| 0.003 | 0.997 | 2b | 0 | 1 |
| 0.999 | 0.001 | 2a | 6 | 0 |
| 0.999 | 0.001 | 2a | 7.42 | 0 |
| 0.999 | 0.001 | 2a | 2.33 | 1 |
| 1.000 | 0.000 | 2a | 1.92 | 0 |
| 0.592 | 0.408 | 2a | 7 | 1 |
| 0.001 | 0.999 | 2b | 0.17 | 1 |
| 0.000 | 1.000 | 2b | 0 | 1 |
| 0.005 | 0.995 | 2b | 11 | 0 |
| 0.000 | 1.000 | 2b | 11 | 0 |
| 0.030 | 0.970 | 2b | 10.75 | 0 |
| 0.001 | 0.999 | 2b | 4.42 | 1 |
| 0.000 | 1.000 | 2b | 10.92 | 0 |
| 0.001 | 0.999 | 2b | 10.92 | 0 |
| 1.000 | 0.000 | 2a | 10.92 | 0 |
| 0.001 | 0.999 | 2b | 10.92 | 0 |
| 0.000 | 1.000 | 2b | 11.67 | 0 |
| 1.000 | 0.000 | 2a | 10.92 | 0 |
| 1.000 | 0.000 | 2a | 10.83 | 0 |
| 1.000 | 0.000 | 2a | 10.83 | 0 |
| 0.754 | 0.246 | 2a | 6.42 | 0 |
| 0.001 | 0.999 | 2b | 0.08 | 0 |

## Claims

1. An *in vitro* method of assigning a grade to a breast tumour corresponding to histological Grade 1 or Grade 3, which grade is indicative of the aggressiveness of the tumour, the method comprising detecting the expression of FLJ11029, GenBank Accession Number BG165011.

2. The *in vitro* method according to Claim 1, in which the method comprises detecting a high level of expression of the gene, preferably above 7.7063, and assigning the survival chances associated with histological Grade 3 to the breast tumour or detecting a low level of expression of the gene, preferably below 7.7063, and assigning the survival chances associated with histological Grade 1 to the breast tumour, in which the level of gene expression is measured as the natural log transform normalised signal intensity measurement for Affymetrix arrays, preferably, global mean normalisation with scaling factor of 500, and in which the breast tumour preferably is a histological Grade 2 breast tumour.

3. The *in vitro* method according to Claim 1 or 2, in which the expression of a plurality of genes is detected, preferably all the genes set out in Table D1 (SWS Classifier 0), preferably in the form of an expression profile of the plurality of genes, in which the expression profile is preferably derived from microarray hybridisation, preferably hybridisation to an Affymetrix microarray preferably comprising a probe set consisting of a probe or probes having Affymetrix ID numbers as set out in Column 6 ("Affi ID") of Table D1, or by real time polymerase chain reaction (RT-PCR).

4. The *in vitro* method according to Claim 1, 2 or 3, in which the expression level of 5 or more genes is detected, in which the 5 or more genes preferably comprises the genes set out in Table D2 (SWS Classifier 1), or in which the expression level of the genes is preferably detected using microarray analysis with a probe set consisting of probes having Affymetrix ID numbers as set out in Column 6 ("Affi ID") of Table D2 (SWS Classifier 1).

5. The *in vitro* method according to Claim 4, in which the method comprises:
(a) detecting a high level of expression of the gene if the expression level of the gene is above the expression level set out in Column 9 ("Cut-Off") of Table D2 (SWS Classifier 1) and assigning the grade set out in Column 7 ("Grade with Higher Expression") of Table D2 (SWS Classifier 1) to the breast tumour; or
(b) detecting a low level of expression of the gene if the expression level of the gene is below the expression level set out in Column 9 ("Cut-Off") of Table D2 (SWS Classifier 1) and assigning the grade set out in Column 8 ("Grade with Lower Expression") of Table D2 (SWS Classifier 1) to the breast tumour;
in which the level of gene expression is measured as the natural log transform normalised signal intensity measurement for Affymetrix arrays, preferably, global mean normalisation with scaling factor of 500.

6. The *in vitro* method according to any preceding claim, in which the expression level of 17 or more genes in Table D1 or all the genes in Table D1 is detected, in which the genes preferably comprise the genes set out in Table D3 (SWS Classifier 2), or in which the expression level of the genes is preferably detected using microarray analysis with a probe set consisting of probes having Affymetrix ID numbers as set out in Column 6 ("Affi ID") of Table D3.

7. The *in vitro* method according to Claim 6, in which the method comprises:
(a) detecting a high level of expression of the gene if the expression level of the gene is above the expression level set out in Column 9 ("Cut-Off") of Table D3 (SWS Classifier 2) and assigning the grade set out in Column 7 ("Grade with Higher Expression") of Table D3 (SWS Classifier 2) to the breast tumour; or
(b) detecting a low level of expression of the gene if the expression level of the gene is below the expression level set out in Column 9 ("Cut-Off") of Table D3 (SWS Classifier 2), and assigning the grade set out in Column 8 ("Grade with Lower Expression") of Table D3 (SWS Classifier 2) to the breast tumour;
in which the level of gene expression is measured as the natural log transform normalised signal intensity measurement for Affymetrix arrays , preferably, global mean normalisation with scaling factor of 500.

8. The *in vitro* method according to any of Claims 3 to 7, in which the grade is assigned by applying:
(a) a class prediction algorithm comprising a nearest shrunken centroid method to the expression data of the plurality of genes;
(b) a class prediction algorithm comprising Prediction Analysis of Microarrays (PAM) to the expression data of the plurality of genes;
(c) a class prediction algorithm comprising the steps of:
(i) obtaining a set of predictor parameters;
(ii) re-coding the parameters to obtain discrete-valued variables;
(iii) selecting statistically robust discrete-valued variables and combinations thereof;
(iv) obtaining a sum of the statistically weighted discrete-valued variables and combinations thereof; and
(v) obtaining a predictive outcome of breast cancer subtype based on the sum; or
(c) a class prediction algorithm comprising Statistically Weighted Syndromes (SWS) to the gene expression data.

9. An *in vitro* method of classifying a histological Grade 2 breast tumour into a low aggressiveness tumour or a high aggressiveness tumour, the method comprising assigning the grade to the histological Grade 2 breast tumour corresponding to histological Grade 1 or Grade 3 according to any preceding claim, in which the histological Grade 2 breast tumour that is assigned the low aggressiveness grade has preferably at least one feature of a histological Grade 1 breast tumour, and/or in which the breast tumour that is assigned the high aggressiveness grade has preferably at least one feature of a histological Grade 3 breast tumour, preferably in which the feature comprises any one or more of: (a) likelihood of tumour recurrence post-surgery or survival rate, preferably disease free survival rate; and (b) susceptibility to treatment.

10. The *in vitro* method according to any preceding claim, in which the method is capable of classifying a breast tumour preferably a histological Grade 1 and histological Grade 3 tumour to an accuracy of at least 85%, at least 90% accuracy, or at least 95% accuracy, with reference to the grade obtained of the breast tumour by histological grading.

11. The *in vitro* method according to any preceding claim, in which the histological grading comprises the Nottingham Grading System (NGS) or the Elston-Ellis Modified Scarff, Bloom, Richardson Grading System.

12. A computer implemented *in vitro* method of assigning a grade indicative of aggressiveness to a breast tumour, the method comprising processing expression data for FLJ11029, GenBank Accession Number BG165011, detecting a high level of expression of the gene, preferably above 7.7063, and assigning the survival chances associated with histological Grade 3 to the breast tumour or detecting a low level of expression of the gene, preferably below 7.7063, and assigning the survival chances associated with histological Grade 1 to the breast tumour, in which the level of gene expression is measured as the natural log transform normalised signal intensity measurement for Affymetrix arrays, preferably, global mean normalisation with scaling factor of 500.

13. A program storage device readable by a machine, tangibly embodying a program of instructions executable by the machine to perform method of assigning a grade indicative of aggressiveness to a breast tumour, the method comprising: processing expression data for FLJ11029, GenBank Accession Number BG165011, detecting a high level of expression of the gene, preferably above 7.7063, and assigning the survival chances associated with histological Grade 3 to the breast tumour or detecting a low level of expression of the gene, preferably below 7.7063, and assigning the survival chances associated with histological Grade 1 to the breast tumour, in which the level of gene expression is measured as the natural log transform normalised signal intensity measurement for Affymetrix arrays.

## Patentansprüche

1. *In vitro* Verfahren zum Zuweisen eines Grads an einen Brusttumor, entsprechend dem histologischen Grad 1 oder Grad 3, wobei dieser Grad die Aggressivität des Tumors anzeigt, wobei das Verfahren das Nachweisen der Expression von FLJ11029, GenBank Accession Number BG165011, umfasst.

2. *In vitro* Verfahren nach Anspruch 1, wobei das Verfahren das Nachweisen eines hohen Expressionsniveaus des Gens umfasst, vorzugsweise über 7,7063, und das Zuweisen der Überlebenschancen, die mit dem histologischen Grad 3 assoziiert sind, an den Brusttumor, oder das Nachweisen eines niedrigen Expressionsniveaus des Gens, vorzugsweise unter 7,7063, und das Zuweisen der Überlebenschancen, die mit dem histologischen Grad 1 assoziiert sind, an den Brusttumor, wobei das Niveau der Genexpression als die Messung der normierten Signalintensität der natürlichen Logumwandlung für Affymetrix-Arrays gemessen wird, vorzugsweise der globalen mittleren Normierung mit dem Skalierfaktor 500, und wobei der Brusttumor vorzugsweise ein Brusttumor mit dem histologischen Grad 2 ist.

3. *In vitro* Verfahren nach Anspruch 1 oder 2, wobei die Expression einer Vielzahl von Genen nachgewiesen wird, vorzugsweise aller Gene, die in Tabelle D1 (SWS-Klassifizierer 0) angegeben sind, vorzugsweise in Form eines Expressionsprofils der Vielzahl von Genen, wobei das Expressionsprofil vorzugsweise von einer Microarray-Hybridisierung abgeleitet ist, vorzugsweise einer Hybridisierung zu einem Affymetrix-Microarray, vorzugsweise umfassend einen Sondensatz, der aus einer Sonde oder aus Sonden besteht, die Affymetrix ID-Nummern aufweisen, wie in Spalte 6 ("Affi ID") von Tabelle D1 angegeben, oder durch Echtzeit-Polymerasekettenreaktion (RT-PCR).

4. *In vitro* Verfahren nach Anspruch 1, 2 oder 3, wobei das Expressionsniveau von 5 oder mehr Genen nachgewiesen wird, wobei die 5 oder mehr Gene vorzugsweise die Gene umfassen, die in Tabelle D2 (SWS-Klassifizierer 1) angegeben sind, oder wobei das Expressionsniveau der Gene vorzugsweise unter Verwendung einer Microarray-Analyse nachgewiesen wird, mit einem Sondensatz, bestehend aus Sonden, die Affymetrix ID-Nummern aufweisen, wie in Spalte 6 ("Affi ID") von Tabelle D2 (SWS-Klassifizierer 1) angegeben.

5. *In vitro* Verfahren nach Anspruch 4, wobei das Verfahren Folgendes umfasst:
(a) Nachweisen eines hohen Expressionsniveaus des Gens, wenn das Expressionsniveau des Gens über dem Expressinonsniveau liegt, angegeben in Spalte 9 ("Cut-Off") von Tabelle D2 (SWS-Klassifizierer 1), und Zuweisen des Grads, angegeben in Spalte 7 ("Grad mit höherer Expression") von Tabelle D2 (SWS-Klassifizierer 1) an den Brusttumor; oder
(b) Nachweisen eines niedrigen Expressionsniveaus des Gens, wenn das Expressionsniveau des Gens unter dem Expressionsniveau liegt, angegeben in Spalte 9 ("Cut-Off") von Tabelle D2 (SWS-Klassifizierer 1), und Zuweisen des Grads, angegeben in Spalte 8 ("Grad mit geringerer Expression") von Tabelle D2 (SWS-Klassifizierer 1) an den Brusttumor;
wobei das Niveau der Genexpression als die Messung der normierten Signalintensität der natürlichen Logumwandlung für Affymetrix-Arrays gemessen wird, vorzugsweise der globalen mittleren Normalisierung mit dem Skalierfaktor 500.

6. *In vitro* Verfahren nach einem der vorstehenden Ansprüche, wobei das Expressionsniveau von 17 oder mehr Genen in Tabelle D1 oder aller Gene in Tabelle D1 nachgewiesen wird, wobei die Gene vorzugsweise die Gene umfassen, die in Tabelle D3 angegeben sind (SWS-Klassifizierer 2), oder wobei das Expressionsniveau der Gene vorzugsweise unter Verwendung einer Microarray-Analyse mit einem Sondensatz nachgewiesen wird, bestehend aus Sonden, die Affymetrix ID-Nummern aufweisen, wie in Spalte 6 ("Affi ID") von Tabelle D3 angegeben.

7. *In vitro* Verfahren nach Anspruch 6, wobei das Verfahren Folgendes umfasst:
(a) Nachweisen eines hohen Expressionsniveaus des Gens, wenn das Expressionsniveau des Gens über dem Expressionsniveau liegt, angegeben in Spalte 9 ("Cut-Off") von Tabelle D3 (SWS-Klassifizierer 2), und Zuweisen des Grads, angegeben in Spalte 7 ("Grad mit höherer Expression") von Tabelle D3 (SWS-Klassifizierer 2) an den Brusttumor; oder
(b) Nachweisen eines niedrigen Expressionsniveaus des Gens, wenn das Expressionsniveau des Gens unter dem Expressionsniveau liegt, angegeben in Spalte 9 ("Cut-Off") von Tabelle D3 (SWS-Klassifizierer 2), und Zuweisen des Grads, angegeben in Spalte 8 ("Grad mit geringerer Expression") von Tabelle D3 (SWS-Klassifizierer 2) an den Brusttumor;
wobei das Niveau der Genexpression als die Messung der normierten Signalintensität der natürlichen Logumwandlung für Affymetrix-Arrays gemessen wird, vorzugsweise der globalen mittleren Normalisierung mit dem Skalierfaktor 500.

8. *In vitro* Verfahren nach einem der Ansprüche 3 bis 7, wobei der Grad zugewiesen wird durch Anwendung:
(a) eines Algorithmus zur Vorhersage der Klasse, umfassend ein Nearest-Shrunken-Centroid-Verfahren, auf die Expressionsdaten der Vielzahl von Genen;
(b) eines Algorithmus zur Vorhersage der Klasse, umfassend Vorhersageanalyse von Microarrays (PAM), auf die Expressionsdaten der Vielzahl von Genen;
(c) eines Algorithmus zur Vorhersage der Klasse, umfassend die folgenden Schritte:
(i) Erhalten eines Satzes von Vorhersageparametern;
(ii) Neucodieren der Parameter, um wertediskrete Variablen zu erhalten;
(iii) Auswählen von statistisch robusten wertediskreten Variablen und Kombinationen davon;
(iv) Erhalten einer Summe von statistisch gewichteten wertediskreten Variablen und Kombinationen davon; und
(v) Erhalten eines prädiktiven Ausgangs der Brustkrebs-Unterart basierend auf der Summe; oder
(c) eines Algorithmus zur Vorhersage der Klasse, umfassend Statistisch Gewichtete Syndrome (SWS) auf die Genexpressionsdaten.

9. *In vitro* Verfahren zum Klassifizieren eines Brusttumors mit dem histologischen Grad 2 zu einem Tumor mit niedriger Aggressivität oder einem Tumor mit hoher Aggressivität, wobei das Verfahren das Zuweisen des Grads zum Brusttumor mit dem histologischen Grad 2 entsprechend dem histologischen Grad 1 oder Grad 3 gemäß einem der vorstehenden Ansprüche umfasst, wobei der Brusttumor mit dem histologischen Grad 2, dem der Grad mit niedriger Aggressivität zugewiesen wird, vorzugsweise mindestens ein Merkmal des Brusttumors mit dem histologischen Grad 1 aufweist, und/oder wobei der Brusttumor, dem der Grad mit hoher Aggressivität zugewiesen wird, vorzugsweise mindestens ein Merkmal eines Brusttumors mit dem histologischen Grad 3 aufweist, vorzugsweise wobei das Merkmal eines oder mehrere des Folgenden umfasst: (a) Wahrscheinlichkeit des Wiederauftretens des Tumors nach dem chirurgischen Eingriff oder die Überlebensrate, vorzugsweise die erkrankungsfreie Überlebensrate; und (b) Möglichkeit der Behandlung.

10. *In vitro* Verfahren nach einem der vorstehenden Ansprüche, wobei das Verfahren dazu in der Lage ist, einen Brusttumor vorzugsweise als einen Tumor mit dem histologischen Grad 1 und dem histologischen Grads 3 mit einer Genauigkeit von mindestens 85 %, einer Genauigkeit von mindestens 90 % oder einer Genauigkeit von mindestens 95 % mit Bezug auf den Grad zu klassifizieren, der vom Brusttumor durch histologische Gradierung erhalten wurde.

11. *In vitro* Verfahren nach einem der vorstehenden Ansprüche, wobei die histologische Gradierung das Nottingham Grading System (NGS) oder das Elston-Ellis Modified Scarff, Bloom, Richardson Grading System umfasst.

12. Computer-implementiertes *in vitro* Verfahren des Zuweisens eines Grads, der die Aggressivität angibt, an einen Brusttumor, wobei das Verfahren das Verarbeiten der Expressionsdaten für FLJ11029, GenBank Accession Number BG165011, das Nachweisen eines hohen Expressionsniveaus des Gens, vorzugsweise über 7,7063, und das Zuweisen der Überlebenschancen, die mit dem histologischen Grad 3 assoziiert sind, an den Brusttumor, oder das Nachweisen eines niedrigen Expressionsniveaus des Gens, vorzugsweise unter 7,7063, und das Zuweisen der Überlebenschancen, die mit dem histologischen Grad 1 assoziiert sind, an den Brusttumor umfassen, wobei das Niveau der Genexpression als die Messung der normierten Signalintensität der natürlichen Logumwandlung für Affymetrix-Arrays gemessen wird, vorzugsweise der globalen mittleren Normalisierung mit dem Skalierfaktor 500.

13. Programmspeichervorrichtung, die von einer Maschine lesbar ist, die eindeutig ein Programm von Anweisungen ausführt, das von der Maschine durchgeführt werden kann, um das Verfahren des Zuweisens eines Grads, der die Aggressivität angibt, an einen Brusttumor durchzuführen, wobei das Verfahren Folgendes umfasst: Verarbeiten der Expressionsdaten für FLJ11029, GenBank Accession Number BG165011, Nachweisen eines hohen Expressionsniveaus des Gens, vorzugsweise über 7,7063, und Zuweisen der Überlebenschancen, die mit dem histologischen Grad 3 assoziiert sind, an den Brusttumor, oder Nachweisen eines niedrigen Expressionsniveaus des Gens, vorzugsweise unter 7,7063, und Zuweisen der Überlebenschancen, die mit dem histologischen Grad 1 assoziiert sind, an den Brusttumor, wobei das Genexpressionsniveau als die Messung der normierten Signalintensität der natürlichen Logumwandlung für Affymetrix-Arrays gemessen wird

## Revendications

1. Procédé *in vitro* pour attribuer un grade à une tumeur du sein correspondant au Grade 1 ou au Grade 3 histologique, ce grade étant indicatif de l'agressivité de la tumeur, le procédé comprenant la détection de l'expression du FLJ11029, Numéro d'accès GenBank BG165011.

2. Procédé *in vitro* selon la revendication 1, dans lequel le procédé comprend la détection d'un niveau élevé d'expression du gène, de préférence au-dessus de 7,7063, et l'attribution des chances de survie associées au Grade 3 histologique à la tumeur du sein ou la détection d'un niveau d'expression faible du gène, de préférence en dessous de 7,7063, et attribuer les chances de survie associées au Grade 1 histologique à la tumeur du sein, dans laquelle le niveau d'expression du gène est mesuré en tant que mesure d'intensité de signal normalisé par transformation du logarithme naturel pour des réseaux Affymetrix, de préférence, une normalisation moyenne globale avec un facteur de mise à l'échelle de 500, et dans lequel la tumeur du sein est de préférence une tumeur de Grade 2 histologique.

3. Procédé *in vitro* selon la revendication 1 ou 2, dans lequel l'expression d'une pluralité de gènes est détectée, de préférence tous les gènes définis dans le Tableau D1 (Classificateur SWS 0), de préférence sous la forme d'un profil d'expression de la pluralité de gènes, dans lequel le profil d'expression est de préférence dérivé d'une hybridation de micro-réseau, de préférence une hybridation d'un micro-réseau Affymetrix comprenant de préférence un ensemble de sonde(s) constitué d'une sonde ou de sondes ayant des numéros d'Affymetrix ID tels que définis dans la colonne 6 ("Affi ID") du Tableau D1, ou par réaction en chaîne par polymérase en temps réel (RT-PCR).

4. Procédé *in vitro* selon la revendication 1, 2 ou 3, dans lequel le niveau d'expression de 5 gènes ou plus est détecté, dans lequel les 5 gènes ou plus comprennent de préférence les gènes définis dans le Tableau D2 (Classificateur SWS 1), ou dans lequel le niveau d'expression des gènes est de préférence détecté à l'aide d'une analyse de micro-réseau avec un ensemble de sondes constitué de sondes ayant des numéros d'identification Affymetrix tels que définis dans la colonne 6 ("Affi ID") du Tableau D2 (Classificateur SWS 1).

5. Procédé *in vitro* selon la revendication 4, dans lequel le procédé comprend :
(a) détecter un niveau élevé d'expression du gène si le niveau d'expression du gène est supérieur au niveau d'expression défini dans la colonne 9 ("Seuil") du Tableau D2 (Classificateur SWS 1) et attribuer le grade défini dans la colonne 7 ("Grade avec un niveau d'expression supérieure") du Tableau D2 (Classificateur SWS 1) à la tumeur du sein ; ou
(b) détecter un niveau d'expression faible du gène si le niveau d'expression du gène est inférieur au niveau d'expression défini dans la colonne 9 («Seuil») du tableau D2 (Classificateur SWS 1) et en attribuant le grade défini dans la colonne 8 ("Grade avec un niveau d'expression inférieur") du tableau D2 (Classificateur SWS 1) à la tumeur du sein ;
dans lequel le niveau de l'expression du gène est mesuré en tant que mesure d'intensité de signal normalisé par transformation du logarithme naturel pour des micro-réseaux Affymetrix, de préférence, une normalisation moyenne globale avec un facteur de mise à l'échelle de 500.

6. Procédé *in vitro* selon l'une quelconque des revendications précédentes, dans lequel le niveau d'expression de 17 gènes ou plus dans le Tableau D1 ou tous les gènes du Tableau D1 est détecté, dans lequel les gènes comprennent de préférence les gènes définis dans le Tableau D3 (Classificateur SWS 2), ou dans lequel le niveau d'expression des gènes est de préférence détecté en utilisant une analyse de micro-réseau avec un ensemble de sonde composé de sondes ayant des numéros d'identification Affymetrix comme définis dans la colonne 6 ("Affi ID") du Tableau D3.

7. Procédé *in vitro* selon la revendication 6, dans lequel le procédé comprend :
(a) détecter un niveau élevé d'expression du gène si le niveau d'expression du gène est supérieur au niveau d'expression défini dans la colonne 9 ("Seuil") du Tableau D3 (Classificateur SWS 2) et attribuer le grade défini dans la colonne 7 («Grade avec une expression supérieure») du Tableau D3 (Classificateur SWS 2) à la tumeur du sein ;
(b) détecter un niveau d'expression faible du gène si le niveau d'expression du gène est inférieur au niveau d'expression défini dans la colonne 9 ("Seuil") du Tableau D3 (Classificateur SWS 2) et attribuer le grade défini dans la colonne 8 («Grade avec une expression inférieure») du Tableau D3 (Classificateur SWS C2) à la tumeur du sein ;
dans lequel le niveau de l'expression du gène est mesuré en tant que mesure d'intensité de signal normalisé par transformation du logarithme naturel pour des micro-réseaux Affymetrix, de préférence, une normalisation moyenne globale avec un facteur de mise à l'échelle de 500.

8. Procédé *in vitro* selon l'une quelconque des revendications 3 à 7, dans lequel le grade est attribué en appliquant :
(a) un algorithme de prédiction de classe comprenant un procédé de détermination de centroïde rétréci le plus proche des données d'expression de la pluralité de gènes ;
(b) un algorithme de prédiction de classe comprenant une analyse de prédiction des micro-réseaux (PAM) aux données d'expression de la pluralité de gènes ;
(c) un algorithme de prédiction de classe comprenant les étapes de :
(i) obtenir un ensemble de paramètres prédictifs ;
(ii) recoder les paramètres pour obtenir des variables à valeurs discrètes ;
(iii) sélectionner des variables à valeurs discrètes statistiquement fiables et des combinaisons de celles-ci ;
(iv) obtenir une somme des variables à valeurs discrètes pondérées statistiquement et de combinaisons de celles-ci ; et
(v) obtenir un résultat prédictif du sous-type de cancer du sein en fonction de la somme ;
ou
(c) un algorithme de prédiction de classe comprenant des Syndromes pondérés statistiquement (SWS) aux données d'expression de gènes.

9. Procédé *in vitro* de classement d'une tumeur du sein de Grade 2 histologique en une tumeur de faible agressivité ou une tumeur de forte agressivité, le procédé comprenant l'attribution du grade à la tumeur du sein de Grade 2 histologique correspondant au Grade histologique 1 ou 3 selon l'une quelconque des revendications précédentes, dans lequel la tumeur du sein de Grade 2 histologique qui a reçu le grade d'agressivité faible a de préférence au moins une caractéristique d'une tumeur du sein de Grade 1 histologique, et/ou dans lequel la tumeur du sein qui a reçu le grade d'agressivité élevée a au moins une caractéristique d'une tumeur du sein de Grade 3 histologique, de préférence la caractéristique comprenant une ou plusieurs des caractéristiques suivantes : (a) une probabilité de récidive tumorale post-chirurgie ou un taux de survie, de préférence un taux de survie sans maladie ; et (b) une sensibilité au traitement.

10. Procédé *in vitro* selon l'une quelconque des revendications précédentes, dans lequel le procédé est capable de classer une tumeur du sein, de préférence une tumeur de Grade 1 histologique et une tumeur de Grade 3 histologique avec une précision d'au moins 85%, une précision d'au moins 90%, ou une précision d'au moins 95%, en référence au grade obtenu pour la tumeur du sein par un classement histologique.

11. Procédé *in vitro* selon l'une quelconque des revendications précédentes, dans lequel le classement histologique comprend le système d'attribution de grade de Nottingham (NGS) ou le système d'attribution de grade de Scarff, Bloom et Richardson modifié par Elston et Ellis.

12. Procédé *in vitro* mis en oeuvre par un ordinateur pour attribuer un grade indicatif d'agressivité à une tumeur du sein, le procédé comprenant le traitement de données d'expression du FLJ11029, Numéro d'accès GenBank BG165011, la détection d'un niveau d'expression élevé du gène, de préférence au-dessus de 7,7063, et l'attribution des chances de survie associées au Grade 3 histologique à la tumeur du sein ou la détection d'un niveau d'expression faible du gène, de préférence inférieur à 7,7063, et l'attribution des chances de survie associées à un Grade 1 histologique à la tumeur du sein, dans lequel le niveau d'expression génétique est mesuré en tant que mesure d'intensité de signal normalisé par transformation du logarithme naturel pour des réseaux Affymetrix, de préférence, la normalisation moyenne globale avec un facteur de mise à l'échelle de 500.

13. Dispositif de stockage de programme lisible par une machine, réalisant de manière tangible un programme d'instructions exécutable par la machine pour effectuer un procédé d'attribution d'un grade indicatif d'agressivité à une tumeur du sein, le procédé comprenant : le traitement de données d'expression du FLJ11029, Numéro d'accès GenBank BG165011, la détection d'un niveau d'expression élevé du gène, de préférence au-dessus de 7,7063, et l'attribution des chances de survie associées au Grade 3 histologique à la tumeur du sein ou la détection d'un niveau d'expression faible du gène, de préférence inférieur à 7,7063, et l'attribution des chances de survie associées au Grade 1 histologique de Grade 1 à la tumeur du sein, dans lequel le niveau d'expression du gène est mesuré en tant que mesure d'intensité de signal normalisé par transformation du logarithme naturel pour des réseaux Affymetrix.
